(19) Europäisches Patentamt
European Patent Office
Office européen des brevets

(11) EP 4 692 077 A1

(12) **EUROPEAN PATENT APPLICATION**
published in accordance with Art. 153(4) EPC

(43) Date of publication:
**11.02.2026 Bulletin 2026/07**

(21) Application number: **24780491.7**

(22) Date of filing: **27.03.2024**

(51) International Patent Classification (IPC):
***C07D 403/14*** (2006.01) ***C07D 519/00*** (2006.01)
***C09K 11/06*** (2006.01)

(52) Cooperative Patent Classification (CPC):
**C07D 403/14; C07D 519/00; C09K 11/06**

(86) International application number:
**PCT/JP2024/012318**

(87) International publication number:
**WO 2024/204389 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.03.2023 JP 2023050631**

(71) Applicant: **Kyulux, Inc.**
**Fukuoka-shi, Fukuoka 819-0388 (JP)**

(72) Inventors:
- **CHO Yong Joo**
  **Fukuoka-shi, Fukuoka 819-0388 (JP)**
- **MORIO Momoko**
  **Fukuoka-shi, Fukuoka 819-0388 (JP)**
- **SUZUKI Yoshitake**
  **Fukuoka-shi, Fukuoka 819-0388 (JP)**
- **HWANG Songhye**
  **Fukuoka-shi, Fukuoka 819-0388 (JP)**
- **OZAWA Hiroaki**
  **Fukuoka-shi, Fukuoka 819-0388 (JP)**
- **KANAHARA Kousei**
  **Fukuoka-shi, Fukuoka 819-0388 (JP)**
- **SUZUKI Iori**
  **Fukuoka-shi, Fukuoka 819-0388 (JP)**
- **HAMASAKI Taro**
  **Fukuoka-shi, Fukuoka 819-0388 (JP)**
- **SHIMOI Yuko**
  **Fukuoka-shi, Fukuoka 819-0388 (JP)**

(74) Representative: **Zimmermann & Partner Patentanwälte mbB**
**P.O. Box 330 920**
**80069 München (DE)**

(54) **COMPOUND, LIGHT EMITTING MATERIAL, AND DELAYED FLUORESCENT BODY**

(57) The compound of the following general formula has excellent light emission characteristics. $R^1$ to $R^5$ are H, D, an alkyl group, an aryl group, a donor group, a triazinyl group substituted with $Ar^3$ and $Ar^4$, or the like, $R^2$ or $R^3$ is the triazinyl group or the like, two or more of $R^1$ to $R^5$ are donor groups, and $Ar^1$ to $Ar^4$ are an aryl group or a heteroaryl group. At least one of $R^1$ to $R^5$ is a donor group having a fused ring structure of four or more rings, or at least one of $Ar^1$ to $Ar^4$ is a heteroaryl group.

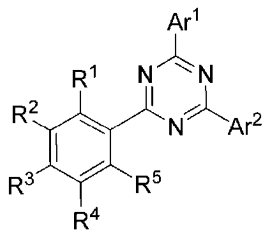

EP 4 692 077 A1

## Description

Technical Field

The present invention relates to a compound having a skeleton in which a triazine ring and a benzene ring are bonded, and a light-emitting material and a delayed fluorescent substance using the compound.

Background Art

Research has been actively conducted to develop light-emitting materials for organic electroluminescent devices (organic EL devices) and the like. Fluorescent materials, phosphorescent materials, and fluorescent materials have been known as light-emitting materials for a long time. However, fluorescent materials have a problem of low light emission efficiency, and phosphorescent materials have a problem of being expensive because they contain rare metals and being difficult to emit deep blue light. In recent years, a delayed fluorescent material has been developed as a light-emitting material that addresses these problems.

A delayed fluorescent material is a material which, in an excited state, after having undergone reverse intersystem crossing from an excited triplet state to an excited singlet state, emits fluorescence when returning back from the excited singlet state to a ground state thereof. Fluorescence through the route is observed later than fluorescence from the excited singlet state directly occurring from the ground state (ordinary fluorescence), and is therefore referred to as delayed fluorescence. Here, for example, in the case where a light-emitting compound is excited through carrier injection thereinto, the occurring probability of the excited singlet state to the excited triplet state is statistically 25%/75%, and therefore improvement of light emission efficiency by the fluorescence alone from the directly occurring excited singlet state is limited. On the other hand, in a delayed fluorescent material, not only the excited singlet state thereof but also the excited triplet state can be utilized for fluorescent emission through the route via the above-mentioned reverse intersystem crossing, and therefore as compared with an ordinary fluorescent material, a delayed fluorescent material can realize a higher light emission efficiency.

Since such a principle has been clarified, various researches have led to the discovery of various delayed fluorescent materials. Among these, many compounds in which a benzene ring is substituted with a donor group and an acceptor group are included. For example, a compound having a skeleton, in which the benzene ring is substituted with a carbazol-9-yl group of a donor group and with a cyano group and a substituted triazinyl group of acceptor groups, has been proposed (see PTL 1).

Citation List

Patent Literature

PTL 1:WO2019/191665A1

Summary of Invention

Technical Problem

Even when a material emits delayed fluorescence, one having extremely good characteristics and having no problem in practical use has not yet been provided. Therefore, it is more useful if a delayed fluorescent material having more excellent characteristics can be provided. However, the improvement of delayed fluorescent materials is in the stage of trial and error, and it is not easy to generalize the chemical structure of useful light-emitting materials.

Under such circumstances, the present inventors have conducted research for the purpose of providing a compound more useful as a delayed fluorescent material for a light-emitting device. Then, the present inventors have conducted intensive studies for the purpose of deriving and generalizing a general formula of a compound more useful as a delayed fluorescent material.

Solution to Problem

As a result of intensive studies to achieve the above-mentioned object, the present inventors have found that a compound having a structure satisfying specific conditions is useful as a material for organic light-emitting devices in that the compound can improve light emission characteristics. The present invention has been proposed based on these findings, and specifically has the following configuration.

[1] A compound represented by the following general formula (1):

General Formula (1)

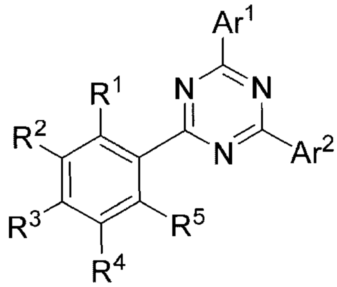

in which $R^1$ to $R^5$ each independently represent a hydrogen atom, a deuterium atom, a substituted or unsubstituted alkyl group, a substituted or unsubstituted aryl group, a donor group, or a group represented by the following formula (2); $R^2$ or $R^3$ is a group represented by the following general formula (2), and two or more of $R^1$ to $R^5$ are donor groups; $Ar^1$ and $Ar^2$ each independently represent a substituted or unsubstituted aryl group, or a substituted or unsubstituted heteroaryl group;

General Formula (2)

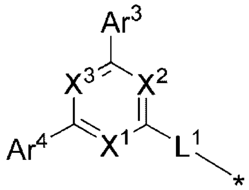

in which $X^1$ to $X^3$ each independently represent N or C(R), and at least one of $X^1$ to $X^3$ is N; R represents a hydrogen atom, a deuterium atom, or a substituent; $Ar^3$ and $Ar^4$ each independently represent a substituted or unsubstituted aryl group, or a substituted or unsubstituted heteroaryl group; $L^1$ represents a single bond or a divalent linking group; * represents a bonding position;

provided that, in the compound represented by the general formula (1), at least one of $R^1$ to $R^5$ is a donor group having a fused ring structure of four or more rings, at least one of $Ar^1$ to $Ar^4$ is a substituted or unsubstituted heteroaryl group (excluding a nitrogen-containing 6-membered ring group), or $R^1$ to $R^5$ and $Ar^1$ to $Ar^4$ satisfy both of these conditions.

[2] The compound according to [1], in which at least one of $R^1$ to $R^5$ is a donor group having a fused ring structure of four or more rings.

[3] The compound according to [2], in which at least one of $R^1$ to $R^5$ is a donor group represented by the following general formula (3):

General Formula (3)

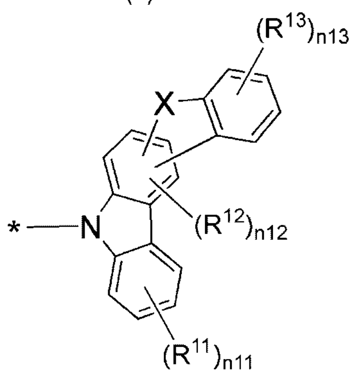

in which X represents O, S or N-$R^{14}$; $R^{11}$ to $R^{13}$ each independently represent a deuterium atom, or a substituent; $R^{14}$ represents an aryl group optionally substituted with one or more selected from the group consisting of a deuterium

atom, an alkyl group and an aryl group, or an alkyl group optionally substituted with one or more selected from the group consisting of a deuterium atom and an aryl group; $R^{11}$'s, $R^{12}$'s, and $R^{13}$'s each may be bonded to each other to form a cyclic structure; n11 and n13 each independently represent an integer of 0 to 4, and n12 represents an integer of 0 to 2.

[4] The compound according to [2] or [3], in which $Ar^1$ to $Ar^4$ are each independently a substituted or unsubstituted aryl group.

[5] The compound according to any one of [1] to [3], in which at least one of $Ar^1$ to $Ar^4$ is a substituted or unsubstituted heteroaryl group (excluding a nitrogen-containing 6-membered ring group).

[6] The compound according to [5], in which at least one of $Ar^1$ to $Ar^4$ is a heteroaryl group having a 5-membered ring bonded via a nitrogen atom.

[7] The compound according to any one of [1] to [6], in which $R^2$ is a group represented by the general formula (2).

[8] The compound according to any one of [1] to [7], in which $R^3$ is a group represented by the general formula (2).

[9] The compound according to any one of [1] to [8], in which the donor group is a substituted or unsubstituted carbazol-9-yl group.

[10] The compound according to any one of [1] to [9], in which three of $R^1$ to $R^5$ are donor groups.

[11] The compound according to any one of [1] to [10], in which $X^1$ to $X^3$ are N.

[12] The compound according to any one of [1] to [11], in which $L^1$ is a single bond.

[13] The compound according to any one of [1] to [12], in which $R^1$ is a hydrogen atom.

[14] The compound according to any one of [1] to [13], in which the compound has at least one deuterium atom.

[15] A light-emitting material including the compound according to any one of [1] to [14].

[16] A delayed fluorescent substance including the compound according to any one of [1] to [14].

Advantageous Effects of Invention

The compound of the present invention is useful as a material for an organic light-emitting device.

Description of Embodiments

The contents of the present invention will be described in detail below. The following description of constitutional elements may be made based on representative embodiments and specific examples of the present invention, but the present invention is not limited to such embodiments and specific examples. In the description herein, a numerical range expressed using "to" means a range that includes the numerical values described before and after "to" as the lower limit and the upper limit. A part or all of hydrogen atoms existing in the molecule of the compound for use in the present invention can be substituted with deuterium atoms ($^2$H, deuterium D). In the chemical structural formulae in the description herein, the hydrogen atom is expressed as H, or the expression thereof is omitted. For example, when expression of the atoms bonding to the ring skeleton-constituting carbon atoms of a benzene ring is omitted, H is considered to bond to the ring skeleton-constituting carbon atom at the site having the omitted expression. In the description herein, the term "substituent" means an atom or an atomic group except a hydrogen atom and a deuterium atom. The term "substituted or unsubstituted" means that a hydrogen atom may be substituted with a deuterium atom or a substituent.

[Compound Represented by General Formula (1)]

The compound represented by the following general formula (1) is described.

General Formula (1)

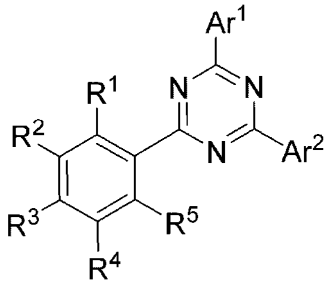

In the general formula (1), $R^1$ to $R^5$ each independently represent a hydrogen atom, a deuterium atom, a substituted or unsubstituted alkyl group, a substituted or unsubstituted aryl group, a donor group, or a group represented by the following formula (2). $R^2$ or $R^3$ is a group represented by the following general formula (2), and two or more of $R^1$ to $R^3$

are donor groups; $Ar^1$ and $Ar^2$ each independently represent a substituted or unsubstituted aryl group, or a substituted or unsubstituted heteroaryl group. In the compound represented by the general formula (1), at least one of $R^1$ to $R^5$ is a donor group having a fused ring structure of four or more rings, at least one of $Ar^1$ to $Ar^4$ is a substituted or unsubstituted heteroaryl group (excluding a nitrogen-containing 6-membered ring group), or $R^1$ to $R^5$ and $Ar^1$ to $Ar^4$ satisfy both of these conditions. Here, $Ar^3$ and $Ar^4$ are groups present in the general formula (2).

The alkyl group that $R^1$ to $R^5$ can adopt may be linear, branched or cyclic. Two or more of a linear moiety, a cyclic moiety, and a branched moiety may be in the group as mixed. The carbon number of the alkyl group can be, for example, 1 or more, 2 or more, or 4 or more. The carbon number can also be 30 or less, 20 or less, 10 or less, 6 or less, or 4 or less. Specific examples of the alkyl group include a methyl group, an ethyl group, an n-propyl group, an isopropyl group, an n-butyl group, an isobutyl group, a tert-butyl group, an n-pentyl group, an isopentyl group, an n-hexyl group, an isohexyl group, a 2-ethylhexyl group, an n-heptyl group, an isoheptyl group, an n-octyl group, an isooctyl group, an n-nonyl group, an isononyl group, an n-decanyl group, an isodecanyl group, a cyclopentyl group, a cyclohexyl group, and a cycloheptyl group. The alkyl group which is the substituent may be further substituted with, for example, a deuterium atom, an aryl group, an alkoxy group, an aryloxy group, and a halogen atom. In one aspect of the present invention, the substituent for the alkyl group is one or more selected from the group consisting of an aryl group and a deuterium atom. In one preferred aspect of the present invention, the alkyl group is unsubstituted, and, for example, can be selected from the group consisting of a methyl group, an ethyl group, an isopropyl group, and a tert-butyl group.

The aryl group that $R^1$ to $R^5$ and $Ar^1$ and $Ar^2$ can adopt each may be a monocyclic ring or may be a fused ring in which two or more rings are fused. In the case of a fused ring, the number of fused rings is preferably 2 to 6, and can be selected from, for example, 2 to 4. Specific examples of the ring include a benzene ring, a naphthalene ring, an anthracene ring, a phenanthrene ring, and a triphenylene ring. In one aspect of the present invention, the aryl group is a substituted or unsubstituted phenyl group, a substituted or unsubstituted naphthalen-1-yl group, or a substituted or unsubstituted naphthalen-2-yl group, and is preferably a substituted or unsubstituted phenyl group. For example, the substituent for the aryl group may be selected from Substituent Group A, may be selected from Substituent Group B, may be selected from Substituent Group C, may be selected from Substituent Group D, or may be selected from Substituent Group E. In one aspect of the present invention, the substituent for the aryl group is one or more selected from the group consisting of an alkyl group, an aryl group, and a deuterium atom. In one preferred aspect of the present invention, the aryl group is substituted with at least one deuterium atom. In one aspect of the present invention, the aryl group is unsubstituted.

Specific examples of the substituted or unsubstituted aryl group that $R^1$ to $R^5$ and $Ar^1$ and $Ar^2$ can adopt are shown below. However, the aryl group which can be employed in the present invention should not be limitatively interpreted by the following specific examples. In the following specific examples, * indicates a bonding position. The expression of a methyl group is omitted. Consequently, Ar2 to Ar7 represent structures substituted with a methyl group.

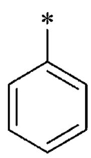
Ar1
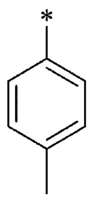
Ar2
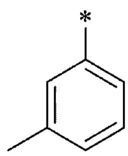
Ar3
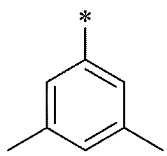
Ar4
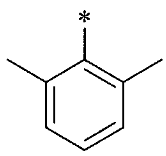
Ar5
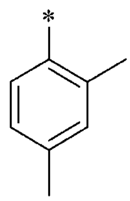
Ar6

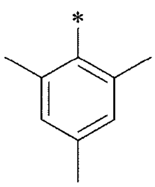
Ar7
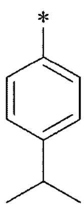
Ar8
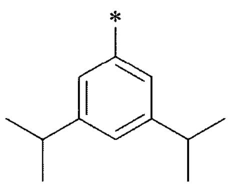
Ar9
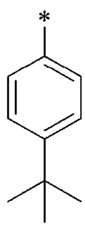
Ar10
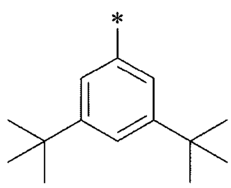
Ar11
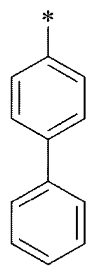
Ar12

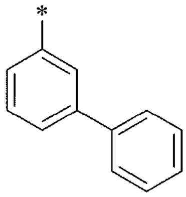
Ar13

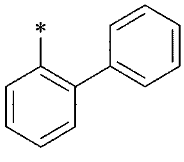
Ar14

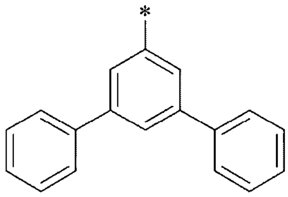
Ar15

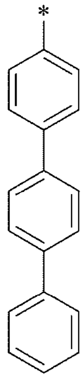
Ar16

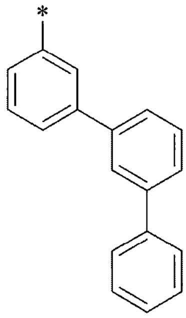
Ar17

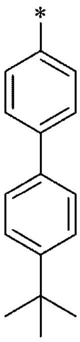
Ar18

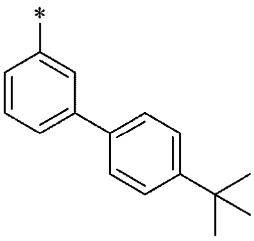
Ar19

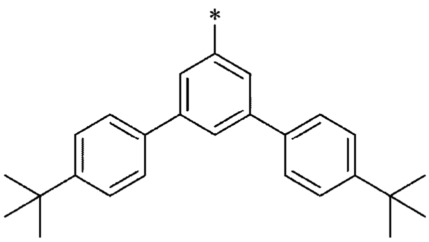
Ar20

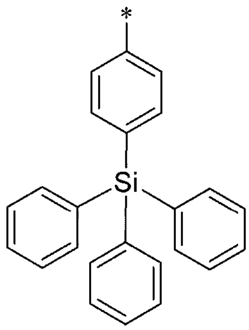
Ar21

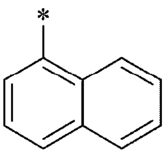
Ar22

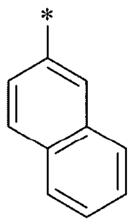
Ar23

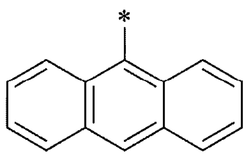
Ar24

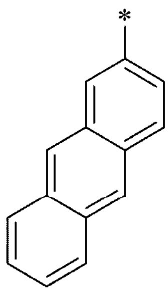
Ar25

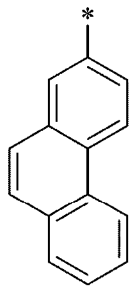
Ar26

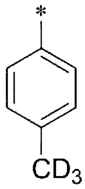
Ar27

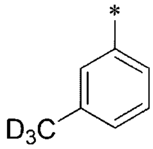
Ar28

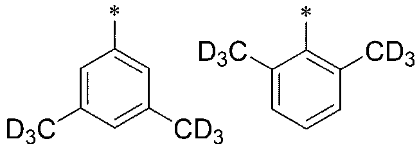
Ar29 Ar30

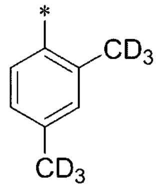
Ar31

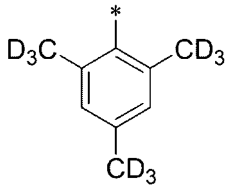
Ar32

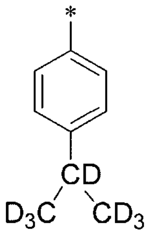
Ar33

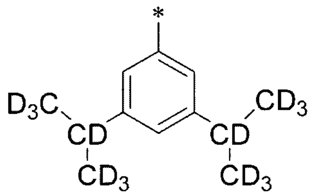
Ar34

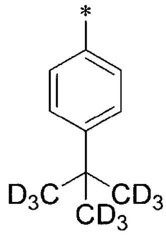
Ar35

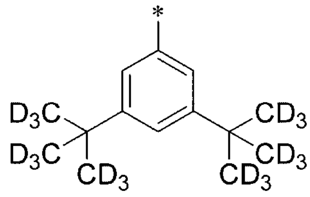
Ar36

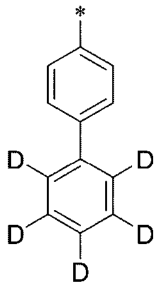
Ar37

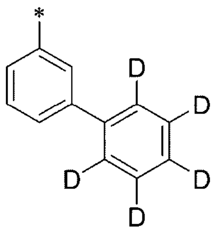
Ar38

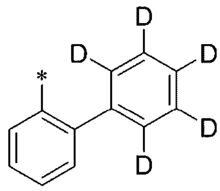
Ar39

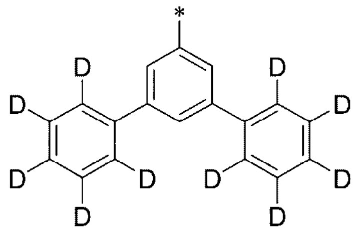
Ar40

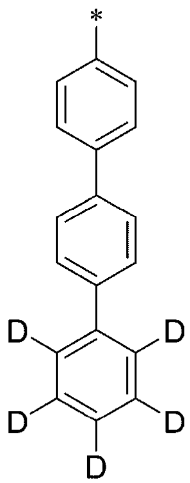
Ar41

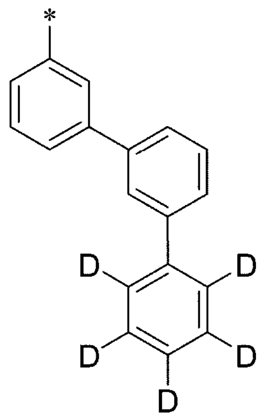
Ar42

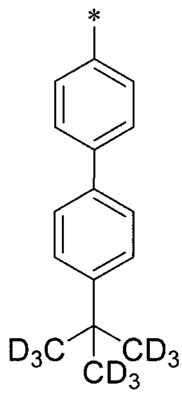
Ar43

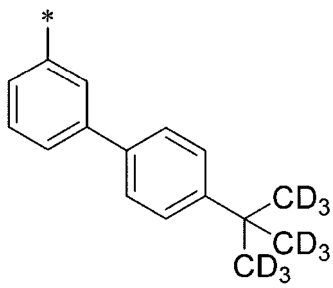
Ar44

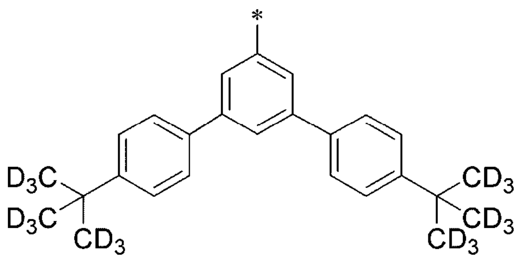
Ar45

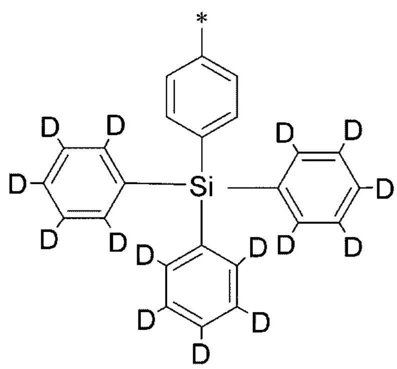
Ar46

In addition to the above-mentioned specific examples, groups obtained by substituting all hydrogen atoms present in Ar1 to Ar26 with deuterium atoms are exemplified as Ar47 to Ar72, respectively.

In one aspect of the present invention, the aryl group that $R^1$ to $R^5$ can adopt is selected from the group consisting of Ar1 to Ar72. In one aspect of the present invention, the aryl group that $R^1$ to $R^5$ can adopt is Ar1 or Ar47. In one aspect of the present invention, the aryl group that $R^1$ to $R^5$ can adopt is selected from the group consisting of Ar2 to Ar11, Ar27 to Ar36, and Ar48 to Ar57. In one aspect of the present invention, the aryl group that $R^1$ to $R^5$ can adopt is selected from the group consisting of Ar12 to Ar20, Ar37 to Ar45, and Ar58 to Ar66. In one aspect of the present invention, the aryl group that $R^1$ to $R^5$ can adopt is selected from the group consisting of Ar22 to Ar26 and Ar68 to Ar72. In one preferred aspect of the present invention, the aryl group that $R^1$ to $R^5$ can adopt is selected from the group consisting of Ar1, Ar12 to Ar15, Ar24, Ar37 to Ar40, Ar47, Ar58 to Ar61, and Ar70.

In one aspect of the present invention, the aryl group that $Ar^1$ and $Ar^2$ can adopt is selected from the group consisting of Ar1 to Ar72. In one aspect of the present invention, the aryl group that $Ar^1$ and $Ar^2$ can adopt is Ar1 or Ar47. In one aspect of the present invention, the aryl group that $Ar^1$ or $Ar^2$ can adopt is selected from the group consisting of Ar2 to Ar11, Ar27 to Ar36, and Ar48 to Ar57. In one aspect of the present invention, the aryl group that $Ar^1$ or $Ar^2$ can adopt is selected from the group consisting of Ar12 to Ar20, Ar37 to Ar45, and Ar58 to Ar66. In one preferred aspect of the present invention, the aryl group that $R^1$ to $R^5$ can adopt is selected from the group consisting of Ar1, Ar12 to Ar14, Ar37 to Ar40, Ar47, and Ar58 to Ar61.

At least two of $R^1$ to $R^5$ in the general formula (1) are donor groups. The donor group that $R^1$ to $R^5$ can adopt does not include a substituted or unsubstituted aryl group.

The "donor group" can be selected from groups having a negative Hammett's σp value. The "acceptor group" can be selected from groups having a positive Hammett's σp value. The Hammett's σp value is proposed by L. P. Hammett and quantifies the influence of a substituent on the reaction rate or equilibrium of a para-substituted benzene derivative. Specifically, the value is a constant (σp) specific to the substituent in the following equations that is established between substituents and reaction rate constants or equilibrium constants in para-substituted benzene derivatives:

$$\log(k/k_0) = \rho\sigma p$$

or

$\log(K/K_0) = \rho\sigma p$. In the above equations, $k_0$ represents a rate constant of a benzene derivative not having a substituent; k represents a rate constant of a benzene derivative substituted with a substituent; $K_0$ represents an equilibrium constant of a benzene derivative not having a substituent; K represents an equilibrium constant of a benzene derivative substituted with a substituent; and ρ represents a reaction constant to be determined by the type and the condition of reaction. Regarding the description relating to the "Hammett's σp value" and the numerical value of each substituent in the present invention, reference can be made to the description relating to σp value in Hansch, C. et. al., Chem. Rev., 91, 165-195 (1991).

The donor group that $R^1$ to $R^5$ can adopt preferably has σp of -0.3 or less, more preferably -0.5 or less, and still more preferably -0.7 or less. For example, the value may be selected from a range of -0.9 or less, or from a range of -1.1 or less.

The donor group in the present invention is preferably a group containing a substituted amino group. The donor group may be a substituted amino group, or may be a substituted amino group-bonded aryl group, especially a substituted amino group-bonded phenyl group. In one preferred aspect of the present invention, the donor group is a substituted amino group.

The substituent bonding to the nitrogen atom of a substituted amino group is preferably a substituted or unsubstituted alkyl group, a substituted or unsubstituted alkenyl group, a substituted or unsubstituted aryl group, or a substituted or unsubstituted heteroaryl group, more preferably a substituted or unsubstituted aryl group or a substituted or unsubstituted heteroaryl group. Especially, the substituted amino group is preferably a substituted or unsubstituted diarylamino group, or a substituted or unsubstituted diheteroarylamino group. The two aryl groups constituting the diarylamino group referred to herein may be bonded to each other, and the two heteroaryl groups constituting the diheteroarylamino group may be bonded to each other.

The donor group that $R^1$ to $R^5$ can adopt is preferably a group represented by the following general formula (a).

General Formula (a)

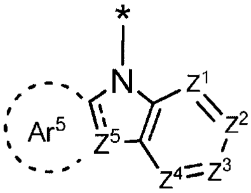

In the general formula (a), $Z^1$ represents C-$R^{14}$ or N, $Z^2$ represents C-$R^{15}$ or N, $Z^3$ represents C-$R^{16}$ or N, and $Z^4$ represents C-$R^{17}$ or N. $Z^5$ represents C or N, $Ar^5$ represents a substituted or unsubstituted aromatic ring or a substituted or unsubstituted heteroaromatic ring. $R^{14}$ and $R^{15}$, $R^{15}$ and $R^{16}$, and $R^{16}$ and $R^{17}$ each may be bonded to each other to form a cyclic structure.

Among $Z^1$ to $Z^4$, the number of groups represented by N is preferably 0 to 3, and preferably 0 to 2. In one aspect of the present invention, among $Z^1$ to $Z^4$, the number of groups represented by N is 1. In one aspect of the present invention, among $Z^1$ to $Z^4$, the number of groups represented by N is 0.

$R^{14}$ to $R^{17}$ each independently represent a hydrogen atom, a deuterium atom, or a substituent.

For example, the substituent may be selected from Substituent Group A, may be selected from Substituent Group B, may be selected from Substituent Group C, may be selected from Substituent Group D, or may be selected from Substituent Group E. When two or more of $R^{14}$ to $R^{17}$ represent substituents, the two or more substituents may be the same or different. Zero to two of $R^{14}$ to $R^{17}$ are preferably substituents, and for example, one may be a substituent, or zero may be a substituent ($R^{14}$ to $R^{17}$ are a hydrogen atom or a deuterium atom).

$R^{14}$ and $R^{15}$, $R^{15}$ and $R^{16}$, and $R^{16}$ and $R^{17}$ each may be bonded to each other to form a cyclic structure. The cyclic structure may be any of an aromatic ring, an heteroaromatic ring, an aliphatic hydrocarbon ring, and an aliphatic heterocyclic ring, and may be a ring obtained by fusing these rings. The structure is preferably an aromatic ring or a heteroaromatic ring. Examples of the aromatic ring include a substituted or unsubstituted benzene ring. Another benzene ring may be further fused to the benzene ring, and a heterocyclic ring such as a pyridine ring may be fused to the benzene ring. The heteroaromatic ring means a ring exhibiting aromaticity including a hetero atom as a ring skeleton-constituting atom, and is preferably a 5- to 7-membered ring, and for example, a 5-membered ring or a 6-membered ring can be employed. In one aspect of the present invention, a furan ring, a thiophene ring, or a pyrrole ring can be employed as the heteroaromatic ring. In one preferred aspect of the present invention, the cyclic structure is a furan ring of a substituted or unsubstituted benzofuran, a thiophene ring of a substituted or unsubstituted benzothiophene, or a pyrrole ring of a substituted or unsubstituted indole. The benzofuran, benzothiophene, and indole referred to herein may be unsubstituted, may be substituted with a substituent selected from Substituent Group A, may be substituted with a substituent selected from Substituent Group B, may be substituted with a substituent selected from Substituent Group C, may be substituted with a substituent selected from Substituent Group D, and may be substituted with a substituent selected from Substituent Group E. It is preferable that a substituted or unsubstituted aryl group is bonded to the nitrogen atom constituting the pyrrole ring of indole, and examples of the substituent include a substituent selected from any of Substituent Group A to Substituent Group E. The cyclic structure may be a substituted or unsubstituted cyclopentadiene ring. In one aspect of the present invention, a pair of $R^{14}$ and $R^{15}$, $R^{15}$ and $R^{16}$, and $R^{16}$ and $R^{17}$ are bonded to each other to form a cyclic structure. In one aspect of the present invention, none of $R^{14}$ and $R^{15}$, $R^{15}$ and $R^{16}$, and $R^{16}$ and $R^{17}$ are bonded to each other to form a cyclic structure.

In the general formula (a), $Z^5$ represents C or N, $Ar^5$ represents a substituted or unsubstituted aromatic ring or a substituted or unsubstituted heteroaromatic ring. In one aspect of the present invention, $Z^5$ is C, and $Ar^5$ is a substituted or unsubstituted aromatic ring or a substituted or unsubstituted heteroaromatic ring. In one aspect of the present invention, $Z^5$ is N, and $Ar^5$ is a substituted or unsubstituted heteroaromatic ring.

Examples of the aromatic ring that $Ar^5$ can adopt include a benzene ring. Another benzene ring may be further fused to the benzene ring, and a heterocyclic ring such as a pyridine ring may be fused to the benzene ring. The heteroaromatic ring that $Ar^5$ can adopt is preferably a 5- to 7-membered ring, and for example, a 5-membered ring or a 6-membered ring can be employed. In one aspect of the present invention, as the heteroaromatic ring, a furan ring, a thiophene ring, a pyrrole ring, an imidazole ring, a pyridine ring, a pyridazine ring, a pyrimidine ring, or a pyrazine ring can be employed. In one aspect of the present invention, $Z^5$ is C, and the heteroaromatic ring is a furan ring of a substituted or unsubstituted benzofuran, a thiophene ring of a substituted or unsubstituted benzothiophene, a pyridine ring of a substituted or unsubstituted quinoline, or a pyridine ring of a substituted or unsubstituted isoquinoline. In one aspect of the present invention, $Z^5$ is N, and the heteroaromatic ring is a pyrrole ring of a substituted or unsubstituted indole, or an imidazole ring of a substituted or unsubstituted benzimidazole. The benzofuran, benzothiophene, quinoline, isoquinoline, indole, and benzimidazole referred to herein may be unsubstituted, or may be substituted with a substituent selected from Substituent Group A, may be substituted with a substituent selected from Substituent Group B, may be substituted with a

substituent selected from Substituent Group C, may be substituted with a substituent selected from Substituent Group D, and may be substituted with a substituent selected from Substituent Group E.

When $Z^5$ in the general formula (a) is C, a group represented by the following general formula (b) is preferred.

General Formula (b)

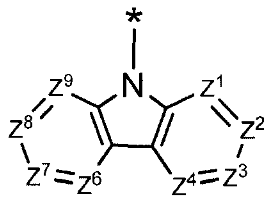

In the general formula (b), $Z^1$ represents C-$R^{14}$ or N, $Z^2$ represents C-$R^{15}$ or N, $Z^3$ represents C-$R^{16}$ or N, $Z^4$ represents C-$R^{17}$ or N, $Z^6$ represents C-$R^{18}$ or N, $Z^7$ represents C-$R^{19}$ or N, $Z^8$ represents C-$R^{20}$ or N, and $Z^9$ represents C-$R^{21}$ or N. $R^{14}$ and $R^{15}$, $R^{15}$ and $R^{16}$, $R^{16}$ and $R^{17}$, $R^{18}$ and $R^{19}$, $R^{19}$ and $R^{20}$, and $R^{20}$ and $R^{21}$ each may be bonded to each other to form a cyclic structure.

Regarding $Z^1$ to $Z^4$ and $R^{14}$ to $R^{17}$ in the general formula (b), reference can be made to the corresponding description of the general formula (a). $Z^6$ to $Z^9$ and $R^{18}$ to $R^{21}$ in the general formula (b) correspond to $Z^1$ to $Z^4$ and $R^{14}$ to $R^{17}$ in the general formula (a), respectively, and regarding the contents thereof, reference can be made to the descriptions of $Z^1$ to $Z^4$ and $R^{14}$ to $R^{17}$ in the general formula (a).

In one aspect of the present invention, among $Z^1$ to $Z^4$ and $Z^6$ to $Z^9$, the number of groups represented by N is preferably 0 to 2, and more preferably 0 or 1. In one aspect of the present invention, among $Z^1$ to $Z^4$ and $Z^6$ to $Z^9$, the number of groups represented by N is 1. In one preferred aspect of the present invention, among $Z^1$ to $Z^4$ and $Z^6$ to $Z^9$, the number of groups represented by N is 0. When the number is 0, the formula represents a substituted or unsubstituted carbazol-9-yl group.

The donor group that $R^1$ to $R^5$ can adopt is preferably a substituted or unsubstituted carbazol-9-yl group. The carbazol-9-yl group referred to herein may be unsubstituted, or may be substituted with a substituent selected from Substituent Group A, may be substituted with a substituent selected from Substituent Group B, may be substituted with a substituent selected from Substituent Group C, may be substituted with a substituent selected from Substituent Group D, and may be substituted with a substituent selected from Substituent Group E. In addition, one or more rings may be further fused to the two benzene rings constituting the carbazol-9-yl group. In one preferred aspect of the present invention, the donor group that $R^1$ to $R^5$ can adopt is a carbazol-9-yl group optionally substituted with a group selected from Substituent Group E, and optionally fused with one or more rings. In the case where the carbazol-9-yl group not fused with a ring is substituted, the substitution site is not specifically limited, but is preferably at least one of 2- to 7-positions, more preferably at least one of 3- to 6-positions, and still more preferably a 3-position and a 6-position.

In one aspect of the present invention, the donor group that $R^1$ to $R^5$ can adopt is a carbazol-9-yl group fused with one or more rings, and hereinafter, this will be referred to as a "ring-fused carbazol-9-yl group". The ring-fused carbazol-9-yl group that $R^1$ to $R^5$ can adopt may be unsubstituted, or may be substituted with a substituent selected from Substituent Group A, may be substituted with a substituent selected from Substituent Group B, may be substituted with a substituent selected from Substituent Group C, may be substituted with a substituent selected from Substituent Group D, and may be substituted with a substituent selected from Substituent Group E. Preferably, the group is unsubstituted, or substituted with a substituent selected from Substituent Group E. In one aspect of the present invention, the ring-fused carbazol-9-yl group is unsubstituted. In one preferred aspect of the present invention, the ring-fused carbazol-9-yl group is substituted with an aryl group optionally substituted with one atom or group selected from the group consisting of a deuterium atom, an alkyl group, and an aryl group or with a group formed by combining two or more thereof.

The total number of fused rings in the ring-fused carbazol-9-yl group is 4 or more, preferably 5 or more, more preferably 5 to 9, and still more preferably 5 to 7. In one preferred aspect of the present invention, the number of rings constituting the fused ring is 5. Here, the number of rings includes the number of rings of carbazole to be fused (i.e. 3).

The ring-fused carbazol-9-yl group is a group bonded via a nitrogen atom constituting the ring skeleton of carbazole, and has a structure in which a ring is fused to at least one of the two benzene rings constituting carbazole. The fused ring may be any of an aromatic hydrocarbon ring, an aromatic heterocyclic ring, an aliphatic hydrocarbon ring, and an aliphatic heterocyclic ring, and may be a ring obtained by further fusing these rings. Preferred are an aromatic hydrocarbon ring and an aromatic heterocyclic ring. Examples of the aromatic hydrocarbon ring include a substituted or unsubstituted benzene ring. Another benzene ring may be further fused to the benzene ring, and a heterocyclic ring such as a pyridine ring may be fused to the benzene ring. The aromatic heterocyclic ring means a ring exhibiting aromaticity including a hetero atom as a ring skeleton-constituting atom, and is preferably a 5- to 7-membered ring, and for example, a 5-membered ring or a 6-membered ring can be employed. In one aspect of the present invention, a furan ring, a thiophene ring, or a pyrrole

ring can be employed as the aromatic heterocyclic ring. In one aspect of the present invention, the fused ring is a furan ring of a substituted or unsubstituted benzofuran, a thiophene ring of a substituted or unsubstituted benzothiophene, or a pyrrole ring of a substituted or unsubstituted indole. It is preferable that a substituent selected from Substituent Group E (but except for a deuterium atom alone) is bonded to the nitrogen atom of the pyrrole ring, and it is more preferable that an aryl group optionally substituted with an alkyl group or an aryl group is bonded. In the present invention, it is preferable to employ a carbazol-9-yl group in which a ring having one or more atoms selected from the group consisting of an oxygen atom, a sulfur atom, and a nitrogen atom as a ring skeleton-constituting atom is fused. Above all, preferably employed are a benzofuro structure-fused carbazol-9-yl group, a benzothieno structure-fused carbazol-9-yl group, and an indolo structure-fused carbazol-9-yl group. In one aspect of the present invention, the compound has at least one benzofuro structure-fused carbazol-9-yl group, and for example, has two or more such groups. In one aspect of the present invention, the compound has at least one benzothieno structure-fused carbazol-9-yl group, and for example, has two or more such groups.

The ring-fused carbazol-9-yl group employable herein includes a substituted or unsubstituted benzofuro[2,3-a]carbazol-12-yl group, a substituted or unsubstituted benzofuro[3,2-a]carbazol-12-yl group, a substituted or unsubstituted benzofuro[2,3-b]carbazol-7-yl group, a substituted or unsubstituted benzofuro[3,2-b]carbazol-11-yl group, a substituted or unsubstituted benzofuro[2,3-c]carbazol-8-yl group, and a substituted or unsubstituted benzofuro[3,2-c]carbazol-5-yl group. Further, the ring-fused carbazol-9-yl group employable herein includes a substituted or unsubstituted benzothieno[2,3-a]carbazol-12-yl group, a substituted or unsubstituted benzothieno[3,2-a]carbazol-12-yl group, a substituted or unsubstituted benzothieno[2,3-b]carbazol-7-yl group, a substituted or unsubstituted benzothieno[3,2-b]carbazol-11-yl group, a substituted or unsubstituted benzothieno[2,3-c]carbazol-8-yl group, and a substituted or unsubstituted benzothieno[3,2-c]carbazol-5-yl group. Furthermore, the ring-fused carbazol-9-yl group employable herein includes a substituted or unsubstituted 11-phenylindolo[2,3-a]carbazol-12-yl group, a substituted or unsubstituted 5-phenylindolo[3,2-a]carbazol-12-yl group, a substituted or unsubstituted 5-phenylindolo[2,3-b]carbazol-7-yl group, a substituted or unsubstituted 5-phenylindolo[3,2-b]carbazol-11-yl group, a substituted or unsubstituted 5-phenylindolo[2,3-c]carbazol-8-yl group, and a substituted or unsubstituted 12-phenylindolo[3,2-a]carbazol-5-yl group.

The number of the substituents substituted on the ring-fused carbazol-9-yl group is preferably 1 to 10, more preferably 1 to 6, still more preferably 1 to 4, and may be, for example 1, or may be, for example 2. In one preferred aspect of the present invention, any of the 3-position or the 6-position of the ring-fused carbazol-9-yl group is substituted. In one preferred aspect of the present invention, the compound has at least one substituent on the para-position of the benzene ring viewed from the hetero atom present in the ring-fused carbazol-9-yl group. In one preferred aspect of the present invention, the compound has at least one substituent only on the para-position of the benzene ring viewed from the hetero atom present in the ring-fused carbazol-9-yl group. In one preferred aspect of the present invention, the compound has substituents on all the substitutable para-positions of the benzene ring viewed from the hetero atom present in the ring-fused carbazol-9-yl group.

Specific examples of the donor group that $R^1$ to $R^5$ in the general formula (1) can adopt are shown below. However, the donor group which can be employed in the present invention shall not be construed as being limited by the following specific examples.

First, specific examples of the substituted or unsubstituted carbazol-9-yl group in which the donor group has a fused ring structure of three rings are shown below. In the following specific examples, Ph represents a phenyl group ($C_6H_5$), and * indicates a bonding position. Since the expression of a methyl group is omitted, for example, D2 has one methyl group. A deuterated methyl group is expressed as $CD_3$. In addition, $C_6D_5$ represents a phenyl group in which all hydrogen atoms are deuterated. D represents a deuterium atom.

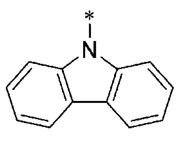
D1

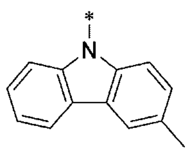
D2

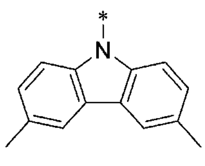
D3

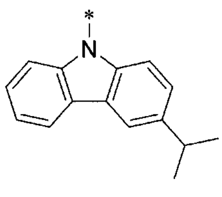
D4

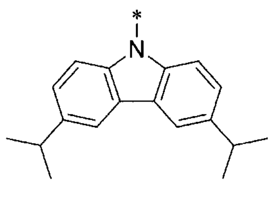
D5

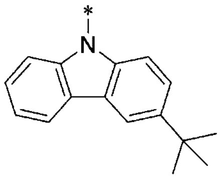
D6

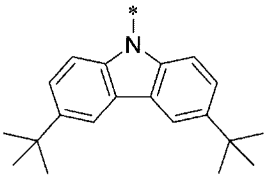
D7

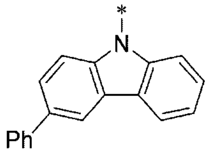
D8

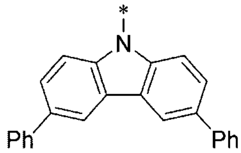
D9

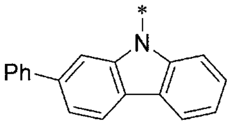
D10

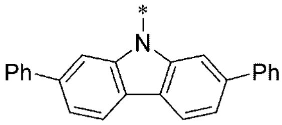
D11

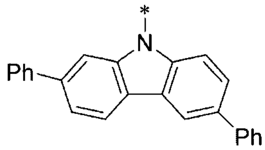
D12

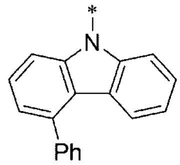
D13

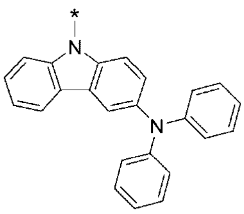
D14

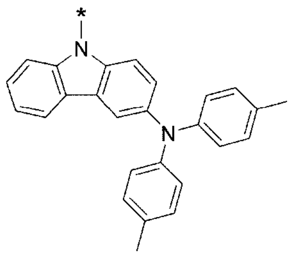
D15

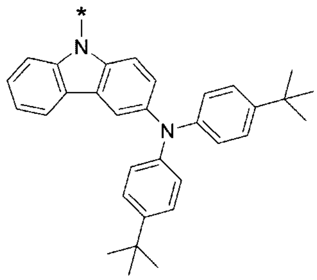
D16

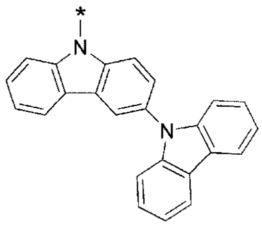
D17

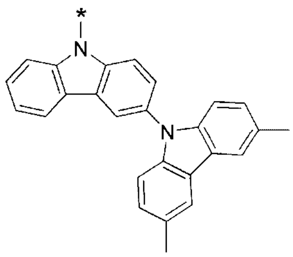
D18

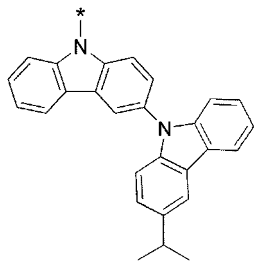
D19

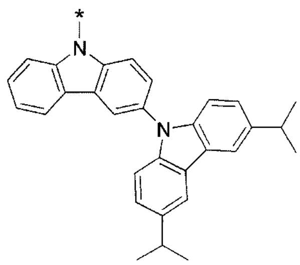
D20

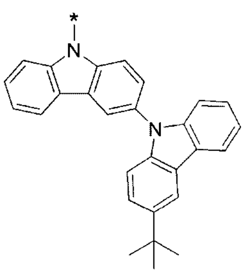
D21

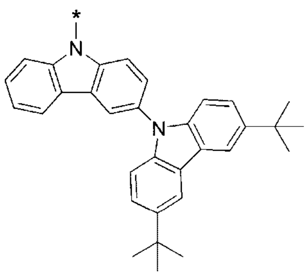
D22

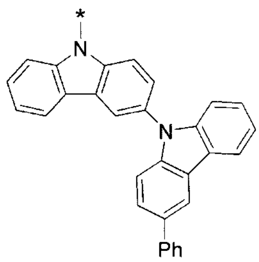
D23

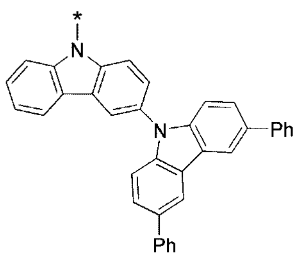
D24

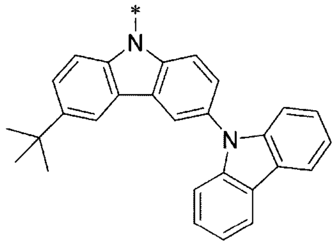 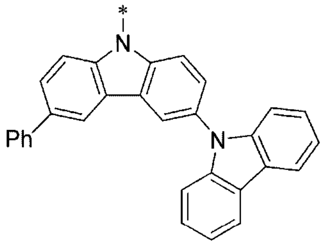 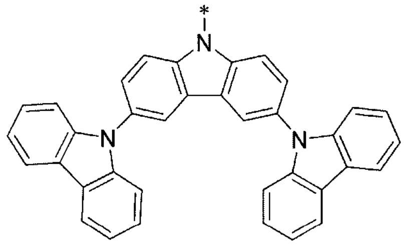

D25 D26 D27

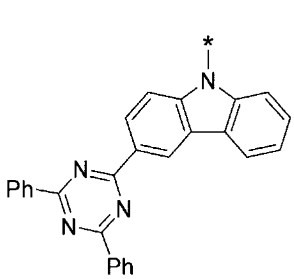 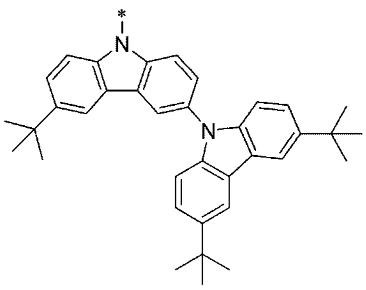 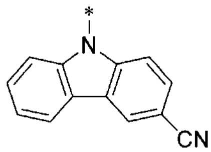

D28 D29 D30

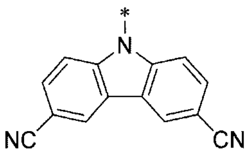 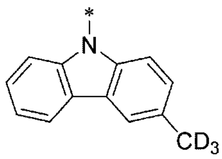 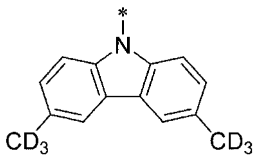 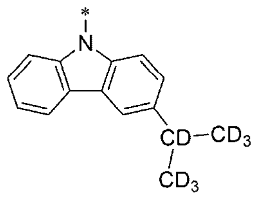

D31 D32 D33 D34

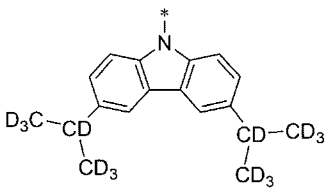 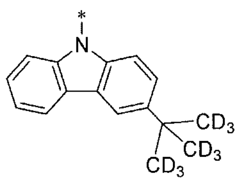 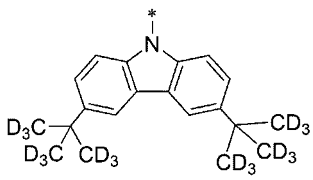

D35 D36 D37

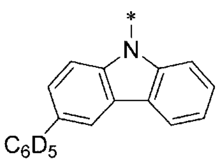 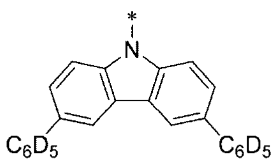 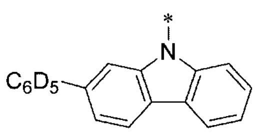 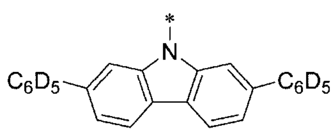

D38 D39 D40 D41

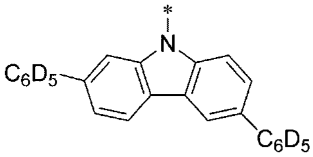

D42

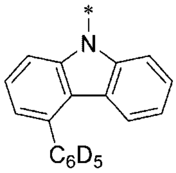

D43

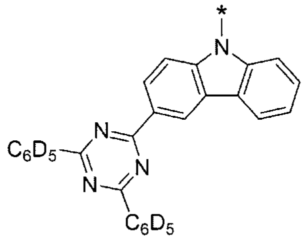

D44

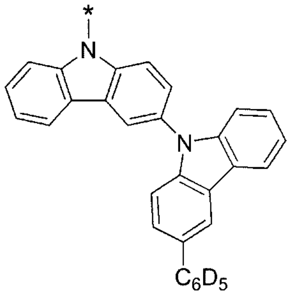

D45

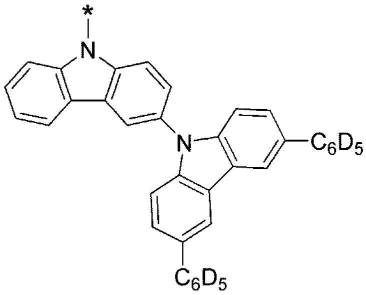

D46

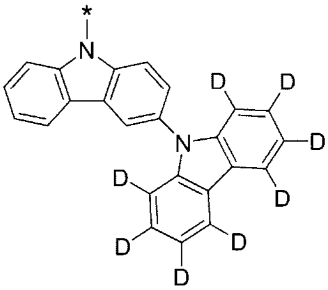

D47

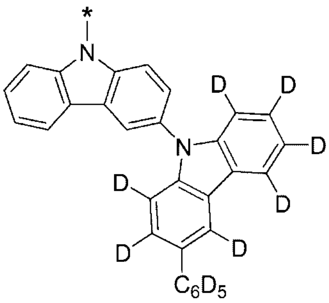

D48

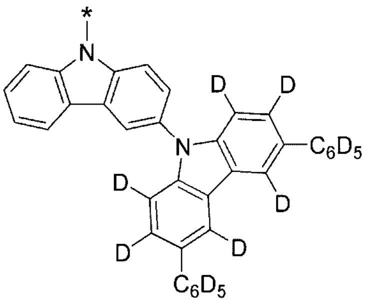

D49

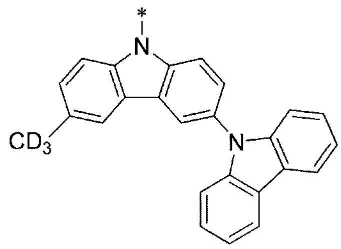

D50

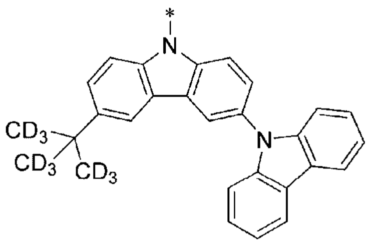

D51

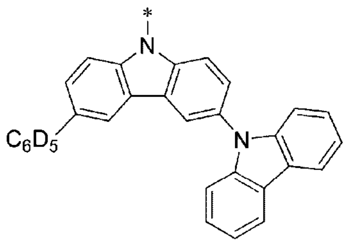

D52

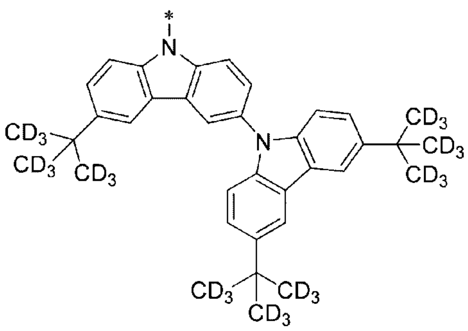

D53

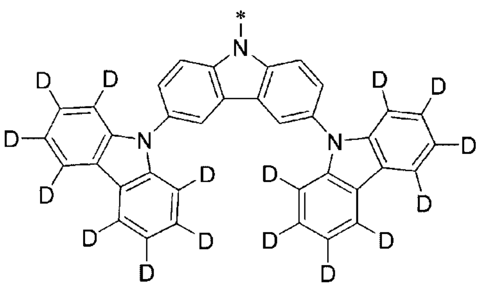

D54

Groups obtained by substituting all hydrogen atoms present in the above D1 to D31 with deuterium atoms are disclosed as D55 to D85.

Next, specific examples of the donor group having a fused ring structure of four or more rings are shown below.

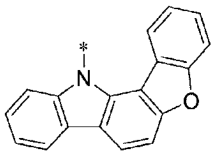

D86

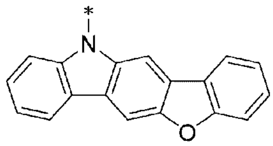

D87

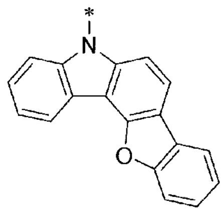

D88

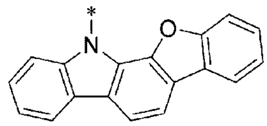

D89

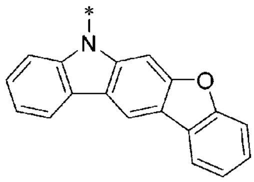

D90

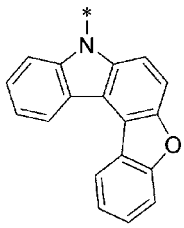

D91

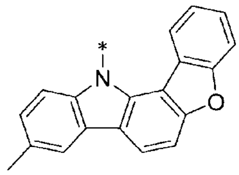

D92

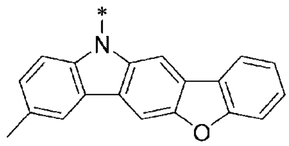

D93

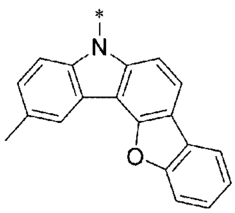

D94

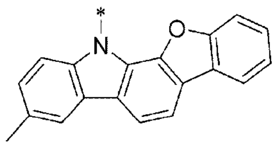

D95

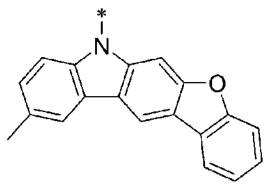

D96

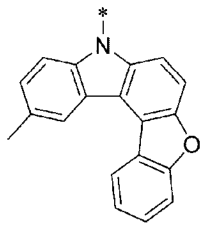

D97

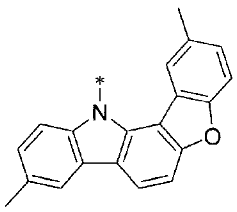

D98

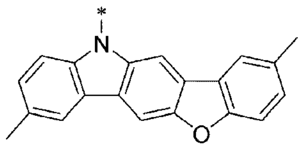

D99

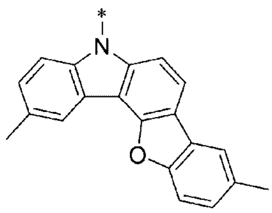

D100

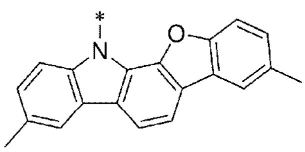

D101

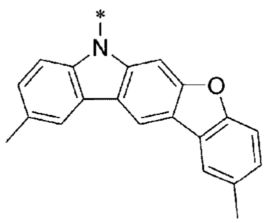

D102

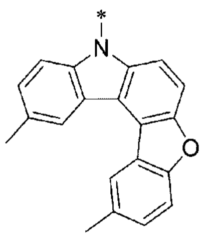

D103

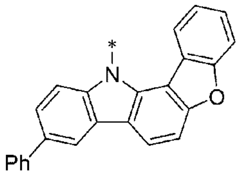

D104

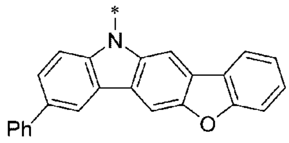

D105

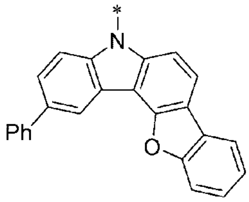

D106

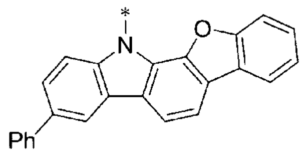

D107

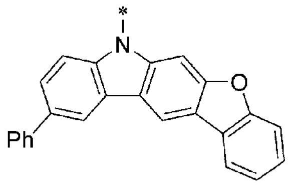

D108

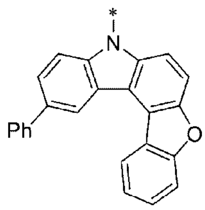

D109

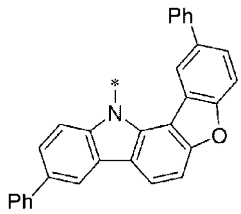

D110

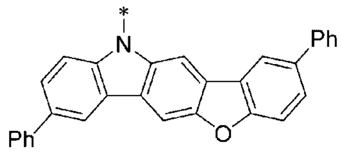

D111

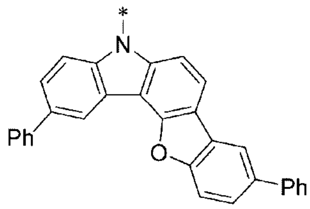

D112

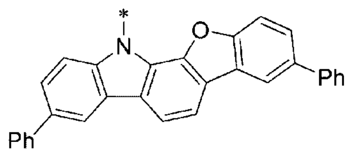

D113

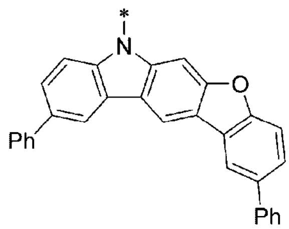

D114

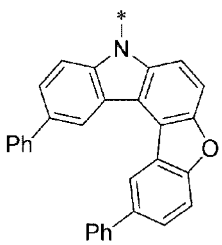

D115

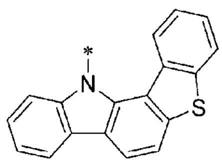

D116

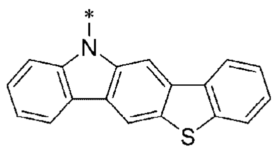

D117

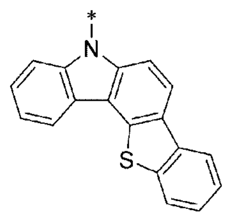

D118

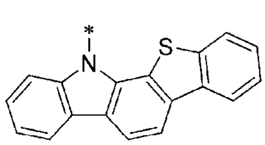

D119

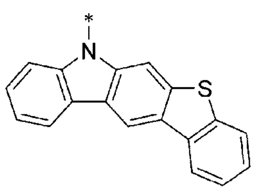

D120

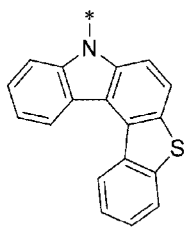

D121

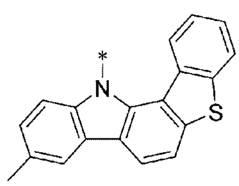

D122

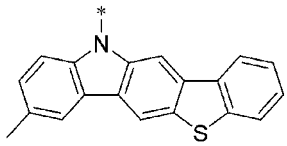
D123

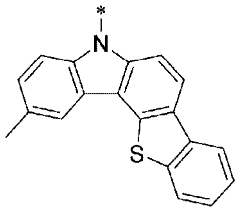
D124

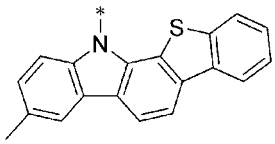
D125

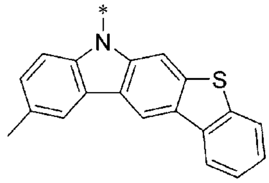
D126

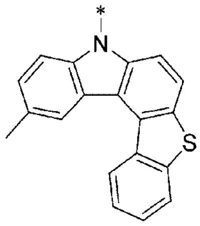
D127

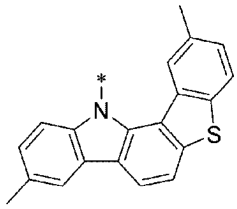
D128

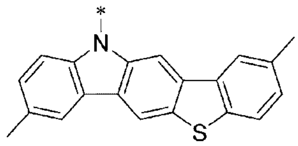
D129

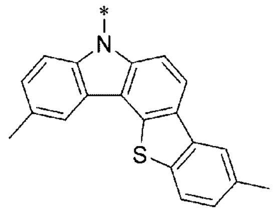
D130

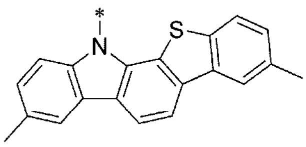
D131

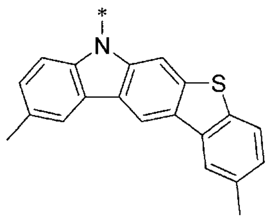
D132

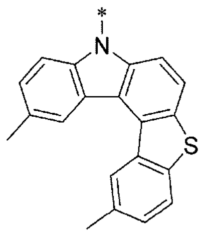
D133

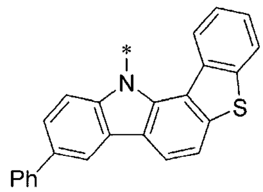
D134

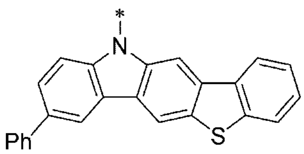
D135

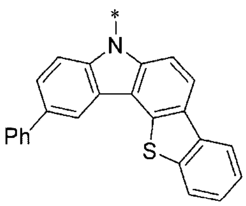
D136

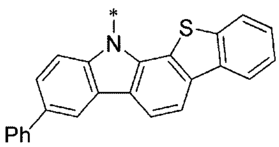
D137

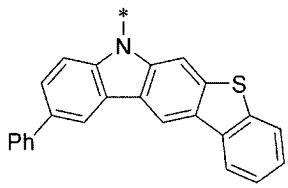
D138

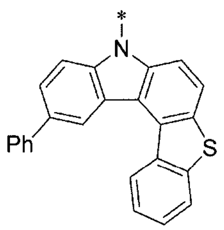
D139

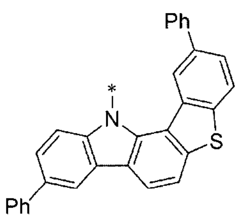
D140

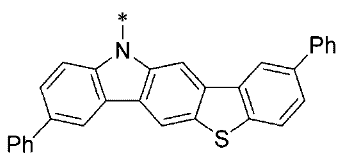
D141

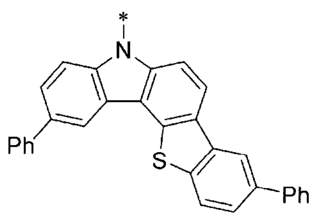
D142

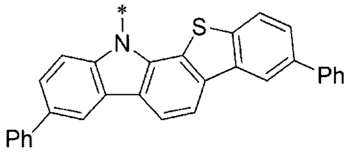

D143

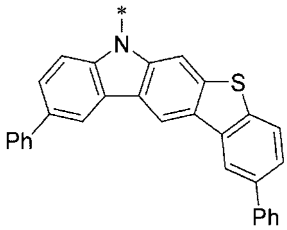

D144

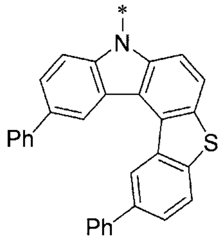

D145

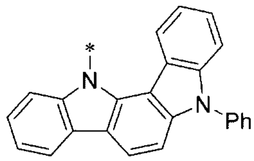

D146

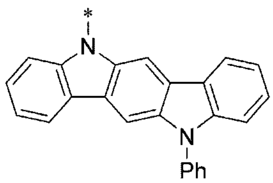

D147

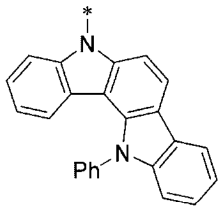

D148

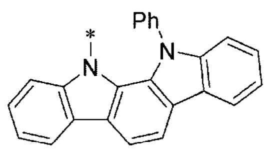

D149

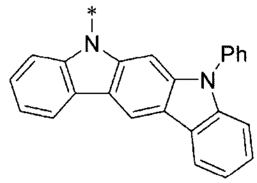

D150

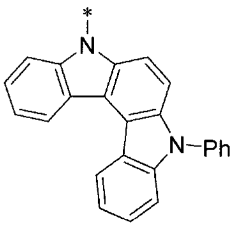

D151

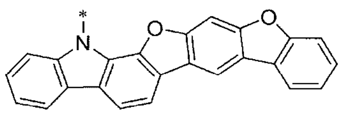

D152

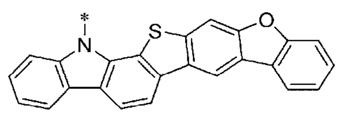

D153

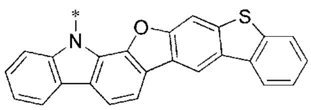

D154

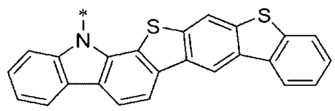

D155

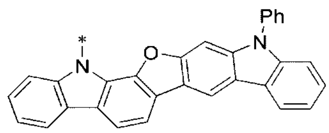

D156

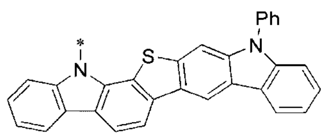

D157

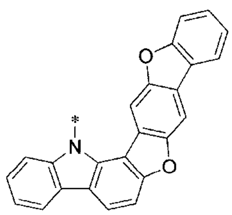

D158

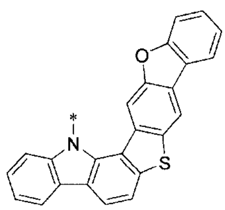

D159

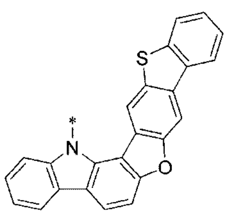

D160

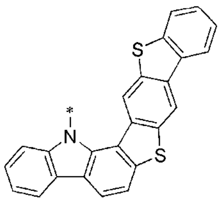
D161

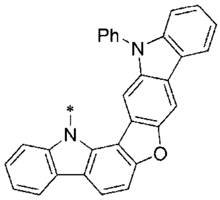
D162

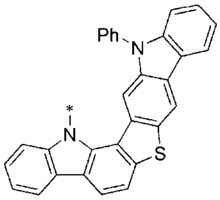
D163

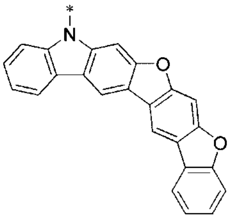
D164

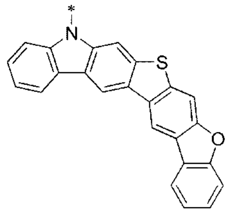
D165

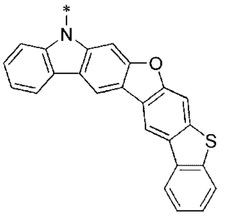
D166

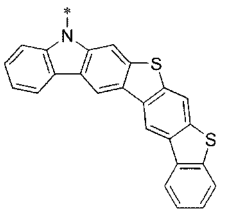
D167

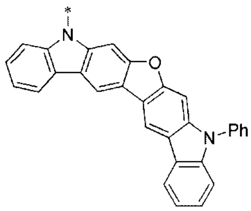
D168

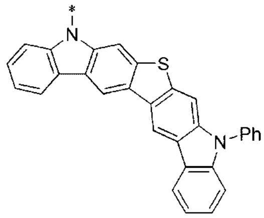
D169

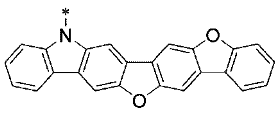
D170

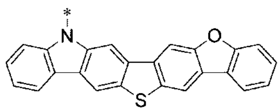
D171

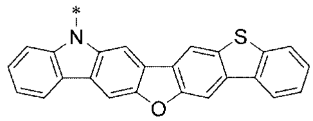
D172

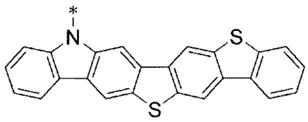
D173

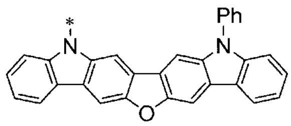
D174

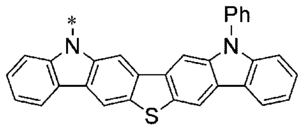
D175

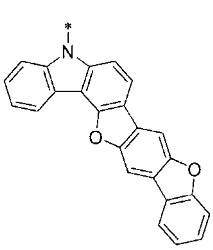
D176

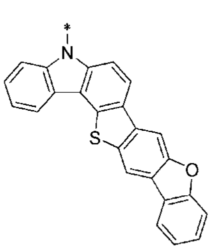
D177

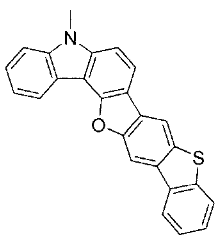
D178

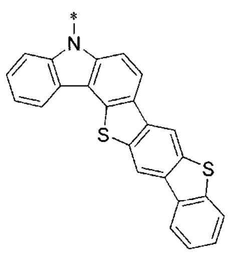
D179

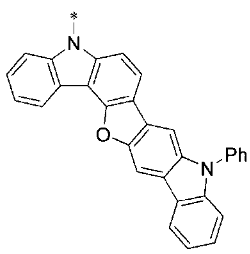
D180

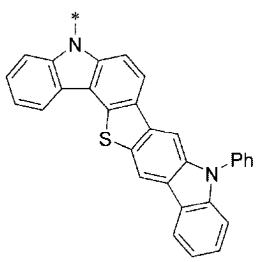

D181

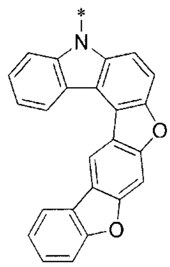

D182

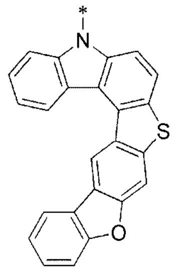

D183

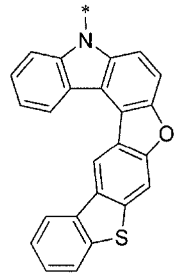

D184

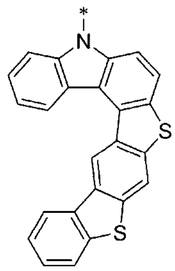

D185

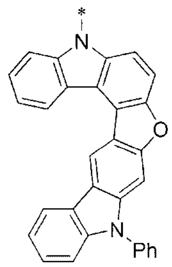

D186

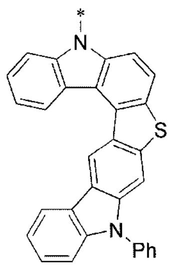

D187

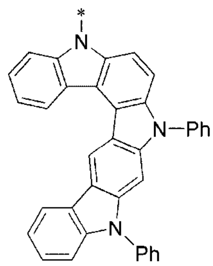

D188

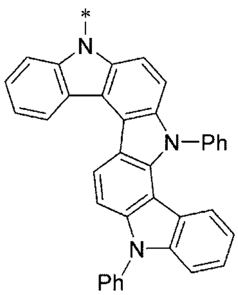

D189

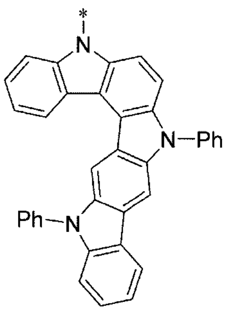

D190

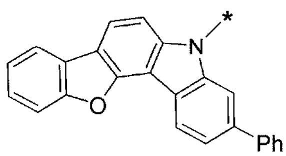

D191

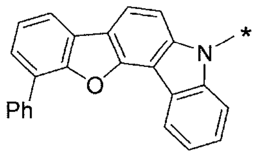

D192

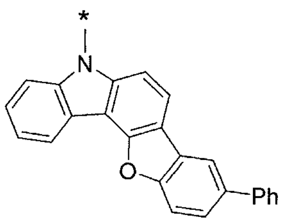

D193

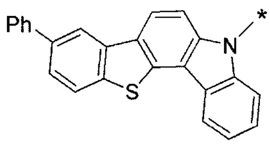

D194

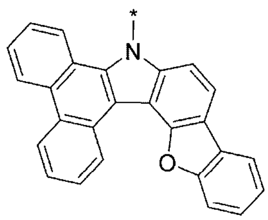

D195

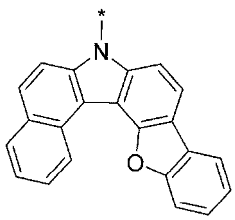

D196

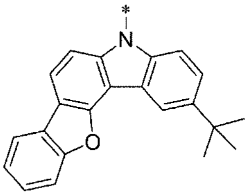

D197

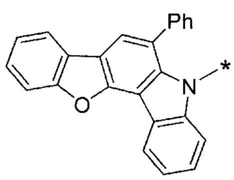

D198

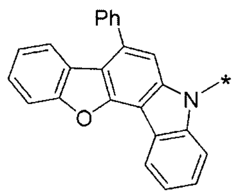

D199

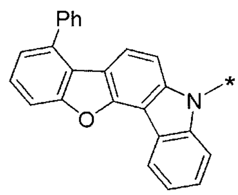

D200

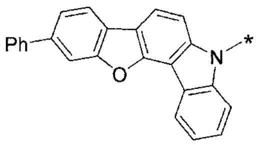
D201

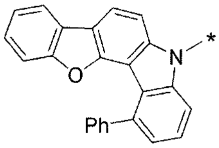
D202

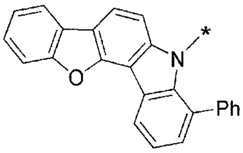
D203

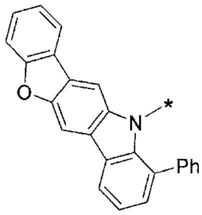
D204

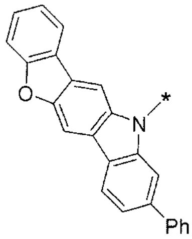
D205

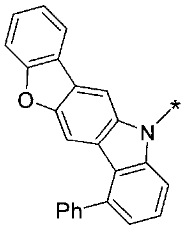
D206

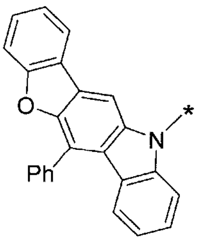
D207

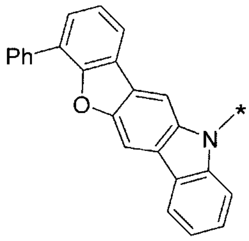
D208

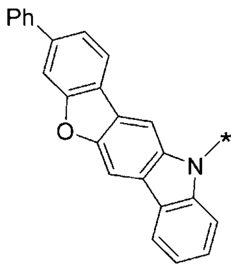
D209

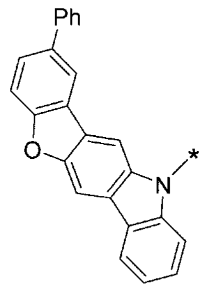
D210

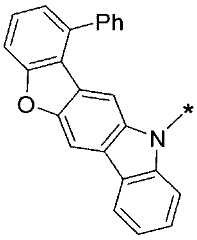
D211

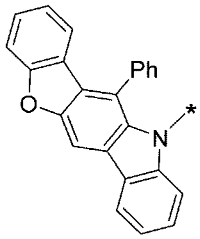
D212

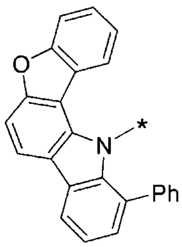
D213

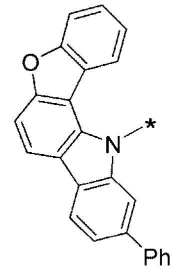
D214

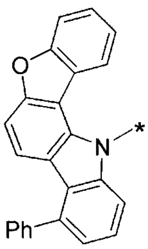
D215

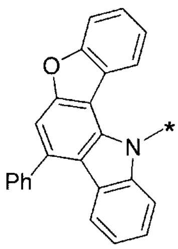
D216

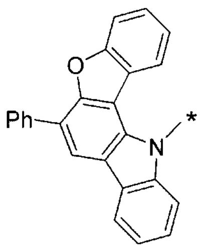
D217

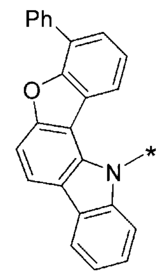
D218

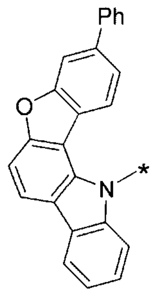
D219

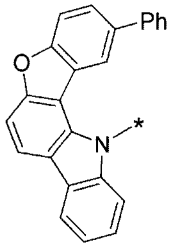
D220

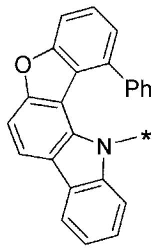
D221

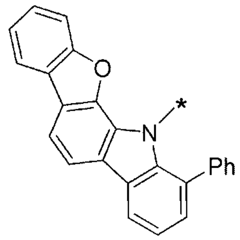
D222

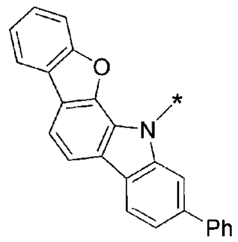
D223

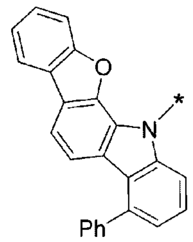
D224

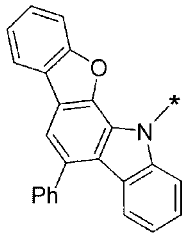
D225

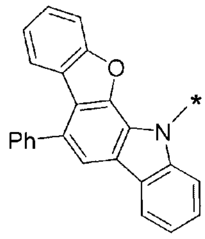
D226

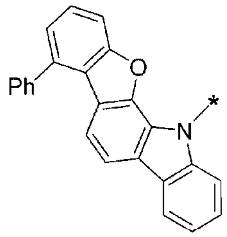
D227

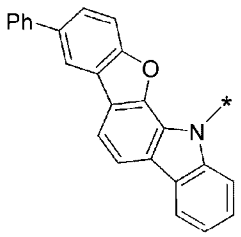
D228

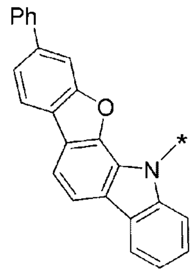
D229

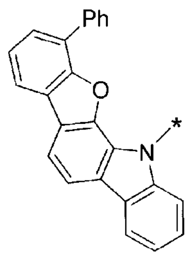
D230

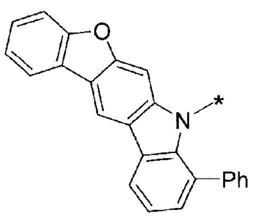
D231

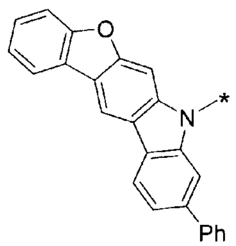
D232

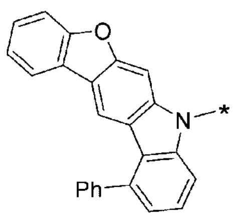
D233

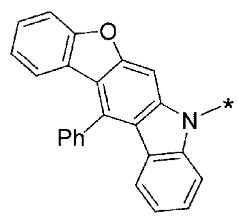
D234

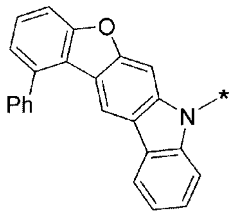

D235

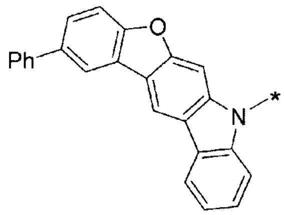

D236

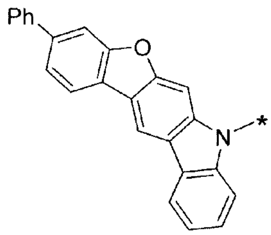

D237

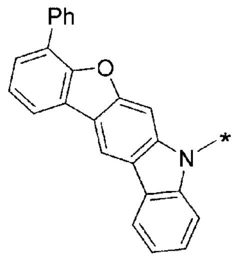

D238

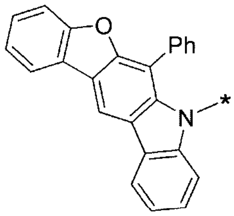

D239

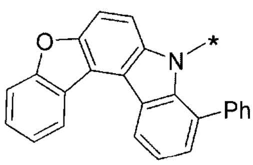

D240

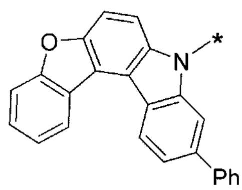

D241

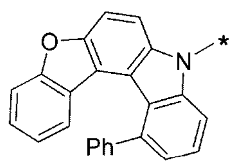

D242

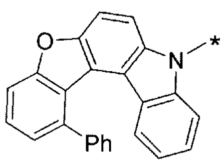

D243

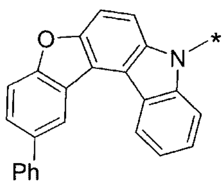

D244

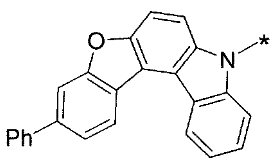

D245

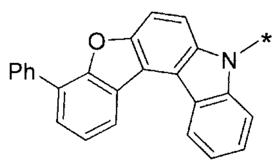

D246

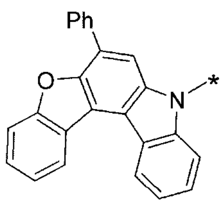

D247

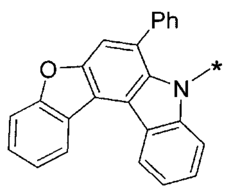

D248

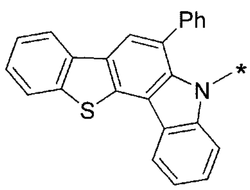

D249

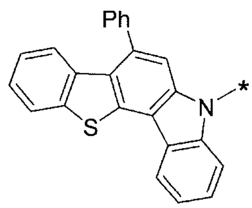

D250

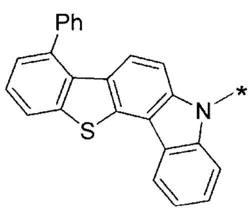

D251

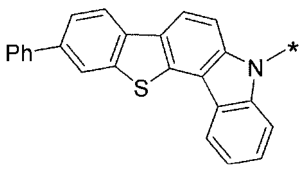

D252

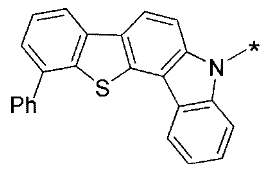

D253

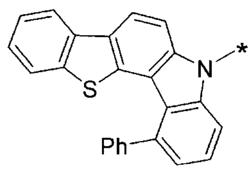

D254

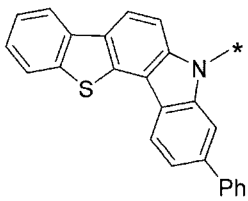

D255

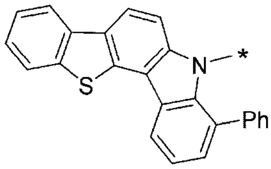

D256

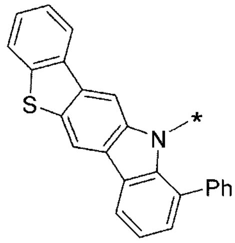

D257

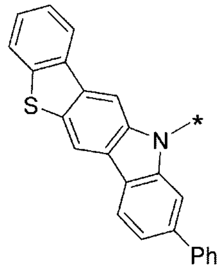

D258

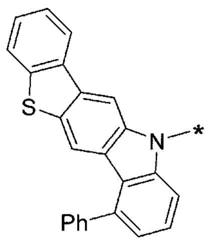

D259

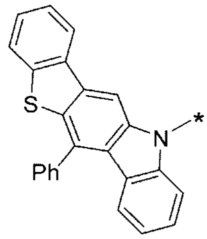

D260

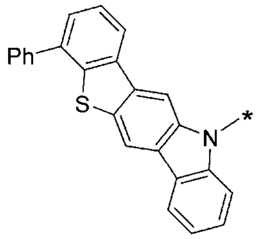

D261

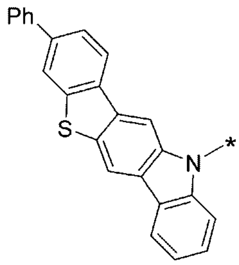

D262

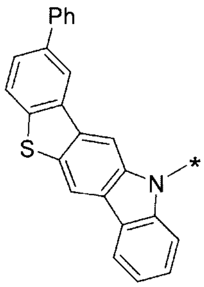

D263

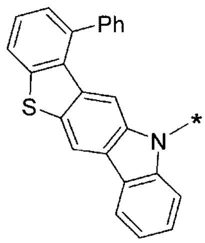

D264

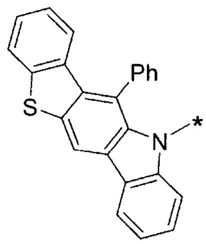

D265

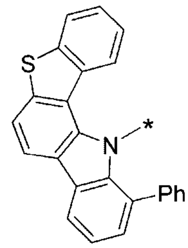

D266

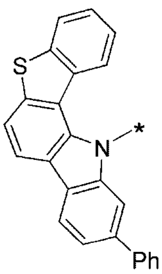

D267

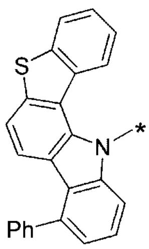

D268

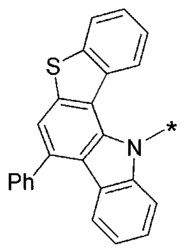

D269

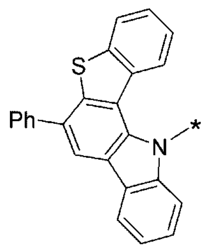

D270

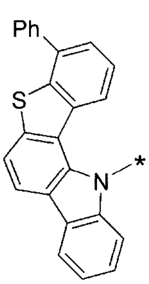
D271

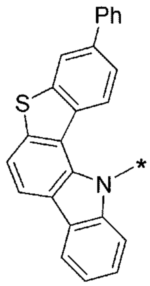
D272

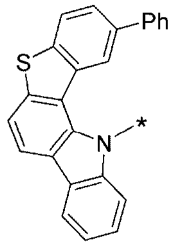
D273

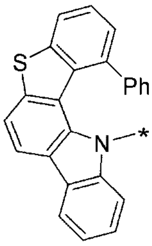
D274

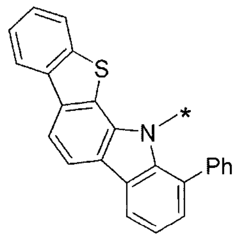
D275

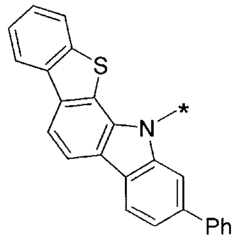
D276

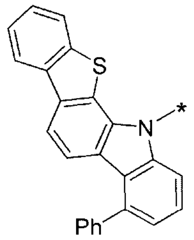
D277

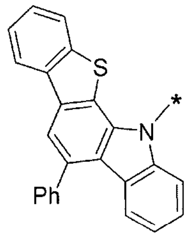
D278

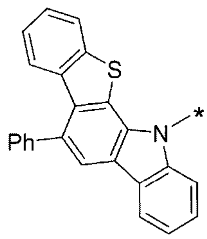
D279

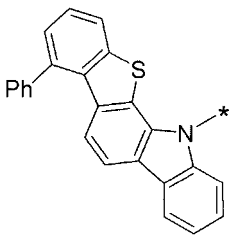
D280

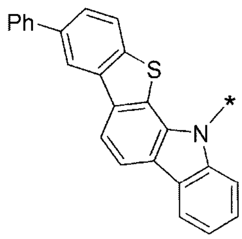
D281

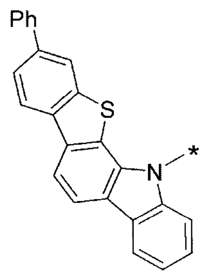
D282

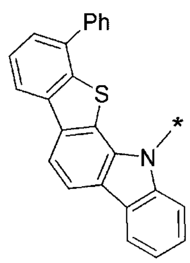
D283

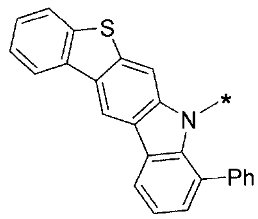
D284

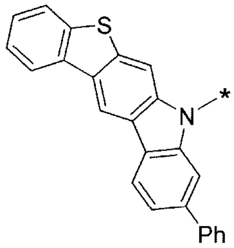
D285

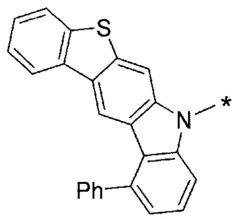
D286

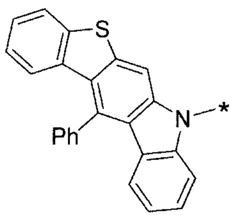
D287

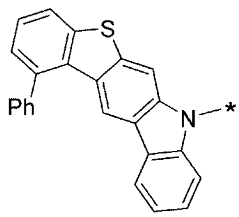
D288

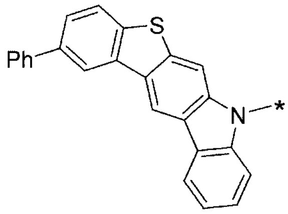
D289

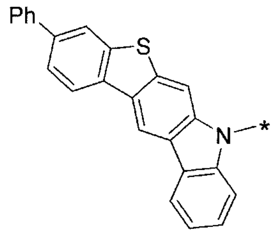
D290

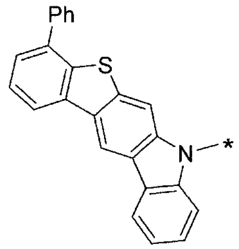
D291

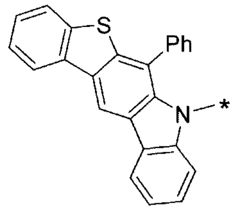
D292

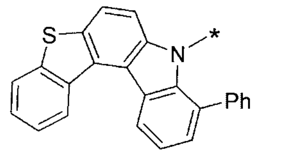
D293

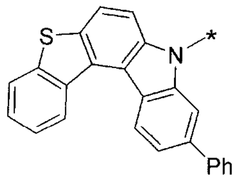
D294

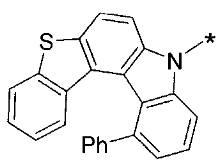
D295

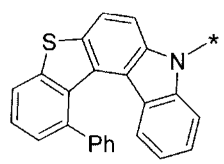
D296

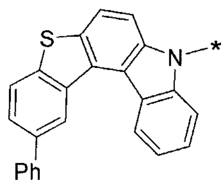
D297

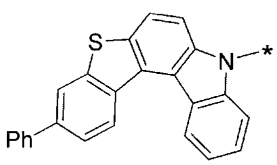
D298

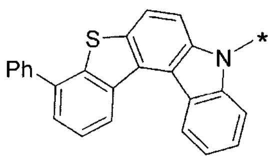
D299

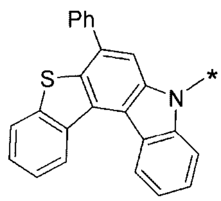
D300

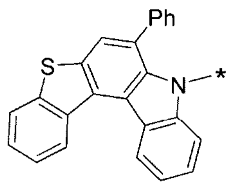
D301

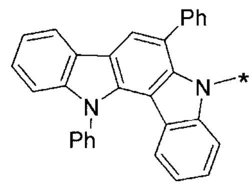
D302

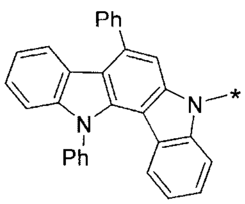
D303

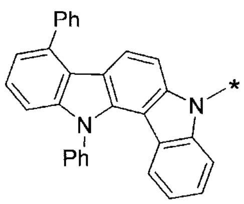
D304

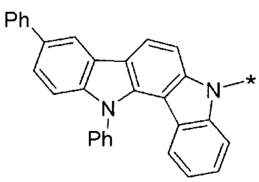
D305

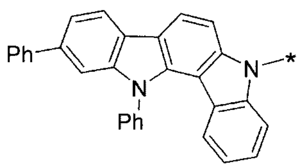
D306

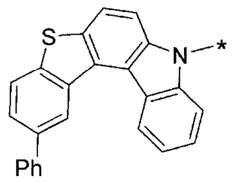

D307

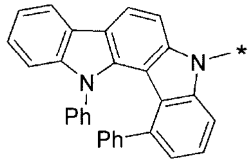

D308

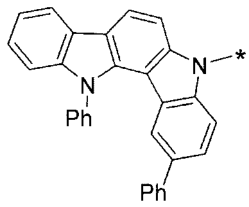

D309

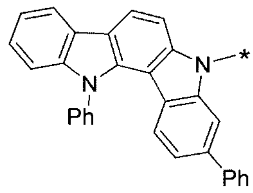

D310

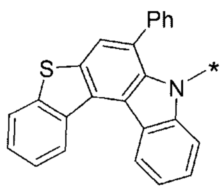

D311

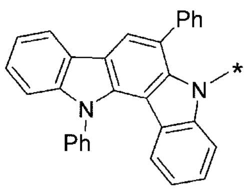

D312

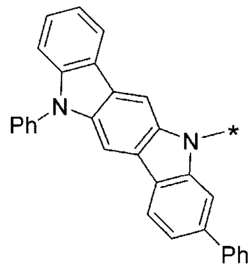

D313

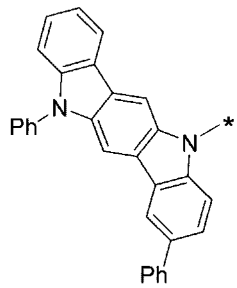

D314

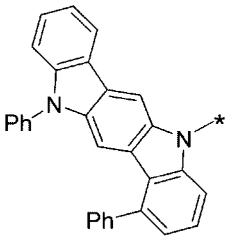

D315

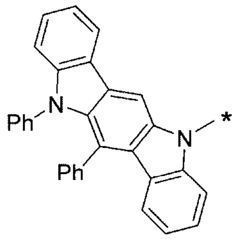

D316

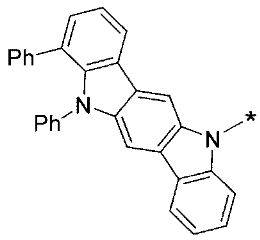

D317

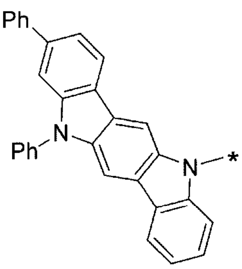

D318

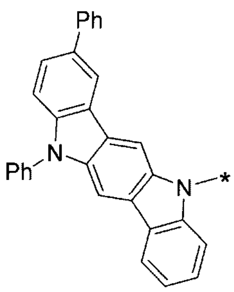

D319

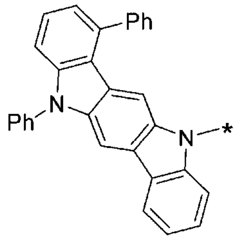

D320

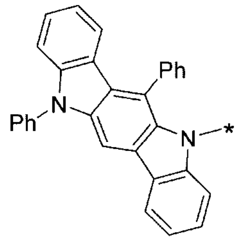

D321

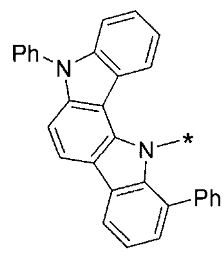

D322

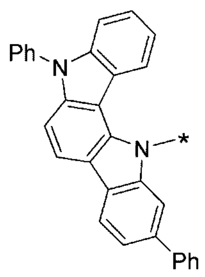
D323

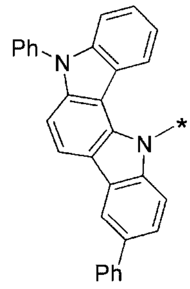
D324

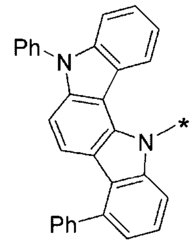
D325

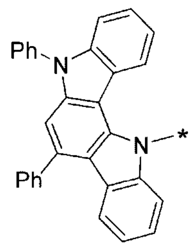
D326

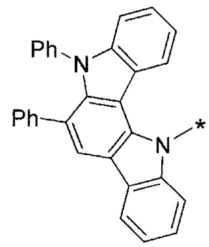
D327

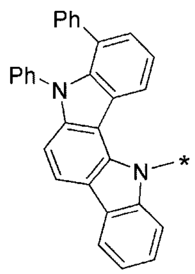
D328

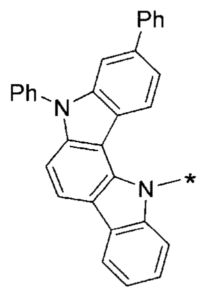
D329

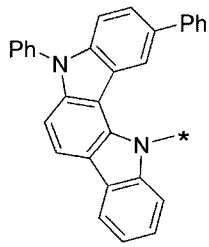
D330

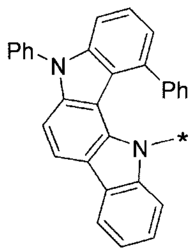
D331

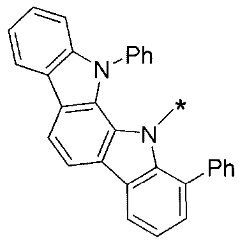
D332

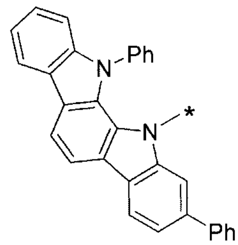
D333

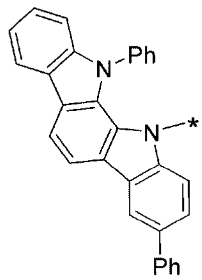
D334

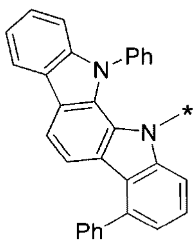
D335

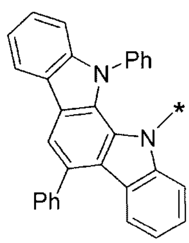
D336

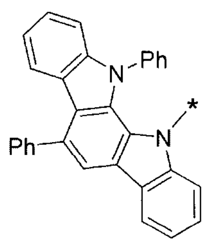
D337

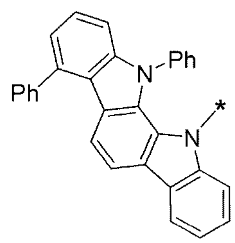
D338

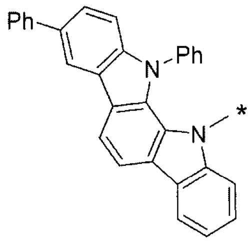

D339

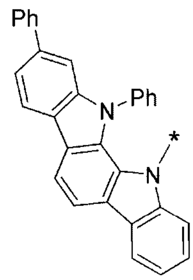

D340

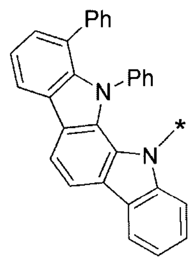

D341

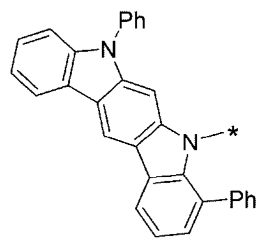

D342

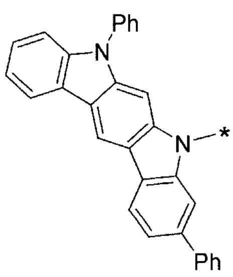

D343

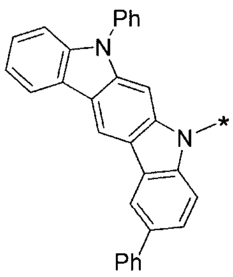

D344

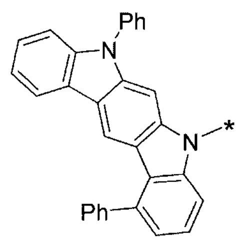

D345

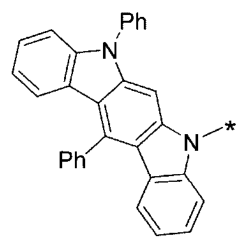

D346

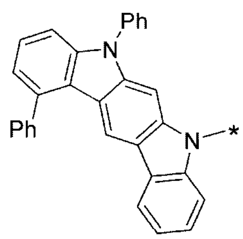

D347

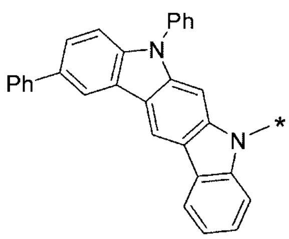

D348

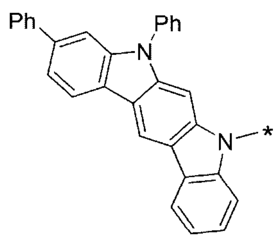

D349

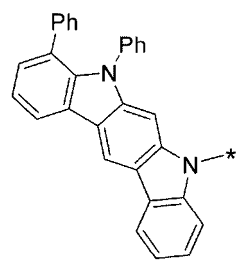

D350

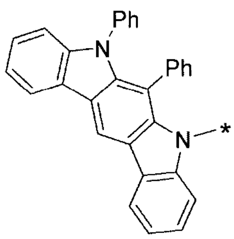

D351

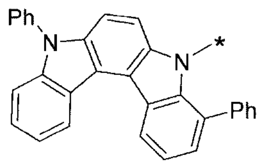

D352

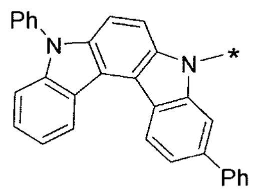

D353

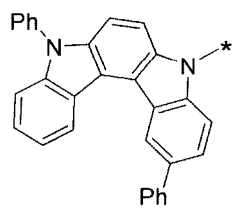

D354

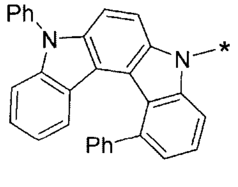

D355

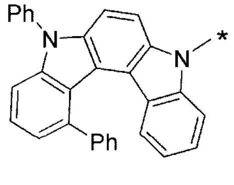

D356

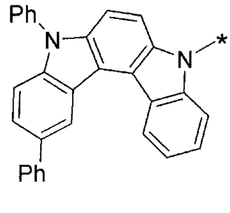

D357

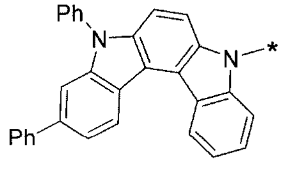

D358

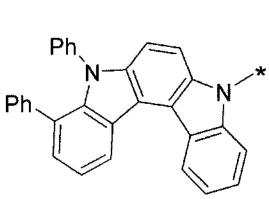 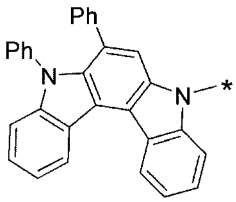 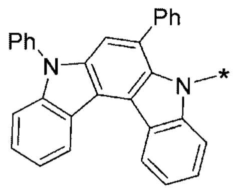 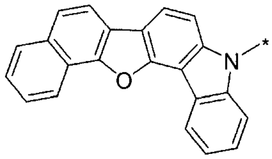

D359 D360 D361 D362

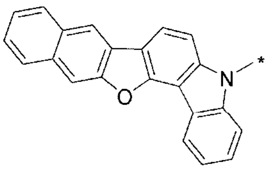 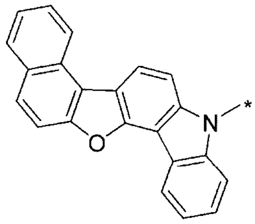 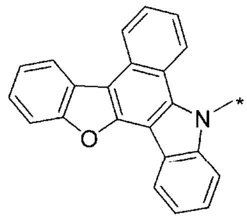 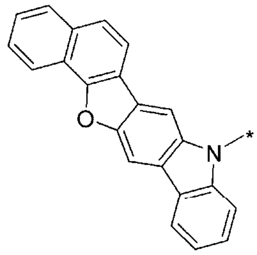

D363 D364 D365 D366

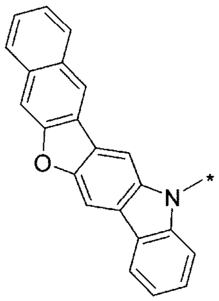 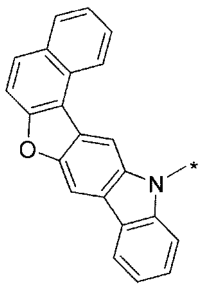 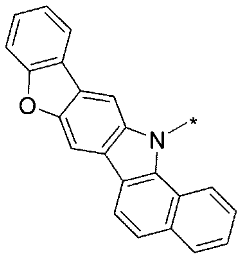 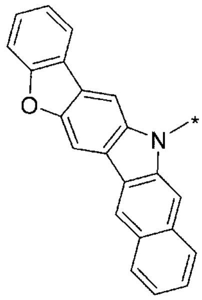 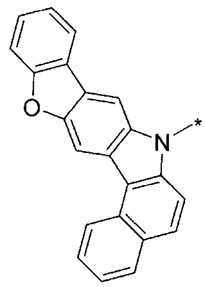

D367 D368 D369 D370 D371

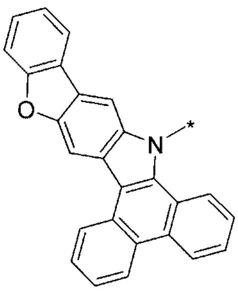 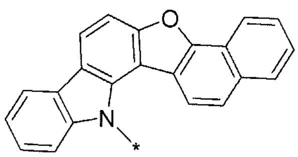 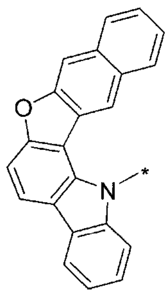 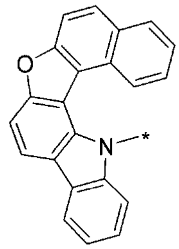

D372 D373 D374 D375

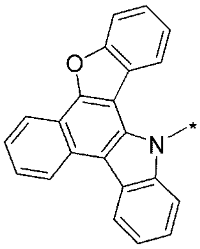
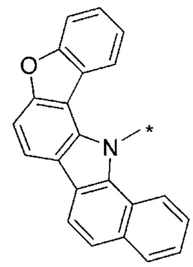
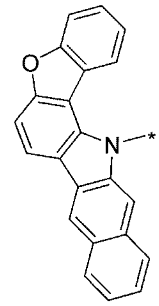
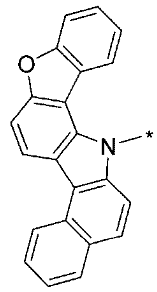

D376 D377 D378 D379

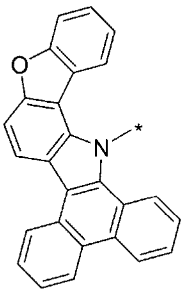
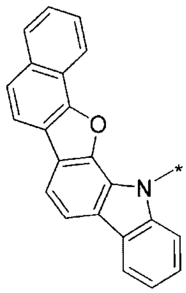
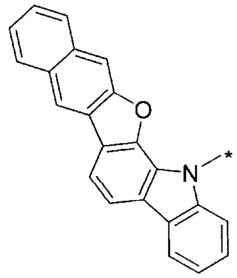
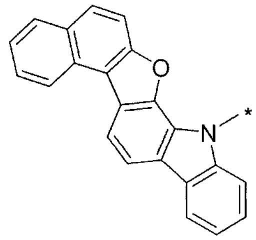

D380 D381 D382 D383

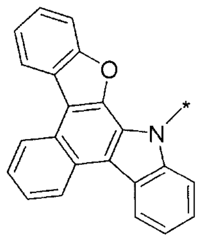
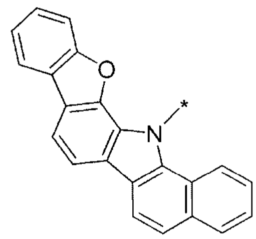
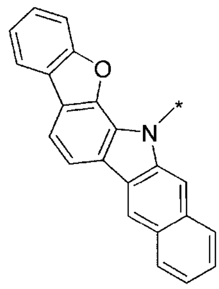
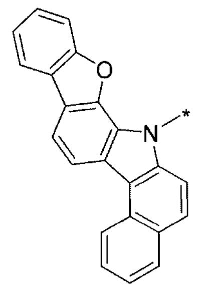

D384 D385 D386 D387

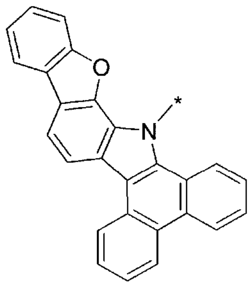
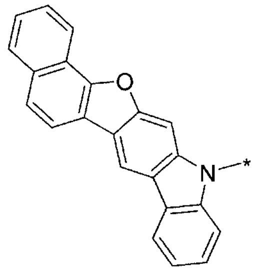
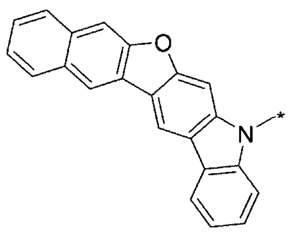
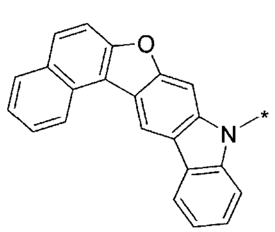

D388 D389 D390 D391

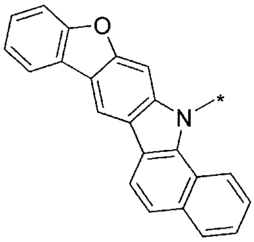
D392

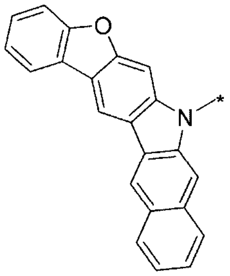
D393

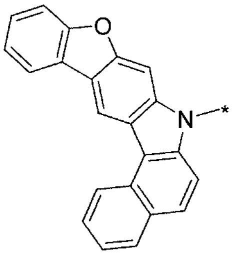
D394

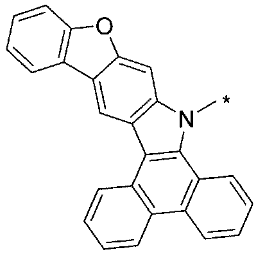
D395

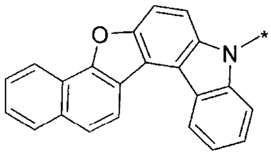
D396

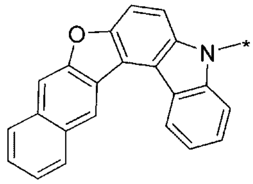
D397

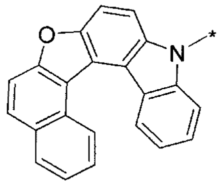
D398

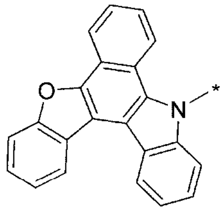
D399

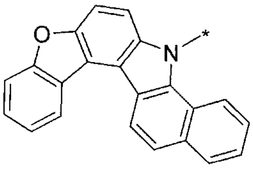
D400

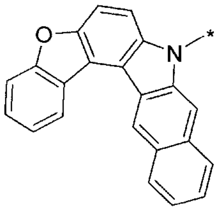
D401

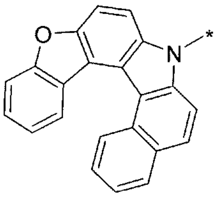
D402

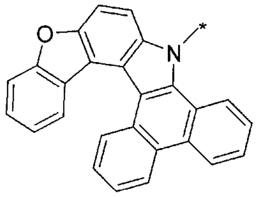
D403

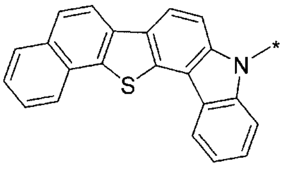
D404

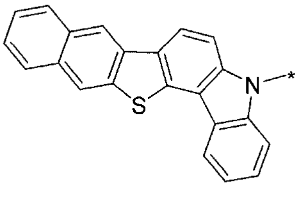
D405

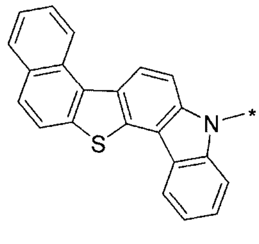
D406

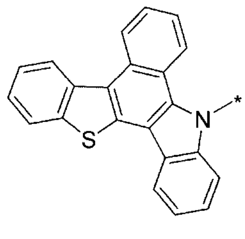
D407

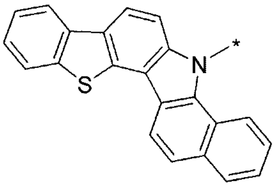
D408

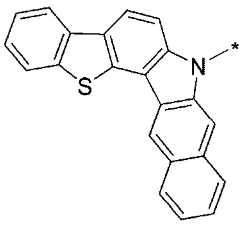
D409

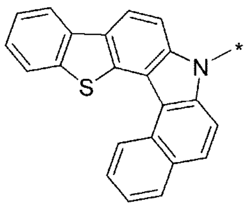
D410

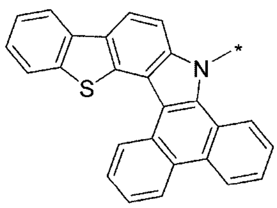
D411

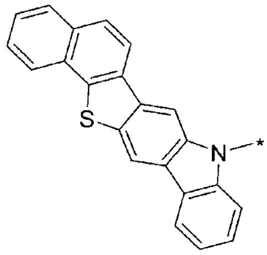 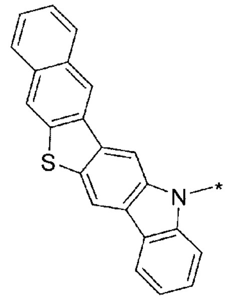 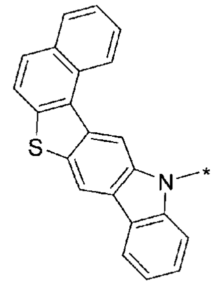 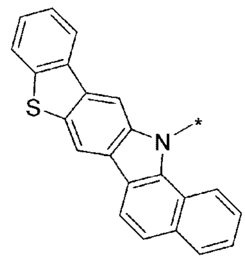

D412 D413 D414 D415

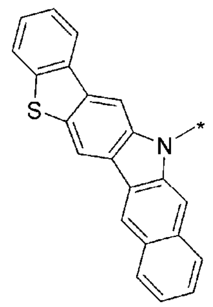 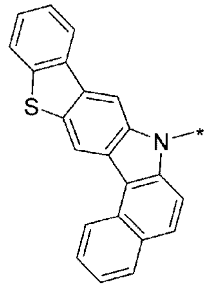 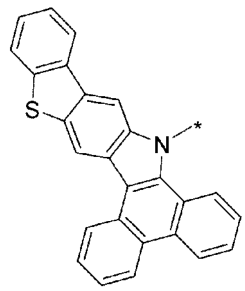 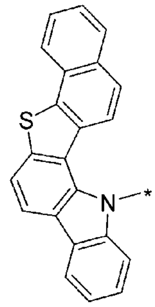

D416 D417 D418 D419

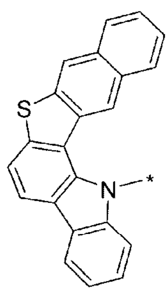 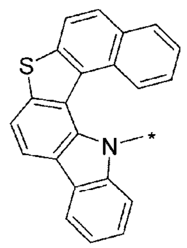 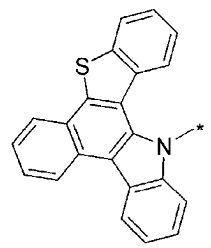 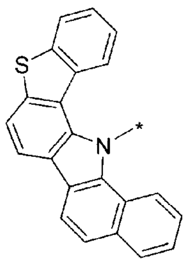

D420 D421 D422 D423

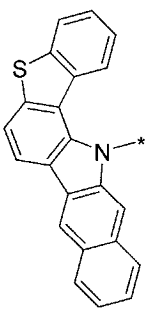 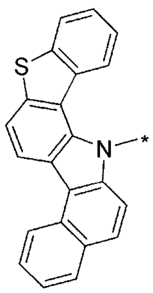 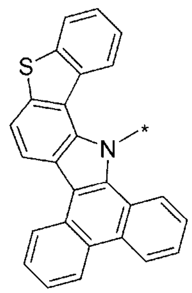 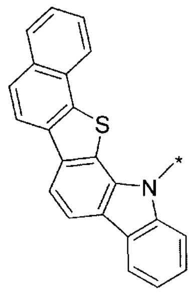

D424 D425 D426 D427

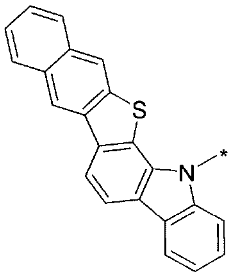

D428

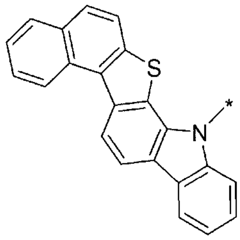

D429

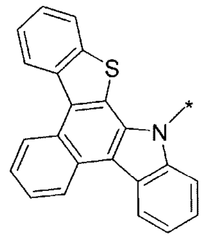

D430

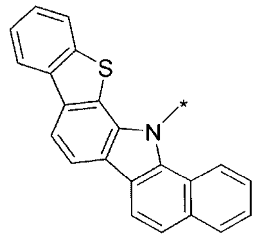

D431

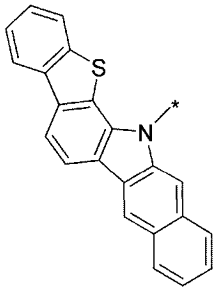

D432

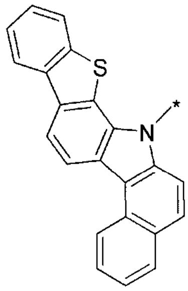

D433

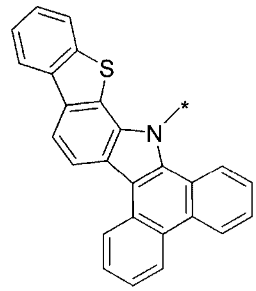

D434

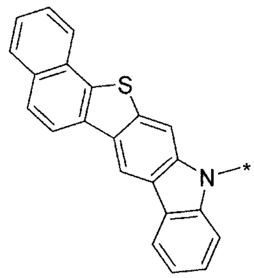

D435

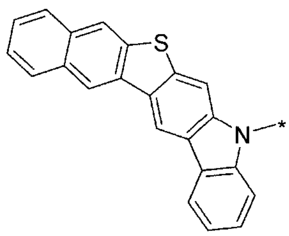

D436

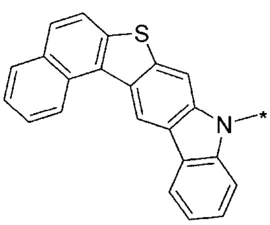

D437

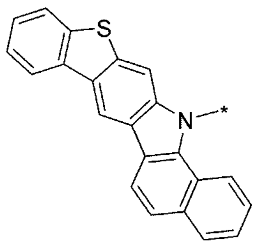

D438

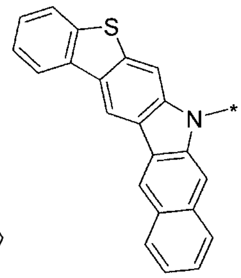

D439

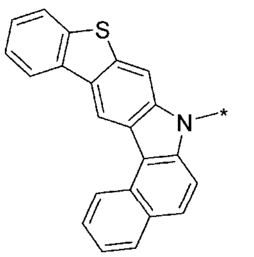

D440

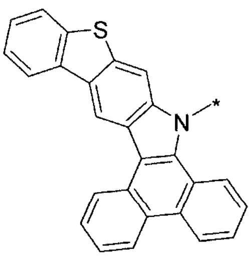

D441

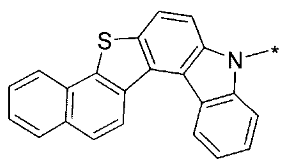

D442

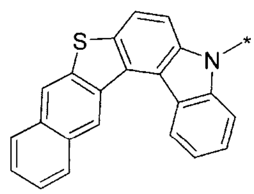

D443

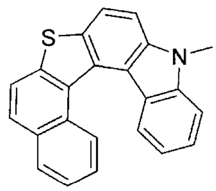

D444

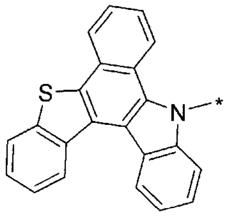

D445

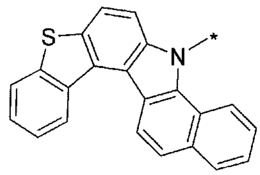

D446

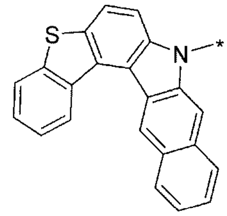

D447

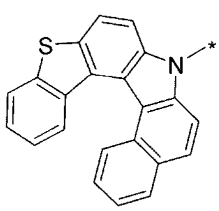

D448

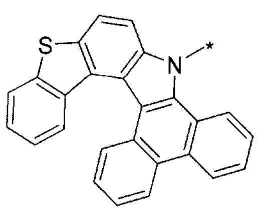

D449

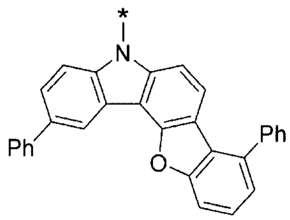

D450

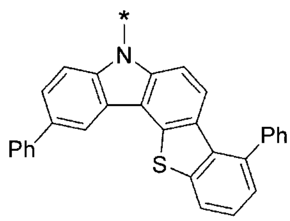

D451

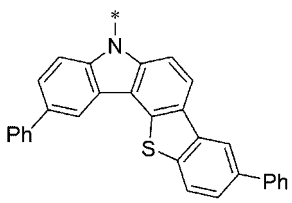

D452

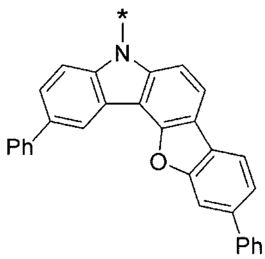

D453

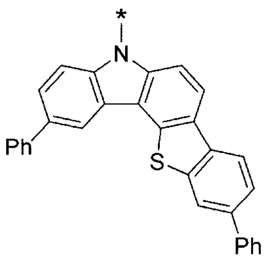

D454

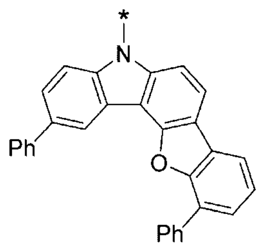

D455

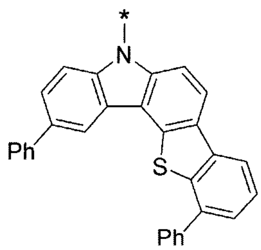

D456

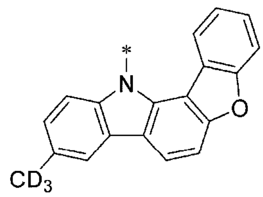

D457

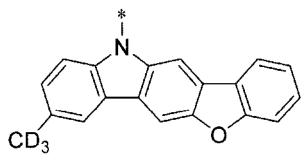

D458

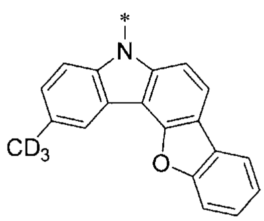

D459

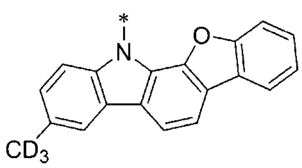

D460

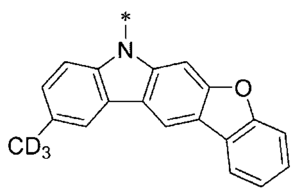

D461

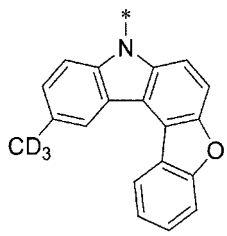

D462

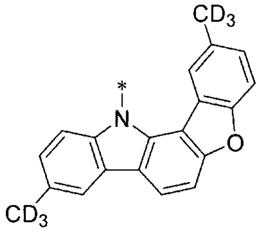
D463

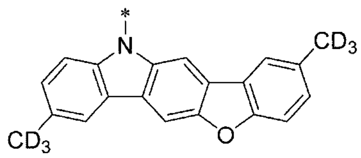
D464

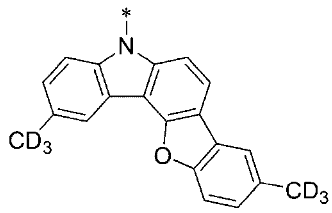
D465

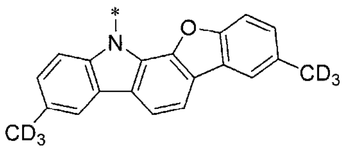
D466

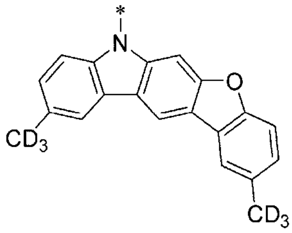
D467

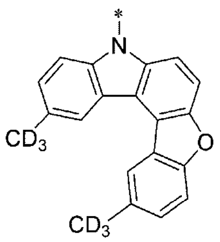
D468

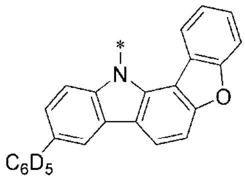
D469

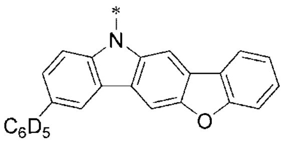
D470

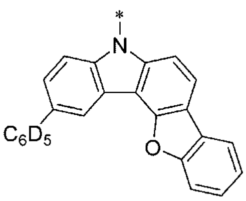
D471

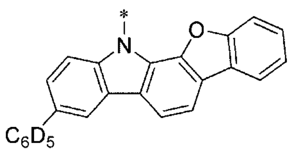
D472

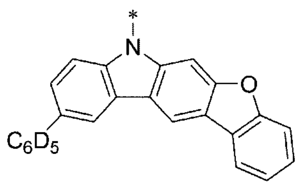
D473

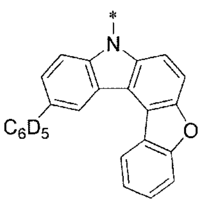
D474

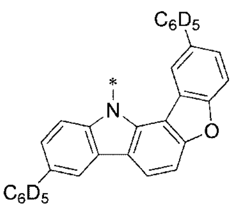
D475

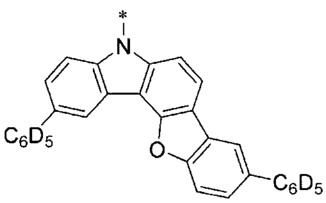
D476

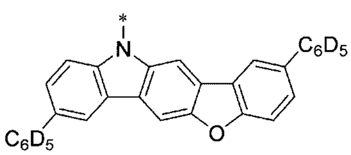
D477

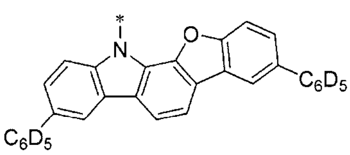
D478

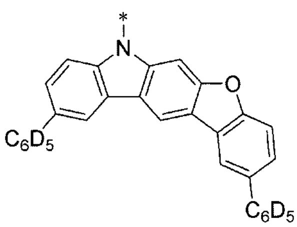
D479

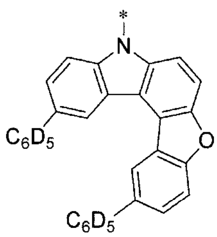
D480

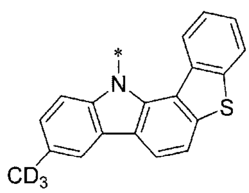
D481

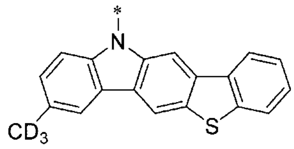 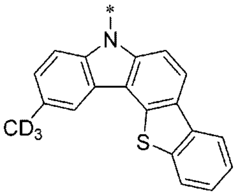 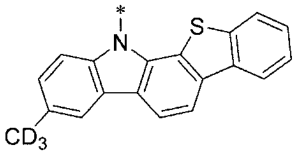 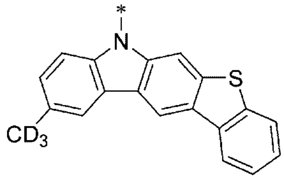

D482 D483 D484 D485

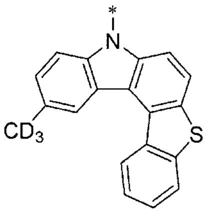 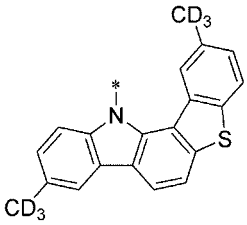 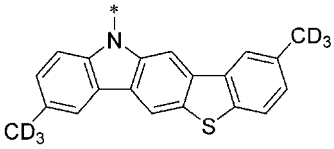 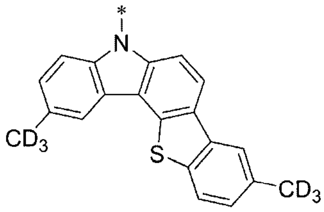

D486 D487 D488 D489

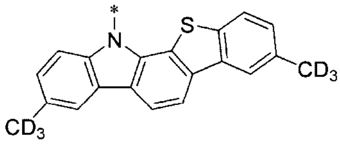 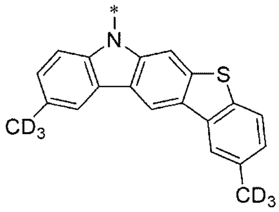 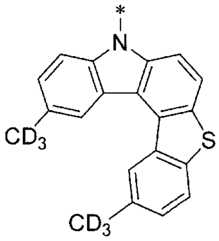 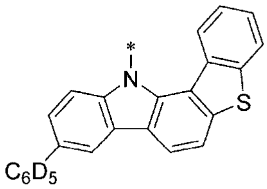

D490 D491 D492 D493

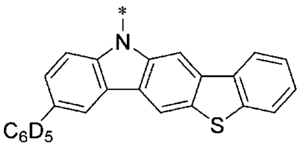 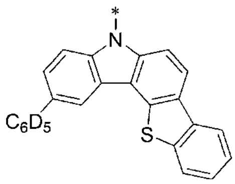 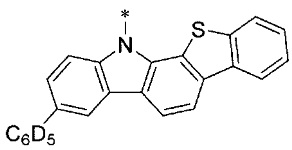 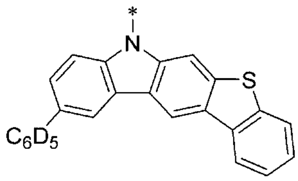

D494 D495 D496 D497

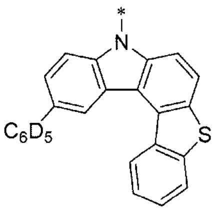 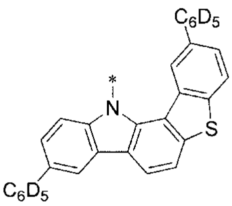 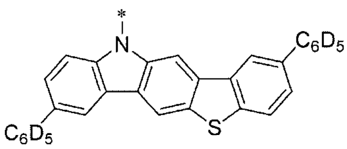 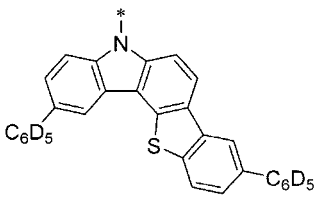

D498 D499 D500 D501

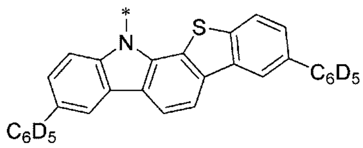

D502

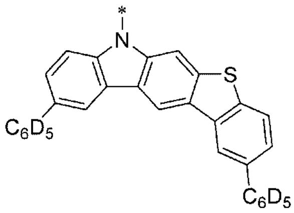

D503

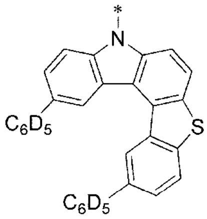

D504

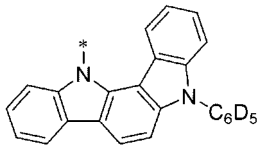

D505

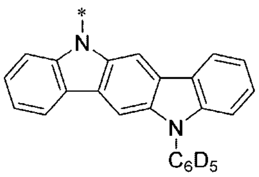

D506

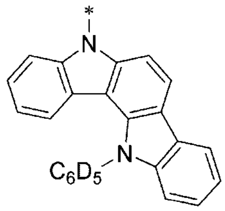

D507

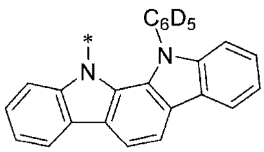

D508

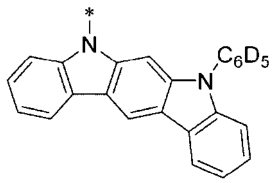

D509

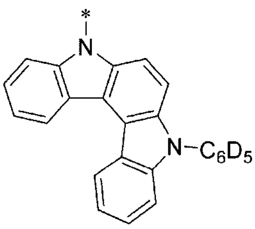

D510

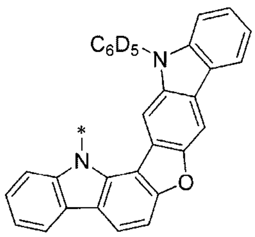

D511

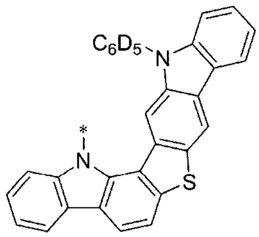

D512

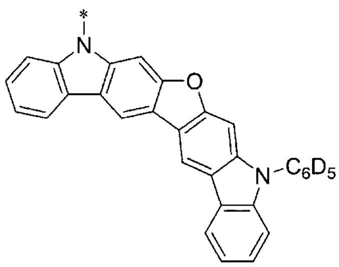

D513

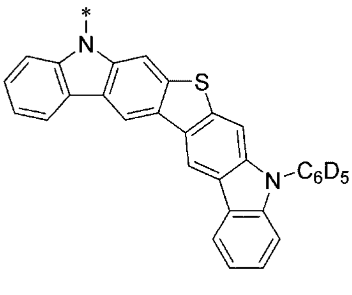

D514

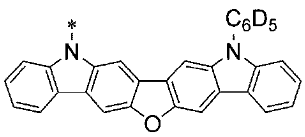

D515

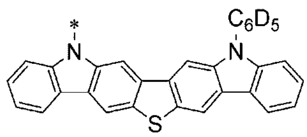

D516

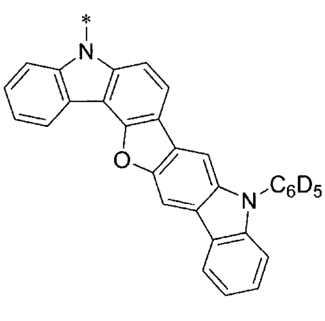

D517

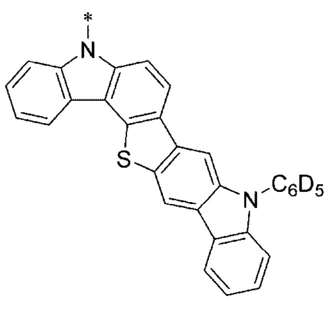

D518

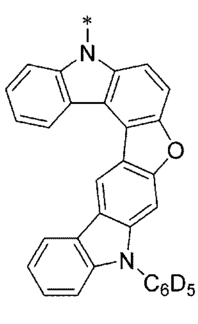

D519

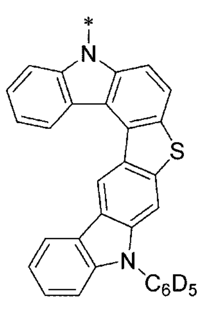

D520

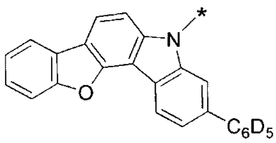 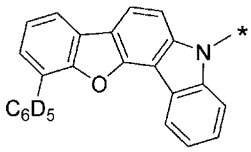 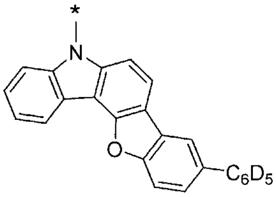 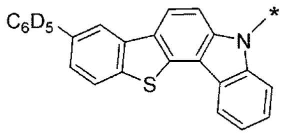

D521 D522 D523 D524

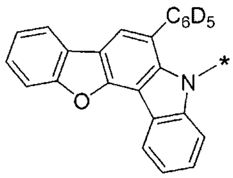 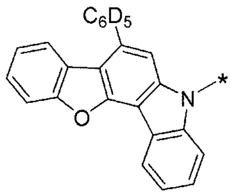 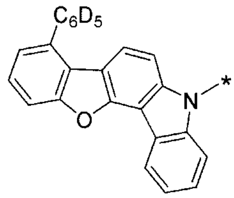 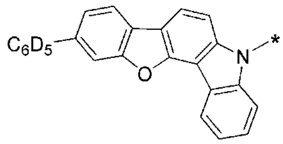

D525 D526 D527 D528

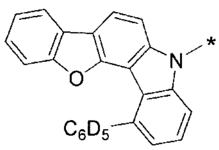 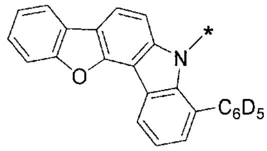 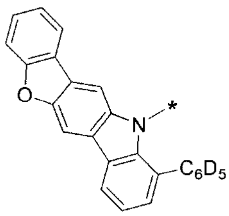 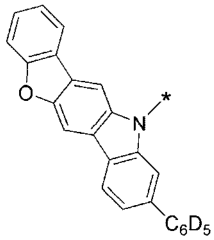 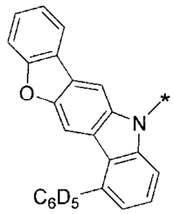

D529 D530 D531 D532 D533

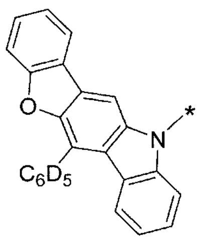 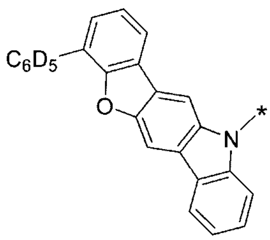 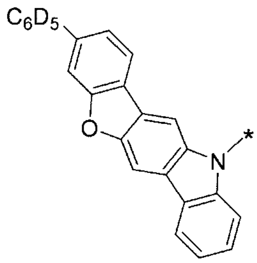 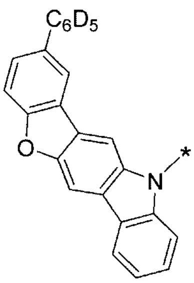

D534 D535 D536 D537

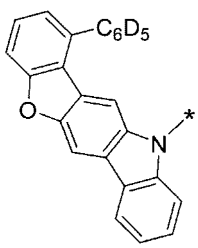 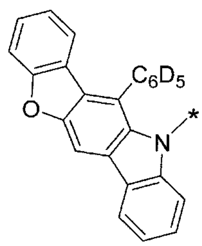 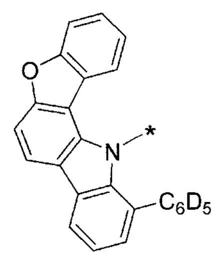 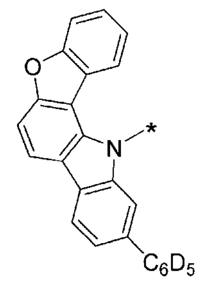

D538 D539 D540 D541

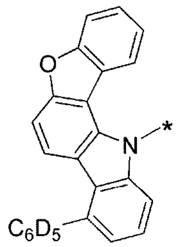
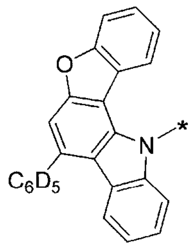
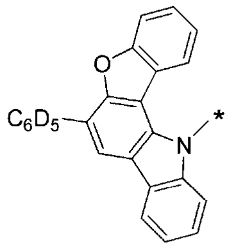
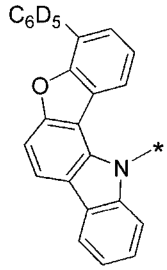

D542 D543 D544 D545

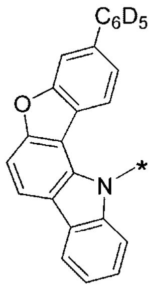
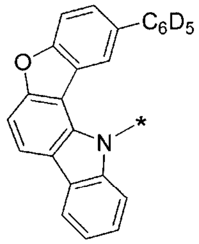
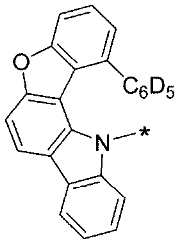
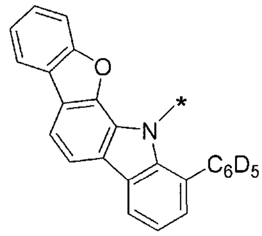

D546 D547 D548 D549

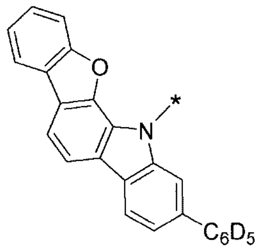
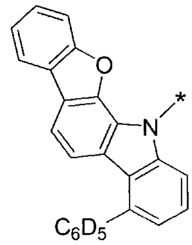
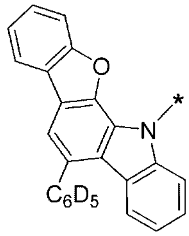
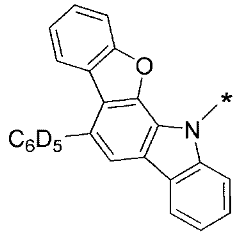

D550 D551 D552 D553

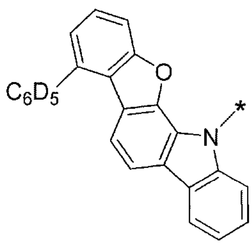
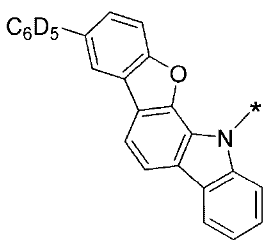
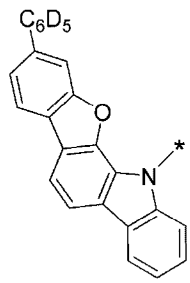
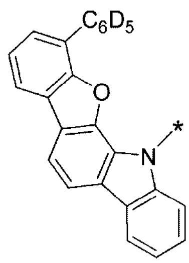

D554 D555 D556 D557

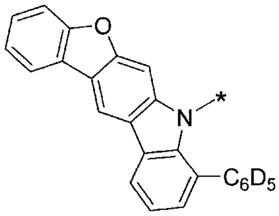

D558

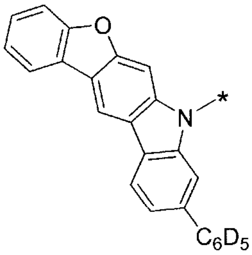

D559

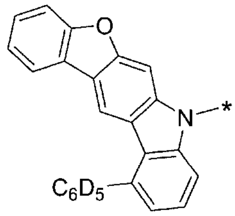

D560

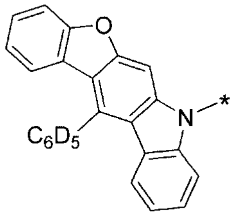

D561

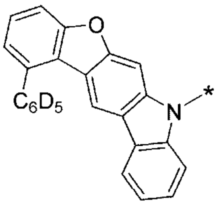

D562

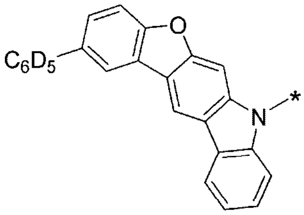

D563

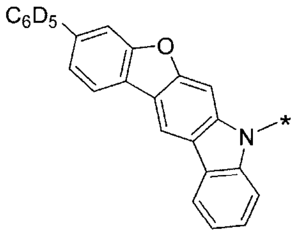

D564

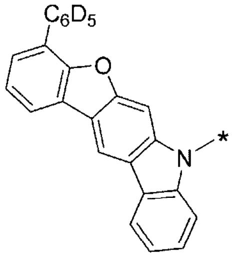

D565

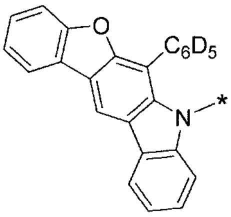

D566

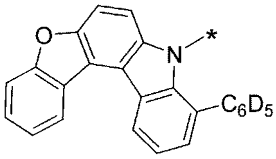

D567

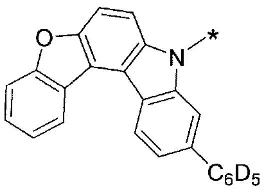

D568

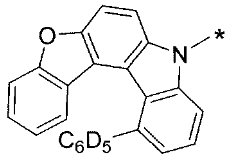

D569

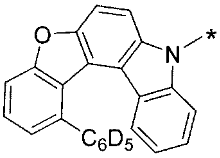

D570

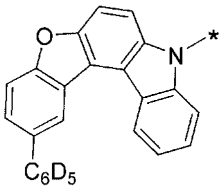

D571

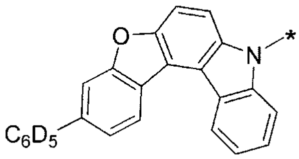

D572

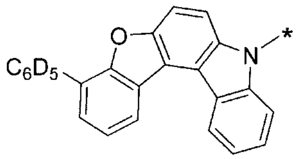

D573

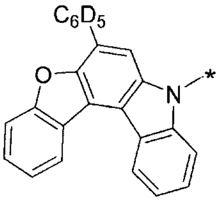

D574

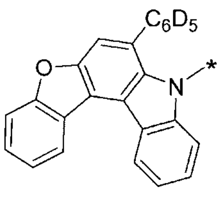

D575

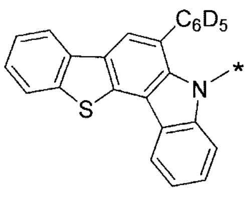

D576

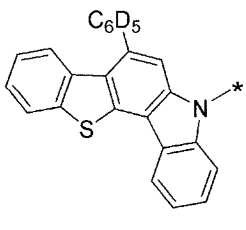

D577

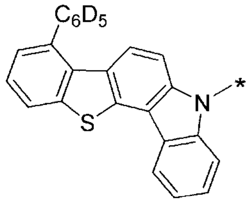 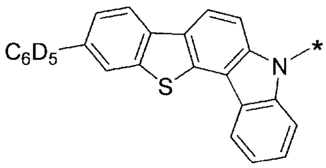 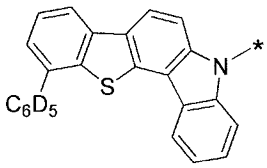 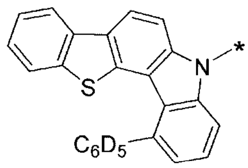

D578 D579 D580 D581

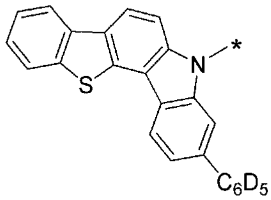 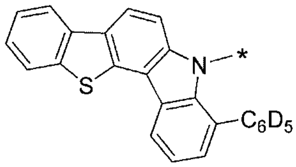 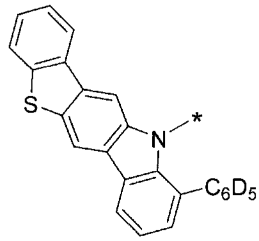 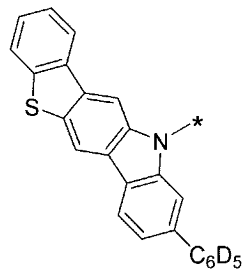

D582 D583 D584 D585

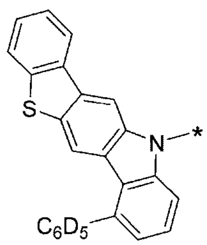 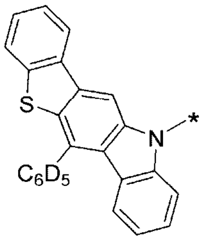 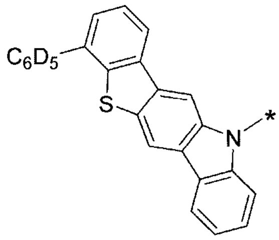 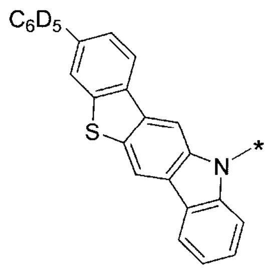

D586 D587 D588 D589

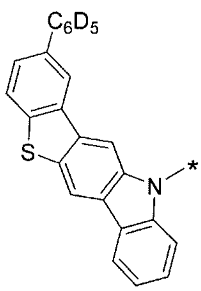 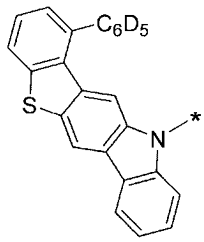 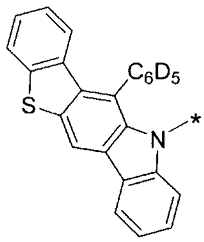 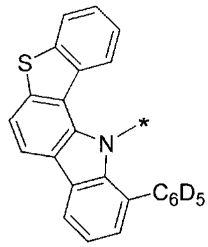

D590 D591 D592 D593

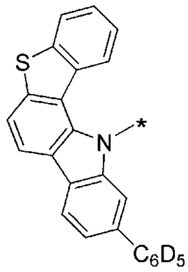
D594

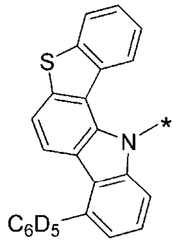
D595

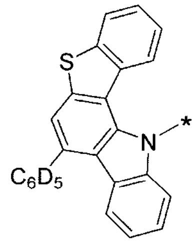
D596

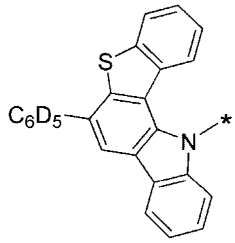
D597

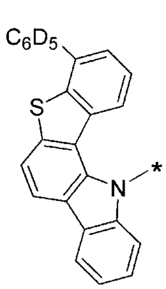
D598

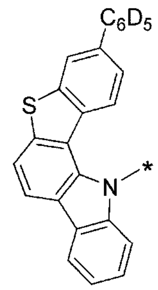
D599

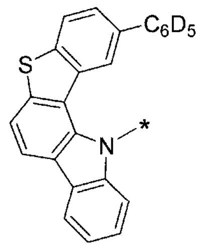
D600

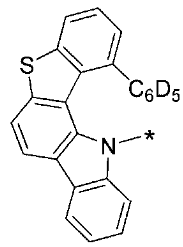
D601

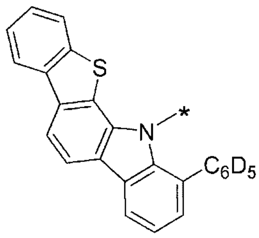
D602

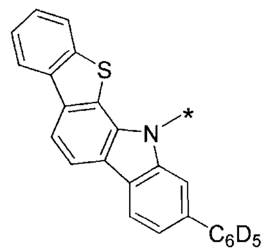
D603

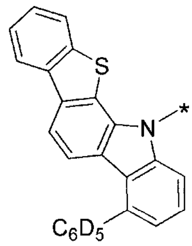
D604

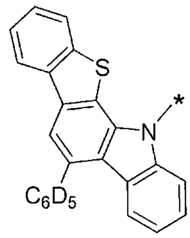
D605

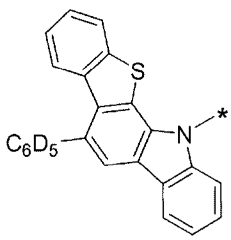
D606

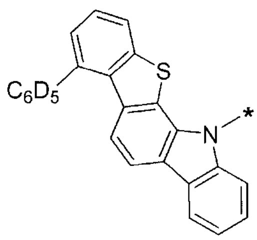
D607

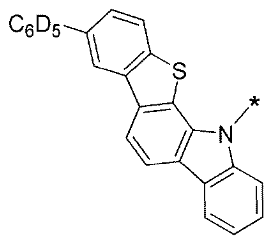
D608

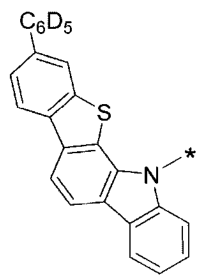
D609

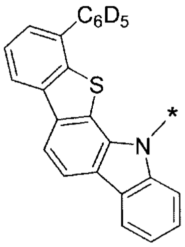
D610

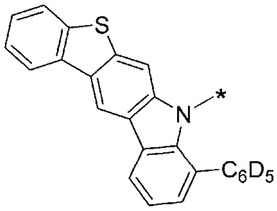
D611

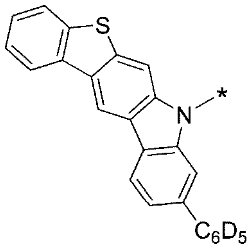
D612

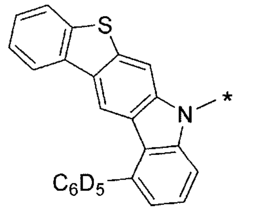
D613

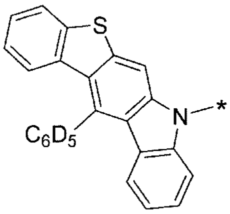
D614

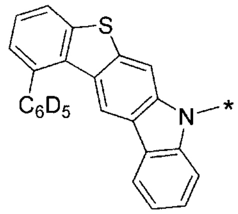
D615

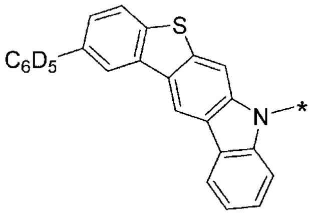
D616

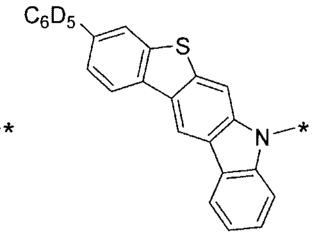
D617

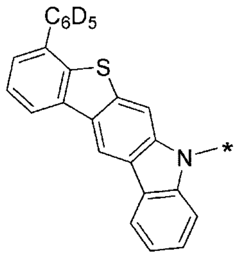
D618

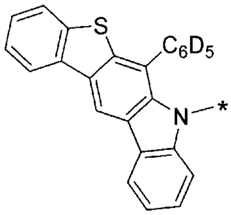
D619

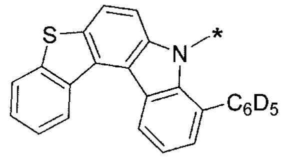
D620

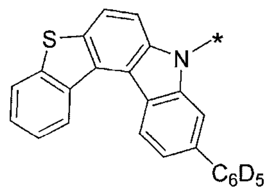
D621

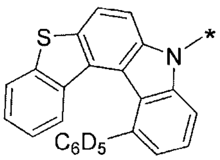
D622

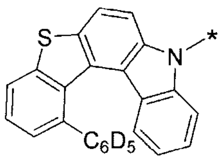
D623

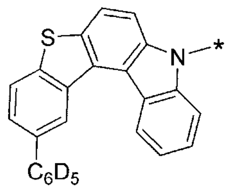
D624

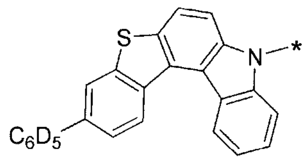
D625

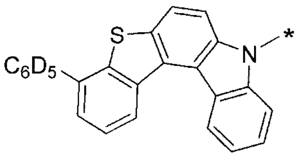
D626

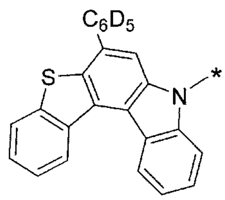
D627

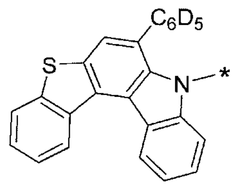
D628

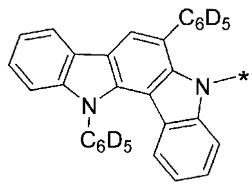
D629

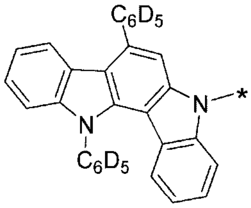 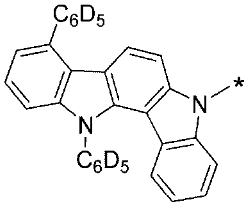 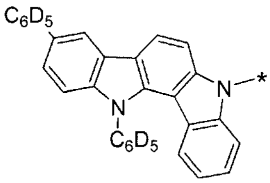 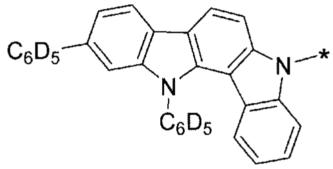

D630 D631 D632 D633

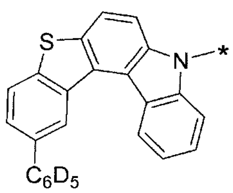 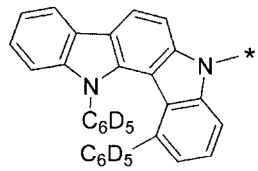 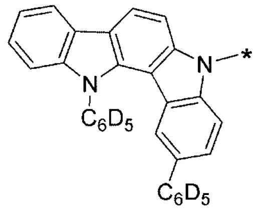 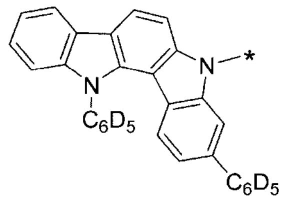

D634 D635 D636 D637

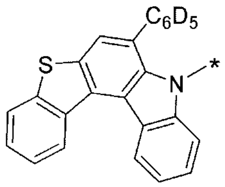 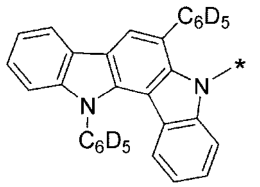 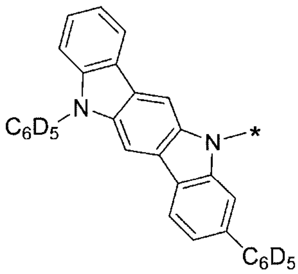 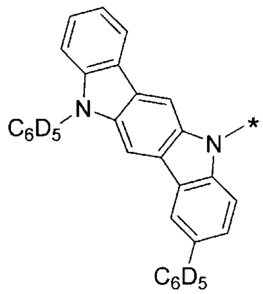

D638 D639 D640 D641

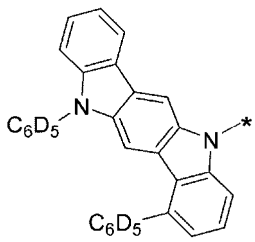 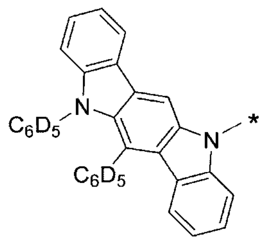 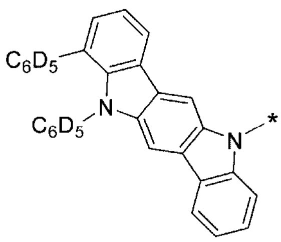 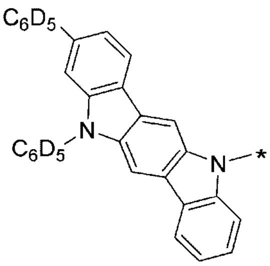

D642 D643 D644 D645

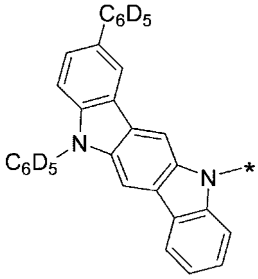 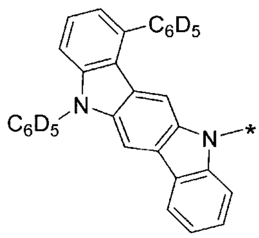 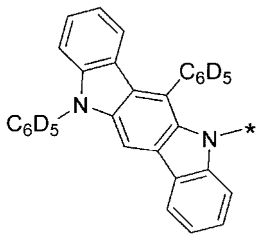 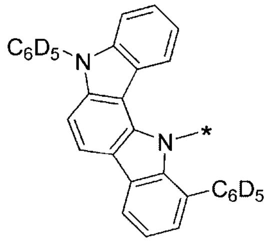

D646 D647 D648 D649

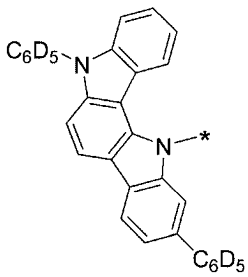

D650

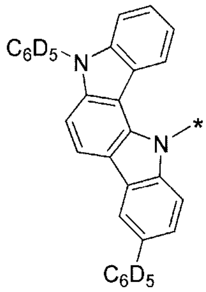

D651

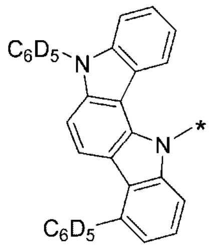

D652

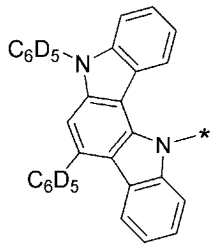

D653

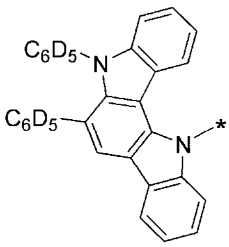

D654

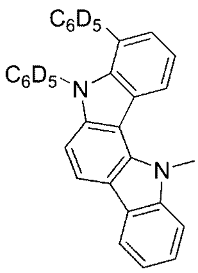

D655

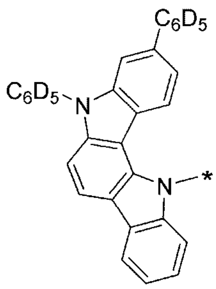

D656

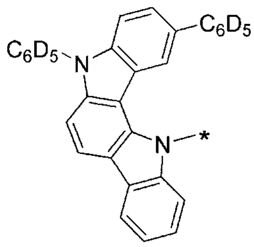

D657

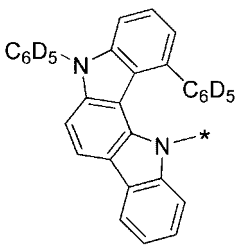

D658

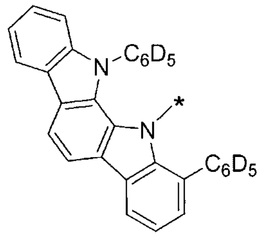

D659

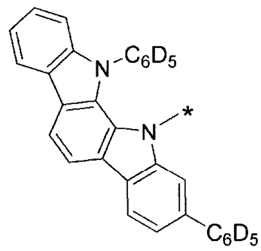

D660

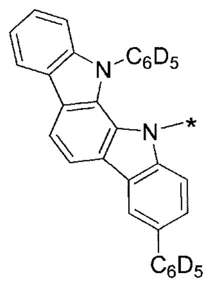

D661

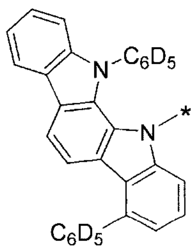

D662

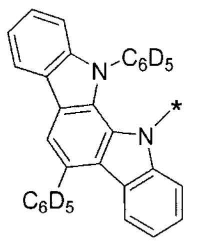

D663

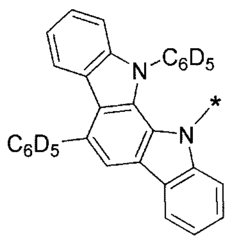

D664

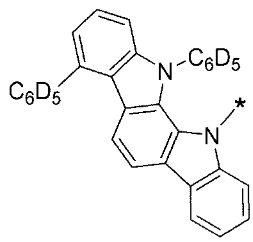

D665

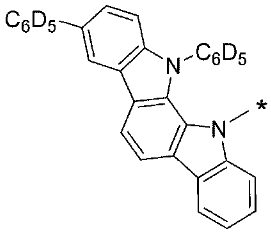

D666

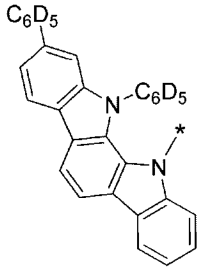

D667

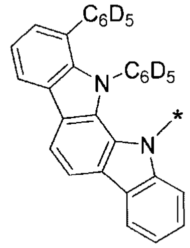

D668

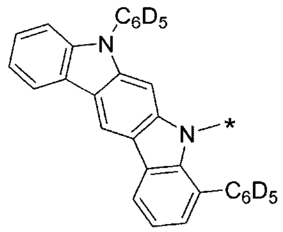

D669

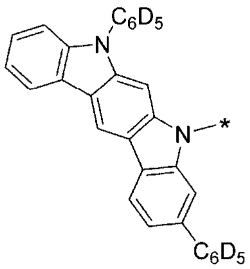

D670

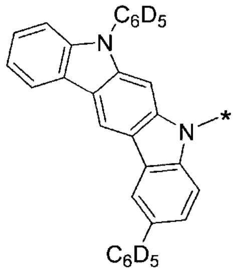

D671

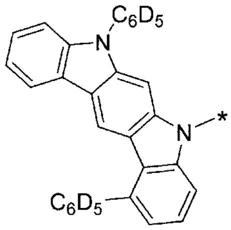

D672

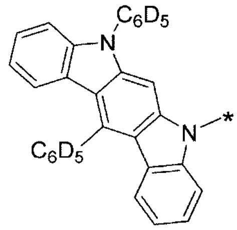

D673

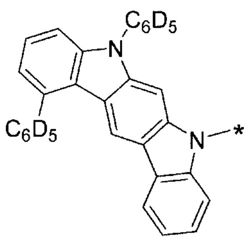

D674

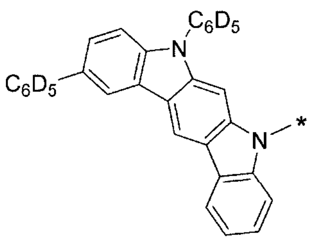

D675

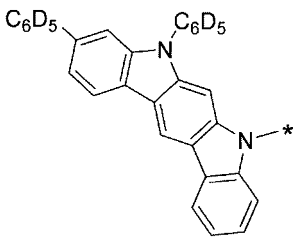

D676

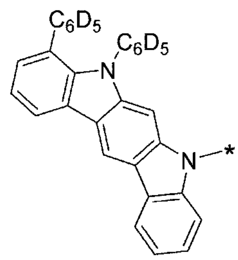

D677

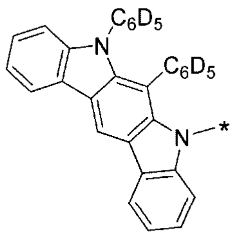

D678

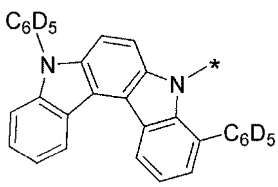

D679

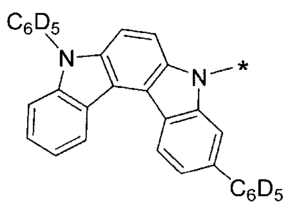

D680

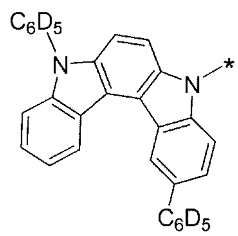

D681

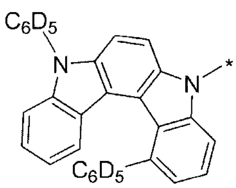

D682

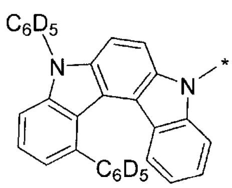

D683

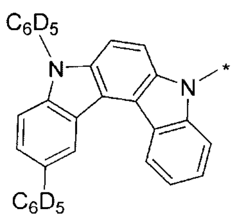

D684

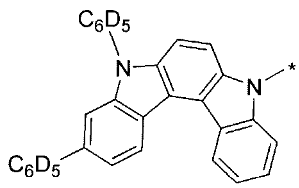

D685

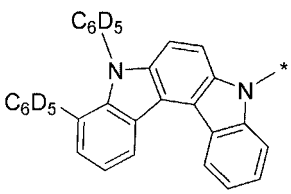
D686

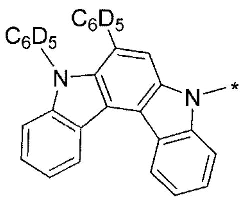
D687

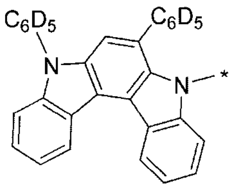
D688

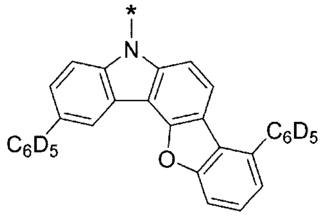
D689

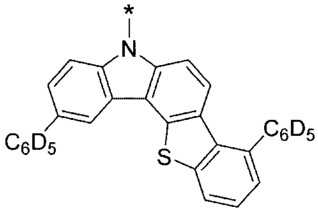
D690

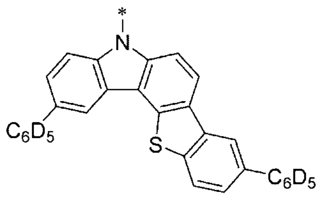
D691

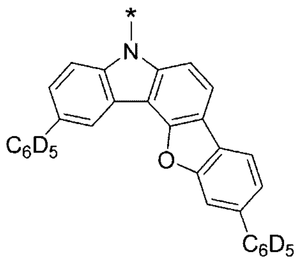
D692

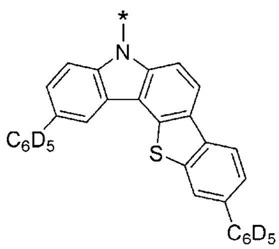
D693

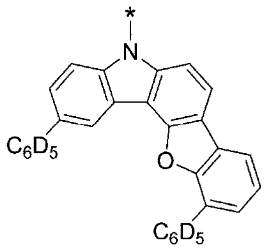
D694

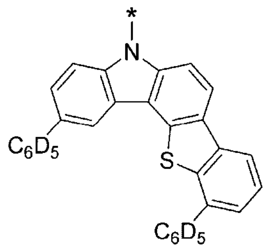
D695

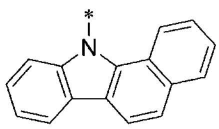
D696

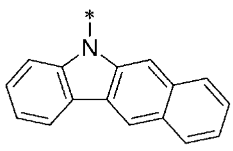
D697

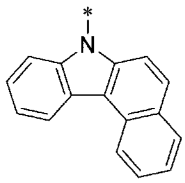
D698

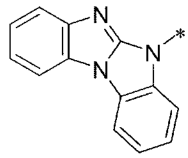
D699

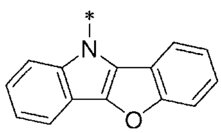
D700

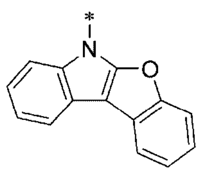
D701

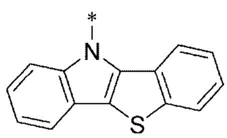
D702

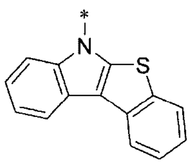
D703

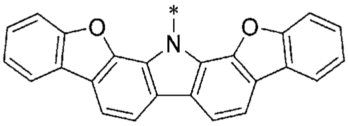

D704

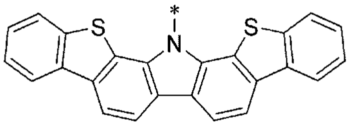

D705

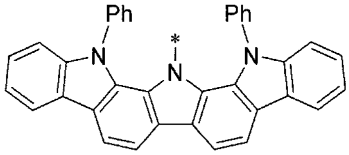

D706

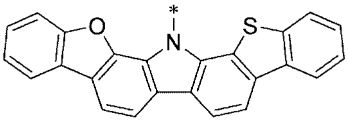

D707

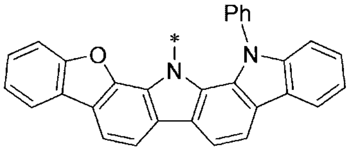

D708

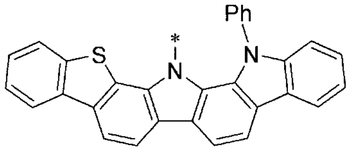

D709

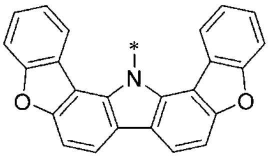

D710

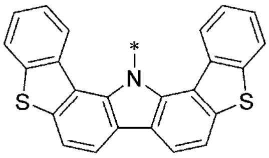

D711

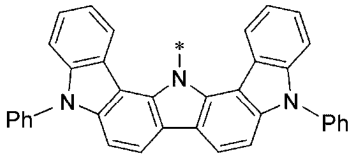

D712

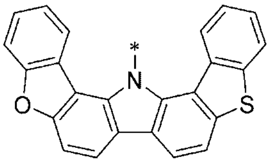

D713

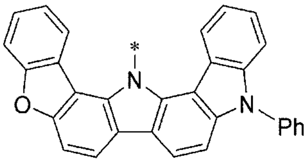

D714

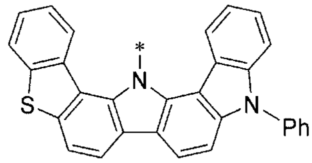

D715

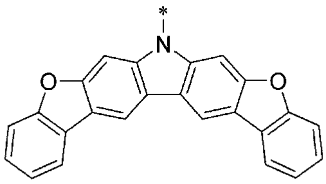

D716

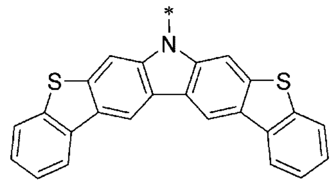

D717

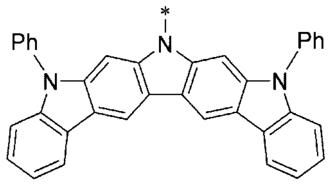

D718

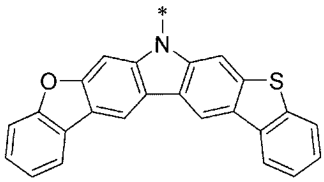

D719

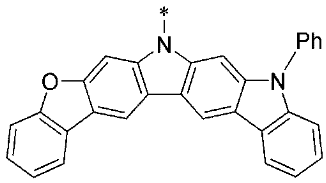

D720

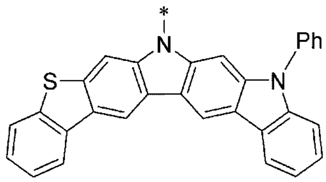

D721

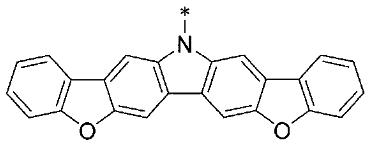
D722

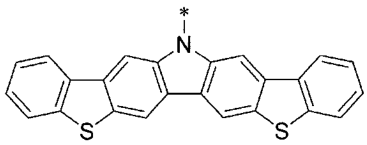
D723

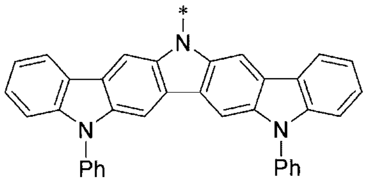
D724

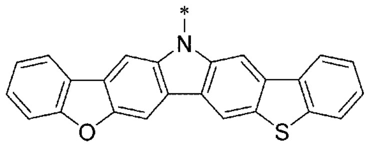
D725

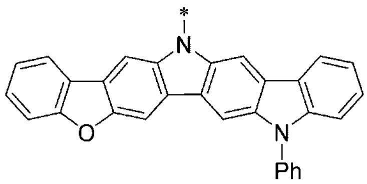
D726

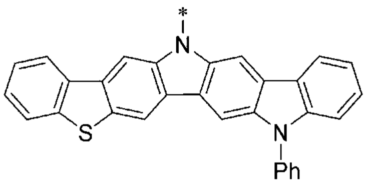
D727

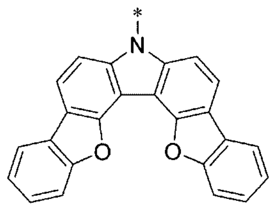
D728

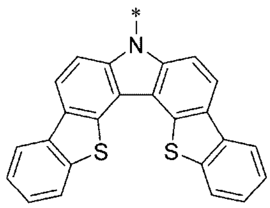
D729

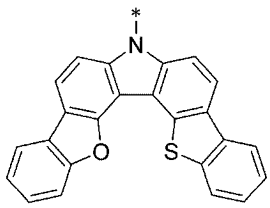
D730

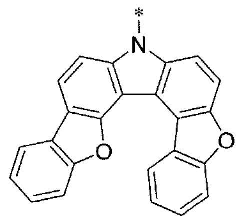
D731

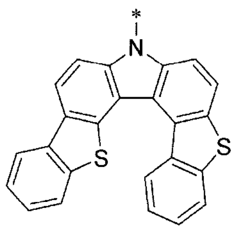
D732

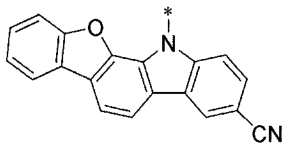
D733

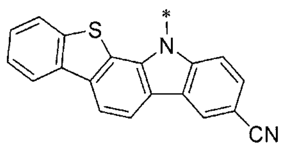
D734

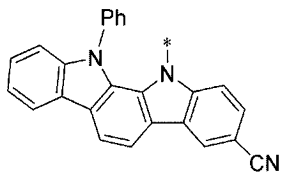
D735

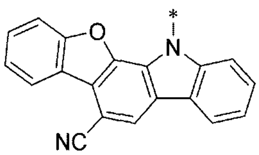
D736

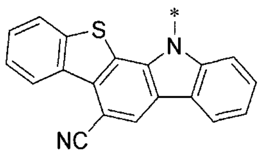
D737

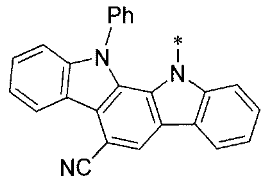
D738

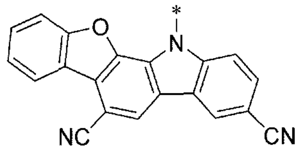
D739

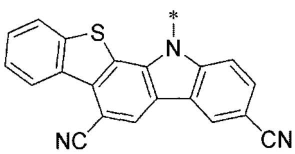
D740

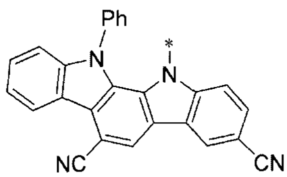
D741

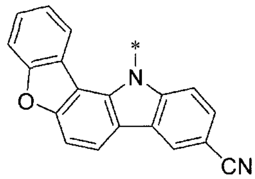
D742

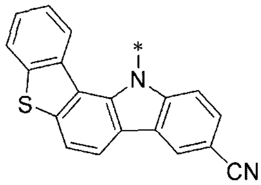
D743

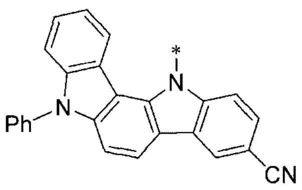
D744

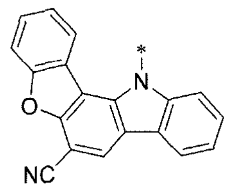
D745

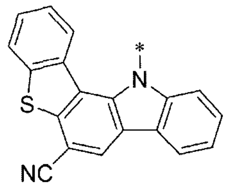
D746

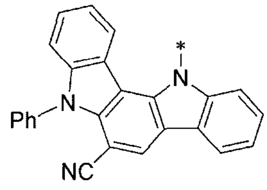
D747

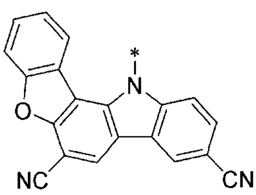
D748

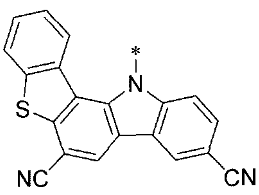
D749

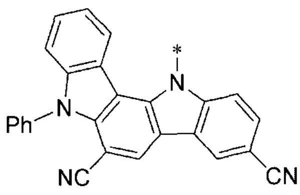
D750

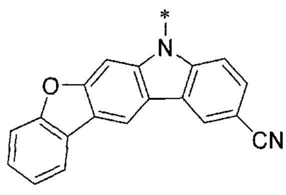
D751

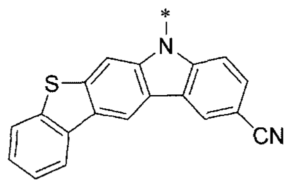
D752

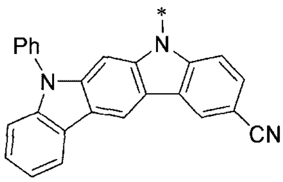
D753

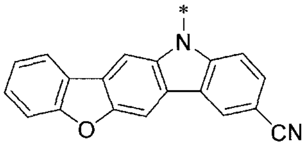
D754

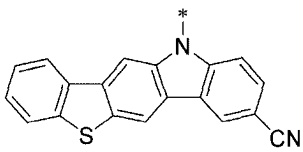
D755

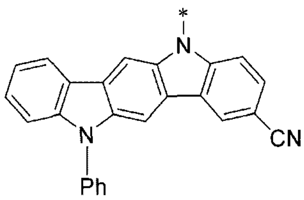
D756

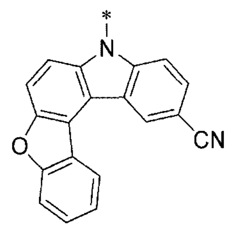
D757

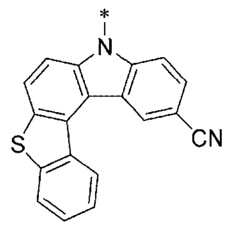
D758

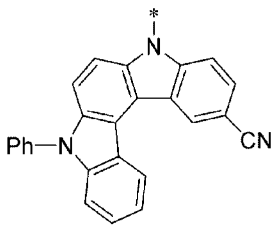
D759

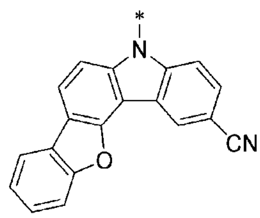
D760

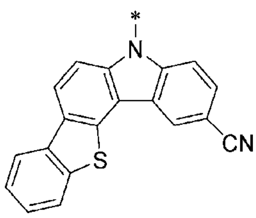
D761

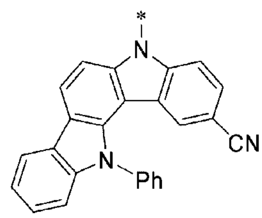
D762

Groups obtained by substituting all hydrogen atoms present in the above D86 to D456 and D696 to D762 with deuterium atoms are disclosed as D763 to D1210.

In one aspect of the present invention, the donor group that $R^1$ to $R^5$ can adopt is selected from the group consisting of D1 to D1210. In one aspect of the present invention, the donor group that $R^1$ to $R^5$ can adopt is selected from the group consisting of D1 to D85. In one aspect of the present invention, the donor group that $R^1$ to $R^5$ can adopt is selected from the group consisting of D86 to D1210. In one aspect of the present invention, the donor group that $R^1$ to $R^5$ can adopt is selected from the group consisting of D86 to D695, D704 to D1133, and D1142 to D1210. In one aspect of the present invention, the donor group that $R^1$ to $R^5$ can adopt is selected from the group consisting of D696 to D703, and D1134 to D1141.

Two or more of $R^1$ to $R^5$ in the general formula (1) are donor groups. In one aspect of the present invention, two of three of $R^1$ to $R^5$ are donor groups. In one preferred aspect of the present invention, two of $R^1$ to $R^5$ are donor groups. In one preferred aspect of the present invention, three of $R^1$ to $R^5$ are donor groups. In one aspect of the present invention, at least $R^3$ is a donor group. In one aspect of the present invention, at least $R^4$ is a donor group. In one aspect of the present invention, at least $R^5$ is a donor group. In one aspect of the present invention, only $R^3$ is a donor group. In one aspect of the

present invention, only $R^4$ is a donor group. In one aspect of the present invention, only $R^5$ is a donor group. In one aspect of the present invention, only $R^3$ and $R^5$ are donor groups. In one aspect of the present invention, only $R^2$ and $R^5$ are donor groups. In one aspect of the present invention, only $R^2$ and $R^4$ are donor groups. In one aspect of the present invention, only $R^3$, $R^4$, and $R^5$ are donor groups. In one aspect of the present invention, only $R^2$, $R^4$, and $R^5$ are donor groups. When two or more of $R^1$ to $R^5$ are donor groups, these can be the same or different.

The number of $R^1$ to $R^5$ that are a hydrogen atom of a deuterium atom is 0 to 2, preferably 0 of 1, and is, for example, 1, or for example, 0. For example, $R^1$ is a hydrogen atom or a deuterium atom. These show more excellent light emission characteristics than the compounds where the number of $R^1$ to $R^5$ that are a hydrogen atom of a deuterium atom is 3. The number of $R^1$ to $R^5$ that are a substituted or unsubstituted aryl group is 0 or 1, and is preferably 1. The number may be 0. The number of $R^1$ to $R^5$ that are a substituted or unsubstituted alkyl group is 0 to 3, preferably 0 to 2, and may be 1 or may be 0.

In one aspect of the present invention, $R^1$ to $R^5$ are each independently selected from a hydrogen atom, a deuterium atom, a substituted or unsubstituted alkyl group, a donor group, and a group represented by the general formula (2). In one aspect of the present invention, $R^1$ to $R^5$ are each independently selected from a hydrogen atom, a deuterium atom, a donor group, and a group represented by the general formula (2). In one aspect of the present invention, $R^1$ to $R^5$ are each independently selected from a hydrogen atom, a deuterium atom, a substituted or unsubstituted carbazol-9-yl group which may be fused, and a group represented by the general formula (2).

The heteroaryl group that $Ar^1$ and $Ar^2$ in the general formula (1) can adopt may be a monocyclic ring or may be a fused ring in which two or more rings are fused. In the case of a fused ring, the number of fused rings is preferably 2 to 6, and can be selected from, for example, 2 to 4. Specific examples of the ring include a pyridine ring, a pyrimidine ring, and a pyrrole ring, and these rings may be fused with any other ring. Specific examples of the heteroaryl group include a 2-pyridyl group, a 3-pyridyl group, a 4-pyridyl group, a carbazol-9-yl group, a carbazol-1-yl group, a carbazol-2-yl group, a carbazol-3-yl group, and a carbazol-4-yl group. The number of the ring skeleton-constituting atoms of the heteroaryl group is preferably 4 to 40, more preferably 5 to 20, and may be selected from a range of 5 to 16, or may be selected from a range of 5 to 12.

In one aspect of the present invention, $Ar^1$ and $Ar^2$ are substituted or unsubstituted aryl groups.

In the general formula (1), $R^2$ or $R^3$ is a group represented by the following general formula (2). In addition, $R^1$, $R^4$, and $R^5$ can also adopt a group represented by the following general formula (2).

General Formula (2)

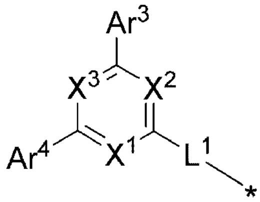

in which $X^1$ to $X^3$ each independently represent N or C(R), and at least one of $X^1$ to $X^3$ is N; R represents a hydrogen atom, a deuterium atom, of a substituent; $Ar^3$ and $Ar^4$ each independently represent a substituted or unsubstituted aryl group, of a substituted or unsubstituted heteroaryl group; $L^1$ represents a single bond, a substituted of unsubstituted arylene group, of a substituted or unsubstituted heteroarylene group. * represents a bonding position;

In the general formula (2), $L^1$ represents a single bond of a divalent linking group. Examples of the divalent linking group include a substituted of unsubstituted arylene group, and a substituted or unsubstituted heteroarylene group. In one preferred aspect of the present invention, $L^1$ is a single bond. In one aspect of the present invention, $L^1$ is a substituted or unsubstituted arylene group. In one aspect of the present invention, $L^1$ is a substituted or unsubstituted heteroarylene group. Regarding the aryl moiety constituting the arylene group, reference can be made to the description and the preferred range of the aryl group in the description section of $R^1$ to $R^5$ mentioned above. Examples of the heteroarylene group include a linking group formed by substituting at least one ring skeleton carbon atom constituting the arylene group with a nitrogen atom.

Specific examples of $L^1$ are shown below. However, $L^1$ which can be employed in the present invention shall not be construed as being limited by the following specific examples. In the following specific examples, the expression of a methyl group is omitted. Consequently, for example, L3 to L5 are substituted with a methyl group. * indicates a bonding position. L1 is a single bond.

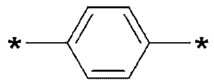
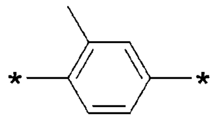
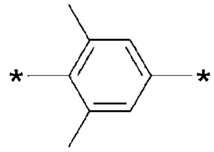
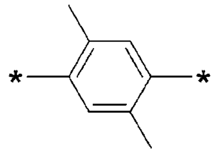

L1 L2 L3 L4 L5

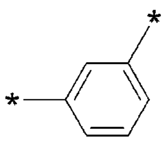
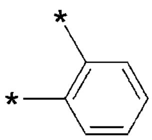
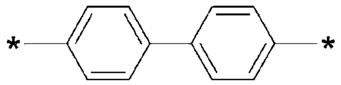
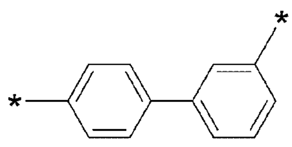

L6 L7 L8 L9

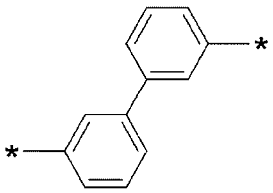
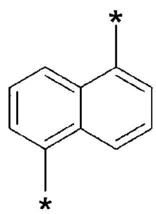
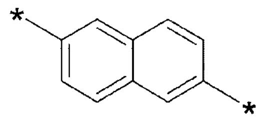
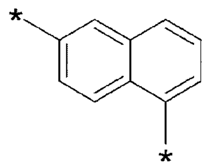

L10 L11 L12 L13

Groups obtained by substituting all hydrogen atoms present in the above L2 to L13 with deuterium atoms are disclosed as L14 to L25. In one aspect of the present invention, $L^1$ is selected from the group consisting of L1 to L25. In one aspect of the present invention, $L^1$ is selected from the group consisting of L1 to L7, and L14 to L19. In one aspect of the present invention, $L^1$ is selected from the group consisting of L1, L8 to L13, and L20 to L25. In one aspect of the present invention, $L^1$ is selected from the group consisting of L2 to L25.

In the general formula (2), $X^1$ to $X^3$ each independently represent N or C(R). However, at least one of $X^1$ to $X^3$ is N. R represents a hydrogen atom, a deuterium atom, or a substituent. The substituent referred to herein may be selected from Substituent Group A, may be selected from Substituent Group B, may be selected from Substituent Group C, may be selected from Substituent Group D, or may be selected from Substituent Group E. In one preferred aspect of the present invention, $X^1$ to $X^3$ are N. In one aspect of the present invention, $X^1$ and $X^3$ are N, and $X^2$ is C(R). In one aspect of the present invention, $X^1$ and $X^2$ are N, and $X^3$ is C(R). In one aspect of the present invention, $X^1$ is N, and $X^2$ and $X^3$ are C(R). In one aspect of the present invention, $X^2$ is N, and $X^1$ and $X^3$ are C(R). In one aspect of the present invention, R is a hydrogen atom or a deuterium atom. In one aspect of the present invention, R is an alkyl group optionally substituted with a deuterium atom. In one aspect of the present invention, R is an aryl group optionally substituted with a deuterium atom, an alkyl group, or an aryl group.

Regarding specific examples and preferred ranges of $Ar^3$ and $Ar^4$ in the general formula (2), reference can be made to the corresponding description of $Ar^1$ and $Ar^2$ in the general formula (1). In one aspect of the present invention, $Ar^1$ and $Ar^3$ are the same. In one preferred aspect of the present invention, $Ar^1$ and $Ar^3$ are the same, and $Ar^2$ and $Ar^4$ are the same. In one aspect of the present invention, $Ar^1$ to $Ar^4$ are the same.

In one preferred aspect of the present invention, $X^1$ to $X^3$ are N, and $L^1$ is a single bond. In one aspect of the present invention, $X^1$ to $X^3$ are N, and $L^1$ is a substituted or unsubstituted arylene group, preferably a substituted or unsubstituted phenylene group, and more preferably an unsubstituted phenylene group (for example, L2, for example, L6).

In one aspect of the present invention, $X^1$ to $X^3$ are N, $L^1$ is a single bond, and $Ar^3$ and $Ar^4$ are the same. In one aspect of the present invention, $X^1$ to $X^3$ are N, $L^1$ is a single bond, and $Ar^3$ and $Ar^4$ are each independently a substituted or unsubstituted aryl group. In one aspect of the present invention, $X^1$ to $X^3$ are N, $L^1$ is a single bond, and $Ar^3$ and $Ar^4$ are each independently a substituted or unsubstituted heteroaryl group (excluding a nitrogen-containing 6-membered ring group), preferably a donor heteroaryl group bonded via a nitrogen atom, and more preferably a substituted or unsubstituted carbazol-9-yl group. In one aspect of the present invention, $X^1$ to $X^3$ are N, $L^1$ is a single bond, $Ar^3$ is a substituted or unsubstituted heteroaryl group (excluding a nitrogen-containing 6-membered ring group), preferably a donor heteroaryl group bonded via a nitrogen atom, and more preferably a substituted or unsubstituted carbazol-9-yl group, and $Ar^4$ is a substituted or unsubstituted aryl group.

In one preferred aspect of the present invention, only $R^2$ is a group represented by the general formula (2). In one

preferred aspect of the present invention, only $R^3$ is a group represented by the general formula (2). In one aspect of the present invention, only one of $R^1$, $R^3$, $R^4$, and $R^5$ and $R^2$ are each independently a group represented by the general formula (2). In one aspect of the present invention, only one of $R^1$, $R^2$, $R^4$, and $R^5$ and $R^3$ are each independently a group represented by the general formula (2).

In the compound represented by the general formula (1), at least one of $R^1$ to $R^5$ is a donor group having a fused ring structure of four or more rings, at least one of $Ar^1$ to $Ar^4$ is a substituted or unsubstituted heteroaryl group (excluding a nitrogen-containing 6-membered ring group), or $R^1$ to $R^5$ and $Ar^1$ to $Ar^4$ satisfy both of these conditions.

The "donor group having a fused ring structure of four or more rings" as referred to herein is a donor group having a fused ring structure of four or more rings and bonding via one atom of the ring skeleton-constituting atoms of the fused ring structure. The one atom is preferably a carbon atom or a nitrogen atom, and more preferably a nitrogen atom. The donor group as referred to herein means a group having a negative Hammett's $\sigma p$ value.

The donor group having a fused ring structure of four or more rings preferably has a fused ring structure of five or more rings, and more preferably has a fused ring structure of five to seven rings. Examples thereof include groups having a fused ring structure of five rings and groups having a fused ring structure of seven rings.

As the donor group having a fused ring structure of four or more rings, a ring-fused carbazol-9-yl group is preferable. Specific examples thereof include D86 to D1210.

In particular, a group having a structure represented by the following general formula (3) is preferable.

General Formula (3)

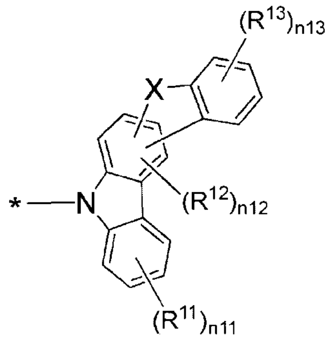

In the general formula (3), X represents O, S, or N-$R^{14}$. $R^{11}$ to $R^{13}$ each independently represent a deuterium atom, or a substituent. $R^{14}$ represents an aryl group optionally substituted with one or more selected from the group consisting of a deuterium atom, an alkyl group and an aryl group, or an alkyl group optionally substituted with one or more selected from the group consisting of a deuterium atom and an aryl group. $R^{11}$'s, $R^{12}$'s, and $R^{13}$'s each may be bonded to each other to form a cyclic structure. n11 and n13 each independently represent an integer of 0 to 4, and n12 represents an integer of 0 to 2.

In the general formula (3), X represents O, S, or N-$R^{14}$. $R^{14}$ represents an aryl group optionally substituted with one or more selected from the group consisting of a deuterium atom, an alkyl group and an aryl group, or an alkyl group optionally substituted with one or more selected from the group consisting of a deuterium atom and an aryl group. Here, the aryl group as a substituent for the aryl group and the alkyl group can be selected from, for example, an aryl group having 6 to 22 carbon atoms, and the alkyl group as a substituent for the alkyl group and the aryl group can be selected from, for example, an alkyl group having 1 to 20 carbon atoms. In one preferred aspect of the present invention, X is O. In one preferred aspect of the present invention, X is N-$R^{14}$. For example $R^{14}$ of N-$R^{14}$ is an aryl group (for example, having 6 to 22 carbon atoms). The aryl group may be not substituted with or may be substituted with at least one atom or group selected from the group consisting of a deuterium atom, an alkyl group (for example, having 1 to 20 carbon atoms), and an aryl group (for example, having 6 to 22 carbon atoms). X may be an oxygen atom or a sulfur atom.

In the general formula (3), the two bonds bonding to one benzene ring of the carbazole ring bond to the neighboring positions of the benzene ring to form a fused ring structure of the carbazole ring and the hetero-fused ring containing X. For example, in the case where X is O, a benzofurocarbazole ring is formed as a fused ring structure, in the case where X is S, a benzothienocarbazole ring is formed as a fused ring structure, and in the case where X is N-$R^{14}$, an indolocarbazole ring is formed as a fused ring structure. The positions to which the two bonds bond may be the 1-position and the 2-position of the carbazole ring, or may be the 2-position and the 3-position of the carbazole ring, or may be the 3-position and the 4-position of the carbazole ring. In the case where the bonding positions of the two bonds are the 1-position and the 2-position, the position at which the bond of X bonds may be the 1-position or the 2-position, in the case where the bonding positions of the two bonds are the 2-position and the 3-position, the position at which the bond of X bonds may be the 2-position or the 3-position, and in the case where the bonding positions of the two bonds are the 3-position and the 4-position, the position at which the bond of X bonds may be the 3-position or the 4-position.

In the general formula (3), * represents a bonding position.

In the general formula (3), $R^{11}$ to $R^{13}$ each independently represent a deuterium atom or a substituent. $R^{11}$'s, $R^{12}$'s, and $R^{13}$'s each may be bonded to each other to form a cyclic structure, but $R^{11}$ is not bonded to any one of $R^{12}$ to $R^{14}$ to form a cyclic structure, $R^{12}$ is not bonded to any one of $R^{13}$ and $R^{14}$ to form a cyclic structure, and $R^{13}$ is not bonded to $R^{14}$ to form a cyclic structure. For example, the substituent may be selected from Substituent Group A, may be selected from Substituent Group B, may be selected from Substituent Group C, may be selected from Substituent Group D, or may be selected from Substituent Group E. In one preferred aspect of the present invention, the substituent means one group or a combination of two or more groups selected from the group consisting of an alkyl group (for example, having 1 to 20 carbon atoms), and an aryl group (for example, having 6 to 22 carbon atoms).

n11 and n13 each independently represent an integer of 0 to 4, and n12 represents an integer of 0 to 2. When n11 is 2 or more, two or more $R^{11}$'s may be the same or different. When n13 is 2 or more, two or more $R^{13}$'s may be the same or different. When n12 is 2, two $R^{12}$'s may be the same or different. n11 and n13 may be any number of 0, 1, 2, 3, or 4, and n12 may be any number of 0, 1, or 2. When n11 is 1, $R^{11}$ may be a deuterium atom, or may be a substituent. When n11 is 2 or more, all two or more $R^{11}$'s may be deuterium atoms, or all may be substituents, or a part thereof may be deuterium atoms and the remaining ones may be substituents. When n13 is 1, $R^{13}$ may be a deuterium atom, or may be a substituent. When n13 is 2 or more, all two or more $R^{13}$'s may be deuterium atoms, or all may be substituents, or a part thereof may be deuterium atoms and the remaining ones may be substituents. When n12 is 1, $R^{12}$ may be a deuterium atom, or may be a substituent. When n12 is 2, both two $R^{12}$'s may be deuterium atoms, or both may be substituents, or one of the two may be a deuterium atom and the other may be a substituent.

In the case of having a donor group having a fused ring structure of four or more rings, two or more of $R^1$ to $R^5$ are preferably donor groups having a fused ring structure of four or more rings, and for example, two of R1 to R5 are donor groups having a fused ring structure of four or more rings, or for example, three of of R1 to R5 are donor groups having a fused ring structure of four or more rings. When two or more of $R^1$ to $R^5$ are donor groups having a fused ring structure of four or more rings, they may be the same or different, but preferably they are the same. In one aspect of the present invention, at least $R^3$ is a donor group having a fused ring structure of four or more rings. In one aspect of the present invention, at least $R^4$ is a donor group having a fused ring structure of four or more rings. In one aspect of the present invention, at least $R^5$ is a donor group having a fused ring structure of four or more rings. In one aspect of the present invention, $R^3$ and $R^5$ are donor groups having a fused ring structure of four or more rings. In one aspect of the present invention, $R^3$, $R^4$, and $R^5$ are donor groups having a fused ring structure of four or more rings.

In one preferred aspect of the present invention, at least one of $Ar^1$ to $Ar^4$ is a substituted or unsubstituted heteroaryl group (excluding a nitrogen-containing 6-membered ring group). The number of the substituted or unsubstituted heteroaryl groups (excluding a nitrogen-containing 6-membered ring group) may be only one, may be two, may be three, or may be all of $Ar^1$ to $Ar^4$. When there are two or more, they may be the same or different, but are preferably the same. The substituted or unsubstituted heteroaryl group (excluding a nitrogen-containing 6-membered ring group) is preferably a heteroaryl group bonded via a nitrogen atom which is one of ring skeleton-constituting atoms, more preferably a heteroaryl group having a 5-membered ring bonded via a nitrogen atom (a group bonded via a nitrogen atom of a pyrrole ring, and the pyrrole ring may be substituted and is preferably fused), and still more preferably a substituted or unsubstituted carbazol-9-yl group. In one preferred aspect of the present invention, the carbazol-9-yl group as referred to herein may be substituted with an alkyl group or an aryl group optionally substituted with a group selected from the group consisting of a deuterium atom, an alkyl group and an aryl group, and can also be substituted with a deuterium atom. For the description and details of the carbazol-9-yl group, reference can be made to the description of the carbazol-9-yl group that $R^1$ to $R^5$ can adopt, the description of the ring-fused carbazol-9-yl group, and specific examples D1 to D1210.

In one preferred aspect of the present invention, $Ar^1$ to $Ar^4$ are each independently a substituted or unsubstituted aryl group. All of $Ar^1$ to $Ar^4$ may be the same, and examples thereof include an unsubstituted phenyl group and a perdeuterated phenyl group. When $Ar^1$ to $Ar^4$ are each independently a substituted or unsubstituted aryl group, at least one of $R^1$ to $R^5$ is a donor group having a fused ring structure of four or more rings. The number of the donor groups having a fused ring structure of four or more rings may be 2 or more, for example, 2, and for example, 3. As the donor group having a fused ring structure of four or more rings, specific examples D86 to D1210 can be referred to. Among these, a donor group having a fused ring structure of five or more rings (for example, five rings, for example, seven rings) can be preferably employed. In one preferred aspect of the present invention, when $Ar^1$ to $Ar^4$ are each independently a substituted or unsubstituted aryl group, at least one of $R^1$ to $R^5$ has a deuterium atom. For example, the compound can have a deuterated donor group having a fused ring structure of three rings (for example, a perdeuterated carbazol-9-yl group), or can have a deuterated aryl group (for example, a perdeuterated phenyl group).

The compound represented by the general formula (1) preferably does not contain a metal atom, and may be a compound composed only of atoms selected from the group consisting of a carbon atom, a hydrogen atom, a deuterium atom, a nitrogen atom, an oxygen atom, and a sulfur atom. In one preferred aspect of the present invention, the compound represented by the general formula (1) is composed only of atoms selected from the group consisting of a carbon atom, a hydrogen atom, a deuterium atom, a nitrogen atom, and an oxygen atom. In addition, the compound represented by the

general formula (1) may be a compound composed only of atoms selected from the group consisting of a carbon atom, a hydrogen atom, a deuterium atom, a nitrogen atom, and a sulfur atom. The compound represented by the general formula (1) may be a compound composed only of atoms selected from the group consisting of a carbon atom, a hydrogen atom, a deuterium atom, and a nitrogen atom. The compound represented by the general formula (1) may be a compound composed only of atoms selected from the group consisting of a carbon atom, a hydrogen atom, and a nitrogen atom. Further, the compound represented by the general formula (1) may be a compound which does not contain a hydrogen atom but contains a deuterium atom.

In the description herein, the term "Substituent Group A" means one atom or group or a combination of two or more thereof selected from the group consisting of a deuterium atom, a hydroxyl group, a halogen atom (for example, a fluorine atom, a chlorine atom, a bromine atom, and an iodine atom), an alkyl group (for example, having 1 to 40 carbon atoms), an alkoxy group (for example, having 1 to 40 carbon atoms), an alkylthio group (for example, having 1 to 40 carbon atoms), an aryl group (for example, having 6 to 30 carbon atoms), an aryloxy group (for example, having 6 to 30 carbon atoms), an arylthio group (for example, having 6 to 30 carbon atoms), a heteroaryl group (for example, having 5 to 30 ring skeleton-constituting atoms), a heteroaryloxy group (for example, having 5 to 30 ring skeleton-constituting atoms), a heteroarylthio group (for example, having 5 to 30 ring skeleton-constituting atoms), an acyl group (for example, having 1 to 40 carbon atoms), an alkenyl group (for example, having 1 to 40 carbon atoms), an alkynyl group (for example, having 1 to 40 carbon atoms), an alkoxycarbonyl group (for example, having 1 to 40 carbon atoms), an aryloxycarbonyl group (for example, having 1 to 40 carbon atoms), a heteroaryloxycarbonyl group (for example, having 1 to 40 carbon atoms), a silyl group (for example, a trialkylsilyl group having 1 to 40 carbon atoms), and a nitro group.

In the description herein, the term "Substituent Group B" means one atom or group or a combination of two or more thereof selected from the group consisting of a deuterium atom, an alkyl group (for example, having 1 to 40 carbon atoms), an alkoxy group (for example, having 1 to 40 carbon atoms), an aryl group (for example, having 6 to 30 carbon atoms), an aryloxy group (for example, having 6 to 30 carbon atoms), a heteroaryl group (for example, having 5 to 30 ring skeleton-constituting atoms), a heteroaryloxy group (for example, having 5 to 30 ring skeleton-constituting atoms), and a diarylaminoamino group (for example, having 0 to 20 carbon atoms).

In the description herein, the term "Substituent Group C" means one atom or group or a combination of two or more thereof selected from the group consisting of a deuterium atom, an alkyl group (for example, having 1 to 20 carbon atoms), an aryl group (for example, having 6 to 22 carbon atoms), a heteroaryl group (for example, having 5 to 20 ring skeleton-constituting atoms), and a diarylamino group (for example, having 12 to 20 carbon atoms).

In the description herein, the term "Substituent Group D" means one atom or group or a combination of two or more thereof selected from the group consisting of a deuterium atom, an alkyl group (for example, having 1 to 20 carbon atoms), an aryl group (for example, having 6 to 22 carbon atoms), and a heteroaryl group (for example, having 5 to 20 ring skeleton-constituting atoms).

In the description herein, the term "Substituent Group E" means one atom or group or a combination of two or more groups selected from the group consisting of a deuterium atom, an alkyl group (for example, having 1 to 20 carbon atoms), and an aryl group (for example, having 6 to 22 carbon atoms).

In the description herein, the substituent meant by an expression of "substituted or unsubstituted" or "optionally substituted" may be selected, for example, from Substituent Group A, may be selected from Substituent Group B, may be selected from Substituent Group C, may be selected from Substituent Group D, or may be selected from Substituent Group E.

Specific examples of the compound represented by the general formula (1) are shown in the following Tables 1 to 8. However, the compound represented by the general formula (1) that can be used in the present invention should not be construed as being limited by these specific examples.

In Table 1, structures of the compounds are individually shown by specifying $R^3$ to $R^5$ of the following general formula (1a) for each compound. That is, structures in which $R^2$ is a group represented by the general formula (2), $Ar^1$ and $Ar^3$ are perdeuterated carbazol-9-yl groups (D55), $Ar^2$ and $Ar^4$ are perdeuterated phenyl groups (Ar47), $X^1$ to $X^3$ are nitrogen atoms (N), $L^1$ is a single bond (L1), $R^1$ is a hydrogen atom, and $R^3$ to $R^5$ are groups specified in Table 1 are individually shown as structures of Compounds 1 to 170.

General Formula (1a)

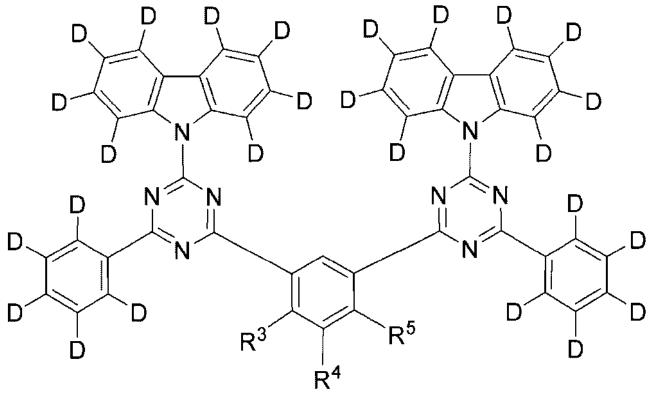

[Table 1]

| No. | $R^3$ | $R^4$ | $R^5$ |
|---|---|---|---|
| 1 | D1 | D1 | D1 |
| 2 | D2 | D1 | D2 |
| 3 | D3 | D1 | D3 |
| 4 | D4 | D1 | D4 |
| 5 | D5 | D1 | D5 |
| 6 | D6 | D1 | D6 |
| 7 | D7 | D1 | D7 |
| 8 | D8 | D1 | D8 |
| 9 | D9 | D1 | D9 |
| 10 | D10 | D1 | D10 |
| 11 | D11 | D1 | D11 |
| 12 | D12 | D1 | D12 |
| 13 | D13 | D1 | D13 |
| 14 | D14 | D1 | D14 |
| 15 | D15 | D1 | D15 |
| 16 | D16 | D1 | D16 |
| 17 | D17 | D1 | D17 |
| 18 | D18 | D1 | D18 |
| 19 | D19 | D1 | D19 |
| 20 | D20 | D1 | D20 |
| 21 | D21 | D1 | D21 |
| 22 | D22 | D1 | D22 |
| 23 | D23 | D1 | D23 |
| 24 | D24 | D1 | D24 |
| 25 | D25 | D1 | D25 |
| 26 | D26 | D1 | D26 |
| 27 | D27 | D1 | D27 |
| 28 | D28 | D1 | D28 |

(continued)

| No. | $R^3$ | $R^4$ | $R^5$ |
|---|---|---|---|
| 29 | D29 | D1 | D29 |
| 30 | D30 | D1 | D30 |
| 31 | D31 | D1 | D31 |
| 32 | D32 | D1 | D32 |
| 33 | D33 | D1 | D33 |
| 34 | D34 | D1 | D34 |
| 35 | D35 | D1 | D35 |
| 36 | D36 | D1 | D36 |
| 37 | D37 | D1 | D37 |
| 38 | D38 | D1 | D38 |
| 39 | D39 | D1 | D39 |
| 40 | D40 | D1 | D40 |
| 41 | D41 | D1 | D41 |
| 42 | D42 | D1 | D42 |
| 43 | D43 | D1 | D43 |
| 44 | D44 | D1 | D44 |
| 45 | D45 | D1 | D45 |
| 46 | D46 | D1 | D46 |
| 47 | D47 | D1 | D47 |
| 48 | D48 | D1 | D48 |
| 49 | D49 | D1 | D49 |
| 50 | D50 | D1 | D50 |
| 51 | D51 | D1 | D51 |
| 52 | D52 | D1 | D52 |
| 53 | D53 | D1 | D53 |
| 54 | D54 | D1 | D54 |
| 55 | D55 | D1 | D55 |
| 56 | D56 | D1 | D56 |
| 57 | D57 | D1 | D57 |
| 58 | D58 | D1 | D58 |
| 59 | D59 | D1 | D59 |
| 60 | D60 | D1 | D60 |
| 61 | D61 | D1 | D61 |
| 62 | D62 | D1 | D62 |
| 63 | D63 | D1 | D63 |
| 64 | D64 | D1 | D64 |
| 65 | D65 | D1 | D65 |
| 66 | D66 | D1 | D66 |
| 67 | D67 | D1 | D67 |

(continued)

| No. | $R^3$ | $R^4$ | $R^5$ |
|---|---|---|---|
| 68 | D68 | D1 | D68 |
| 69 | D69 | D1 | D69 |
| 70 | D70 | D1 | D70 |
| 71 | D71 | D1 | D71 |
| 72 | D72 | D1 | D72 |
| 73 | D73 | D1 | D73 |
| 74 | D74 | D1 | D74 |
| 75 | D75 | D1 | D75 |
| 76 | D76 | D1 | D76 |
| 77 | D77 | D1 | D77 |
| 78 | D78 | D1 | D78 |
| 79 | D79 | D1 | D79 |
| 80 | D80 | D1 | D80 |
| 81 | D81 | D1 | D81 |
| 82 | D82 | D1 | D82 |
| 83 | D83 | D1 | D83 |
| 84 | D84 | D1 | D84 |
| 85 | D85 | D1 | D85 |
| 86 | D1 | D2 | D1 |
| 87 | D2 | D2 | D2 |
| 88 | D3 | D2 | D3 |
| 89 | D4 | D2 | D4 |
| 90 | D5 | D2 | D5 |
| 91 | D6 | D2 | D6 |
| 92 | D7 | D2 | D7 |
| 93 | D8 | D2 | D8 |
| 94 | D9 | D2 | D9 |
| 95 | D10 | D2 | D10 |
| 96 | D11 | D2 | D11 |
| 97 | D12 | D2 | D12 |
| 98 | D13 | D2 | D13 |
| 99 | D14 | D2 | D14 |
| 100 | D15 | D2 | D15 |
| 101 | D16 | D2 | D16 |
| 102 | D17 | D2 | D17 |
| 103 | D18 | D2 | D18 |
| 104 | D19 | D2 | D19 |
| 105 | D20 | D2 | D20 |
| 106 | D21 | D2 | D21 |

(continued)

| No. | $R^3$ | $R^4$ | $R^5$ |
|---|---|---|---|
| 107 | D22 | D2 | D22 |
| 108 | D23 | D2 | D23 |
| 109 | D24 | D2 | D24 |
| 110 | D25 | D2 | D25 |
| 111 | D26 | D2 | D26 |
| 112 | D27 | D2 | D27 |
| 113 | D28 | D2 | D28 |
| 114 | D29 | D2 | D29 |
| 115 | D30 | D2 | D30 |
| 116 | D31 | D2 | D31 |
| 117 | D32 | D2 | D32 |
| 118 | D33 | D2 | D33 |
| 119 | D34 | D2 | D34 |
| 120 | D35 | D2 | D35 |
| 121 | D36 | D2 | D36 |
| 122 | D37 | D2 | D37 |
| 123 | D38 | D2 | D38 |
| 124 | D39 | D2 | D39 |
| 125 | D40 | D2 | D40 |
| 126 | D41 | D2 | D41 |
| 127 | D42 | D2 | D42 |
| 128 | D43 | D2 | D43 |
| 129 | D44 | D2 | D44 |
| 130 | D45 | D2 | D45 |
| 131 | D46 | D2 | D46 |
| 132 | D47 | D2 | D47 |
| 133 | D48 | D2 | D48 |
| 134 | D49 | D2 | D49 |
| 135 | D50 | D2 | D50 |
| 136 | D51 | D2 | D51 |
| 137 | D52 | D2 | D52 |
| 138 | D53 | D2 | D53 |
| 139 | D54 | D2 | D54 |
| 140 | D55 | D2 | D55 |
| 141 | D56 | D2 | D56 |
| 142 | D57 | D2 | D57 |
| 143 | D58 | D2 | D58 |
| 144 | D59 | D2 | D59 |
| 145 | D60 | D2 | D60 |

(continued)

| No. | $R^3$ | $R^4$ | $R^5$ |
|---|---|---|---|
| 146 | D61 | D2 | D61 |
| 147 | D62 | D2 | D62 |
| 148 | D63 | D2 | D63 |
| 149 | D64 | D2 | D64 |
| 150 | D65 | D2 | D65 |
| 151 | D66 | D2 | D66 |
| 152 | D67 | D2 | D67 |
| 153 | D68 | D2 | D68 |
| 154 | D69 | D2 | D69 |
| 155 | D70 | D2 | D70 |
| 156 | D71 | D2 | D71 |
| 157 | D72 | D2 | D72 |
| 158 | D73 | D2 | D73 |
| 159 | D74 | D2 | D74 |
| 160 | D75 | D2 | D75 |
| 161 | D76 | D2 | D76 |
| 162 | D77 | D2 | D77 |
| 163 | D78 | D2 | D78 |
| 164 | D79 | D2 | D79 |
| 165 | D80 | D2 | D80 |
| 166 | D81 | D2 | D81 |
| 167 | D82 | D2 | D82 |
| 168 | D83 | D2 | D83 |
| 169 | D84 | D2 | D84 |
| 170 | D85 | D2 | D85 |

In Table 2, structures of Compounds 1 to 442987 are shown by collectively displaying $R^3$ to $R^5$ of a plurality of compounds in each row. For example, in the row of Compounds 1 to 85 in Table 2, compounds in which $R^4$ is fixed to D1 (carbazol-9-yl group), $R^3$ and $R^5$ are the same, and D1 to D85 are referred to as Compounds 1 to 85, respectively. Similarly, in the row of Compounds 86 to 170 in Table 2, compounds in which $R^4$ is fixed to D2 (3-methylcarbazol-9-yl group), $R^3$ and $R^5$ are the same, and D1 to D85 are referred to as Compounds 86 to 170, respectively. That is, the row of Compounds 1 to 85 and the row of Compounds 86 to 170 in Table 2 collectively represent Compounds 1 to 170 specified in Table 1 in two rows. Similarly, in the row of Compounds 171 to 255 in Table 2, compounds in which $R^4$ is fixed to D3 (3,6-dimethyl-carbazol-9-yl group), $R^3$ and $R^5$ are the same, and D1 to D85 are referred to as Compounds 171 to 255, respectively. In the same manner, Compounds 256 to 442987 in Table 2 are also specified.

[Table 2]

| No. | $R^3$ | $R^4$ | $R^5$ | = |
|---|---|---|---|---|
| 1~ 85 | D1~D85 | D1 | D1~D85 | |
| 86 ~ 170 | D1~D85 | D2 | D1~D85 | |
| 171 ~ 255 | D1~D85 | D3 | D1~D85 | |
| 256 ~ 340 | D1~D85 | D4 | D1~D85 | |

(continued)

| No. | $R^3$ | $R^4$ | $R^5$ | = |
|---|---|---|---|---|
| 341 ~ 425 | D1~D85 | D5 | D1~D85 | $R^3 = R^5$ |
| 426 ~ 510 | D1~D85 | D6 | D1~D85 | |
| 511 ~ 595 | D1~D85 | D7 | D1~D85 | |
| 596 ~ 680 | D1~D85 | D8 | D1~D85 | |
| 681 ~ 765 | D1~D85 | D9 | D1~D85 | |
| 766 ~ 850 | D1~D85 | D10 | D1~D85 | |
| 851 ~ 935 | D1~D85 | D11 | D1~D85 | |
| 936 ~ 1020 | D1~D85 | D12 | D1~D85 | |
| 1021 ~ 1105 | D1~D85 | D13 | D1~D85 | |
| 1106 ~ 1190 | D1~D85 | D14 | D1~D85 | |
| 1191 ~ 1275 | D1~D85 | D15 | D1~D85 | |
| 1276 ~ 1360 | D1~D85 | D16 | D1~D85 | |
| 1361 ~ 1445 | D1~D85 | D17 | D1~D85 | |
| 1446 ~ 1530 | D1~D85 | D18 | D1~D85 | |
| 1531 ~ 1615 | D1~D85 | D19 | D1~D85 | |
| 1616 ~ 1700 | D1~D85 | D20 | D1~D85 | |
| 1701 ~ 1785 | D1~D85 | D21 | D1~D85 | |
| 1786 ~ 1870 | D1~D85 | D22 | D1~D85 | |
| 1871 ~ 1955 | D1~D85 | D23 | D1~D85 | |
| 1956 ~ 2040 | D1~D85 | D24 | D1~D85 | |
| 2041 ~ 2125 | D1~D85 | D25 | D1~D85 | |
| 2126 ~ 2210 | D1~D85 | D26 | D1~D85 | |
| 2211 ~ 2295 | D1~D85 | D27 | D1~D85 | |
| 2296 ~ 2380 | D1~D85 | D28 | D1~D85 | |
| 2381 ~ 2465 | D1~D85 | D29 | D1~D85 | |
| 2466 ~ 2550 | D1~D85 | D30 | D1~D85 | |
| 2551 ~ 2635 | D1~D85 | D31 | D1~D85 | |
| 2636 ~ 2720 | D1~D85 | D32 | D1~D85 | |
| 2721 ~ 2805 | D1~D85 | D33 | D1~D85 | |
| 2806 ~ 2890 | D1~D85 | D34 | D1~D85 | |
| 2891 ~ 2975 | D1~D85 | D35 | D1~D85 | |
| 2976 ~ 3060 | D1~D85 | D36 | D1~D85 | |
| 3061 ~ 3145 | D1~D85 | D37 | D1~D85 | |
| 3146 ~ 3230 | D1~D85 | D38 | D1~D85 | |
| 3231 ~ 3315 | D1~D85 | D39 | D1~D85 | |
| 3316 ~ 3400 | D1~D85 | D40 | D1~D85 | |
| 3401 ~ 3485 | D1~D85 | D41 | D1~D85 | |
| 3486 ~ 3570 | D1~D85 | D42 | D1~D85 | |
| 3571 ~ 3655 | D1~D85 | D43 | D1~D85 | |

(continued)

| No. | $R^3$ | $R^4$ | $R^5$ | = |
|---|---|---|---|---|
| 3656 ~ 3740 | D1~D85 | D44 | D1~D85 | |
| 3741 ~ 3825<br>3826 ~ 3910 | D1~D85<br>D1~D85 | D45<br>D46 | D1~D85<br>D1~D85 | |
| 3911 ~ 3995 | D1~D85 | D47 | D1~D85 | |
| 3996 ~ 4080 | D1~D85 | D48 | D1~D85 | |
| 4081 ~ 4165 | D1~D85 | D49 | D1~D85 | |
| 4166 ~ 4250 | D1~D85 | D50 | D1~D85 | |
| 4251 ~ 4335 | D1~D85 | D51 | D1~D85 | |
| 4336 ~ 4420 | D1~D85 | D52 | D1~D85 | |
| 4421 ~ 4505 | D1~D85 | D53 | D1~D85 | |
| 4506 ~ 4590 | D1~D85 | D54 | D1~D85 | |
| 4591 ~ 4675 | D1~D85 | D55 | D1~D85 | |
| 4676 ~ 4760 | D1~D85 | D56 | D1~D85 | |
| 4761 ~ 4845 | D1~D85 | D57 | D1~D85 | |
| 4846 ~ 4930 | D1~D85 | D58 | D1~D85 | |
| 4931 ~ 5015 | D1~D85 | D59 | D1~D85 | |
| 5016 ~ 5100 | D1~D85 | D60 | D1~D85 | |
| 5101 ~ 5185 | D1~D85 | D61 | D1~D85 | |
| 5186 ~ 5270 | D1~D85 | D62 | D1~D85 | |
| 5271 ~ 5355 | D1~D85 | D63 | D1~D85 | |
| 5356 ~ 5440 | D1~D85 | D64 | D1~D85 | |
| 5441 ~ 5525 | D1~D85 | D65 | D1~D85 | |
| 5526 ~ 5610 | D1~D85 | D66 | D1~D85 | |
| 5611 ~ 5695 | D1~D85 | D67 | D1~D85 | |
| 5696 ~ 5780 | D1~D85 | D68 | D1~D85 | |
| 5781 ~ 5865 | D1~D85 | D69 | D1~D85 | |
| 5866 ~ 5950 | D1~D85 | D70 | D1~D85 | |
| 5951 ~ 6035 | D1~D85 | D71 | D1~D85 | |
| 6036 ~ 6120 | D1~D85 | D72 | D1~D85 | |
| 6121 ~ 6205 | D1~D85 | D73 | D1~D85 | |
| 6206 ~ 6290 | D1~D85 | D74 | D1~D85 | |
| 6291 ~ 6375 | D1~D85 | D75 | D1~D85 | |
| 6376 ~ 6460 | D1~D85 | D76 | D1~D85 | |
| 6461 ~ 6545<br>6546 ~ 6630 | D1~D85<br>D1~D85 | D77<br>D78 | D1~D85<br>D1~D85 | |
| 6631 ~ 6715 | D1~D85 | D79 | D1~D85 | $R^3 = R^5$ |
| 6716 ~ 6800 | D1~D85 | D80 | D1~D85 | |
| 6801 ~ 6885 | D1~D85 | D81 | D1~D85 | |
| 6886 ~ 6970 | D1~D85 | D82 | D1~D85 | |
| 6971 ~ 7055 | D1~D85 | D83 | D1~D85 | |

(continued)

| No. | $R^3$ | $R^4$ | $R^5$ | = |
|---|---|---|---|---|
| 7056 ~ 7140 | D1~D85 | D84 | D1~D85 | |
| 7141 ~ 7225 | D1~D85 | D85 | D1~D85 | |
| 7226 ~ 7310 | D1~D85 | Ar1 | D1~D85 | |
| 7311 ~ 7395 | D1~D85 | Ar14 | D1~D85 | |
| 7396 ~ 7480 | D1~D85 | Ar15 | D1~D85 | |
| 7481 ~ 7565 | D1~D85 | Ar39 | D1~D85 | |
| 7566 ~ 7650 | D1~D85 | Ar40 | D1~D85 | |
| 7651 ~ 7735 | D1~D85 | Ar47 | D1~D85 | |
| 7736 ~ 7820 | Ar1 | D1~D85 | D1~D85 | $R^4 = R^5$ |
| 7821 ~ 7905 | Ar14 | D1~D85 | D1~D85 | |
| 7906 ~ 7990 | Ar15 | D1~D85 | D1~D85 | |
| 7991 ~ 8075 | Ar39 | D1~D85 | D1~D85 | |
| 8076 ~ 8160 | Ar40 | D1~D85 | D1~D85 | |
| 8161 ~ 8245 | Ar47 | D1~D85 | D1~D85 | |
| 8246 ~ 8330 | D1~D85 | D1~D85 | Ar1 | $R^3 = R^4$ |
| 8331 ~ 8415 | D1~D85 | D1~D85 | Ar14 | |
| 8416 ~ 8500 | D1~D85 | D1~D85 | Ar15 | |
| 8501 ~ 8585 | D1~D85 | D1~D85 | Ar39 | |
| 8586 ~ 8670 | D1~D85 | D1~D85 | Ar40 | |
| 8671 ~ 8755 | D1~D85 | D1~D85 | Ar47 | |
| 8756 ~ 9880 | D86~D1210 | D86-D1210 | D86~D1210 | $R^3 = R^4 = R^5$ |
| 9881 ~ 11005 | D86~D1210 | H | D86~D1210 | $R^3 = R^5$ |
| 11006 ~ 12130 | D86~D1210 | D1 | D86~D1210 | |
| 12131 ~ 13255 | D86~D1210 | D2 | D86~D1210 | |
| 13256 ~ 14380 | D86~D1210 | D3 | D86-01210 | |
| 14381 ~ 15505 | D86~D1210 | D4 | D86~D1210 | |
| 15506 ~ 16630 | D86~D1210 | D5 | D86~D1210 | |
| 16631 ~ 17755 | D86~D1210 | D6 | D86~D1210 | |
| 17756 ~ 18880 | D86~D1210 | D7 | D86~D1210 | |
| 18881 ~ 20005 | D86~D1210 | D8 | D86~D1210 | |
| 20006 ~ 21130 | D86~D1210 | D9 | D86~D1210 | |
| 21131 ~ 22255 | D86~D1210 | D10 | D86~D1210 | |
| 22256 ~ 23380 | D86~D1210 | D11 | D86~D1210 | |
| 23381 ~ 24505 | D86~D1210 | D12 | D86~D1210 | |
| 24506 ~ 25630 | D86~D1210 | D13 | D86~D1210 | |
| 25631 ~ 26755 | D86~D1210 | D14 | D86~D1210 | |
| 26756 ~ 27880 | D86~D1210 | D15 | D86~D1210 | |
| 27881 ~ 29005<br>29006 ~ 30130 | D86~D1210<br>D86~D1210 | D16<br>D17 | D86-01210<br>D86~D1210 | |
| 30131 ~ 31255 | D86~D1210 | D18 | D86~D1210 | |

(continued)

| No. | $R^3$ | $R^4$ | $R^5$ | = |
|---|---|---|---|---|
| 31256 ~ 32380 | D86~D1210 | D19 | D86~D1210 | $R^3 = R^5$ |
| 32381 ~ 33505 | D86~D1210 | D20 | D86~D1210 | |
| 33506 ~ 34630 | D86~D1210 | D21 | D86~D1210 | |
| 34631 ~ 35755 | D86~D1210 | D22 | D86~D1210 | |
| 35756 ~ 36880 | D86~D1210 | D23 | D86~D1210 | |
| 36881 ~ 38005 | D86~D1210 | D24 | D86~D1210 | |
| 38006 ~ 39130 | D86~D1210 | D25 | D86~D1210 | |
| 39131 ~ 40255 | D86~D1210 | D26 | D86~D1210 | |
| 40256 ~ 41380 | D86~D1210 | D27 | D86~D1210 | |
| 41381 ~ 42505 | D86~D1210 | D28 | D86~D1210 | |
| 42506 ~ 43630 | D86~D1210 | D29 | D86~D1210 | |
| 43631 ~ 44755 | D86~D1210 | D30 | D86~D1210 | |
| 44756 ~ 45880 | D86~D1210 | D31 | D86~D1210 | |
| 45881 ~ 47005 | D86~D1210 | D32 | D86~D1210 | |
| 47006 ~ 48130 | D86~D1210 | D33 | D86~D1210 | |
| 48131 ~ 49255 | D86~D1210 | D34 | D86~D1210 | |
| 49256 ~ 50380 | D86~D1210 | D35 | D86~D1210 | |
| 50381 ~ 51505 | D86~D1210 | D36 | D86~D1210 | |
| 51506 ~ 52630<br>52631 ~ 53755 | D86~D1210<br>D86~D1210 | D37<br>D38 | D86~D1210<br>D86~D1210 | |
| 53756 ~ 54880 | D86~D1210 | D39 | D86~D1210 | |
| 54881 ~ 56005 | D86~D1210 | D40 | D86~D1210 | |
| 56006 ~ 57130 | D86~D1210 | D41 | D86~D1210 | |
| 57131 ~ 58255 | D86~D1210 | D42 | D86~D1210 | |
| 58256 ~ 59380 | D86~D1210 | D43 | D86~D1210 | |
| 59381 ~ 60505 | D86~D1210 | D44 | D86~D1210 | |
| 60506 ~ 61630 | D86~D1210 | D45 | D86~D1210 | |
| 61631 ~ 62755 | D86~D1210 | D46 | D86~D1210 | |
| 62756 ~ 63880 | D86~D1210 | D47 | D86~D1210 | |
| 63881 ~ 65005 | D86~D1210 | D48 | D86~D1210 | |
| 65006 ~ 66130 | D86~D1210 | D49 | D86~D1210 | |
| 66131 ~ 67255 | D86~D1210 | D50 | D86~D1210 | |
| 67256 ~ 68380 | D86~D1210 | D51 | D86~D1210 | |
| 68381 ~ 69505 | D86~D1210 | D52 | D86~D1210 | |
| 69506 ~ 70630 | D86~D1210 | D53 | D86~D1210 | |
| 70631 ~ 71755 | D86~D1210 | D54 | D86~D1210 | |
| 71756 ~ 72880 | D86~D1210 | D55 | D86~D1210 | |
| 72881 ~ 74005 | D86~D1210 | D56 | D86~D1210 | |
| 74006 ~ 75130 | D86~D1210 | D57 | D86~D1210 | |
| 75131 ~ 76255 | D86~D1210 | D58 | D86~D1210 | |

(continued)

| No. | $R^3$ | $R^4$ | $R^5$ | = |
|---|---|---|---|---|
| 76256 ~ 77380 | D86~D1210 | D59 | D86~D1210 | |
| 77381 ~ 78505 | D86~D1210 | D60 | D86~D1210 | |
| 78506 ~ 79630 | D86~D1210 | D61 | D86~D1210 | |
| 79631 ~ 80755 | D86~D1210 | D62 | D86~D1210 | |
| 80756 ~ 81880 | D86~D1210 | D63 | D86~D1210 | |
| 81881 ~ 83005 | D86~D1210 | D64 | D86~D1210 | |
| 83006 ~ 84130 | D86~D1210 | D65 | D86~D1210 | |
| 84131 ~ 85255 | D86~D1210 | D66 | D86~D1210 | |
| 85256 ~ 86380 | D86~D1210 | D67 | D86~D1210 | |
| 86381 ~ 87505 | D86~D1210 | D68 | D86~D1210 | |
| 87506 ~ 88630 | D86~D1210 | D69 | D86~D1210 | |
| 88631 ~ 89755 | D86~D1210 | D70 | D86~D1210 | |
| 89756~ 90880 | D86~D1210 | D71 | D86~D1210 | |
| 90881~ 92005 | D86~D1210 | D72 | D86~D1210 | |
| 92006 ~ 93130 | D86~D1210 | D73 | D86~D1210 | |
| 93131 ~ 94255 | D86~D1210 | D74 | D86~D1210 | |
| 94256 ~ 95380 | D86~D1210 | D75 | D86~D1210 | |
| 95381 ~ 96505 | D86~D1210 | D76 | D86~D1210 | |
| 96506 ~ 97630 | D86~D1210 | D77 | D86~D1210 | |
| 97631 ~ 98755 | D86~D1210 | D78 | D86~D1210 | |
| 98756 ~ 99880 | D86~D1210 | D79 | D86~D1210 | |
| 99881 ~ 101005 | D86~D1210 | D80 | D86~D1210 | |
| 101006 ~ 102130 | D86~D1210 | D81 | D86~D1210 | |
| 102131 ~ 103255 | D86~D1210 | D82 | D86~D1210 | |
| 103256 ~ 104380 | D86~D1210 | D83 | D86~D1210 | |
| 104381 ~ 105505 | D86~D1210 | D84 | D86~D1210 | |
| 105506 ~ 106630 | D86~D1210 | D85 | D86~D1210 | $R^3 = R^5$ |
| 106631 ~ 107755 | D86~D1210 | Ar1 | D86~D1210 | |
| 107756 ~ 108880 | D86~D1210 | Ar14 | D86~D1210 | |
| 108881 ~ 110005 | D86~D1210 | Ar15 | D86~D1210 | |
| 110006 ~ 111130 | D86~D1210 | Ar39 | D86~D1210 | |
| 111131 ~ 112255 | D86~D1210 | Ar40 | D86~D1210 | |
| 112256 ~ 113380 | D86~D1210 | Ar47 | D86~D1210 | |
| 113381 ~ 114505 | H | D86~D1210 | D86~D1210 | |
| 114506 ~ 115630<br>115631 ~ 116755 | D1<br>D2 | D86~D1210<br>D86~D1210 | D86~D1210<br>D86~D1210 | |
| 116756 ~ 117880 | D3 | D86~D1210 | D86~D1210 | |
| 117881 ~ 119005 | D4 | D86~D1210 | D86~D1210 | |
| 119006 ~ 120130 | D5 | D86~D1210 | D86~D1210 | |
| 120131 ~ 121255 | D6 | D86~D1210 | D86~D1210 | |

(continued)

| No. | $R^3$ | $R^4$ | $R^5$ | = |
|---|---|---|---|---|
| 121256 ~ 122380 | D7 | D86~D1210 | D86~D1210 | $R^4 = R^5$ |
| 122381 ~ 123505 | D8 | D86~D1210 | D86~D1210 | |
| 123506 ~ 124630 | D9 | D86~D1210 | D86~D1210 | |
| 124631 ~ 125755 | D10 | D86~D1210 | D86~D1210 | |
| 125756 ~ 126880 | D11 | D86~D1210 | D86~D1210 | |
| 126881 ~ 128005<br>128006 ~ 129130 | D12<br>D13 | D86~D1210<br>D86~D1210 | D86~D1210<br>D86~D1210 | |
| 129131 ~ 130255 | D14 | D86~D1210 | D86~D1210 | |
| 130256 ~ 131380 | D15 | D86~D1210 | D86~D1210 | |
| 131381 ~ 132505 | D16 | D86~D1210 | D86~D1210 | |
| 132506 ~ 133630 | D17 | D86~D1210 | D86~D1210 | |
| 133631 ~ 134755 | D18 | D86~D1210 | D86~D1210 | |
| 134756 ~ 135880 | D19 | D86~D1210 | D86~D1210 | |
| 135881 ~ 137005 | D20 | D86~D1210 | D86~D1210 | |
| 137006 ~ 138130 | D21 | D86~D1210 | D86~D1210 | |
| 138131 ~ 139255 | D22 | D86~D1210 | D86~D1210 | |
| 139256 ~ 140380 | D23 | D86~D1210 | D86~D1210 | |
| 140381 ~ 141505 | D24 | D86~D1210 | D86~D1210 | |
| 141506 ~ 142630 | D25 | D86~D1210 | D86~D1210 | |
| 142631 ~ 143755 | D26 | D86~D1210 | D86~D1210 | |
| 143756 ~ 144880 | D27 | D86~D1210 | D86~D1210 | |
| 144881 ~ 146005 | D28 | D86~D1210 | D86~D1210 | |
| 146006 ~ 147130 | D29 | D86~D1210 | D86~D1210 | |
| 147131 ~ 148255 | D30 | D86~D1210 | D86~D1210 | |
| 148256 ~ 149380 | D31 | D86~D1210 | D86~D1210 | |
| 149381 ~ 150505 | D32 | D86~D1210 | D86~D1210 | |
| 150506 ~ 151630 | D33 | D86~D1210 | D86~D1210 | |
| 151631 ~ 152755 | D34 | D86~D1210 | D86~D1210 | |
| 152756 ~ 153880 | D35 | D86~D1210 | D86~D1210 | |
| 153881 ~ 155005<br>155006 ~ 156130 | D36<br>D37 | D86~D1210<br>D86~D1210 | D86~D1210<br>D86~D1210 | |
| 156131 ~ 157255 | D38 | D86~D1210 | D86~D1210 | |
| 157256 ~ 158380 | D39 | D86~D1210 | D86~D1210 | |
| 158381 ~ 159505 | D40 | D86~D1210 | D86~D1210 | |
| 159506 ~ 160630 | D41 | D86~D1210 | D86~D1210 | |
| 160631 ~ 161755 | D42 | D86~D1210 | D86~D1210 | |
| 161756 ~ 162880 | D43 | D86~D1210 | D86~D1210 | |
| 162881 ~ 164005 | D44 | D86~D1210 | D86~D1210 | |
| 164006 ~ 165130 | D45 | D86~D1210 | D86~D1210 | |
| 165131 ~ 166255 | D46 | D86~D1210 | D86~D1210 | |

(continued)

| No. | $R^3$ | $R^4$ | $R^5$ | = |
|---|---|---|---|---|
| 166256 ~ 167380 | D47 | D86~D1210 | D86~D1210 | |
| 167381 ~ 168505 | D48 | D86~D1210 | D86~D1210 | |
| 168506 ~ 169630 | D49 | D86~D1210 | D86~D1210 | |
| 169631 ~ 170755 | D50 | D86~D1210 | D86~D1210 | $R^4 = R^5$ |
| 170756 ~ 171880 | D51 | D86~D1210 | D86~D1210 | |
| 171881 ~ 173005 | D52 | D86~D1210 | D86~D1210 | |
| 173006 ~ 174130 | D53 | D86~D1210 | D86~D1210 | |
| 174131 ~ 175255 | D54 | D86~D1210 | D86~D1210 | |
| 175256 ~ 176380 | D55 | D86~D1210 | D86~D1210 | |
| 176381 ~ 177505 | D56 | D86~D1210 | D86~D1210 | |
| 177506 ~ 178630 | D57 | D86~D1210 | D86~D1210 | |
| 178631 ~ 179755 | D58 | D86~D1210 | D86~D1210 | |
| 179756 ~ 180880 | D59 | D86~D1210 | D86~D1210 | |
| 180881 ~ 182005 | D60 | D86~D1210 | D86~D1210 | |
| 182006 ~ 183130 | D61 | D86~D1210 | D86~D1210 | |
| 183131 ~ 184255 | D62 | D86~D1210 | D86~D1210 | |
| 184256 ~ 185380 | D63 | D86~D1210 | D86~D1210 | |
| 185381 ~ 186505 | D64 | D86~D1210 | D86~D1210 | |
| 186506 ~ 187630 | D65 | D86~D1210 | D86~D1210 | |
| 187631 ~ 188755 | D66 | D86~D1210 | D86~D1210 | |
| 188756 ~ 189880 | D67 | D86-D1210 | D86~D1210 | |
| 189881 ~ 191005 | D68 | D86~D1210 | D86~D1210 | |
| 191006 ~ 192130 | D69 | D86~D1210 | D86~D1210 | |
| 192131 ~ 193255 | D70 | D86~D1210 | D86~D1210 | |
| 193256 ~ 194380 | D71 | D86~D1210 | D86~D1210 | |
| 194381 ~ 195505 | D72 | D86~D1210 | D86~D1210 | |
| 195506 ~ 196630 | D73 | D86~D1210 | D86~D1210 | |
| 196631 ~ 197755 | D74 | D86~D1210 | D86~D1210 | |
| 197756 ~ 198880 | D75 | D86~D1210 | D86~D1210 | |
| 198881 ~ 200005 | D76 | D86~D1210 | D86~D1210 | |
| 200006 ~ 201130 | D77 | D86~D1210 | D86~D1210 | |
| 201131 ~ 202255 | D78 | D86~D1210 | D86~D1210 | |
| 202256 ~ 203380 | D79 | D86~D1210 | D86~D1210 | |
| 203381 ~ 204505 | D80 | D86~D1210 | D86~D1210 | |
| 204506 ~ 205630<br>205631 ~ 206755 | D81<br>D82 | D86~D1210<br>D86~D1210 | D86~D1210<br>D86~D1210 | |
| 206756 ~ 207880 | D83 | D86~D1210 | D86~D1210 | |
| 207881 ~ 209005 | D84 | D86-D1210 | D86~D1210 | |
| 209006 ~ 210130 | D85 | D86~D1210 | D86~D1210 | |
| 210131 ~ 211255 | Ar1 | D86~D1210 | D86~D1210 | |

(continued)

| No. | $R^3$ | $R^4$ | $R^5$ | = |
|---|---|---|---|---|
| 211256 ~ 212380 | Ar14 | D86~D1210 | D86~D1210 | |
| 212381 ~ 213505 | Ar15 | D86~D1210 | D86~D1210 | |
| 213506 ~ 214630 | Ar39 | D86~D1210 | D86~D1210 | |
| 214631 ~ 215755 | Ar40 | D86~D1210 | D86~D1210 | |
| 215756 ~ 216880 | Ar47 | D86~D1210 | D86~D1210 | |
| 216881 ~ 218005 | D86~D1210 | D86~D1210 | H | $R^3 = R^4$ |
| 218006 ~ 219130 | D86~D1210 | D86~D1210 | D1 | |
| 219131 ~ 220255 | D86~D1210 | D86~D1210 | D2 | |
| 220256 ~ 221380 | D86~D1210 | D86~D1210 | D3 | |
| 221381 ~ 222505 | D86~D1210 | D86~D1210 | D4 | |
| 222506 ~ 223630 | D86~D1210 | D86~D1210 | D5 | |
| 223631 ~ 224755 | D86~D1210 | D86~D1210 | D6 | |
| 224756 ~ 225880 | D86~D1210 | D86~D1210 | D7 | |
| 225881 ~ 227005 | D86~D1210 | D86~D1210 | D8 | |
| 227006 ~ 228130 | D86~D1210 | D86~D1210 | D9 | |
| 228131 ~ 229255 | D86~D1210 | D86~D1210 | D10 | |
| 229256 ~ 230380 | D86~D1210 | D86~D1210 | D11 | |
| 230381 ~ 231505 | D86~D1210 | D86~D1210 | D12 | |
| 231506 ~ 232630 | D86~D1210 | D86~D1210 | D13 | |
| 232631 ~ 233755 | D86~D1210 | D86~D1210 | D14 | |
| 233756 ~ 234880 | D86~D1210 | D86~D1210 | D15 | |
| 234881 ~ 236005 | D86~D1210 | D86~D1210 | D16 | |
| 236006 ~ 237130 | D86~D1210 | D86~D1210 | D17 | |
| 237131 ~ 238255 | D86~D1210 | D86~D1210 | D18 | |
| 238256 ~ 239380 | D86~D1210 | D86~D1210 | D19 | |
| 239381 ~ 240505 | D86~D1210 | D86~D1210 | D20 | |
| 240506 ~ 241630 | D86~D1210 | D86~D1210 | D21 | |
| 241631 ~ 242755 | D86~D1210 | D86~D1210 | D22 | |
| 242756 ~ 243880 | D86~D1210 | D86~D1210 | D23 | |
| 243881 ~ 245005 | D86~D1210 | D86~D1210 | D24 | |
| 245006 ~ 246130 | D86~D1210 | D86~D1210 | D25 | |
| 246131 ~ 247255 | D86~D1210 | D86~D1210 | D26 | |
| 247256 ~ 248380 | D86~D1210 | D86~D1210 | D27 | |
| 248381 ~ 249505 | D86~D1210 | D86~D1210 | D28 | |
| 249506 ~ 250630 | D86~D1210 | D86~D1210 | D29 | |
| 250631 ~ 251755 | D86~D1210 | D86~D1210 | D30 | |
| 251756 ~ 252880 | D86~D1210 | D86~D1210 | D31 | |
| 252881 ~ 254005 | D86~D1210 | D86~D1210 | D32 | |
| 254006 ~ 255130 | D86~D1210 | D86~D1210 | D33 | |

(continued)

| No. | $R^3$ | $R^4$ | $R^5$ | = |
|---|---|---|---|---|
| 255131 ~ 256255 | D86~D1210 | D86~D1210 | D34 | $R^3 = R^4$ |
| 256256 ~ 257380 | D86~D1210 | D86~D1210 | D35 | |
| 257381 ~ 258505 | D86~D1210 | D86~D1210 | D36 | |
| 258506 ~ 259630 | D86~D1210 | D86~D1210 | D37 | |
| 259631 ~ 260755 | D86~D1210 | D86~D1210 | D38 | |
| 260756 ~ 261880 | D86~D1210 | D86~D1210 | D39 | |
| 261881 ~ 263005 | D86~D1210 | D86~D1210 | D40 | |
| 263006 ~ 264130 | D86~D1210 | D86~D1210 | D41 | |
| 264131 ~ 265255 | D86~D1210 | D86~D1210 | D42 | |
| 265256 ~ 266380 | D86~D1210 | D86~D1210 | D43 | |
| 266381 ~ 267505 | D86~D1210 | D86~D1210 | D44 | |
| 267506 ~ 268630 | D86~D1210 | D86~D1210 | D45 | |
| 268631 ~ 269755 | D86~D1210 | D86~D1210 | D46 | |
| 269756 ~ 270880 | D86~D1210 | D86~D1210 | D47 | |
| 270881 ~ 272005 | D86~D1210 | D86~D1210 | D48 | |
| 272006 ~ 273130 | D86~D1210 | D86~D1210 | D49 | |
| 273131 ~ 274255 | D86~D1210 | D86~D1210 | D50 | |
| 274256 ~ 275380 | D86~D1210 | D86~D1210 | D51 | |
| 275381 ~ 276505 | D86~D1210 | D86~D1210 | D52 | |
| 276506 ~ 277630 | D86~D1210 | D86~D1210 | D53 | |
| 277631 ~ 278755 | D86~D1210 | D86~D1210 | D54 | |
| 278756 ~ 279880 | D86~D1210 | D86~D1210 | D55 | |
| 279881 ~ 281005 | D86~D1210 | D86~D1210 | D56 | |
| 281006 ~ 282130 | D86~D1210 | D86~D1210 | D57 | |
| 282131 ~ 283255 | D86~D1210 | D86~D1210 | D58 | |
| 283256 ~ 284380 | D86~D1210 | D86~D1210 | D59 | |
| 284381 ~ 285505 | D86~D1210 | D86~D1210 | D60 | |
| 285506 ~ 286630 | D86~D1210 | D86~D1210 | D61 | |
| 286631 ~ 287755 | D86~D1210 | D86~D1210 | D62 | |
| 287756 ~ 288880 | D86~D1210 | D86~D1210 | D63 | |
| 288881 ~ 290005 | D86~D1210 | D86~D1210 | D64 | |
| 290006 ~ 291130 | D86~D1210 | D86~D1210 | D65 | |
| 291131 ~ 292255 | D86~D1210 | D86~D1210 | D66 | |
| 292256 ~ 293380 | D86~D1210 | D86~D1210 | D67 | |
| 293381 ~ 294505 | D86~D1210 | D86~D1210 | D68 | |
| 294506 ~ 295630 | D86~D1210 | D86~D1210 | D69 | |
| 295631 ~ 296755 | D86~D1210 | D86~D1210 | D70 | |
| 296756 ~ 297880 | D86~D1210 | D86~D1210 | D71 | |
| 297881 ~ 299005 | D86~D1210 | D86~D1210 | D72 | |

(continued)

| No. | $R^3$ | $R^4$ | $R^5$ | = |
|---|---|---|---|---|
| 299006 ~ 300130 | D86~D1210 | D86~D1210 | D73 | |
| 300131 ~ 301255 | D86~D1210 | D86~D1210 | D74 | |
| 301256 ~ 302380 | D86~D1210 | D86~D1210 | D75 | |
| 302381 ~ 303505 | D86~D1210 | D86~D1210 | D76 | |
| 303506 ~ 304630 | D86~D1210 | D86~D1210 | D77 | |
| 304631 ~ 305755 | D86~D1210 | D86~D1210 | D78 | |
| 305756 ~ 306880 | D86~D1210 | D86~D1210 | D79 | |
| 306881 ~ 308005 | D86~D1210 | D86~D1210 | D80 | |
| 308006 ~ 309130 | D86~D1210 | D86~D1210 | D81 | |
| 309131 ~ 310255 | D86~D1210 | D86~D1210 | D82 | |
| 310256 ~ 311380 | D86~D1210 | D86~D1210 | D83 | |
| 311381 ~ 312505 | D86~D1210 | D86~D1210 | D84 | |
| 312506 ~ 313630 | D86~D1210 | D86~D1210 | D85 | |
| 313631 ~ 314755 | D86~D1210 | D86~D1210 | Ar1 | $R^3 = R^4$ |
| 314756 ~ 315880 | D86~D1210 | D86~D1210 | Ar14 | |
| 315881 ~ 317005 | D86~D1210 | D86~D1210 | Ar15 | |
| 317006 ~ 318130 | D86~D1210 | D86~D1210 | Ar39 | |
| 318131 ~ 319255 | D86~D1210 | D86~D1210 | Ar40 | |
| 319256 ~ 320380 | D86~D1210 | D86~D1210 | Ar47 | |
| 320381 ~ 321505 | D1 | D86~D1210 | D1 | |
| 321506 ~ 322630 | D2 | D86~D1210 | D2 | |
| 322631 ~ 323755 | D3 | D86~D1210 | D3 | |
| 323756 ~ 324880 | D4 | D86~D1210 | D4 | |
| 324881 ~ 326005 | D5 | D86~D1210 | D5 | |
| 326006 ~ 327130 | D6 | D86~D1210 | D6 | |
| 327131 ~ 328255 | D7 | D86~D1210 | D7 | |
| 328256 ~ 329380 | D8 | D86~D1210 | D8 | |
| 329381 ~ 330505 | D9 | D86~D1210 | D9 | |
| 330506 ~ 331630 | D10 | D86~D1210 | D10 | |
| 331631 ~ 332755 | D11 | D86~D1210 | D11 | |
| 332756 ~ 333880 | D12 | D86~D1210 | D12 | |
| 333881 ~ 335005 | D13 | D86~D1210 | D13 | |
| 335006 ~ 336130 | D14 | D86~D1210 | D14 | |
| 336131 ~ 337255 | D15 | D86~D1210 | D15 | $R^3 = R^5$ |
| 337256 ~ 338380 | D16 | D86~D1210 | D16 | |
| 338381 ~ 339505 | D17 | D86~D1210 | D17 | |
| 339506 ~ 340630 | D18 | D86~D1210 | D18 | |
| 340631 ~ 341755 | D19 | D86~D1210 | D19 | |
| 341756 ~ 342880 | D20 | D86~D1210 | D20 | |

(continued)

| No. | $R^3$ | $R^4$ | $R^5$ | = |
|---|---|---|---|---|
| 342881 ~ 344005 | D21 | D86~D1210 | D21 | |
| 344006 ~ 345130 | D22 | D86~D1210 | D22 | |
| 345131 ~ 346255 | D23 | D86~D1210 | D23 | |
| 346256 ~ 347380 | D24 | D86~D1210 | D24 | |
| 347381 ~ 348505 | D25 | D86~D1210 | D25 | |
| 348506 ~ 349630 | D26 | D86~D1210 | D26 | |
| 349631 ~ 350755 | D27 | D86~D1210 | D27 | |
| 350756 ~ 351880 | D28 | D86~D1210 | D28 | |
| 351881 ~ 353005 | D29 | D86~D1210 | D29 | |
| 353006 ~ 354130 | D30 | D86~D1210 | D30 | |
| 354131 ~ 355255 | D31 | D86~D1210 | D31 | |
| 355256 ~ 356380 | D32 | D86~D1210 | D32 | |
| 356381 ~ 357505 | D33 | D86~D1210 | D33 | |
| 357506 ~ 358630 | D34 | D86~D1210 | D34 | |
| 358631 ~ 359755 | D35 | D86~D1210 | D35 | |
| 359756 ~ 360880 | D36 | D86~D1210 | D36 | |
| 360881 ~ 362005 | D37 | D86~D1210 | D37 | |
| 362006 ~ 363130 | D38 | D86~D1210 | D38 | |
| 363131 ~ 364255 | D39 | D86~D1210 | D39 | |
| 364256 ~ 365380 | D40 | D86~D1210 | D40 | |
| 365381 ~ 366505 | D41 | D86~D1210 | D41 | |
| 366506 ~ 367630 | D42 | D86~D1210 | D42 | |
| 367631 ~ 368755 | D43 | D86~D1210 | D43 | |
| 368756 ~ 369880 | D44 | D86~D1210 | D44 | |
| 369881 ~ 371005 | D45 | D86~D1210 | D45 | |
| 371006 ~ 372130 | D46 | D86~D1210 | D46 | |
| 372131 ~ 373255 | D47 | D86~D1210 | D47 | |
| 373256 ~ 374380 | D48 | D86~D1210 | D48 | |
| 374381 ~ 375505 | D49 | D86~D1210 | D49 | |
| 375506 ~ 376630 | D50 | D86~D1210 | D50 | |
| 376631 ~ 377755 | D51 | D86~D1210 | D51 | |
| 377756 ~ 378880 | D52 | D86~D1210 | D52 | |
| 378881 ~ 380005 | D53 | D86~D1210 | D53 | |
| 380006 ~ 381130 | D54 | D86~D1210 | D54 | |
| 381131 ~ 382255 | D55 | D86~D1210 | D55 | |
| 382256 ~ 383380 | D56 | D86~D1210 | D56 | |
| 383381 ~ 384505 | D57 | D86~D1210 | D57 | |
| 384506 ~ 385630 | D58 | D86~D1210 | D58 | |
| 385631 ~ 386755 | D59 | D86~D1210 | D59 | |

(continued)

| No. | $R^3$ | $R^4$ | $R^5$ | = |
|---|---|---|---|---|
| 386756 ~ 387880 | D60 | D86~D1210 | D60 | $R^3 = R^5$ |
| 387881 ~ 389005 | D61 | D86~D1210 | D61 | |
| 389006 ~ 390130<br>390131 ~ 391255 | D62<br>D63 | D86~D1210<br>D86~D1210 | D62<br>D63 | |
| 391256 ~ 392380 | D64 | D86~D1210 | D64 | |
| 392381 ~ 393505 | D65 | D86~D1210 | D65 | |
| 393506 ~ 394630 | D66 | D86~D1210 | D66 | |
| 394631 ~ 395755 | D67 | D86~D1210 | D67 | |
| 395756 ~ 396880 | D68 | D86~D1210 | D68 | |
| 396881 ~ 398005 | D69 | D86~D1210 | D69 | |
| 398006 ~ 399130 | D70 | D86~D1210 | D70 | |
| 399131 ~ 400255 | D71 | D86~D1210 | D71 | |
| 400256 ~ 401380 | D72 | D86~D1210 | D72 | |
| 401381 ~ 402505 | D73 | D86~D1210 | D73 | |
| 402506 ~ 403630 | D74 | D86~D1210 | D74 | |
| 403631 ~ 404755 | D75 | D86~D1210 | D75 | |
| 404756 ~ 405880 | D76 | D86~D1210 | D76 | |
| 405881 ~ 407005 | D77 | D86~D1210 | D77 | |
| 407006 ~ 408130 | D78 | D86~D1210 | D78 | |
| 408131 ~ 409255 | D79 | D86~D1210 | D79 | |
| 409256 ~ 410380 | D80 | D86~D1210 | D80 | |
| 410381 ~ 411505 | D81 | D86~D1210 | D81 | |
| 411506 ~ 412630 | D82 | D86~D1210 | D82 | |
| 412631 ~ 413755 | D83 | D86~D1210 | D83 | |
| 413756 ~ 414880 | D84 | D86~D1210 | D84 | |
| 414881 ~ 416005 | D85 | D86~D1210 | D85 | |
| 416006 ~ 417130 | Ar1 | D86~D1210 | Ar1 | |
| 417131 ~ 418255 | Ar14 | D86~D1210 | Ar14 | |
| 418256 ~ 419380 | Ar15 | D86~D1210 | Ar15 | |
| 419381 ~ 420505 | Ar39 | D86~D1210 | Ar39 | |
| 420506 ~ 421630 | Ar40 | D86~D1210 | Ar40 | |
| 421631 ~ 422755 | Ar47 | D86~D1210 | Ar47 | |
| 422756 ~ 423879 | D88 | D86, D87, D89~D1210 | D88 | $R^3 = R^5$ |
| 423880 ~ 425003 | D106 | D86~D105,<br>D107~D1210 | D106 | |
| 425004 ~ 426127 | D119 | D86~D118,<br>D120~D1210 | D119 | |
| 426128 ~ 427251 | D471 | D86~D470,<br>D472~D1210 | D471 | |
| 427252 ~ 428375 | D705 | D86~D704,<br>D706~D1210 | D705 | |

(continued)

| No. | $R^3$ | $R^4$ | $R^5$ | = |
|---|---|---|---|---|
| 428376～429499 | D737 | D86~D736, D738~D1210 | D737 | |
| 429500～430623 | D760 | D86~D759, D761~D1210 | D760 | |
| 430624～431747 | D763 | D86~D762, D764~D1210 | D763 | |
| 431748～432871 | D783 | D86~D782, D784~D1210 | D783 | |
| 432872～433995 | D86, D87, D89~D1210 | D88 | D86, D87, D89~D1210 | $R^3 = R^5$ |
| 433996～435119 | D86~D105, D107~D1210 | D106 | D86~D105, D107~D1210 | |
| 435120～436243 | D86~D118, D120~D1210 | D119 | D86~D118, D120~D1210 | |
| 436244～437367 | D86~D470, D472~D1210 | D471 | D86~D470, D472~D1210 | |
| 437368～438491 | D86~D704, D706~D1210 | D705 | D86~D704, D706~D1210 | |
| 438492～439615 | D86~D736, D738~D1210 | D737 | D86~D736, D738~D1210 | |
| 439616～440739 | D86~D759, D761~D1210 | D760 | D86~D759, D761~D1210 | |
| 440740～441863 | D86~D762, D764~D1210 | D763 | D86~D762, D764~D1210 | |
| 441864～442987 | D86~D782, D784~D1210 | D783 | D86~D782, D784~D1210 | |

Next, specific examples of the compound having a structure represented by the following general formula (1b) are shown in Table 3. In Table 3, structures of the compounds are shown in the same manner as in Table 2.

General Formula (1b)

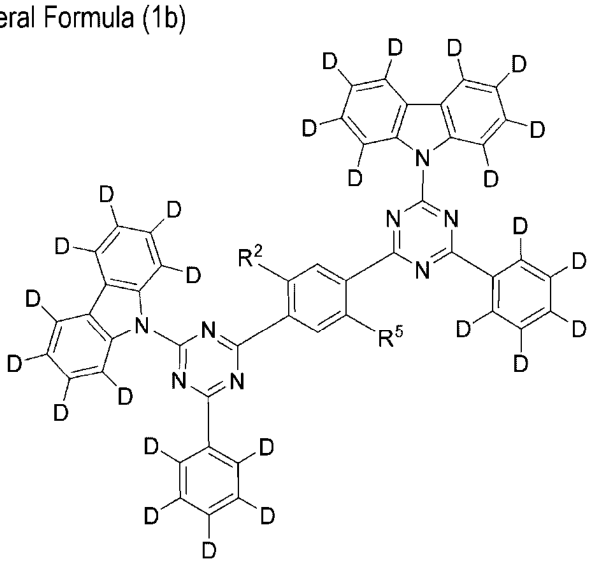

[Table 3]

| No. | $R^2$ | $R^5$ | = |
|---|---|---|---|
| 442988 ~ 444197 | D1~D1210 | D1~D1210 | $R^2 = R^5$ |
| 444198 ~ 445322 | D86~D1210 | D1 | $R^2 \neq R^5$ |
| 445323 ~ 446447 | D86~D1210 | D2 | |
| 446448 ~ 447572 | D86~D1210 | D3 | |
| 447573 ~ 448697 | D86~D1210 | D4 | |
| 448698 ~ 449822 | D86~D1210 | D5 | |
| 449823 ~ 450947 | D86~D1210 | D6 | |
| 450948 ~ 452072 | D86~D1210 | D7 | |
| 452073 ~ 453197 | D86~D1210 | D8 | |
| 453198 ~ 454322 | D86~D1210 | D9 | |
| 454323 ~ 455447 | D86~D1210 | D10 | |
| 455448 ~ 456572 | D86~D1210 | D11 | |
| 456573 ~ 457697 | D86~D1210 | D12 | |
| 457698 ~ 458822 | D86~D1210 | D13 | |
| 458823 ~ 459947 | D86~D1210 | D14 | |
| 459948 ~ 461072 | D86~D1210 | D15 | |
| 461073 ~ 462197 | D86~D1210 | D16 | |
| 462198 ~ 463322 | D86~D1210 | D17 | |
| 463323 ~ 464447 | D86~D1210 | D18 | |
| 464448 ~ 465572 | D86~D1210 | D19 | |
| 465573 ~ 466697 | D86~D1210 | D20 | |
| 466698 ~ 467822 | D86~D1210 | D21 | |
| 467823 ~ 468947 | D86~D1210 | D22 | |
| 468948 ~ 470072 | D86~D1210 | D23 | |
| 470073 ~ 471197 | D86~D1210 | D24 | |
| 471198 ~ 472322 | D86~D1210 | D25 | |
| 472323 ~ 473447 | D86~D1210 | D26 | |
| 473448 ~ 474572 | D86~D1210 | D27 | |
| 474573 ~ 475697 | D86~D1210 | D28 | |
| 475698 ~ 476822 | D86~D1210 | D29 | |
| 476823 ~ 477947 | D86~D1210 | D30 | |
| 477948 ~ 479072 | D86~D1210 | D31 | |
| 479073 ~ 480197 | D86~D1210 | D32 | |
| 480198 ~ 481322 | D86~D1210 | D33 | |
| 481323 ~ 482447 | D86~D1210 | D34 | |
| 482448 ~ 483572 | D86~D1210 | D35 | |
| 483573 ~ 484697 | D86~D1210 | D36 | |
| 484698 ~ 485822 | D86~D1210 | D37 | |
| 485823 ~ 486947 | D86~D1210 | D38 | |

(continued)

| No. | $R^2$ | $R^5$ | = |
|---|---|---|---|
| 486948 ~ 488072 | D86~D1210 | D39 | |
| 488073 ~ 489197 | D86~D1210 | D40 | |
| 489198 ~ 490322 | D86~D1210 | D41 | |
| 490323 ~ 491447 | D86~D1210 | D42 | |
| 491448 ~ 492572 | D86~D1210 | D43 | |
| 492573 ~ 493697 | D86~D1210 | D44 | |
| 493698 ~ 494822 | D86~D1210 | D45 | |
| 494823 ~ 495947 | D86~D1210 | D46 | |
| 495948 ~ 497072 | D86~D1210 | D47 | |
| 497073 ~ 498197 | D86~D1210 | D48 | |
| 498198 ~ 499322 | D86~D1210 | D49 | |
| 499323 ~ 500447 | D86~D1210 | D50 | |
| 500448 ~ 501572 | D86~D1210 | D51 | |
| 501573 ~ 502697 | D86~D1210 | D52 | |
| 502698 ~ 503822 | D86~D1210 | D53 | |
| 503823 ~ 504947 | D86~D1210 | D54 | |
| 504948 ~ 506072 | D86~D1210 | D55 | |
| 506073 ~ 507197 | D86~D1210 | D56 | |
| 507198 ~ 508322 | D86~D1210 | D57 | |
| 508323 ~ 509447 | D86~D1210 | D58 | |
| 509448 ~ 510572 | D86~D1210 | D59 | |
| 510573 ~ 511697 | D86~D1210 | D60 | $R^2 \neq R^5$ |
| 511698 ~ 512822 | D86~D1210 | D61 | |
| 512823 ~ 513947 | D86~D1210 | D62 | |
| 513948 ~ 515072 | D86~D1210 | D63 | |
| 515073 ~ 516197 | D86~D1210 | D64 | |
| 516198 ~ 517322 | D86~D1210 | D65 | |
| 517323 ~ 518447 | D86~D1210 | D66 | |
| 518448 ~ 519572 | D86~D1210 | D67 | |
| 519573 ~ 520697 | D86~D1210 | D68 | |
| 520698 ~ 521822 | D86~D1210 | D69 | |
| 521823 ~ 522947 | D86~D1210 | D70 | |
| 522948 ~ 524072 | D86~D1210 | D71 | |
| 524073 ~ 525197 | D86~D1210 | D72 | |
| 525198 ~ 526322 | D86~D1210 | D73 | |
| 526323 ~ 527447 | D86~D1210 | D74 | |
| 527448 ~ 528572 | D86~D1210 | D75 | |
| 528573 ~ 529697 | D86~D1210 | D76 | |
| 529698 ~ 530822 | D86~01210 | D77 | |
| 530823 ~ 531947 | D86~D1210 | D78 | |

(continued)

| No. | $R^2$ | $R^5$ | = |
|---|---|---|---|
| 531948 ~ 533072 | D86~D1210 | D79 | |
| 533073 ~ 534197 | D86~01210 | D80 | |
| 534198 ~ 535322 | D86~D1210 | D81 | |
| 535323 ~ 536447 | D86-01210 | D82 | |
| 536448 ~ 537572<br>537573 ~ 538697 | D86~D1210<br>D86~D1210 | D83<br>D84 | |
| 538698 ~ 539822 | D86~D1210 | D85 | |

Next, specific examples of the compound having a structure represented by the following general formula (1c) are shown in Table 4. In Table 4, structures of the compounds are shown in the same manner as in Table 2.

General Formula (1c)

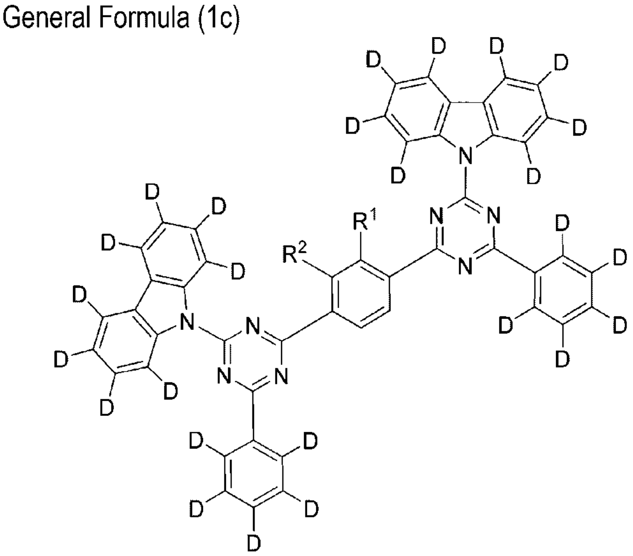

[Table 4]

| No. | $R^1$ | $R^2$ | = |
|---|---|---|---|
| 539823 ~ 541032 | D1~D1210 | D1~D1210 | $R^1 = R^2$ |
| 541033 ~ 542157 | D86~D1210 | D1 | |
| 542158 ~ 543282 | D86~D1210 | D2 | |
| 543283 ~ 544407 | D86~D1210 | D3 | |
| 544408 ~ 545532 | D86~D1210 | D4 | |
| 545533 ~ 546657 | D86~D1210 | D5 | |
| 546658 ~ 547782 | D86~D1210 | D6 | |
| 547783 ~ 548907 | D86~D1210 | D7 | |
| 548908 ~ 550032 | D86~D1210 | D8 | |
| 550033 ~ 551157 | D86~D1210 | D9 | |
| 551158 ~ 552282 | D86~D1210 | D10 | |
| 552283 ~ 553407 | D86~D1210 | D11 | |
| 553408 ~ 554532 | D86~D1210 | D12 | |

(continued)

| No. | $R^1$ | $R^2$ | = |
|---|---|---|---|
| 554533 ~ 555657 | D86~D1210 | D13 | |
| 555658 ~ 556782 | D86~D1210 | D14 | |
| 556783 ~ 557907 | D86~D1210 | D15 | |
| 557908 ~ 559032 | D86~D1210 | D16 | |
| 559033 ~ 560157 | D86~D1210 | D17 | |
| 560158 ~ 561282 | D86~D1210 | D18 | |
| 561283 ~ 562407 | D86~D1210 | D19 | |
| 562408 ~ 563532 | D86~D1210 | D20 | |
| 563533 ~ 564657 | D86~D1210 | D21 | |
| 564658 ~ 565782 | D86~D1210 | D22 | |
| 565783 ~ 566907 | D86~D1210 | D23 | |
| 566908 ~ 568032 | D86~D1210 | D24 | |
| 568033 ~ 569157 | D86~D1210 | D25 | |
| 569158 ~ 570282 | D86~D1210 | D26 | |
| 570283 ~ 571407 | D86~D1210 | D27 | |
| 571408 ~ 572532 | D86~D1210 | D28 | |
| 572533 ~ 573657 | D86~D1210 | D29 | |
| 573658 ~ 574782 | D86~D1210 | D30 | $R^1 \neq R^2$ |
| 574783 ~ 575907 | D86~D1210 | D31 | |
| 575908 ~ 577032 | D86~D1210 | D32 | |
| 577033 ~ 578157 | D86~D1210 | D33 | |
| 578158 ~ 579282 | D86~D1210 | D34 | |
| 579283 ~ 580407 | D86~D1210 | D35 | |
| 580408 ~ 581532 | D86~D1210 | D36 | |
| 581533 ~ 582657 | D86~D1210 | D37 | |
| 582658 ~ 583782 | D86~D1210 | D38 | |
| 583783 ~ 584907 | D86~D1210 | D39 | |
| 584908 ~ 586032 | D86~D1210 | D40 | |
| 586033 ~ 587157 | D86~D1210 | D41 | |
| 587158 ~ 588282 | D86~D1210 | D42 | |
| 588283 ~ 589407 | D86~D1210 | D43 | |
| 589408 ~ 590532 | D86~D1210 | D44 | |
| 590533 ~ 591657 | D86~D1210 | D45 | |
| 591658 ~ 592782<br>592783 ~ 593907 | D86~D1210<br>D86~D1210 | D46<br>D47 | |
| 593908 ~ 595032 | D86~D1210 | D48 | |
| 595033 ~ 596157 | D86~D1210 | D49 | |
| 596158 ~ 597282 | D86~D1210 | D50 | |
| 597283 ~ 598407 | D86~D1210 | D51 | |
| 598408 ~ 599532 | D86~D1210 | D52 | |

(continued)

| No. | $R^1$ | $R^2$ | = |
|---|---|---|---|
| 599533 ~ 600657 | D86~D1210 | D53 | |
| 600658 ~ 601782<br>601783 ~ 602907 | D86~D1210<br>D86~D1210 | D54<br>D55 | |
| 602908 ~ 604032 | D86~D1210 | D56 | |
| 604033 ~ 605157 | D86~D1210 | D57 | |
| 605158 ~ 606282 | D86~D1210 | D58 | |
| 606283 ~ 607407 | D86~D1210 | D59 | |
| 607408 ~ 608532 | D86~D1210 | D60 | $R^1 \neq R^2$ |
| 608533 ~ 609657 | D86~D1210 | D61 | |
| 609658 ~ 610782 | D86~D1210 | D62 | |
| 610783 ~ 611907 | D86~D1210 | D63 | |
| 611908 ~ 613032 | D86~D1210 | D64 | |
| 613033 ~ 614157 | D86~D1210 | D65 | |
| 614158 ~ 615282 | D86~D1210 | D66 | |
| 615283 ~ 616407 | D86~D1210 | D67 | |
| 616408 ~ 617532 | D86~D1210 | D68 | |
| 617533 ~ 618657 | D86~D1210 | D69 | |
| 618658 ~ 619782 | D86~D1210 | D70 | |
| 619783 ~ 620907 | D86~D1210 | D71 | |
| 620908 ~ 622032 | D86~D1210 | D72 | |
| 622033 ~ 623157 | D86~D1210 | D73 | |
| 623158 ~ 624282 | D86~D1210 | D74 | |
| 624283 ~ 625407 | D86~D1210 | D75 | |
| 625408 ~ 626532 | D86~D1210 | D76 | |
| 626533 ~ 627657 | D86~D1210 | D77 | |
| 627658 ~ 628782 | D86~D1210 | D78 | |
| 628783 ~ 629907 | D86~D1210 | D79 | |
| 629908 ~ 631032 | D86~D1210 | D80 | |
| 631033 ~ 632157 | D86~D1210 | D81 | |
| 632158 ~ 633282 | D86~D1210 | D82 | |
| 633283 ~ 634407 | D86~D1210 | D83 | |
| 634408 ~ 635532 | D86~D1210 | D84 | |
| 635533 ~ 636657 | D86~D1210 | D85 | |

Next, specific examples of the compound having a structure represented by the following general formula (1d) are shown in Table 5. In Table 5, structures of the compounds are shown in the same manner as in Table 2.

General Formula (1d)

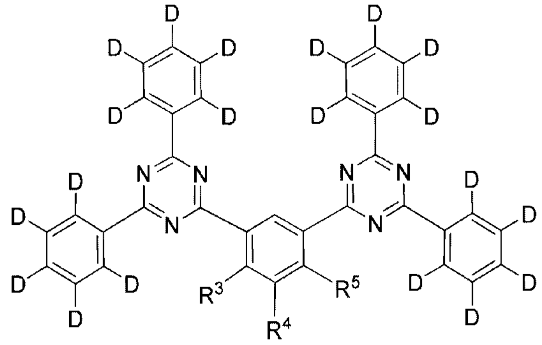

[Table 5]

| No. | $R^3$ | $R^4$ | $R^5$ | = |
|---|---|---|---|---|
| 636658 ~ 637782 | D86~D1210 | D86~D1210 | D86~D1210 | $R^3 = R^4 = R^5$ |
| 637783 ~ 638907 | D86~D1210 | H | D86~D1210 | |
| 638908 ~ 640032 | D86~D1210 | D1 | D86~D1210 | |
| 640033 ~ 641157 | D86~D1210 | D2 | D86~D1210 | |
| 641158 ~ 642282 | D86~D1210 | D3 | D86~D1210 | |
| 642283 ~ 643407 | D86~D1210 | D4 | D86~D1210 | |
| 643408 ~ 644532 | D86~D1210 | D5 | D86~D1210 | |
| 644533 ~ 645657 | D86~D1210 | D6 | D86~D1210 | |
| 645658 ~ 646782 | D86~D1210 | D7 | D86~D1210 | |
| 646783 ~ 647907 | D86~D1210 | D8 | D86~D1210 | |
| 647908 ~ 649032 | D86~D1210 | D9 | D86~D1210 | |
| 649033 ~ 650157 | D86~D1210 | D10 | D86~D1210 | |
| 650158 ~ 651282 | D86~D1210 | D11 | D86~D1210 | |
| 651283 ~ 652407 | D86~D1210 | D12 | D86~D1210 | |
| 652408 ~ 653532 | D86~D1210 | D13 | D86~D1210 | |
| 653533 ~ 654657 | D86~D1210 | D14 | D86~D1210 | |
| 654658 ~ 655782 | D86~D1210 | D15 | D86~D1210 | |
| 655783 ~ 656907 | D86~D1210 | D16 | D86~D1210 | |
| 656908 ~ 658032 | D86~D1210 | D17 | D86~D1210 | |
| 658033 ~ 659157 | D86~D1210 | D18 | D86~D1210 | |
| 659158 ~ 660282 | D86~D1210 | D19 | D86~D1210 | |
| 660283 ~ 661407 | D86~D1210 | D20 | D86~D1210 | |
| 661408 ~ 662532 | D86~D1210 | D21 | D86~D1210 | |
| 662533 ~ 663657 | D86~D1210 | D22 | D86~D1210 | |
| 663658 ~ 664782 | D86~D1210 | D23 | D86~D1210 | |
| 664783 ~ 665907 | D86~D1210 | D24 | D86~D1210 | |
| 665908 ~ 667032 | D86~D1210 | D25 | D86~D1210 | |
| 667033 ~ 668157 | D86~D1210 | D26 | D86~D1210 | |
| 668158 ~ 669282 | D86~D1210 | D27 | D86~D1210 | |

(continued)

| No. | $R^3$ | $R^4$ | $R^5$ | = |
|---|---|---|---|---|
| 669283 ~ 670407 | D86~D1210 | D28 | D86~D1210 | $R^3 = R^5$ |
| 670408 ~ 671532 | D86~D1210 | D29 | D86~D1210 | |
| 671533 ~ 672657<br>672658 ~ 673782 | D86~D1210<br>D86~D1210 | D30<br>D31 | D86~D1210<br>D86~D1210 | |
| 673783 ~ 674907 | D86~D1210 | D32 | D86~D1210 | |
| 674908 ~ 676032 | D86~D1210 | D33 | D86~D1210 | |
| 676033 ~ 677157 | D86~D1210 | D34 | D86~D1210 | |
| 677158 ~ 678282 | D86~D1210 | D35 | D86~D1210 | |
| 678283 ~ 679407 | D86~D1210 | D36 | D86~D1210 | |
| 679408 ~ 680532 | D86~D1210 | D37 | D86~D1210 | |
| 680533 ~ 681657 | D86~D1210 | D38 | D86~D1210 | |
| 681658 ~ 682782 | D86~D1210 | D39 | D86~D1210 | |
| 682783 ~ 683907 | D86~D1210 | D40 | D86~D1210 | |
| 683908 ~ 685032 | D86~D1210 | D41 | D86~D1210 | |
| 685033 ~ 686157 | D86~D1210 | D42 | D86~D1210 | |
| 686158 ~ 687282 | D86~D1210 | D43 | D86~D1210 | |
| 687283 ~ 688407 | D86~D1210 | D44 | D86~D1210 | |
| 688408 ~ 689532 | D86~D1210 | D45 | D86~D1210 | |
| 689533 ~ 690657<br>690658 ~ 691782 | D86~D1210<br>D86~D1210 | D46<br>D47 | D86~D1210<br>D86~D1210 | |
| 691783 ~ 692907 | D86~D1210 | D48 | D86~D1210 | |
| 692908 ~ 694032 | D86~D1210 | D49 | D86~D1210 | |
| 694033 ~ 695157 | D86~D1210 | D50 | D86~D1210 | |
| 695158 ~ 696282 | D86~D1210 | D51 | D86~D1210 | |
| 696283 ~ 697407 | D86~D1210 | D52 | D86~D1210 | |
| 697408 ~ 698532 | D86~D1210 | D53 | D86~D1210 | |
| 698533 ~ 699657 | D86~D1210 | D54 | D86~D1210 | |
| 699658 ~ 700782<br>700783 ~ 701907 | D86~D1210<br>D86~D1210 | D55<br>D56 | D86~D1210<br>D86~D1210 | |
| 701908 ~ 703032 | D86~D1210 | D57 | D86~D1210 | |
| 703033 ~ 704157 | D86~D1210 | D58 | D86~D1210 | |
| 704158 ~ 705282 | D86~D1210 | D59 | D86~D1210 | |
| 705283 ~ 706407 | D86~D1210 | D60 | D86~D1210 | |
| 706408 ~ 707532 | D86~D1210 | D61 | D86~D1210 | |
| 707533 ~ 708657 | D86~D1210 | D62 | D86~D1210 | |
| 708658 ~ 709782 | D86~D1210 | D63 | D86~D1210 | |
| 709783 ~ 710907 | D86~D1210 | D64 | D86~D1210 | |
| 710908 ~ 712032 | D86~D1210 | D65 | D86~D1210 | |
| 712033 ~ 713157 | D86~D1210 | D66 | D86~D1210 | |
| 713158 - 714282 | D86~D1210 | D67 | D86~D1210 | |

(continued)

| No. | $R^3$ | $R^4$ | $R^5$ | = |
|---|---|---|---|---|
| 714283 ~ 715407 | D86~D1210 | D68 | D86~D1210 | $R^3 = R^5$ |
| 715408 ~ 716532 | D86~D1210 | D69 | D86~D1210 | |
| 716533 ~ 717657 | D86~D1210 | D70 | D86~D1210 | |
| 717658 ~ 718782 | D86~D1210 | D71 | D86~D1210 | |
| 718783 ~ 719907 | D86~D1210 | D72 | D86~D1210 | |
| 719908 ~ 721032 | D86~D1210 | D73 | D86~D1210 | |
| 721033 ~ 722157 | D86~D1210 | D74 | D86~D1210 | |
| 722158 ~ 723282 | D86~D1210 | D75 | D86~D1210 | |
| 723283 ~ 724407 | D86~D1210 | D76 | D86~D1210 | |
| **724408** ~ 725532 | D86~D1210 | D77 | D86~D1210 | |
| 725533 ~ 726657 | D86~D1210 | D78 | D86~D1210 | |
| 726658 ~ 727782 | D86~D1210 | D79 | D86~D1210 | |
| 727783 ~ 728907 | D86~D1210 | D80 | D86~D1210 | |
| 728908 ~ 730032 | D86~D1210 | D81 | D86~D1210 | |
| 730033 ~ 731157 | D86~D1210 | D82 | D86~D1210 | |
| 731158 ~ 732282 | D86~D1210 | D83 | D86~D1210 | |
| 732283 ~ 733407 | D86~D1210 | D84 | D86~D1210 | |
| 733408 ~ 734532 | D86~D1210 | D85 | D86~D1210 | |
| 734533 ~ 735657 | D86~D1210 | Ar1 | D86~D1210 | |
| 735658 ~ 736782 | D86~D1210 | Ar14 | D86~D1210 | |
| 736783 ~ 737907 | D86~D1210 | Ar15 | D86~D1210 | |
| 737908 ~ 739032 | D86~D1210 | Ar39 | D86~D1210 | |
| 739033 ~ 740157 | D86~D1210 | Ar40 | D86~D1210 | |
| 740158 ~ 741282 | D86~D1210 | Ar47 | D86~D1210 | |
| 741283 ~ 742407 | H | D86~D1210 | D86~D1210 | |
| 742408 ~ 743532 | D1 | D86~D1210 | D86~D1210 | |
| 743533 ~ 744657 | D2 | D86~D1210 | D86~D1210 | |
| 744658 ~ 745782 | D3 | D86~D1210 | D86~D1210 | |
| 745783 ~ 746907 | D4 | D86~D1210 | D86~D1210 | |
| 746908 ~ 748032 | D5 | D86~D1210 | D86~D1210 | |
| 748033 ~ 749157 | D6 | D86~D1210 | D86~D1210 | |
| 749158 ~ 750282 | D7 | D86~D1210 | D86~D1210 | |
| 750283 ~ 751407 | D8 | D86~D1210 | D86~D1210 | |
| 751408 ~ 752532 | D9 | D86~D1210 | D86~D1210 | |
| 752533 ~ 753657 | D10 | D86~D1210 | D86~D1210 | |
| 753658 ~ 754782 | D11 | D86~D1210 | D86~D1210 | |
| 754783 ~ 755907 | D12 | D86~D1210 | D86~D1210 | |
| 755908 ~ 757032 | D13 | D86 ~ D1210 | D86~D1210 | |
| 757033 - 758157 | D14 | D86~D1210 | D86~D1210 | |
| 758158 ~ 759282 | D15 | D86~D1210 | D86~D1210 | |

(continued)

| No. | $R^3$ | $R^4$ | $R^5$ | = |
|---|---|---|---|---|
| 759283 ~ 760407 | D16 | D86~D1210 | D86~D1210 | $R^4 = R^5$ |
| 760408 ~ 761532 | D17 | D86~D1210 | D86~D1210 | |
| 761533 ~ 762657 | D18 | D86~D1210 | D86~D1210 | |
| 762658 ~ 763782 | D19 | D86~D1210 | D86~D1210 | |
| 763783 ~ 764907 | D20 | D86~D1210 | D86~D1210 | |
| 764908 ~ 766032 | D21 | D86~D1210 | D86~D1210 | |
| 766033 ~ 767157 | D22 | D86~D1210 | D86~D1210 | |
| 767158 ~ 768282 | D23 | D86~D1210 | D86~D1210 | |
| 768283 ~ 769407 | D24 | D86~D1210 | D86~D1210 | |
| 769408 ~ 770532 | D25 | D86-D1210 | D86~D1210 | |
| 770533 ~ 771657 | D26 | D86~D1210 | D86~D1210 | |
| 771658 ~ 772782 | D27 | D86~D1210 | D86~D1210 | |
| 772783 ~ 773907 | D28 | D86~D1210 | D86~D1210 | |
| 773908 ~ 775032 | D29 | D86~D1210 | D86~D1210 | |
| 775033 ~ 776157 | D30 | D86~D1210 | D86~D1210 | |
| 776158 ~ 777282 | D31 | D86~D1210 | D86~D1210 | |
| 777283 ~ 778407 | D32 | D86~D1210 | D86~D1210 | |
| 778408 ~ 779532 | D33 | D86~D1210 | D86~D1210 | |
| 779533 ~ 780657 | D34 | D86~D1210 | D86~D1210 | |
| 780658 ~ 781782 | D35 | D86~D1210 | D86~D1210 | |
| 781783 ~ 782907 | D36 | D86~D1210 | D86~D1210 | |
| 782908 ~ 784032 | D37 | D86~D1210 | D86~D1210 | |
| 784033 ~ 785157 | D38 | D86~D1210 | D86~D1210 | |
| 785158 ~ 786282 | D39 | D86~D1210 | D86~D1210 | |
| 786283 ~ 787407 | D40 | D86~D1210 | D86~D1210 | |
| 787408 ~ 788532 | D41 | D86~D1210 | D86~D1210 | |
| 788533 ~ 789657 | D42 | D86~D1210 | D86~D1210 | |
| 789658 ~ 790782 | D43 | D86~D1210 | D86~D1210 | |
| 790783 ~ 791907 | D44 | D86~D1210 | D86~D1210 | |
| 791908 ~ 793032 | D45 | D86~D1210 | D86~D1210 | |
| 793033 ~ 794157 | D46 | D86~D1210 | D86~D1210 | |
| 794158 ~ 795282 | D47 | D86~D1210 | D86~D1210 | |
| 795283 ~ 796407 | D48 | D86~D1210 | D86~D1210 | |
| 796408 ~ 797532 | D49 | D86~D1210 | D86~D1210 | |
| 797533 ~ 798657 | D50 | D86~D1210 | D86~D1210 | |
| 798658 ~ 799782 | D51 | D86~D1210 | D86~D1210 | |
| 799783 ~ 800907 | D52 | D86~D1210 | D86~D1210 | |
| 800908 ~ 802032 | D53 | D86~D1210 | D86~D1210 | |
| 802033 ~ 803157 | D54 | D86~D1210 | D86~D1210 | |

(continued)

| No. | $R^3$ | $R^4$ | $R^5$ | = |
|---|---|---|---|---|
| 803158 ~ 804282 | D55 | D86~D1210 | D86~D1210 | $R^4 = R^5$ |
| 804283 ~ 805407 | D56 | D86~D1210 | D86~D1210 | |
| 805408 ~ 806532 | D57 | D86~D1210 | D86~D1210 | |
| 806533 ~ 807657 | D58 | D86~D1210 | D86~D1210 | |
| 807658 ~ 808782 | D59 | D86~D1210 | D86~D1210 | |
| 808783 ~ 809907 | D60 | D86~D1210 | D86~D1210 | |
| 809908 ~ 811032 | D61 | D86~D1210 | D86~D1210 | |
| 811033 ~ 812157 | D62 | D86~D1210 | D86~D1210 | |
| 812158 ~ 813282 | D63 | D86~D1210 | D86~D1210 | |
| 813283 ~ 814407 | D64 | D86~D1210 | D86~D1210 | |
| 814408 ~ 815532 | D65 | D86~D1210 | D86~D1210 | |
| 815533 ~ 816657 | D66 | D86~D1210 | D86~D1210 | |
| 816658 ~ 817782 | D67 | D86~D1210 | D86~D1210 | |
| 817783 ~ 818907 | D68 | D86~D1210 | D86~D1210 | |
| 818908 ~ 820032 | D69 | D86~D1210 | D86~D1210 | |
| 820033 ~ 821157 | D70 | D86~D1210 | D86~D1210 | |
| 821158 ~ 822282 | D71 | D86~D1210 | D86~D1210 | |
| 822283 ~ 823407 | D72 | D86~D1210 | D86~D1210 | |
| 823408 ~ 824532 | D73 | D86~D1210 | D86~D1210 | |
| 824533 ~ 825657 | D74 | D86~D1210 | D86~D1210 | |
| 825658 ~ 826782 | D75 | D86~D1210 | D86~D1210 | |
| 826783 ~ 827907 | D76 | D86~D1210 | D86~D1210 | |
| 827908 ~ 829032 | D77 | D86~D1210 | D86~D1210 | |
| 829033 ~ 830157 | D78 | D86~D1210 | D86~D1210 | |
| 830158 ~ 831282 | D79 | D86~D1210 | D86~D1210 | |
| 831283 ~ 832407 | D80 | D86~D1210 | D86~D1210 | |
| 832408 ~ 833532 | D81 | D86~D1210 | D86~D1210 | |
| 833533 ~ 834657 | D82 | D86~D1210 | D86~D1210 | |
| 834658 ~ 835782 | D83 | D86~D1210 | D86~D1210 | |
| 835783 ~ 836907 | D84 | D86~D1210 | D86~D1210 | |
| 836908 ~ 838032 | D85 | D86~D1210 | D86~D1210 | |
| 838033 - 839157 | Ar1 | D86~D1210 | D86~D1210 | |
| 839158 ~ 840282 | Ar14 | D86~D1210 | D86~D1210 | |
| 840283 ~ 841407 | Ar15 | D86~D1210 | D86~D1210 | |
| 841408 ~ 842532 | Ar39 | D86~D1210 | D86~D1210 | |
| 842533 ~ 843657 | Ar40 | D86~D1210 | D86~D1210 | |
| 843658 ~ 844782 | Ar47 | D86~D1210 | D86~D1210 | $R^4 = R^5$ |
| 844783 ~ 845907 | D86~D1210 | D86~D1210 | H | |
| 845908 ~ 847032 | D86~D1210 | D86~D1210 | D1 | |

(continued)

| No. | $R^3$ | $R^4$ | $R^5$ | = |
|---|---|---|---|---|
| 847033 ~ 848157 | D86~D1210 | D86~D1210 | D2 | |
| 848158 ~ 849282 | D86~D1210 | D86~D1210 | D3 | |
| 849283 ~ 850407 | D86~D1210 | D86~D1210 | D4 | |
| 850408 ~ 851532 | D86~D1210 | D86~D1210 | D5 | |
| 851533 ~ 852657 | D86~D1210 | D86~D1210 | D6 | |
| 852658 ~ 853782 | D86~D1210 | D86~D1210 | D7 | |
| 853783 ~ 854907 | D86~D1210 | D86~D1210 | D8 | |
| 854908 ~ 856032 | D86~D1210 | D86~D1210 | D9 | |
| 856033 ~ 857157 | D86~D1210 | D86 ~ D1210 | D10 | |
| 857158 ~ 858282 | D86~D1210 | D86 ~ D1210 | D11 | |
| 858283 ~ 859407 | D86~D1210 | D86~D1210 | D12 | |
| 859408 ~ 860532 | D86~D1210 | D86~D1210 | D13 | |
| 860533 ~ 861657 | D86~D1210 | D86 ~ D1210 | D14 | |
| 861658 ~ 862782 | D86~D1210 | D86~D1210 | D15 | |
| 862783 ~ 863907 | D86~D1210 | D86~D1210 | D16 | |
| 863908 ~ 865032 | D86~D1210 | D86~D1210 | D17 | |
| 865033 ~ 866157 | D86~D1210 | D86~D1210 | D18 | |
| 866158 ~ 867282 | D86~D1210 | D86~D1210 | D19 | |
| 867283 ~ 868407 | D86~D1210 | D86~D1210 | D20 | |
| 868408 ~ 869532 | D86~D1210 | D86~D1210 | D21 | |
| 869533 ~ 870657 | D86~D1210 | D86~D1210 | D22 | |
| 870658 ~ 871782 | D86~D1210 | D86~D1210 | D23 | |
| 871783 ~ 872907 | D86~D1210 | D86~D1210 | D24 | |
| 872908 ~ 874032 | D86~D1210 | D86~D1210 | D25 | |
| 874033 ~ 875157 | D86~D1210 | D86~D1210 | D26 | |
| 875158 ~ 876282 | D86~D1210 | D86~D1210 | D27 | |
| 876283 ~ 877407 | D86~D1210 | D86~D1210 | D28 | |
| 877408 ~ 878532 | D86~D1210 | D86~D1210 | D29 | |
| 878533 ~ 879657 | D86~D1210 | D86~D1210 | D30 | $R^3 = R^4$ |
| 879658 ~ 880782 | D86~D1210 | D86~D1210 | D31 | |
| 880783 ~ 881907 | D86~D1210 | D86~D1210 | D32 | |
| 881908 ~ 883032 | D86~D1210 | D86~D1210 | D33 | |
| 883033 ~ 884157 | D86~D1210 | D86~D1210 | D34 | |
| 884158 ~ 885282 | D86~D1210 | D86~D1210 | D35 | |
| 885283 ~ 886407 | D86~D1210 | D86 ~ D1210 | D36 | |
| 886408 ~ 887532 | D86~D1210 | D86~D1210 | D37 | |
| 887533 ~ 888657 | D86~D1210 | D86~D1210 | D38 | |
| 888658 ~ 889782 | D86~D1210 | D86~D1210 | D39 | |
| 889783 ~ 890907 | D86~D1210 | D86~D1210 | D40 | |

(continued)

| No. | $R^3$ | $R^4$ | $R^5$ | = |
|---|---|---|---|---|
| 890908 ~ 892032 | D86~D1210 | D86~D1210 | D41 | |
| 892033 ~ 893157 | D86~D1210 | D86~D1210 | D42 | |
| 893158 ~ 894282 | D86~D1210 | D86~D1210 | D43 | |
| 894283 ~ 895407 | D86~D1210 | D86~D1210 | D44 | |
| 895408 ~ 896532 | D86~D1210 | D86~D1210 | D45 | |
| 896533 ~ 897657 | D86~D1210 | D86~D1210 | D46 | |
| 897658 ~ 898782 | D86~D1210 | D86~D1210 | D47 | |
| 898783 ~ 899907 | D86~D1210 | D86~D1210 | D48 | |
| 899908 ~ 901032 | D86~D1210 | D86~D1210 | D49 | |
| 901033 ~ 902157 | D86~D1210 | D86~D1210 | D50 | |
| 902158 ~ 903282 | D86~D1210 | D86~D1210 | D51 | |
| 903283 ~ 904407 | D86~D1210 | D86~D1210 | D52 | |
| 904408 ~ 905532 | D86~D1210 | D86~D1210 | D53 | |
| 905533 ~ 906657 | D86~D1210 | D86~D1210 | D54 | |
| 906658 ~ 907782 | D86~D1210 | D86~D1210 | D55 | |
| 907783 ~ 908907 | D86~D1210 | D86~D1210 | D56 | |
| 908908 ~ 910032 | D86~D1210 | D86~D1210 | D57 | |
| 910033 ~ 911157 | D86~D1210 | D86~D1210 | D58 | |
| 911158 ~ 912282 | D86~D1210 | D86~D1210 | D59 | |
| 912283 ~ 913407 | D86~D1210 | D86~D1210 | D60 | |
| 913408 ~ 914532 | D86~D1210 | D86~D1210 | D61 | |
| 914533 ~ 915657 | D86~D1210 | D86~D1210 | D62 | |
| 915658 ~ 916782 | D86~D1210 | D86~D1210 | D63 | |
| 916783 ~ 917907 | D86~D1210 | D86~D1210 | D64 | |
| 917908 ~ 919032 | D86~D1210 | D86~D1210 | D65 | |
| 919033 ~ 920157 | D86~D1210 | D86~D1210 | D66 | |
| 920158 ~ 921282 | D86~D1210 | D86~D1210 | D67 | |
| 921283 ~ 922407 | D86~D1210 | D86~D1210 | D68 | |
| 922408 ~ 923532 | D86~D1210 | D86~D1210 | D69 | |
| 923533 ~ 924657 | D86~D1210 | D86~D1210 | D70 | |
| 924658 ~ 925782 | D86~D1210 | D86~D1210 | D71 | |
| 925783 ~ 926907 | D86~D1210 | D86~D1210 | D72 | |
| 926908 ~ 928032 | D86~D1210 | D86~D1210 | D73 | |
| 928033 ~ 929157 | D86~D1210 | D86~D1210 | D74 | |
| 929158 ~ 930282 | D86~D1210 | D86~D1210 | D75 | |
| 930283 ~ 931407 | D86~D1210 | D86~D1210 | D76 | $R^3 = R^4$ |
| 931408 ~ 932532 | D86~D1210 | D86~D1210 | D77 | |
| 932533 ~ 933657 | D86~D1210 | D86~D1210 | D78 | |
| 933658 ~ 934782 | D86~D1210 | D86~D1210 | D79 | |

(continued)

| No. | $R^3$ | $R^4$ | $R^5$ | = |
|---|---|---|---|---|
| 934783 ~ 935907 | D86~D1210 | D86~D1210 | D80 | |
| 935908 ~ 937032 | D86~D1210 | D86~D1210 | D81 | |
| 937033 ~ 938157 | D86~D1210 | D86~D1210 | D82 | |
| 938158 ~ 939282 | D86~D1210 | D86~D1210 | D83 | |
| 939283 ~ 940407 | D86~D1210 | D86~D1210 | D84 | |
| 940408 ~ 941532 | D86~D1210 | D86~D1210 | D85 | |
| 941533 ~ 942657 | D86~D1210 | D86~D1210 | Ar1 | |
| 942658 ~ 943782 | D86~D1210 | D86~D1210 | Ar14 | |
| 943783 ~ 944907 | D86~D1210 | D86~D1210 | Ar15 | |
| 944908 ~ 946032 | D86~D1210 | D86~D1210 | Ar39 | |
| 946033 ~ 947157 | D86~D1210 | D86~D1210 | Ar40 | |
| 947158 ~ 948282<br>948283 ~ 949407 | D86~D1210<br>D1 | D86~D1210<br>D86~D1210 | Ar47<br>D1 | |
| 949408 ~ 950532 | D2 | D86~D1210 | D2 | |
| 950533 ~ 951657 | D3 | D86~D1210 | D3 | |
| 951658 ~ 952782 | D4 | D86~D1210 | D4 | |
| 952783 ~ 953907 | D5 | D86~D1210 | D5 | |
| 953908 ~ 955032 | D6 | D86~D1210 | D6 | |
| 955033 ~ 956157 | D7 | D86~D1210 | D7 | |
| 956158 ~ 957282 | D8 | D86~D1210 | D8 | |
| 957283 ~ 958407 | D9 | D86~D1210 | D9 | |
| 958408 ~ 959532 | D10 | D86~D1210 | D10 | |
| 959533 ~ 960657 | D11 | D86~D1210 | D11 | |
| 960658 ~ 961782 | D12 | D86~D1210 | D12 | |
| 961783 ~ 962907 | D13 | D86~D1210 | D13 | |
| 962908 ~ 964032<br>964033 ~ 965157 | D14<br>D15 | D86~D1210<br>D86~D1210 | D14<br>D15 | |
| 965158 ~ 966282 | D16 | D86~D1210 | D16 | $R^3 = R^5$ |
| 966283 ~ 967407 | D17 | D86~D1210 | D17 | |
| 967408 ~ 968532 | D18 | D86~D1210 | D18 | |
| 968533 ~ 969657 | D19 | D86~D1210 | D19 | |
| 969658 - 970782 | D20 | D86~D1210 | D20 | |
| 970783 ~ 971907 | D21 | D86~D1210 | D21 | |
| 971908 ~ 973032 | D22 | D86~D1210 | D22 | |
| 973033 ~ 974157 | D23 | D86~D1210 | D23 | |
| 974158 ~ 975282 | D24 | D86~D1210 | D24 | |
| 975283 ~ 976407 | D25 | D86~D1210 | D25 | |
| 976408 ~ 977532 | D26 | D86~D1210 | D26 | |
| 977533 ~ 978657<br>978658 ~ 979782 | D27<br>D28 | D86~D1210<br>D86~D1210 | D27<br>D28 | |

(continued)

| No. | $R^3$ | $R^4$ | $R^5$ | = |
|---|---|---|---|---|
| 979783 ~ 980907 | D29 | D86~D1210 | D29 | |
| 980908 ~ 982032 | D30 | D86~D1210 | D30 | |
| 982033 ~ 983157 | D31 | D86~D1210 | D31 | |
| 983158 ~ 984282 | D32 | D86~D1210 | D32 | $R^3 = R^5$ |
| 984283 ~ 985407 | D33 | D86~D1210 | D33 | |
| 985408 ~ 986532 | D34 | D86~D1210 | D34 | |
| 986533 ~ 987657 | D35 | D86~D1210 | D35 | |
| 987658 ~ 988782 | D36 | D86~D1210 | D36 | |
| 988783 ~ 989907 | D37 | D86~D1210 | D37 | |
| 989908 ~ 991032 | D38 | D86~D1210. | D38 | |
| 991033 ~ 992157 | D39 | D86~D1210 | D39 | |
| 992158 ~ 993282 | D40 | D86~D1210 | D40 | |
| 993283 ~ 994407 | D41 | D86~D1210 | D41 | |
| 994408 ~ 995532 | D42 | D86~D1210 | D42 | |
| 995533 ~ 996657 | D43 | D86~D1210 | D43 | |
| 996658 ~ 997782 | D44 | D86~D1210 | D44 | |
| 997783 ~ 998907 | D45 | D86~D1210 | D45 | |
| 998908 ~ 1000032 | D46 | D86~D1210 | D46 | |
| 1000033 ~ 1001157 | D47 | D86~D1210 | D47 | |
| 1001158 ~ 1002282 | D48 | D86~D1210 | D48 | |
| 1002283 ~ 1003407 | D49 | D86~D1210 | D49 | |
| 1003408 ~ 1004532 | D50 | D86~D1210 | D50 | |
| 1004533 ~ 1005657 | D51 | D86~D1210 | D51 | |
| 1005658 ~ 1006782 | D52 | D86~D1210 | D52 | |
| 1006783 ~ 1007907 | D53 | D86~D1210 | D53 | |
| 1007908 ~ 1009032 | D54 | D86~D1210 | D54 | |
| 1009033 ~ 1010157 | D55 | D86~D1210 | D55 | |
| 1010158 ~ 1011282 | D56 | D86~D1210 | D56 | |
| 1011283 ~ 1012407 | D57 | D86~D1210 | D57 | |
| 1012408 ~ 1013532 | D58 | D86~D1210 | D58 | |
| 1013533 ~ 1014657 | D59 | D86~D1210 | D59 | |
| 1014658 ~ 1015782 | D60 | D86~D1210 | D60 | |
| 1015783 ~ 1016907 | D61 | D86~D1210 | D61 | |
| 1016908 ~ 1018032 | D62 | D86~D1210 | D62 | |
| 1018033 ~ 1019157 | D63 | D86~D1210 | D63 | |
| 1019158 ~ 1020282 | D64 | D86~D1210 | D64 | |
| 1020283 ~ 1021407 | D65 | D86~D1210 | D65 | |
| 1021408 ~ 1022532 | D66 | D86~D1210 | D66 | |
| 1022533 ~ 1023657 | D67 | D86~D1210 | D67 | |

(continued)

| No. | $R^3$ | $R^4$ | $R^5$ | = |
|---|---|---|---|---|
| 1023658 ~ 1024782 | D68 | D86~D1210 | D68 | |
| 1024783 ~ 1025907 | D69 | D86~D1210 | D69 | |
| 1025908 ~ 1027032 | D70 | D86~D1210 | D70 | |
| 1027033 ~ 1028157 | D71 | D86~D1210 | D71 | |
| 1028158 ~ 1029282 | D72 | D86~D1210 | D72 | |
| 1029283 ~ 1030407 | D73 | D86~D1210 | D73 | |
| 1030408 ~ 1031532 | D74 | D86~D1210 | D74 | |
| 1031533 ~ 1032657 | D75 | D86~D1210 | D75 | |
| 1032658 ~ 1033782 | D76 | D86~D1210 | D76 | |
| 1033783 ~ 1034907 | D77 | D86~D1210 | D77 | |
| 1034908 ~ 1036032 | D78 | D86~D1210 | D78 | |
| 1036033 ~ 1037157 | D79 | D86~D1210 | D79 | |
| 1037158 ~ 1038282 | D80 | D86~D1210 | D80 | |
| 1038283 ~ 1039407 | D81 | D86~D1210 | D81 | |
| 1039408 ~ 1040532 | D82 | D86~D1210 | D82 | |
| 1040533 ~ 1041657 | D83 | D86~D1210 | D83 | |
| 1041658 ~ 1042782 | D84 | D86~D1210 | D84 | |
| 1042783 ~ 1043907 | D85 | D868~D1210 | D85 | |
| 1043908 ~ 1045031 | D88 | D86, D87, D89~D1210 | D88 | $R^3 = R^5$ |
| 1045032 ~ 1046155 | D106 | D86~D105, D107~D1210 | D106 | |
| 1046156 ~ 1047279 | D119 | D86~D118, D120~D1210 | D119 | |
| 1047280 ~ 1048403 | D471 | D86~D470, D472~D1210 | D471 | |
| 1048404 ~ 1049527 | D705 | D86~D704, D706~D1210 | D705 | |
| 1049528 ~ 1050651 | D737 | D86~D736. D738~D1210 | D737 | |
| 1050652 ~ 1051775 | D760 | D86~D759, D761~D1210 | D760 | |
| 1051776 ~ 1052899 | D763 | D86~D762, D764~D1210 | D763 | |
| 1052900 ~ 1054023 | D783 | D86~D782, D784~D1210 | D783 | |

| No. | $R^3$ | $R^4$ | $R^5$ | = |
|---|---|---|---|---|
| 1054024 ~ 1055147 | D86, D87, D89~D1210 | D88 | D86, D87, D89~D1210 | $R^3 = R^5$ |
| 1055148 ~ 1056271 | D86~D105, D107~D1210 | D106 | D86~D105, D107~D1210 | |
| 1056272 ~ 1057395 | D86~D118, D120~D1210 | D119 | D86~D118, D120~D1210 | |
| 1057396 ~ 1058519 | D86~D470, D472~D1210 | D471 | D86~D470, D472~D1210 | |
| 1058520 ~ 1059643 | D86~D704, D706~D1210 | D705 | D86~D704, D706~D1210 | |
| 1059644 ~ 1060767 | D86~D736, D738~D1210 | D737 | D86~D736, D738~D1210 | |
| 1060768 ~ 1061891 | D86~D759, D761~D1210 | D760 | D86~D759, D761~D1210 | |
| 1061892 ~ 1063015 | D86~D762, D764~D1210 | D763 | D86~D762, D764~D1210 | |
| 1063016 ~ 1064139 | D86~D782, D784~D1210 | D783 | D86~D782, D784~D1210 | |

Next, specific examples of the compound having a structure represented by the following general formula (1e) are shown in Table 6. In Table 6, structures of the compounds are shown in the same manner as in Table 2.

General Formula (1e)

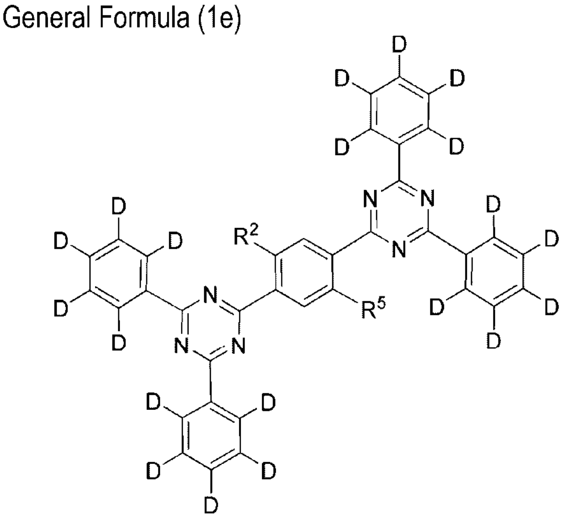

[Table 6]

| No. | $R^2$ | $R^5$ | = |
|---|---|---|---|
| 1064140 ~ 1065264 | D86 ~ D1210 | D86~D1210 | $R^2 = R^5$ |
| 1065265 ~ 1066389 | D86~D1210 | D1 | |
| 1066390 ~ 1067514 | D86~D1210 | D2 | |
| 1067515 ~ 1068639 | D86~D1210 | D3 | |
| 1068640 ~ 1069764 | D86~D1210 | D4 | |
| 1069765 ~ 1070889 | D86~D1210 | D5 | |
| 1070890 ~ 1072014 | D86~D1210 | D6 | |
| 1072015 ~ 1073139 | D86~D1210 | D7 | |
| 1073140 ~ 1074264 | D86~D1210 | D8 | |
| 1074265 ~ 1075389 | D86~D1210 | D9 | |
| 1075390 ~ 1076514 | D86~D1210 | D10 | |
| 1076515 ~ 1077639 | D86~D1210 | D11 | |
| 1077640 ~ 1078764 | D86~D1210 | D12 | |
| 1078765 ~ 1079889 | D86~D1210 | D13 | |
| 1079890 ~ 1081014 | D86~D1210 | D14 | |
| 1081015 ~ 1082139 | D86~D1210 | D15 | |
| 1082140 ~ 1083264 | D86~D1210 | D16 | |
| 1083265 ~ 1084389 | D86~D1210 | D17 | |
| 1084390 ~ 1085514 | D86~D1210 | D18 | |
| 1085515 ~ 1086639 | D86 ~ D1210 | D19 | |
| 1086640 ~ 1087764 | D86~D1210 | D20 | |
| 1087765 ~ 1088889 | D86~D1210 | D21 | |
| 1088890 ~ 1090014 | D86~D1210 | D22 | |
| 1090015 ~ 1091139 | D86~D1210 | D23 | |
| 1091140 ~ 1092264 | D86 ~ D1210 | D24 | |
| 1092265 ~ 1093389 | D86~D1210 | D25 | |

(continued)

| No. | $R^2$ | $R^5$ | = |
|---|---|---|---|
| 1093390 ~ 1094514 | D86~D1210 | D26 | $R^2 \neq R^5$ |
| 1094515 ~ 1095639 | D86~D1210 | D27 | |
| 1095640 ~ 1096764 | D86~D1210 | D28 | |
| 1096765 ~ 1097889 | D86~D1210 | D29 | |
| 1097890 ~ 1099014 | D86 ~ D1210 | D30 | |
| 1099015 ~ 1100139 | D86~D1210 | D31 | |
| 1100140 ~ 1101264 | D86~D1210 | D32 | |
| 1101265 ~ 1102389 | D86~D1210 | D33 | |
| 1102390 ~ 1103514 | D86~D1210 | D34 | |
| 1103515 ~ 1104639 | D86~D1210 | D35 | |
| 1104640 ~ 1105764 | D86 ~ D1210 | D36 | |
| 1105765 ~ 1106889 | D86 ~ D1210 | D37 | |
| 1106890 ~ 1108014 | D86~D1210 | D38 | |
| 1108015 ~ 1109139 | D86~D1210 | D39 | |
| 1109140 ~ 1110264 | D86~D1210 | D40 | |
| 1110265 ~ 1111389 | D86~D1210 | D41 | |
| 1111390 ~ 1112514 | D86~D1210 | D42 | |
| 1112515 ~ 1113639 | D86~D1210 | D43 | |
| 1113640 ~ 1114764 | D86~D1210 | D44 | |
| 1114765 ~ 1115889 | D86~D1210 | D45 | |
| 1115890 ~ 1117014 | D86~D1210 | D46 | |
| 1117015 ~ 1118139 | D86~D1210 | D47 | |
| 1118140 ~ 1119264 | D86~D1210 | D48 | |
| 1119265 ~ 1120389 | D86~D1210 | D49 | |
| 1120390 ~ 1121514 | D86~D1210 | D50 | |
| 1121515 ~ 1122639 | D86~D1210 | D51 | |
| 1122640 ~ 1123764 | D86~D1210 | D52 | |
| 1123765 ~ 1124889 | D86~D1210 | D53 | |
| 1124890 ~ 1126014 | D86 ~ D1210 | D54 | |
| 1126015 ~ 1127139 | D86~D1210 | D55 | |
| 1127140 ~ 1128264 | D86~D1210 | D56 | |
| 1128265 ~ 1129389 | D86~D1210 | D57 | |
| 1129390 ~ 1130514 | D86~D1210 | D58 | |
| 1130515 ~ 1131639 | D86~D1210 | D59 | |
| 1131640 ~ 1132764 | D86~D1210 | D60 | |
| 1132765 ~ 1133889 | D86~D1210 | D61 | |
| 1133890 ~ 1135014 | D86~D1210 | D62 | |
| 1135015 ~ 1136139 | D86~D1210 | D63 | |
| 1136140 ~ 1137264 | D86~D1210 | D64 | |

(continued)

| No. | $R^2$ | $R^5$ | = |
|---|---|---|---|
| 1137265 ~ 1138389 | D86~D1210 | D65 | $R^2 \neq R^5$ |
| 1138390 ~ 1139514 | D86~D1210 | D66 | |
| 1139515 ~ 1140639 | D86~D1210 | D67 | |
| 1140640 ~ 1141764 | D86~D1210 | D68 | |
| 1141765 ~ 1142889 | D86~D1210 | D69 | |
| 1142890 ~ 1144014 | D86~D1210 | D70 | |
| 1144015 ~ 1145139 | D86~D1210 | D71 | |
| 1145140 ~ 1146264 | D86~D1210 | D72 | |
| 1146265 ~ 1147389 | D86~D1210 | D73 | |
| 1147390 ~ 1148514 | D86~D1210 | D74 | |
| 1148515 ~ 1149639 | D86~D1210 | D75 | |
| 1149640 ~ 1150764 | D86~D1210 | D76 | |
| 1150765 ~ 1151889 | D86~D1210 | D77 | |
| 1151890 ~ 1153014 | D86~D1210 | D78 | |
| 1153015 ~ 1154139 | D86~D1210 | D79 | |
| 1154140 ~ 1155264 | D86~D1210 | D80 | |
| 1155265 ~ 1156389 | D86~D1210 | D81 | |
| 1156390 ~ 1157514 | D86~D1210 | D82 | |
| 1157515 ~ 1158639 | D86~D1210 | D83 | |
| 1158640 ~ 1159764 | D86~D1210 | D84 | |
| 1159765 ~ 1160889 | D86~D1210 | D85 | |

Next, specific examples of the compound having a structure represented by the following general formula (1f) are shown in Table 7. In Table 7, structures of the compounds are shown in the same manner as in Table 2.

General Formula (1f)

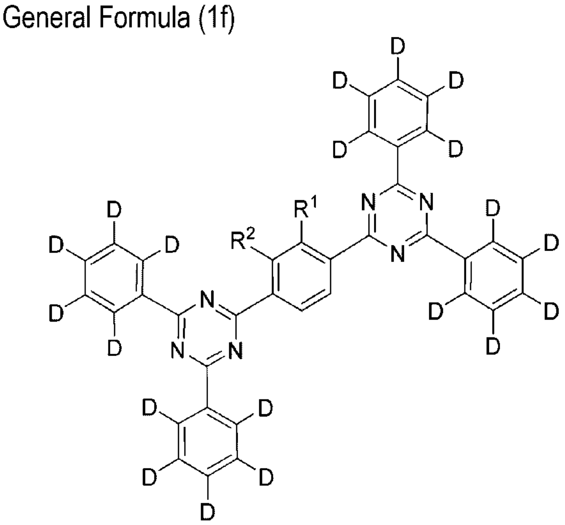

[Table 7]

| No. | $R^1$ | $R^2$ | = |
|---|---|---|---|
| 1160890 ~ 1162014 | D86~D1210 | D86~D1210 | $R^1 = R^2$ |
| 1162015 ~ 1163139 | D86~D1210 | D1 | $R^1 \neq R^2$ |
| 1163140 ~ 1164264 | D86~D1210 | D2 | |
| 1164265 ~ 1165389 | D86~D1210 | D3 | |
| 1165390 ~ 1166514 | D86~D1210 | D4 | |
| 1166515 ~ 1167639 | D86~D1210 | D5 | |
| 1167640 ~ 1168764 | D86~D1210 | D6 | |
| 1168765 ~ 1169889 | D86~D1210 | D7 | |
| 1169890 ~ 1171014 | D86~D1210 | D8 | |
| 1171015 ~ 1172139 | D86~D1210 | D9 | |
| 1172140 ~ 1173264 | D86-D1210 | D10 | |
| 1173265 ~ 1174389 | D86~D1210 | D11 | |
| 1174390 ~ 1175514 | D86~D1210 | D12 | |
| 1175515 ~ 1176639 | D86~D1210 | D13 | |
| 1176640 ~ 1177764 | D86~D1210 | D14 | |
| 1177765 ~ 1178889 | D86~D1210 | D15 | |
| 1178890 ~ 1180014 | D86~D1210 | D16 | |
| 1180015 ~ 1181139 | D86~D1210 | D17 | |
| 1181140 ~ 1182264 | D86~D1210 | D18 | |
| 1182265 ~ 1183389 | D86~D1210 | D19 | |
| 1183390 ~ 1184514 | D86~D1210 | D20 | |
| 1184515 ~ 1185639 | D86~D1210 | D21 | |
| 1185640 ~ 1186764 | D86~D1210 | D22 | |
| 1186765 ~ 1187889 | D86~D1210 | D23 | |
| 1187890 ~ 1189014 | D86~D1210 | D24 | |
| 1189015 ~ 1190139 | D86~D1210 | D25 | |
| 1190140 ~ 1191264 | D86~D1210 | D26 | |
| 1191265 ~ 1192389 | D86~D1210 | D27 | |
| 1192390 ~ 1193514 | D86~D1210 | D28 | |
| 1193515 ~ 1194639 | D86~D1210 | D29 | |
| 1194640 ~ 1195764 | D86~D1210 | D30 | |
| 1195765 ~ 1196889 | D86~D1210 | D31 | |
| 1196890 ~ 1198014 | D86-D1210 | D32 | |
| 1198015 ~ 1199139 | D86~D1210 | D33 | |
| 1199140 ~ 1200264 | D86~D1210 | D34 | |
| 1200265 ~ 1201389 | D86~D1210 | D35 | |
| 1201390 ~ 1202514 | D86~D1210 | D36 | |
| 1202515 ~ 1203639 | D86~D1210 | D37 | |
| 1203640 ~ 1204764 | D86~D1210 | D38 | |

(continued)

| No. | $R^1$ | $R^2$ | = |
|---|---|---|---|
| 1204765 ~ 1205889 | D86~D1210 | D39 | |
| 1205890 ~ 1207014 | D86~D1210 | D40 | |
| 1207015 ~ 1208139 | D86~D1210 | D41 | |
| 1208140 ~ 1209264 | D86~D1210 | D42 | |
| 1209265 ~ 1210389 | D86~D1210 | D43 | |
| 1210390 ~ 1211514 | D86~D1210 | D44 | |
| 1211515 ~ 1212639 | D86-D1210 | D45 | |
| 1212640 ~ 1213764 | D86~D1210 | D46 | |
| 1213765 ~ 1214889 | D86~D1210 | D47 | |
| 1214890 ~ 1216014 | D86~D1210 | D48 | |
| 1216015 ~ 1217139 | D86~D1210 | D49 | |
| 1217140 ~ 1218264 | D86~D1210 | D50 | |
| 1218265 ~ 1219389 | D86~D1210 | D51 | |
| 1219390 ~ 1220514 | D86~D1210 | D52 | |
| 1220515 ~ 1221639 | D86~D1210 | D53 | |
| 1221640 ~ 1222764 | D86~D1210 | D54 | |
| 1222765 ~ 1223889 | D86~D1210 | D55 | |
| 1223890 ~ 1225014 | D86~D1210 | D56 | |
| 1225015 ~ 1226139 | D86~D1210 | D57 | |
| 1226140 ~ 1227264 | D86~D1210 | D58 | |
| 1227265 ~ 1228389 | D86~D1210 | D59 | |
| 1228390 ~ 1229514 | D86~D1210 | D60 | $R^1 \neq R^2$ |
| 1229515 ~ 1230639 | D86~01210 | D61 | |
| 1230640 ~ 1231764 | D86~D1210 | D62 | |
| 1231765 ~ 1232889<br>1232890 ~ 1234014 | D86~D1210<br>D86~D1210 | D63<br>**D64** | |
| 1234015 ~ 1235139 | D86~D1210 | D65 | |
| 1235140 ~ 1236264<br>1236265 ~ 1237389 | D86~D1210<br>D86~01210 | D66<br>D67 | |
| 1237390 ~ 1238514 | D86~D1210 | D68 | |
| 1238515 ~ 1239639<br>1239640 ~ 1240764 | D86~D1210<br>D86~D1210 | D69<br>D70 | |
| 1240765 ~ 1241889 | D86~D1210 | D71 | |
| 1241890 ~ 1243014 | D86~D1210 | D72 | |
| 1243015 ~ 1244139 | D86~01210 | D73 | |
| 1244140 ~ 1245264 | D86~01210 | D74 | |
| 1245265 ~ 1246389 | D86~D1210 | D75 | |
| 1246390 ~ 1247514 | D86~D1210 | D76 | |
| 1247515 ~ 1248639 | D86~01210 | D77 | |
| 1248640 ~ 1249764 | D86~D1210 | D78 | |

(continued)

| No. | $R^1$ | $R^2$ | = |
|---|---|---|---|
| 1249765 ~ 1250889 | D86~D1210 | D79 | |
| 1250890 ~ 1252014 | D86~01210 | D80 | |
| 1252015 ~ 1253139 | D86~D1210 | D81 | |
| 1253140 ~ 1254264 | D86~D1210 | D82 | |
| 1254265 ~ 1255389 | D86~D1210 | D83 | |
| 1255390 ~ 1256514 | D86~D1210 | D84 | |
| 1256515 ~ 1257639 | D86~01210 | D85 | |

In Tables 1 to 4, structures in which $Ar^1$ and $Ar^3$ in the general formula (1) are perdeuterated carbazol-9-yl groups (D55) and $Ar^2$ and $Ar^4$ are perdeuterated phenyl groups (Ar47) were specified as structures of Compounds 1 to 636657. In Tables 5 to 7, structures in which $Ar^1$ to $Ar^4$ in the general formula (1) are perdeuterated phenyl groups (Ar47) were specified as structures of Compounds 636658 to 1257639.

In the following Table 8, together with these Compounds 1 to 1257639, compounds obtained by changing $Ar^1$ to $Ar^4$ of all or a part of Compounds 1 to 636657 as shown in Table 8 are shown in order in the form of a table. In Table 8, Compounds 1 to 636657 are shown in the first row and Compounds 636658 to 1257639 are shown in the second row for clarifying the correspondence relationship. In the third row of Table 8, the compounds in which both $Ar^1$ and $Ar^3$ of Compounds 1 to 636657 are D1 are referred to as Compounds 1(1) to 636657(1), respectively. For example, Compound 1(1) indicates a compound having a structure in which $Ar^1$ and $Ar^3$ of Compound 1 are substituted with D1. Compound 2(1) indicates a compound having a structure in which $Ar^1$ and $Ar^3$ of Compound 2 are substituted with D1. Compound 636657(1) indicates a compound having a structure in which $Ar^1$ and $Ar^3$ of Compound 636657 are substituted with D1. In the fourth row of Table 8, the compounds in which both $Ar^1$ and $Ar^3$ of Compounds 1 to 636657 are D7 are referred to as Compounds 1(2) to 636657(2), respectively. In this manner, compounds in which $Ar^1$ and $Ar^3$, and $Ar^2$ and $Ar^4$ of Compounds 1 to 636657 are listed in Table 8 are specified in order. $X^1$ to $X^3$ of the compounds specified in Table 8 all represent a nitrogen atom (N), $L^1$ represents a single bond (L1), and $R^1$ represents a hydrogen atom. $Ar^1$ and $Ar^3$ are the same, and $Ar^2$ and $Ar^4$ are the same.

[Table 8]

| No. | $Ar^1$,$Ar^3$ | $Ar^2$,$Ar^4$ |
|---|---|---|
| 1 ~ 636657 | D55 | Ar47 |
| 636658 ~ 1257639 | Ar47 | Ar47 |
| 1(1) ~ 636657(1) | D1 | Ar47 |
| 1(2) ~ 636657(2) | D7 | Ar47 |
| 1(3) ~ 636657(3) | D8 | Ar47 |
| 1(4) ~ 636657(4) | D9 | Ar47 |
| 1(5) ~ 636657(5) | D17 | Ar47 |
| 1(6) ~ 636657(6) | D27 | Ar47 |
| 1(7) ~ 636657(7) | D37 | Ar47 |
| 1(8) ~ 636657(8) | D39 | Ar47 |
| 1(9) ~ 636657(9) | D47 | Ar47 |
| 1(10) ~ 636657(10) | D51 | Ar47 |
| 1(11) ~ 636657(11) | D61 | Ar47 |
| 1(12) ~ 636657(12) | D62 | Ar47 |
| 1(13) ~ 636657(13) | D63 | Ar47 |
| 1(14) ~ 636657(14) | D71 | Ar47 |
| 1(15) ~ 636657(15) | D81 | Ar47 |

(continued)

| No. | $Ar^1$,$Ar^3$ | $Ar^2$,$Ar^4$ |
|---|---|---|
| 1(16) ~ 636657(16) | D88 | Ar47 |
| 1(17) ~ 636657(17) | D106 | Ar47 |
| 1(18) ~ 636657(18) | D119 | Ar47 |
| 1(19) ~ 636657(19)<br>1(20) ~ 636657(20) | D765<br>D1 | Ar47<br>Ar1 |
| 1(21) ~ 636657(21) | D7 | Ar1 |
| 1(22) ~ 636657(22) | D8 | Ar1 |
| 1(23) ~ 636657(23) | D9 | Ar1 |
| 1(24) ~ 636657(24) | D17 | Ar1 |
| 1(25) ~ 636657(25) | D27 | Ar1 |
| 1(26) ~ 636657(26) | D37 | Ar1 |
| 1(27) ~ 636657(27) | D39 | Ar1 |
| 1(28) ~ 636657(28) | D47 | Ar1 |
| 1(29) ~ 636657(29) | D51 | Ar1 |
| 1(30) ~ 636657(30) | D55 | Ar1 |
| 1(31) ~ 636657(31)<br>1(32) ~ 636657(32) | D61<br>D62 | Ar1<br>Ar1 |
| 1(33) ~ 636657(33) | D63 | Ar1 |
| 1(34) ~ 636657(34) | D71 | Ar1 |
| 1(35) ~ 636657(35) | D81 | Ar1 |
| 1(36) ~ 636657(36) | D88 | Ar1 |
| 1(37) ~ 636657(37) | D106 | Ar1 |
| 1(38) ~ 636657(38) | D119 | Ar1 |
| 1(39) ~ 636657(39) | D765 | Ar1 |
| 1(40) ~ 636657(40) | D1 | Ar10 |
| 1(41) ~ 636657(41)<br>1(42) ~ 636657(42) | D7<br>D8 | Ar10<br>Ar10 |
| 1(43) ~ 636657(43) | D9 | Ar10 |
| 1(44) - 636657(44) | D17 | Ar10 |
| 1(45) ~ 636657(45) | D27 | Ar10 |
| 1(46) ~ 636657(46) | D37 | Ar10 |
| 1(47) ~ 636657(47) | D39 | Ar10 |
| 1(48) ~ 636657(48) | D47 | Ar10 |
| 1(49) ~ 636657(49) | D51 | Ar10 |
| 1(50) ~ 636657(50) | D55 | Ar10 |
| 1(51) ~ 636657(51) | D61 | Ar10 |
| 1(52) ~ 636657(52) | D62 | Ar10 |
| 1(53) ~ 636657(53) | D63 | Ar10 |
| 1(54) ~ 636657(54) | D71 | Ar10 |
| 1(55) ~ 636657(55) | D81 | Ar10 |

(continued)

| No. | $Ar^1$,$Ar^3$ | $Ar^2$,$Ar^4$ |
|---|---|---|
| 1(56) ~ 636657(56) | D88 | Ar10 |
| 1(57) ~ 636657(57) | D106 | Ar10 |
| 1(58) ~ 636657(58) | D119 | Ar10 |
| 1(59) ~ 636657(59) | D765 | Ar10 |
| 1(60) ~ 636657(60) | D1 | Ar12 |
| 1(61) ~ 636657(61) | D7 | Ar12 |
| 1(62) ~ 636657(62) | D8 | Ar12 |
| 1(63) ~ 636657(63) | D9 | Ar12 |
| 1(64) ~ 636657(64) | D17 | Ar12 |
| 1(65) ~ 636657(65) | D27 | Ar12 |
| 1(66) ~ 636657(66) | D37 | Ar12 |
| 1(67) ~ 636657(67) | D39 | Ar12 |
| 1(68) ~ 636657(68) | D47 | Ar12 |
| 1(69) ~ 636657(69) | D51 | Ar12 |
| 1(70) ~ 636657(70) | D55 | Ar12 |
| 1(71) ~ 636657(71) | D61 | Ar12 |
| 1(72) ~ 636657(72) | D62 | Ar12 |
| 1(73) ~ 636657(73)<br>1(74) ~ 636657(74) | D63<br>D71 | Ar12<br>Ar12 |
| 1(75) ~ 636657(75) | D81 | Ar12 |
| 1(76) ~ 636657(76) | D88 | Ar12 |
| 1(77) ~ 636657(77) | D106 | Ar12 |
| 1(78) ~ 636657(78) | D119 | Ar12 |
| 1(79) ~ 636657(79) | D765 | Ar12 |
| 1(80) ~ 636657(80)<br>1(81) ~ 636657(81) | D1<br>D7 | Ar14<br>Ar14 |
| 1(82) ~ 636657(82) | D8 | Ar14 |
| 1(83) ~ 636657(83) | D9 | Ar14 |
| 1(84) ~ 636657(84) | D17 | Ar14 |
| 1(85) ~ 636657(85) | D27 | Ar14 |
| 1(86) ~ 636657(86) | D37 | Ar14 |
| 1(87) ~ 636657(87) | D39 | Ar14 |
| 1(88) ~ 636657(88) | D47 | Ar14 |
| 1(89) ~ 636657(89) | D51 | Ar14 |
| 1(90) ~ 636657(90) | D55 | Ar14 |
| 1(91) ~ 636657(91) | D61 | Ar14 |
| 1(92) ~ 636657(92)<br>1(93) ~ 636657(93) | D62<br>D63 | Ar14<br>Ar14 |
| 1(94) ~ 636657(94) | D71 | Ar14 |
| 1(95) ~ 636657(95) | D81 | Ar14 |

(continued)

| No. | $Ar^1$,$Ar^3$ | $Ar^2$,$Ar^4$ |
|---|---|---|
| 1(96) ~ 636657(96) | D88 | Ar14 |
| 1(97) ~ 636657(97) | D106 | Ar14 |
| 1(98) ~ 636657(98) | D119 | Ar14 |
| 1(99) ~ 636657(99) | D765 | Ar14 |
| 1(100) ~ 636657(100) | D1 | Ar15 |
| 1(101) ~ 636657(101) | D7 | Ar15 |
| 1(102) ~ 636657(102) | D8 | Ar15 |
| 1(103) ~ 636657(103) | D9 | Ar15 |
| 1(104) ~ 636657(104) | D17 | Ar15 |
| 1(105) ~ 636657(105) | D27 | Ar15 |
| 1(106) ~ 636657(106) | D37 | Ar15 |
| 1(107) ~ 636657(107) | D39 | Ar15 |
| 1(108) ~ 636657(108) | D47 | Ar15 |
| 1(109) ~ 636657(109) | D51 | Ar15 |
| 1(110) ~ 636657(110) | D55 | Ar15 |
| 1(111) ~ 636657(111) | D61 | Ar15 |
| 1(112) ~ 636657(112) | D62 | Ar15 |
| 1(113) ~ 636657(113) | D63 | Ar15 |
| 1(114) ~ 636657(114) | D71 | Ar15 |
| 1(115) ~ 636657(115) | D81 | Ar15 |
| 1(116) ~ 636657(116) | D88 | Ar15 |
| 1(117) ~ 636657(117) | D106 | Ar15 |
| 1(118) ~ 636657(118) | D119 | Ar15 |
| 1(119) ~ 636657(119) | D765 | Ar15 |
| 1(120) ~ 636657(120) | D1 | Ar35 |
| 1(121) ~ 636657(121) | D7 | Ar35 |
| 1(122) ~ 636657(122) | D8 | Ar35 |
| 1(123) ~ 636657(123) | D9 | Ar35 |
| 1(124) ~ 636657(124) | D17 | Ar35 |
| 1(125) ~ 636657(125) | D27 | Ar35 |
| 1(126) ~ 636657(126) | D37 | Ar35 |
| 1(127) ~ 636657(127) | D39 | Ar35 |
| 1(128) ~ 636657(128) | D47 | Ar35 |
| 1(129) ~ 636657(129) | D51 | Ar35 |
| 1(130) ~ 636657(130) | D55 | Ar35 |
| 1(131) ~ 636657(131) | D61 | Ar35 |
| 1(132) ~ 636657(132) | D62 | Ar35 |
| 1(133) ~ 636657(133) | D63 | Ar35 |
| 1(134) ~ 636657(134) | D71 | Ar35 |

(continued)

| No. | $Ar^1,Ar^3$ | $Ar^2,Ar^4$ |
|---|---|---|
| 1(135) ~ 636657(135) | D81 | Ar35 |
| 1(136) ~ 636657(136) | D88 | Ar35 |
| 1(137) ~ 636657(137) | D106 | Ar35 |
| 1(138) ~ 636657(138) | D119 | Ar35 |
| 1(139) ~ 636657(139) | D765 | Ar35 |
| 1(140) ~ 636657(140) | D1 | Ar37 |
| 1(141) ~ 636657(141) | D7 | Ar37 |
| 1(142) ~ 636657(142) | D8 | Ar37 |
| 1(143) ~ 636657(143) | D9 | Ar37 |
| 1(144) ~ 636657(144) | D17 | Ar37 |
| 1(145) ~ 636657(145) | D27 | Ar37 |
| 1(146) ~ 636657(146) | D37 | Ar37 |
| 1(147) ~ 636657(147) | D39 | Ar37 |
| 1(148) ~ 636657(148) | D47 | Ar37 |
| 1(149) ~ 636657(149) | D51 | Ar37 |
| 1(150) ~ 636657(150) | D55 | Ar37 |
| 1(151) ~ 636657(151) | D61 | Ar37 |
| 1(152) ~ 636657(152) | D62 | Ar37 |
| 1(153) ~ 636657(153) | D63 | Ar37 |
| 1(154) ~ 636657(154) | D71 | Ar37 |
| 1(155) ~ 636657(155) | D81 | Ar37 |
| 1(156) ~ 636657(156) | D88 | Ar37 |
| 1(157) ~ 636657(157) | D106 | Ar37 |
| 1(158) ~ 636657(158) | D119 | Ar37 |
| 1(159) ~ 636657(159) | D765 | Ar37 |
| 1(160) ~ 636657(160) | D1 | Ar39 |
| 1(161) ~ 636657(161) | D7 | Ar39 |
| 1(162) ~ 636657(162) | D8 | Ar39 |
| 1(163) ~ 636657(163) | D9 | Ar39 |
| 1(164) ~ 636657(164) | D17 | Ar39 |
| 1(165) ~ 636657(165) | D27 | Ar39 |
| 1(166) ~ 636657(166) | D37 | Ar39 |
| 1(167) ~ 636657(167) | D39 | Ar39 |
| 1(168) ~ 636657(168) | D47 | Ar39 |
| 1(169) ~ 636657(169) | D51 | Ar39 |
| 1(170) ~ 636657(170) | D55 | Ar39 |
| 1(171) ~ 636657(171) | D61 | Ar39 |
| 1(172) ~ 636657(172) | D62 | Ar39 |
| 1(173) ~ 636657(173) | D63 | Ar39 |

(continued)

| No. | $Ar^1$,$Ar^3$ | $Ar^2$,$Ar^4$ |
|---|---|---|
| 1(174) ~ 636657(174) | D71 | Ar39 |
| 1(175) ~ 636657(175) | D81 | Ar39 |
| 1(176) ~ 636657(176) | D88 | Ar39 |
| 1(177) ~ 636657(177) | D106 | Ar39 |
| 1(178) ~ 636657(178) | D119 | Ar39 |
| 1(179) ~ 636657(179) | D765 | Ar39 |
| 1(180) ~ 636657(180) | D1 | Ar40 |
| 1(181) ~ 636657(181) | D7 | Ar40 |
| 1(182) ~ 636657(182) | D8 | Ar40 |
| 1(183) ~ 636657(183) | D9 | Ar40 |
| 1(184) ~ 636657(184) | D17 | Ar40 |
| 1(185) ~ 636657(185) | D27 | Ar40 |
| 1(186) ~ 636657(186) | D37 | Ar40 |
| 1(187) ~ 636657(187) | D39 | Ar40 |
| 1(188) ~ 636657(188) | D47 | Ar40 |
| 1(189) ~ 636657(189) | D51 | Ar40 |
| 1(190) ~ 636657(190) | D55 | Ar40 |
| 1(191) ~ 636657(191) | D61 | Ar40 |
| 1(192) ~ 636657(192) | D62 | Ar40 |
| 1(193) ~ 636657(193) | D63 | Ar40 |
| 1(194) ~ 636657(194) | D71 | Ar40 |
| 1(195) ~ 636657(195) | D81 | Ar40 |
| 1(196) ~ 636657(196) | D88 | Ar40 |
| 1(197) ~ 636657(197) | D106 | Ar40 |
| 1(198) ~ 636657(198) | D119 | Ar40 |
| 1(199) ~ 636657(199) | D765 | Ar40 |
| 1(200) ~ 636657(200) | D1 | Ar56 |
| 1(201) ~ 636657(201) | D7 | Ar56 |
| 1(202) ~ 636657(202) | D8 | Ar56 |
| 1(203) ~ 636657(203) | D9 | Ar56 |
| 1(204) ~ 636657(204) | D17 | Ar56 |
| 1(205) ~ 636657(205) | D27 | Ar56 |
| 1(206) ~ 636657(206) | D37 | Ar56 |
| 1(207) ~ 636657(207) | D39 | Ar56 |
| 1(208) ~ 636657(208) | D47 | Ar56 |
| 1(209) ~ 636657(209) | D51 | Ar56 |
| 1(210) ~ 636657(210) | D55 | Ar56 |
| 1(211) ~ 636657(211) | D61 | Ar56 |
| 1(212) ~ 636657(212) | D62 | Ar56 |

(continued)

| No. | $Ar^1$,$Ar^3$ | $Ar^2$,$Ar^4$ |
|---|---|---|
| 1(213) ~ 636657(213) | D63 | Ar56 |
| 1(214) ~ 636657(214) | D71 | Ar56 |
| 1(215) ~ 636657(215) | D81 | Ar56 |
| 1(216) ~ 636657(216) | D88 | Ar56 |
| 1(217) ~ 636657(217) | D106 | Ar56 |
| 1(218) ~ 636657(218) | D119 | Ar56 |
| 1(219) ~ 636657(219) | D765 | Ar56 |
| 1(220) ~ 636657(220) | D1 | Ar58 |
| 1(221) ~ 636657(221) | D7 | Ar58 |
| 1(222) ~ 636657(222) | D8 | Ar58 |
| 1(223) ~ 636657(223) | D9 | Ar58 |
| 1(224) ~ 636657(224) | D17 | Ar58 |
| 1(225) ~ 636657(225) | D27 | Ar58 |
| 1(226) ~ 636657(226) | D37 | Ar58 |
| 1(227) ~ 636657(227) | D39 | Ar58 |
| 1(228) ~ 636657(228) | D47 | Ar58 |
| 1(229) ~ 636657(229) | D51 | Ar58 |
| 1(230) ~ 636657(230) | D55 | Ar58 |
| 1(231) ~ 636657(231) | D61 | Ar58 |
| 1(232) ~ 636657(232) | D62 | Ar58 |
| 1(233) ~ 636657(233) | D63 | Ar58 |
| 1(234) ~ 636657(234) | D71 | Ar58 |
| 1(235) ~ 636657(235) | D81 | Ar58 |
| 1(236) ~ 636657(236) | D88 | Ar58 |
| 1(237) ~ 636657(237) | D106 | Ar58 |
| 1(238) ~ 636657(238) | D119 | Ar58 |
| 1(239) ~ 636657(239) | D765 | Ar58 |
| 1(240) ~ 636657(240) | D1 | Ar60 |
| 1(241) ~ 636657(241) | D7 | Ar60 |
| 1(242) ~ 636657(242) | D8 | Ar60 |
| 1(243) ~ 636657(243) | D9 | Ar60 |
| 1(244) ~ 636657(244) | D17 | Ar60 |
| 1(245) ~ 636657(245) | D27 | Ar60 |
| 1(246) ~ 636657(246) | D37 | Ar60 |
| 1(247) ~ 636657(247) | D39 | Ar60 |
| 1(248) ~ 636657(248) | D47 | Ar60 |
| 1(249) ~ 636657(249) | D51 | Ar60 |
| 1(250) ~ 636657(250) | D55 | Ar60 |
| 1(251) ~ 636657(251) | D61 | Ar60 |

(continued)

| No. | $Ar^1$,$Ar^3$ | $Ar^2$,$Ar^4$ |
|---|---|---|
| 1(252) ~ 636657(252) | D62 | Ar60 |
| 1(253) ~ 636657(253) | D63 | Ar60 |
| 1(254) ~ 636657(254) | D71 | Ar60 |
| 1(255) ~ 636657(255) | D81 | Ar60 |
| 1(256) ~ 636657(256) | D88 | Ar60 |
| 1(257) ~ 636657(257) | D106 | Ar60 |
| 1(258) ~ 636657(258) | D119 | Ar60 |
| 1(259) ~ 636657(259) | D765 | Ar60 |
| 1(260) ~ 636657(260) | D1 | Ar61 |
| 1(261) ~ 636657(261) | D7 | Ar61 |
| 1(262) ~ 636657(262) | D8 | Ar61 |
| 1(263) ~ 636657(263) | D9 | Ar61 |
| 1(264) ~ 636657(264) | D17 | Ar61 |
| 1(265) ~ 636657(265) | D27 | Ar61 |
| 1(266) ~ 636657(266) | D37 | Ar61 |
| 1(267) ~ 636657(267) | D39 | Ar61 |
| 1(268) ~ 636657(268) | D47 | Ar61 |
| 1(269) ~ 636657(269) | D51 | Ar61 |
| 1(270) ~ 636657(270) | D55 | Ar61 |
| 1(271) ~ 636657(271) | D61 | Ar61 |
| 1(272) ~ 636657(272) | D62 | Ar61 |
| 1(273) ~ 636657(273) | D63 | Ar61 |
| 1(274) ~ 636657(274) | D71 | Ar61 |
| 1(275) ~ 636657(275) | D81 | Ar61 |
| 1(276) ~ 636657(276) | D88 | Ar61 |
| 1(277) ~ 636657(277) | D106 | Ar61 |
| 1(278) ~ 636657(278) | D119 | Ar61 |
| 1(279) ~ 636657(279) | D765 | Ar61 |
| 1(280) ~ 636657(280) | D1 | D55 |
| 1(281) ~ 636657(281) | D7 | D55 |
| 1(282) ~ 636657(282) | D8 | D55 |
| 1(283) ~ 636657(283) | D9 | D55 |
| 1(284) ~ 636657(284) | D17 | D55 |
| 1(285) ~ 636657(285) | D27 | D55 |
| 1(286) ~ 636657(286) | D37 | D55 |
| 1(287) ~ 636657(287) | D39 | D55 |
| 1(288) ~ 636657(288) | D47 | D55 |
| 1(289) ~ 636657(289) | D51 | D55 |
| 1(290) ~ 636657(290) | D55 | D55 |

(continued)

| No. | $Ar^1$,$Ar^3$ | $Ar^2$,$Ar^4$ |
|---|---|---|
| 1(291) ~ 636657(291) | D61 | D55 |
| 1(292) ~ 636657(292) | D62 | D55 |
| 1(293) ~ 636657(293) | D63 | D55 |
| 1(294) ~ 636657(294) | D71 | D55 |
| 1(295) ~ 636657(295) | D81 | D55 |
| 1(296) ~ 636657(296) | D88 | D55 |
| 1(297) ~ 636657(297) | D106 | D55 |
| 1(298) ~ 636657(298) | D119 | D55 |
| 1(299) ~ 636657(299) | D765 | D55 |
| 8756(300) ~ 442987(300) | Ar1 | Ar1 |
| 8756(301) ~ 442987(301) | Ar10 | Ar10 |
| 8756(302) ~ 442987(302) | Ar12 | Ar12 |
| 8756(303) ~ 442987(303) | Ar14 | Ar14 |
| 8756(304) ~ 442987(304) | Ar15 | Ar15 |
| 8756(305) ~ 442987(305) | Ar35 | Ar35 |
| 8756(306) ~ 442987(306) | Ar37 | Ar37 |
| 8756(307) ~ 442987(307) | Ar39 | Ar39 |
| 8756(308) ~ 442987(308) | Ar40 | Ar40 |
| 8756(309) ~ 442987(309) | Ar56 | Ar56 |
| 8756(310) ~ 442987(310) | Ar58 | Ar58 |
| 8756(311) ~ 442987(311) | Ar61 | Ar61 |
| 443073(312) ~ 539822(312) | Ar1 | Ar1 |
| 443073(313) ~ 539822(313) | Ar10 | Ar10 |
| 443073(314) ~ 539822(314) | Ar12 | Ar12 |
| 443073(315) ~ 539822(315) | Ar14 | Ar14 |
| 443073(316) ~ 539822(316) | Ar15 | Ar15 |
| 443073(317) ~ 539822(317) | Ar35 | Ar35 |
| 443073(318) ~ 539822(318) | Ar37 | Ar37 |
| 443073(319) ~ 539822(319) | Ar39 | Ar39 |
| 443073(320) ~ 539822(320) | Ar40 | Ar40 |
| 443073(321) ~ 539822(321) | Ar56 | Ar56 |
| 443073(322) ~ 539822(322) | Ar58 | Ar58 |
| 443073(323) ~ 539822(323) | Ar61 | Ar61 |
| 539908(324) ~ 636657(324) | Ar1 | Ar1 |
| 539908(325) ~ 636657(325) | Ar10 | Ar10 |
| 539908(326) ~ 636657(326) | Ar12 | Ar12 |
| 539908(327) ~ 636657(327) | Ar14 | Ar14 |
| 539908(328) ~ 636657(328) | Ar15 | Ar15 |
| 539908(329) ~ 636657(329) | Ar35 | Ar35 |

(continued)

| No. | $Ar^1,Ar^3$ | $Ar^2,Ar^4$ |
|---|---|---|
| 539908(330) ~ 636657(330) | Ar37 | Ar37 |
| 539908(331) ~ 636657(331) | Ar39 | Ar39 |
| 539908(332) ~ 636657(332) | Ar40 | Ar40 |
| 539908(333) ~ 636657(333) | Ar56 | Ar56 |
| 539908(334) ~ 636657(334) | Ar58 | Ar58 |
| 539908(335) ~ 636657(335) | Ar61 | Ar61 |
| 8756(336) ~ 442987(336) | Ar47 | Ar1 |
| 8756(337) ~ 442987(337) | Ar47 | Ar10 |
| 8756(338) ~ 442987(338) | Ar47 | Ar12 |
| 8756(339) ~ 442987(339) | Ar47 | Ar14 |
| 8756(340) ~ 442987(340) | Ar47 | Ar15 |
| 8756(341) ~ 442987(341) | Ar47 | Ar35 |
| 8756(342) ~ 442987(342) | Ar47 | Ar37 |
| 8756(343) ~ 442987(343) | Ar47 | Ar39 |
| 8756(344) ~ 442987(344) | Ar47 | Ar40 |
| 8756(345) ~ 442987(345) | Ar47 | Ar56 |
| 8756(346) ~ 442987(346) | Ar47 | Ar58 |
| 8756(347) ~ 442987(347) | Ar47 | Ar61 |
| 443073(348) ~ 539822(348) | Ar47 | Ar1 |
| 443073(349) ~ 539822(349) | Ar47 | Ar10 |
| 443073(350) ~ 539822(350) | Ar47 | Ar12 |
| 443073(351) ~ 539822(351) | Ar47 | Ar14 |
| 443073(352) ~ 539822(352) | Ar47 | Ar15 |
| 443073(353) ~ 539822(353) | Ar47 | Ar35 |
| 443073(354) ~ 539822(354) | Ar47 | Ar37 |
| 443073(355) ~ 539822(355) | Ar47 | Ar39 |
| 443073(356) ~ 539822(356) | Ar47 | Ar40 |
| 443073(357) ~ 539822(357) | Ar47 | Ar56 |
| 443073(358) ~ 539822(358) | Ar47 | Ar58 |
| 443073(359) ~ 539822(359) | Ar47 | Ar61 |
| 539908(360) ~ 636657(360) | Ar47 | Ar1 |
| 539908(361) ~ 636657(361) | Ar47 | Ar10 |
| 539908(362) ~ 636657(362) | Ar47 | Ar12 |
| 539908(363) ~ 636657(363) | Ar47 | Ar14 |
| 539908(364) ~ 636657(364) | Ar47 | Ar15 |
| 539908(365) ~ 636657(365) | Ar47 | Ar35 |
| 539908(366) ~ 636657(366) | Ar47 | Ar37 |
| 539908(367) ~ 636657(367) | Ar47 | Ar39 |
| 539908(368) ~ 636657(368) | Ar47 | Ar40 |
| 539908(369) ~ 636657(369) | Ar47 | Ar56 |

(continued)

| No. | $Ar^1$,$Ar^3$ | $Ar^2$,$Ar^4$ |
|---|---|---|
| 539908(370) ~ 636657(370) | Ar47 | Ar58 |
| 539908(371) ~ 636657(371) | Ar47 | Ar61 |

Compound 1 to Compound 636657(371) listed in Table 8 are compounds in which $R^1$ in the general formula (1) is a hydrogen atom. Compounds having a structure in which $R^1$ of these compounds is substituted with a deuterium atom are referred to as Compound 1d to Compound 636657(371)d in order. Compounds having a structure in which $R^1$ of these compounds is substituted with a perdeuterated phenyl group (Ar47) are referred to as Compound 1D to Compound 636657(371)D in order.

All of the compounds specified by the numbers in Tables 1 to 8, Compound 1d to Compound 636657(371)d, and Compound 1D to Compound 636657(371)D are individually disclosed. In addition, among the specific examples of the compounds, in the case where a rotamer is present, a mixture of rotamers and each separated rotamer are also disclosed in the description herein.

In one aspect of the present invention, the compound is selected from the group of compounds specified in Table 8.

An example of a preferred group of compounds represented by the general formula (1) is given below.

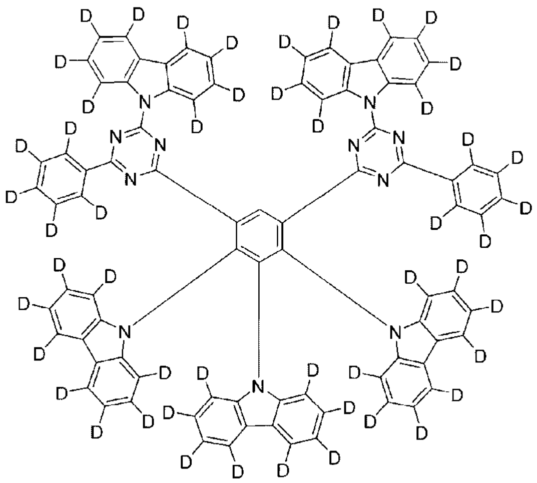

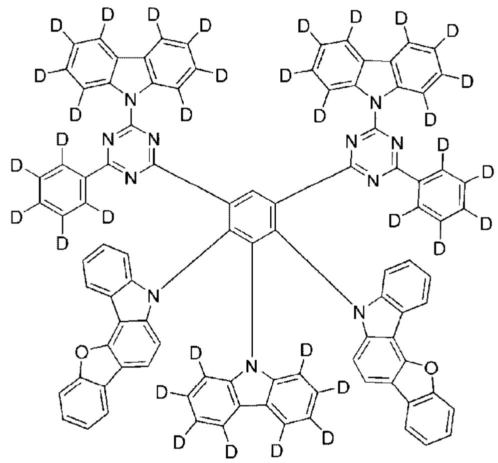

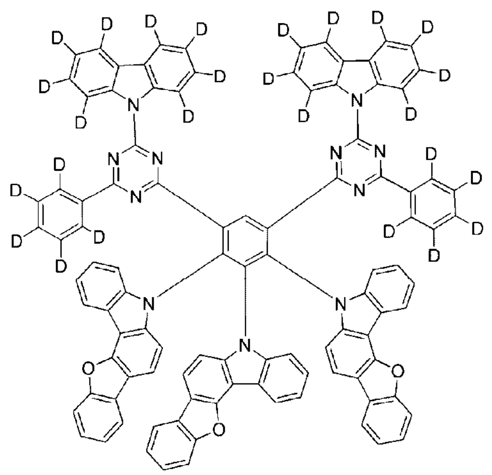

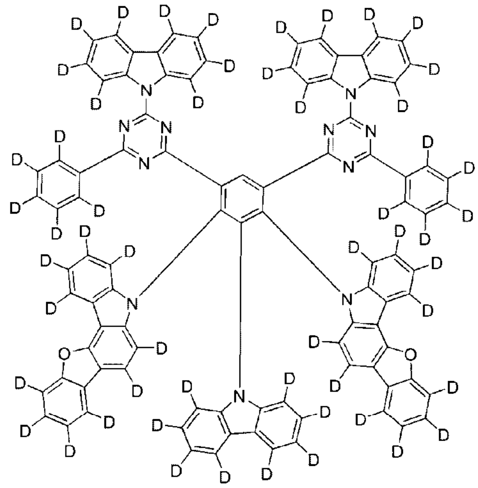

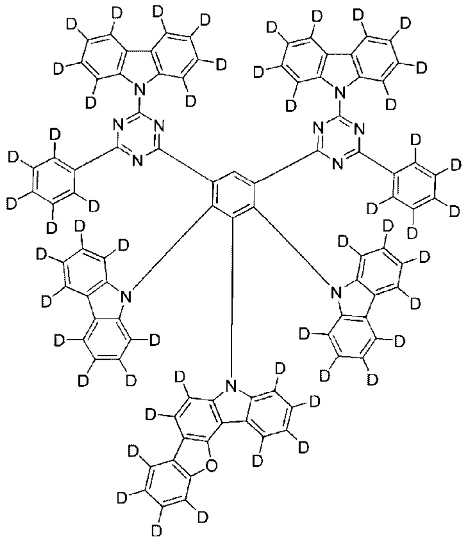

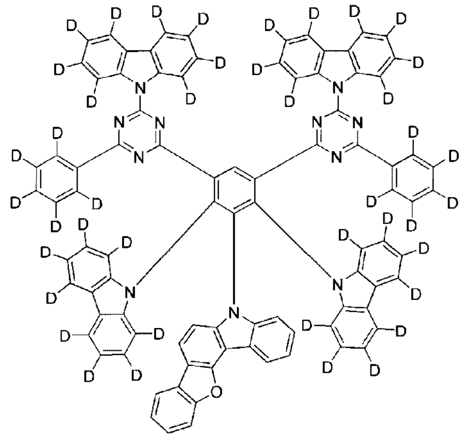

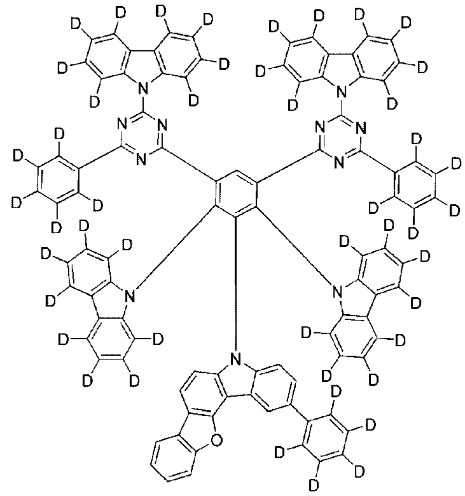

An example of another preferred group of compounds represented by the general formula (1) is given below.

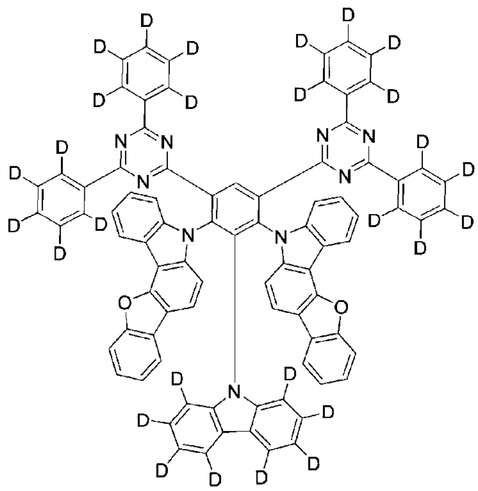

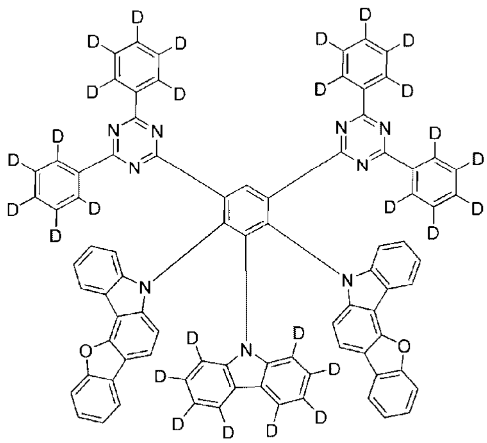

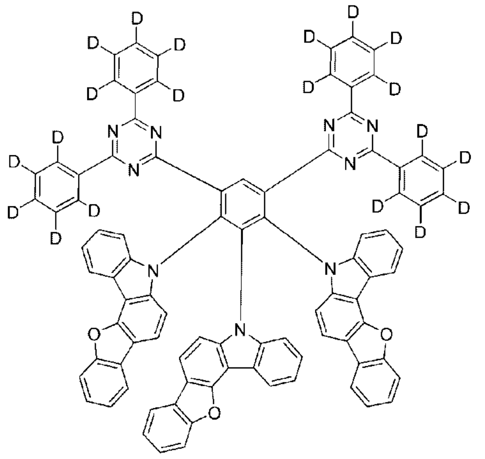

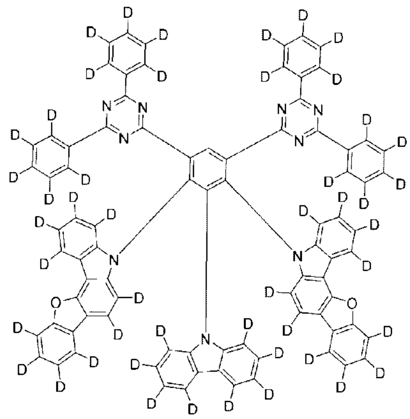

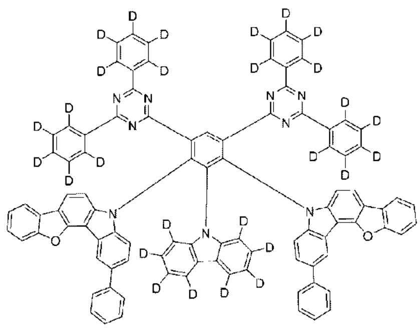

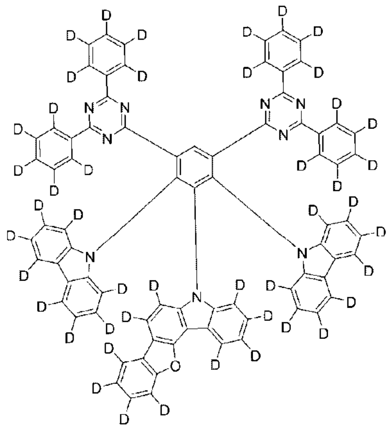

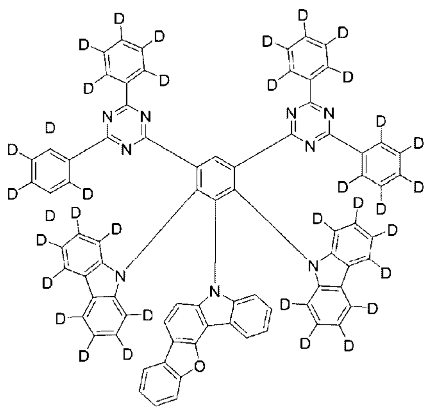

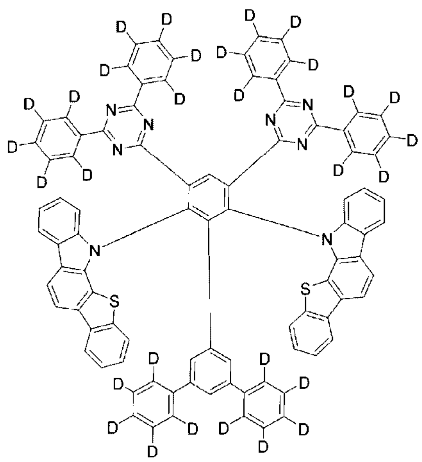

An example of another preferred group of compounds represented by the general formula (1) is given below.

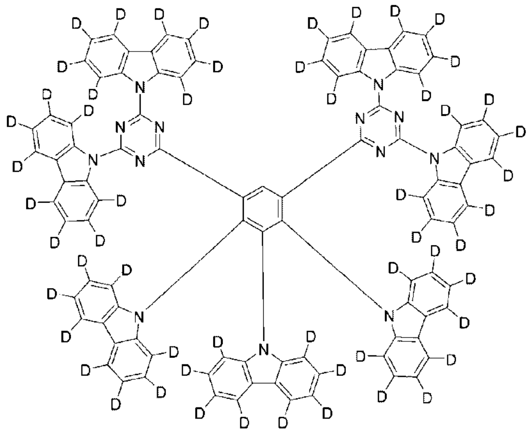

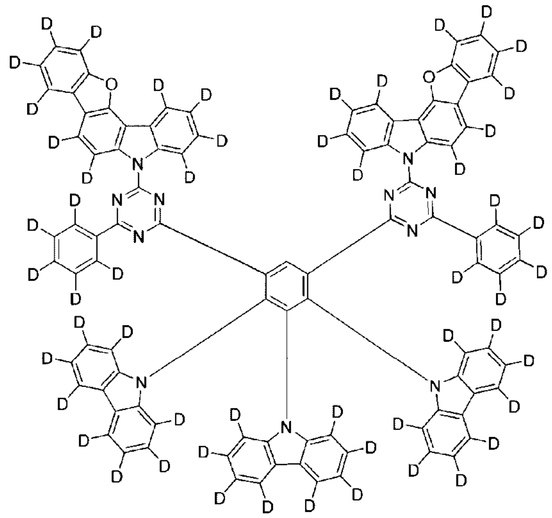

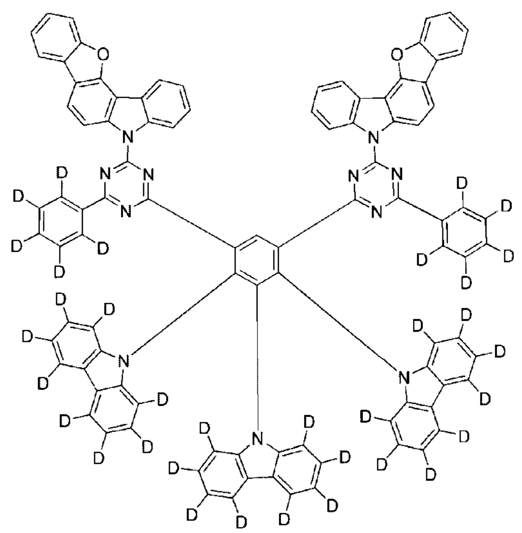

An example of another preferred group of compounds represented by the general formula (1) is given below.

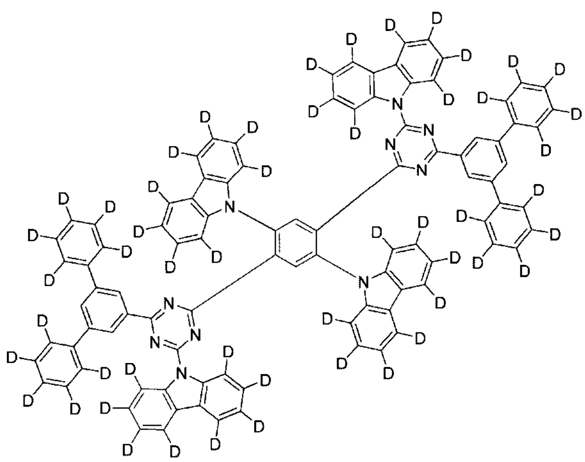

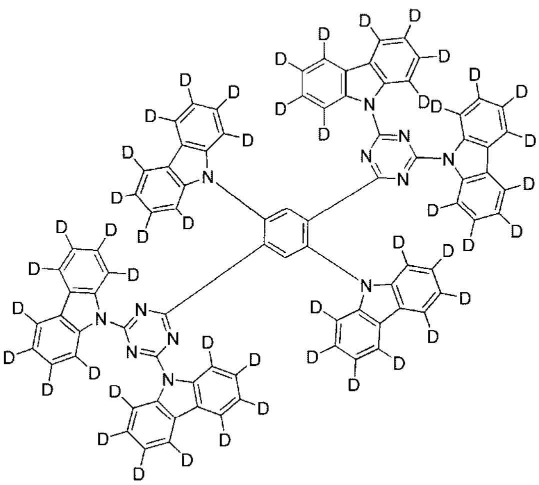

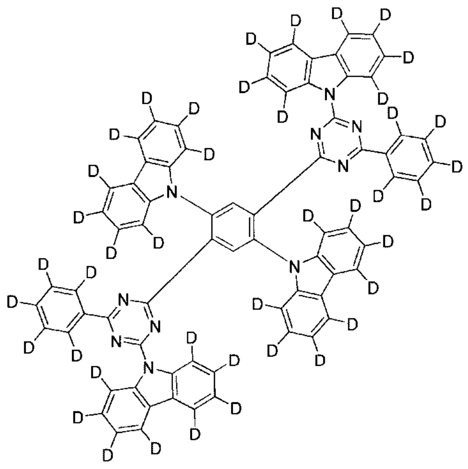

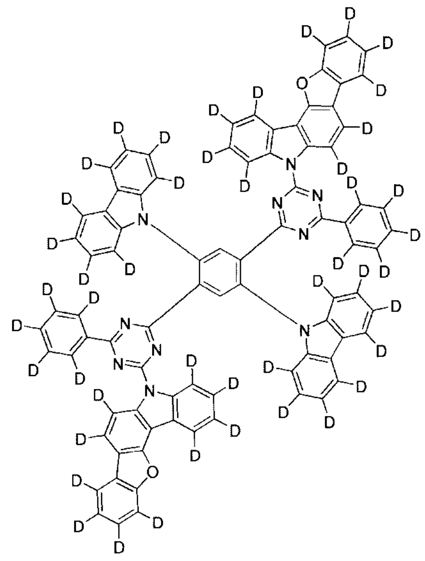

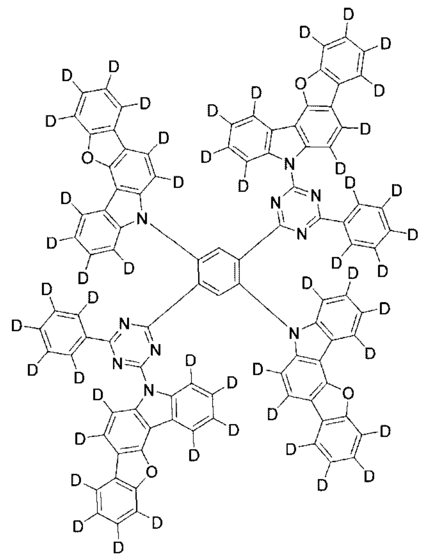

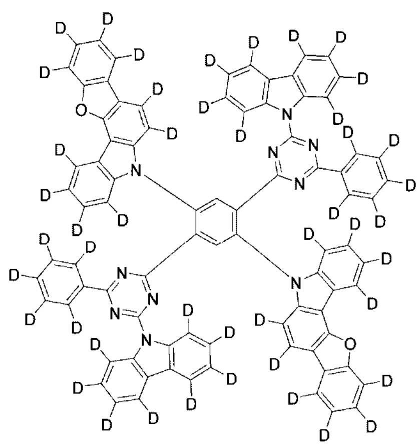

An example of another preferred group of compounds represented by the general formula (1) is given below.

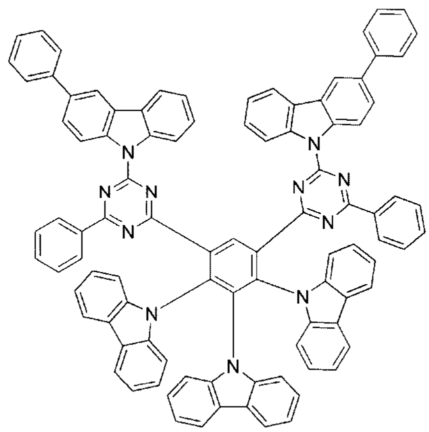

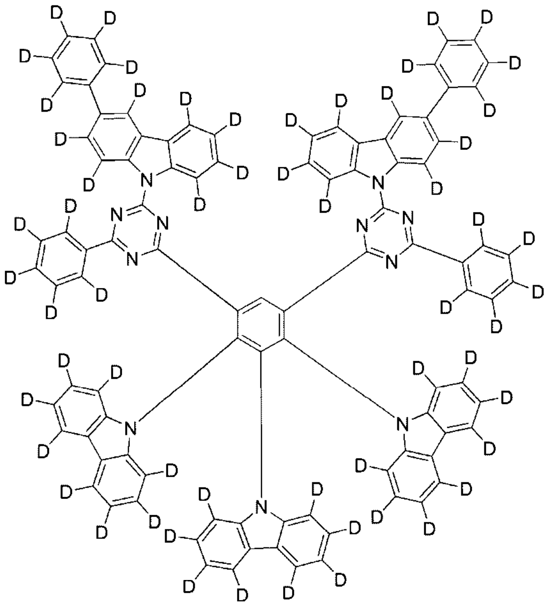

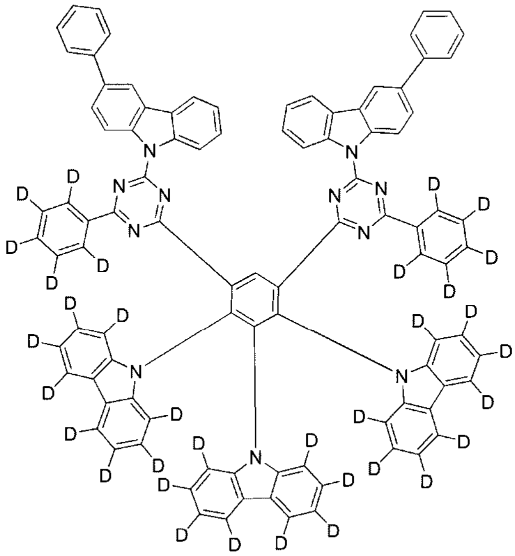

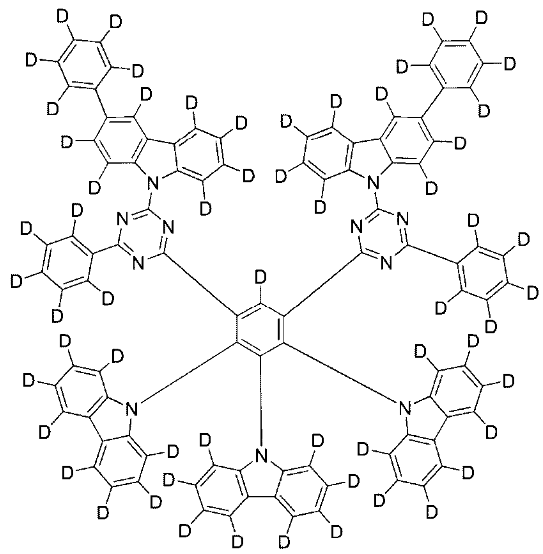

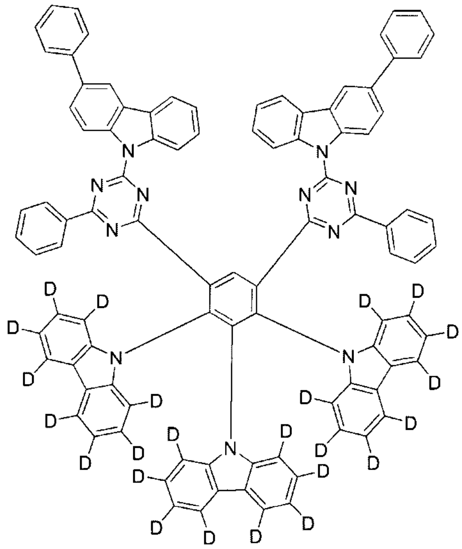

An example of another preferred group of compounds represented by the general formula (1) is given below.

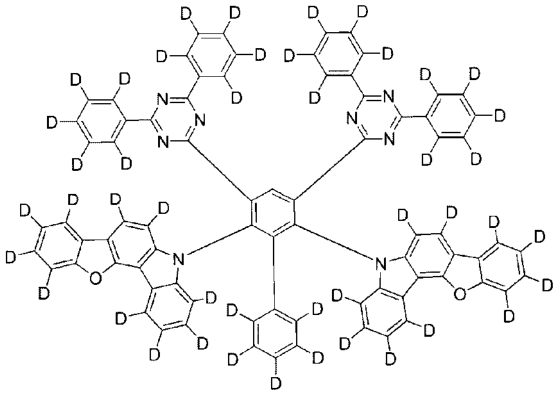

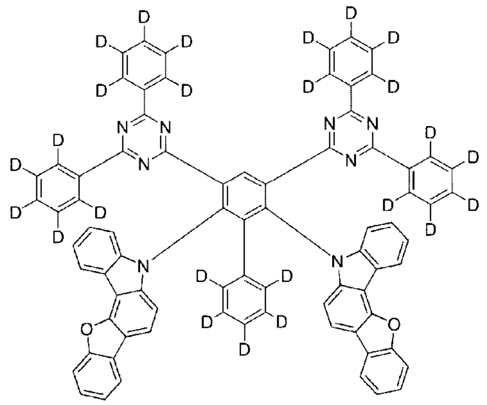

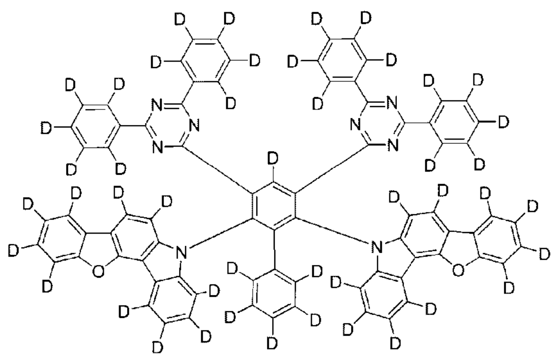

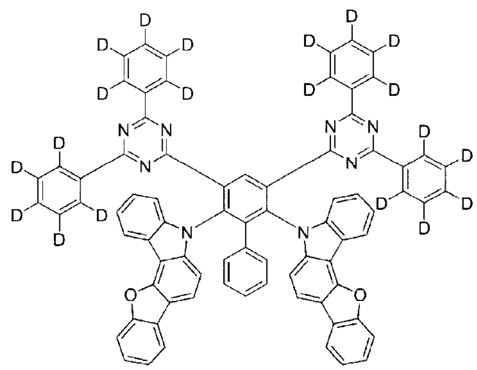

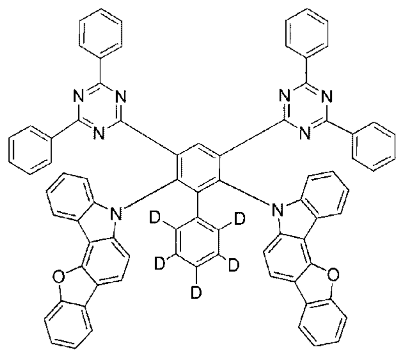

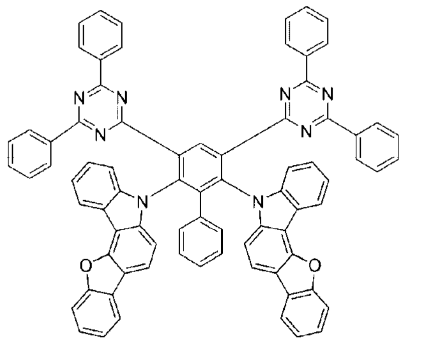

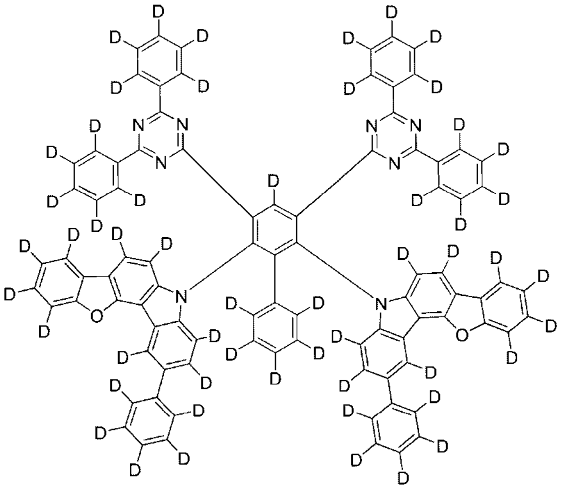

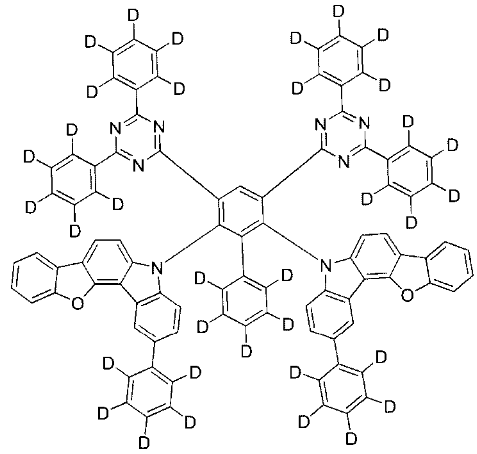

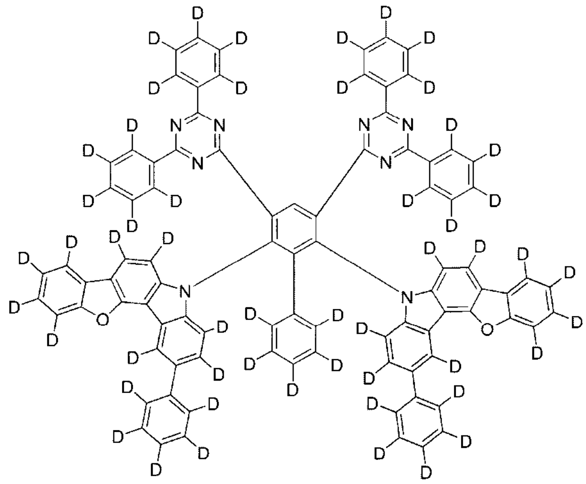

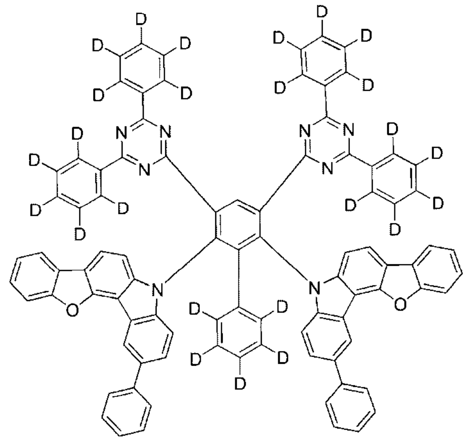

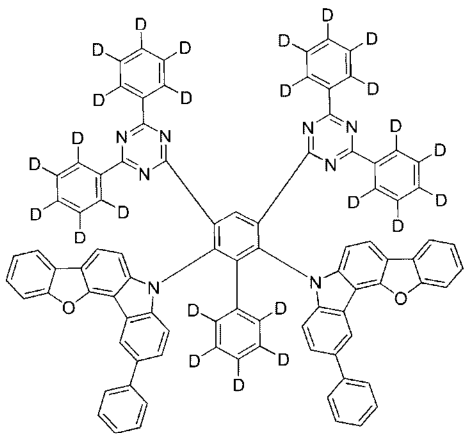

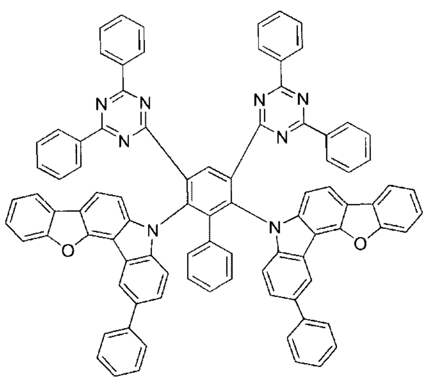

An example of another preferred group of compounds represented by the general formula (1) is given below.

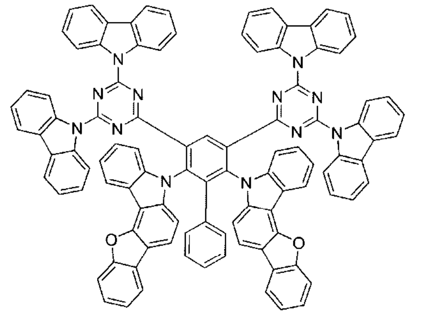

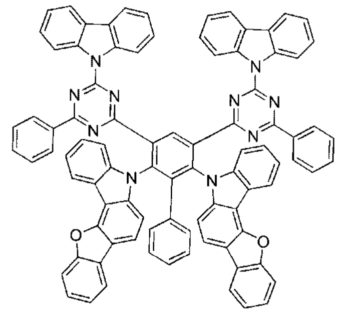

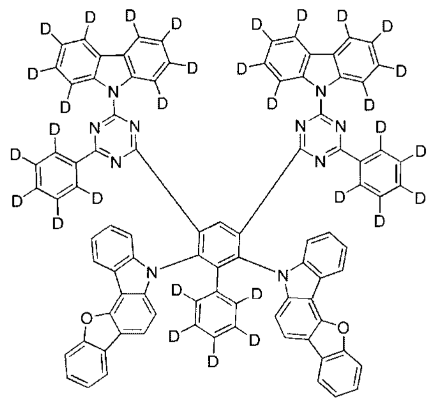

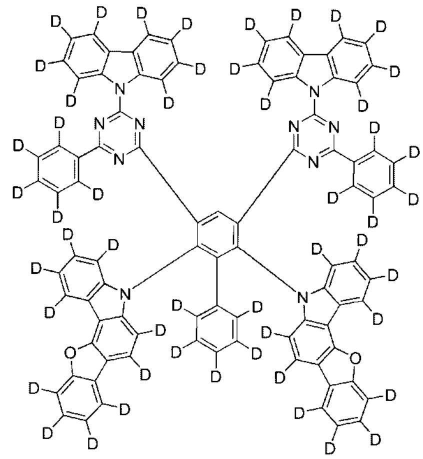

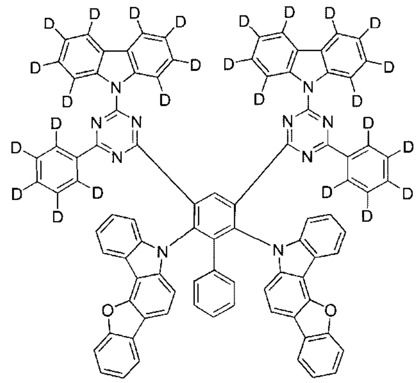

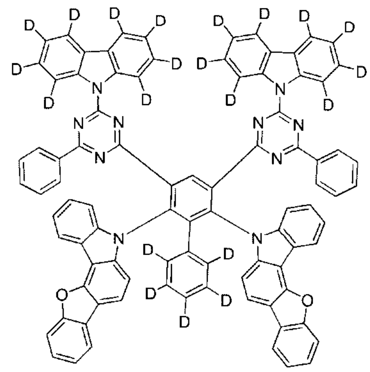

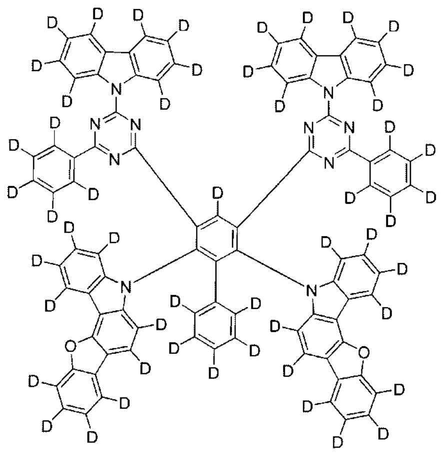

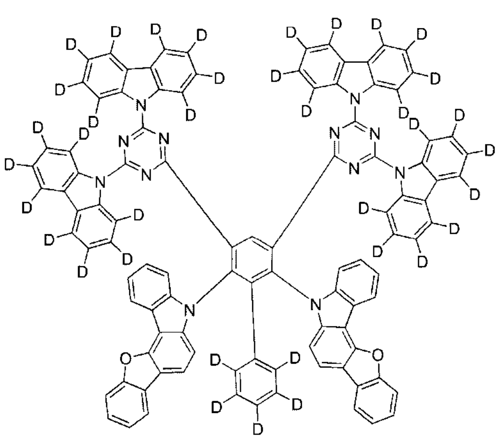

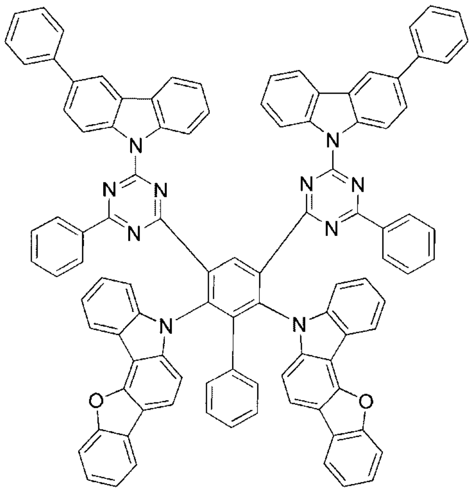

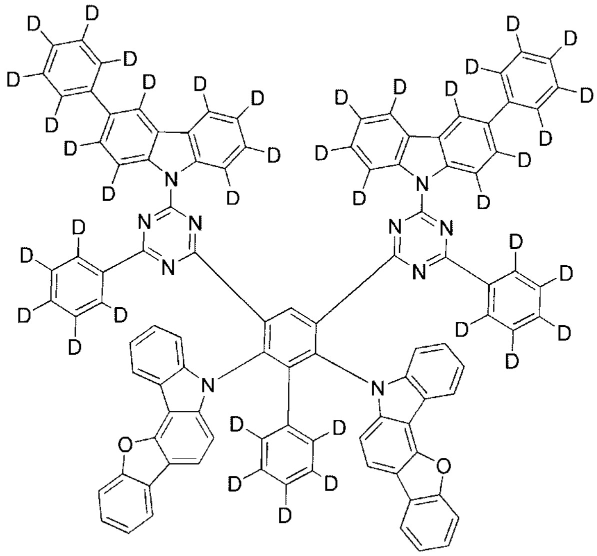

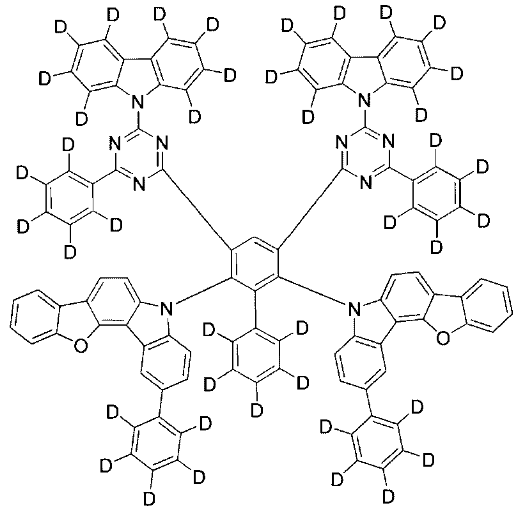

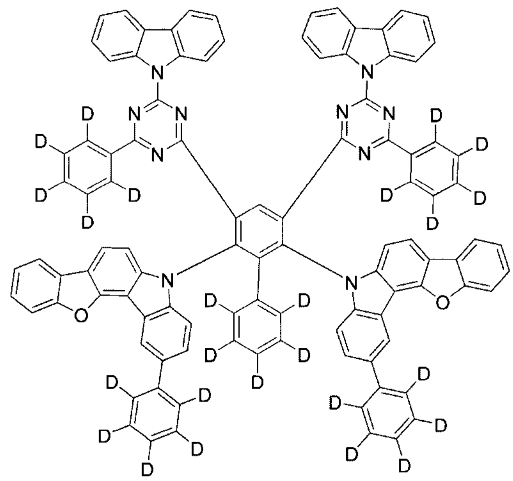

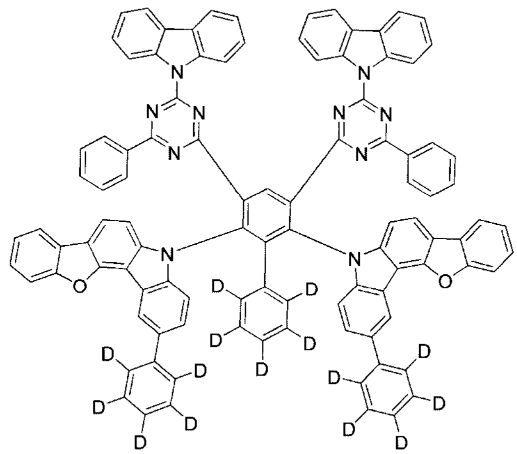

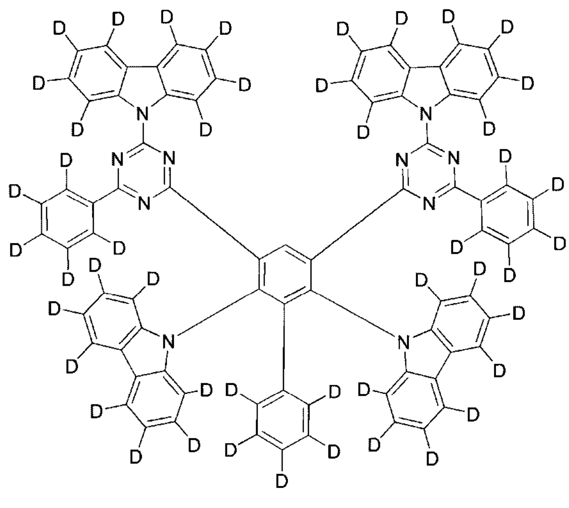

An example of another preferred group of compounds represented by the general formula (1) is given below.

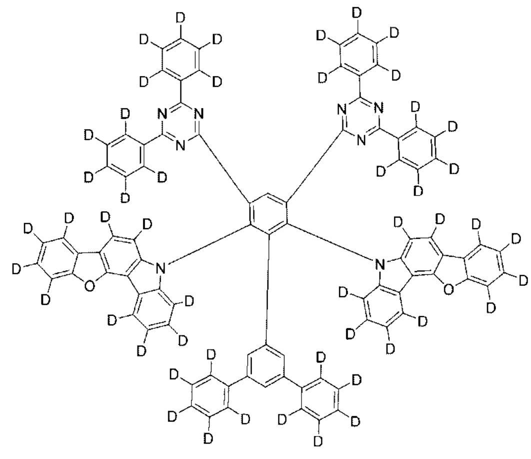

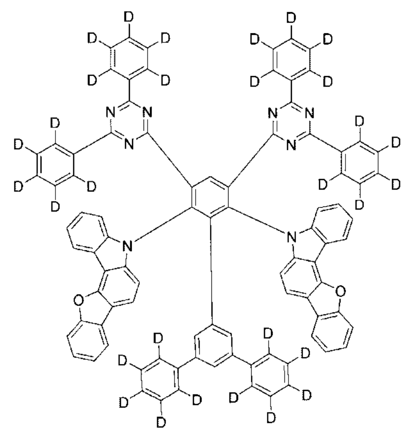

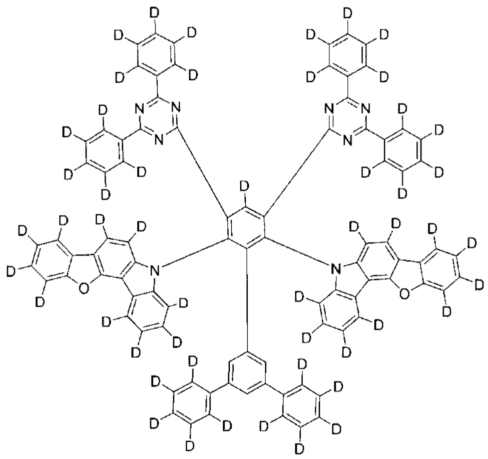

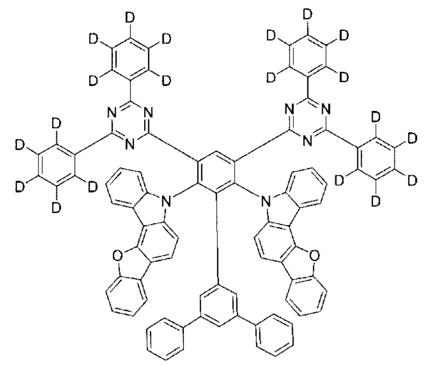

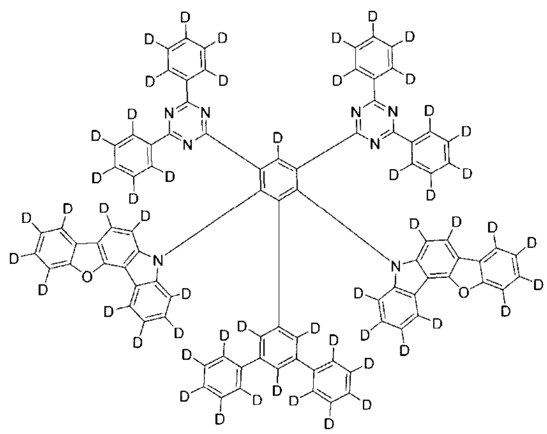

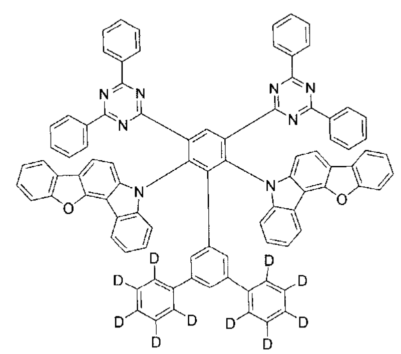

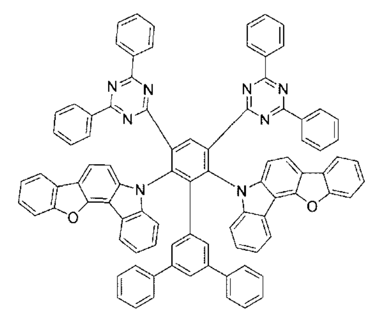

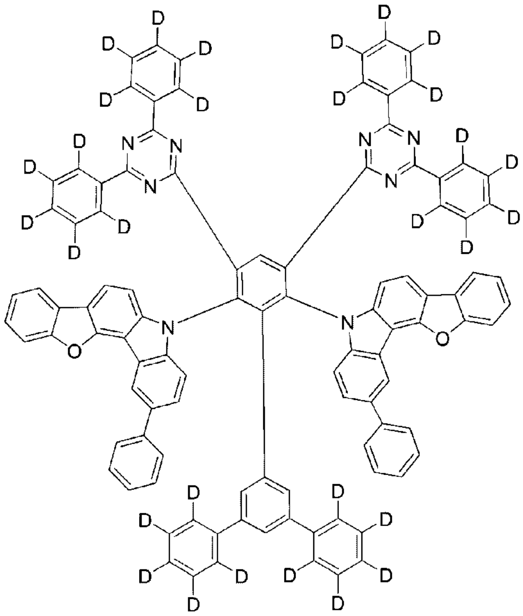

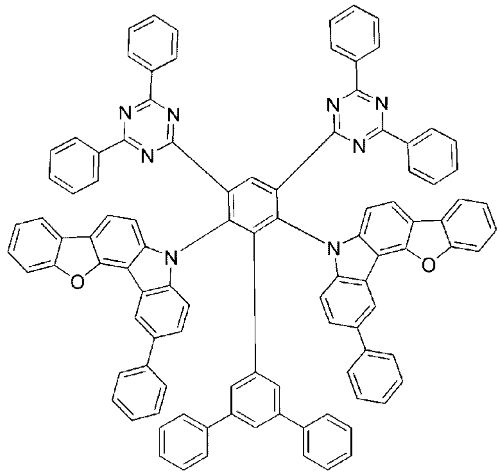

An example of another preferred group of compounds represented by the general formula (1) is given below.

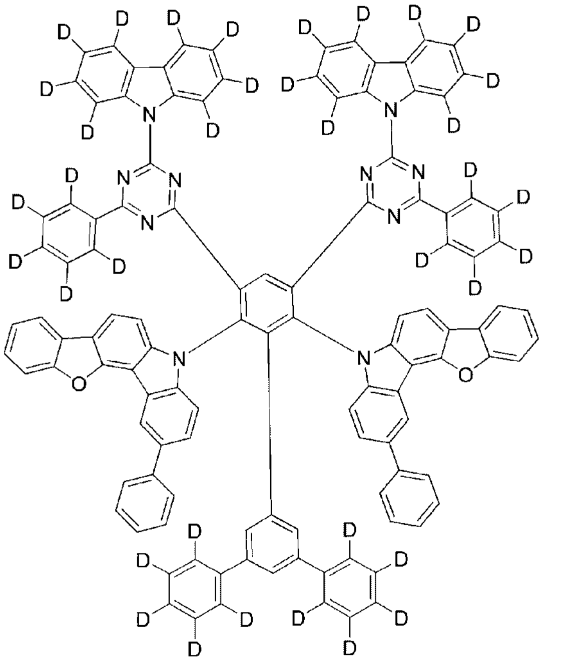

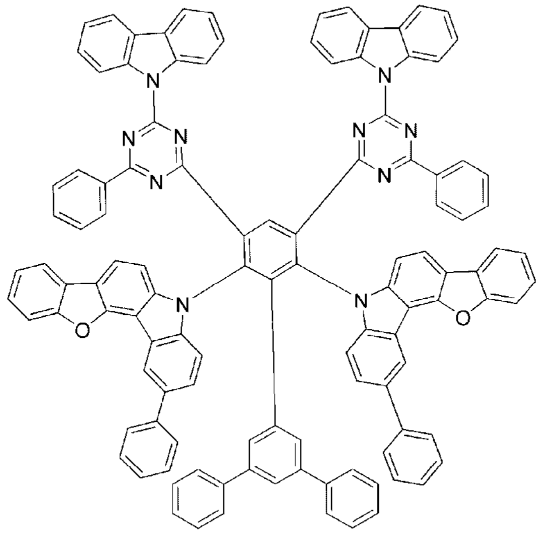

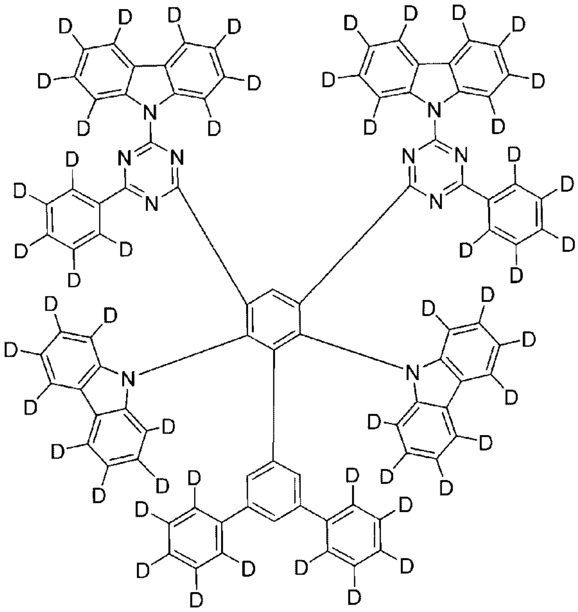

An example of another preferred group of compounds represented by the general formula (1) is given below.

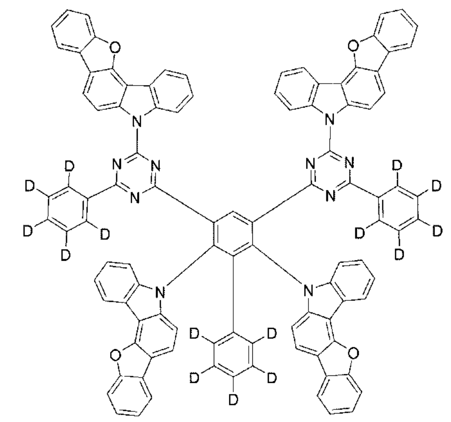

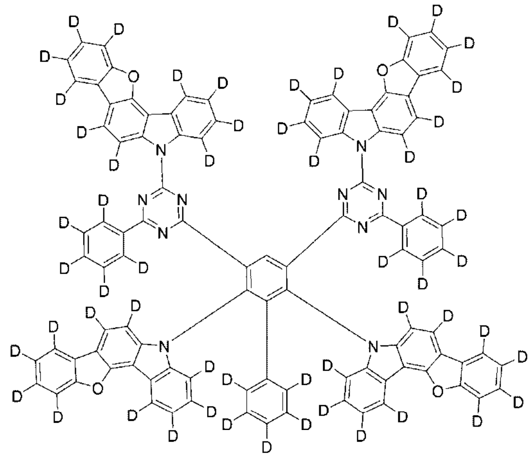

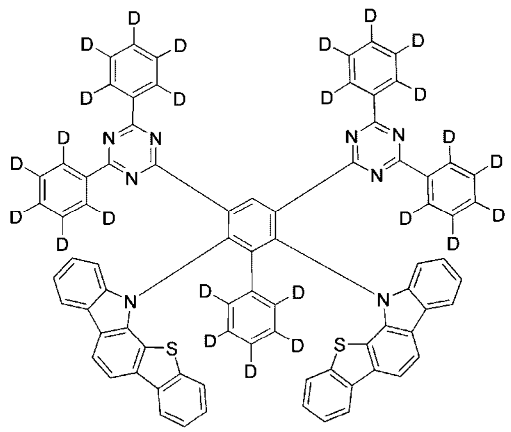

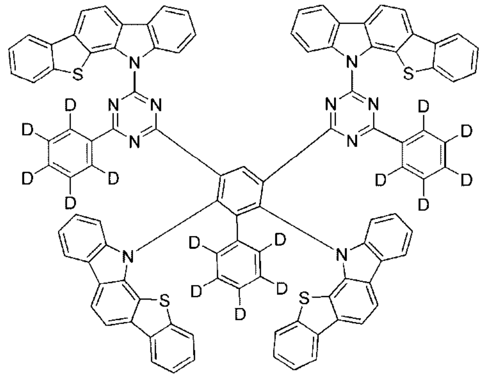

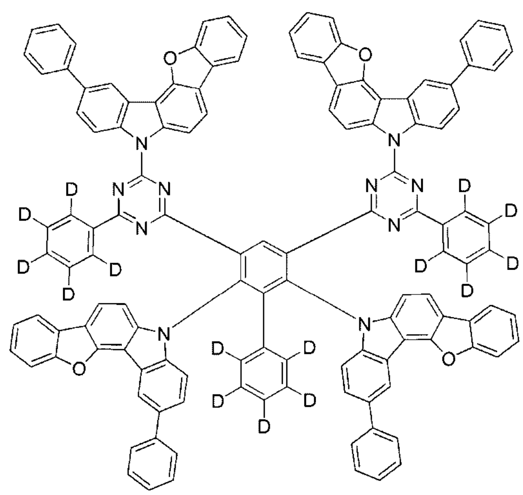

An example of another preferred group of compounds represented by the general formula (1) is given below.

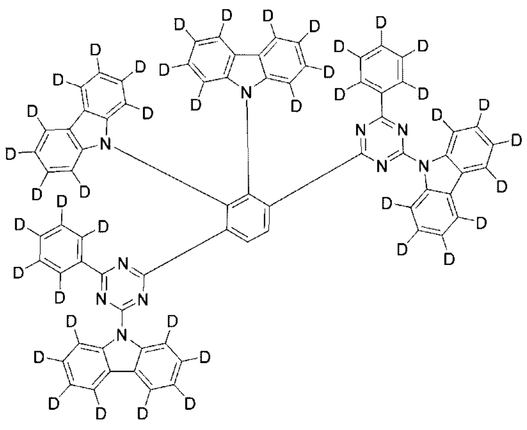

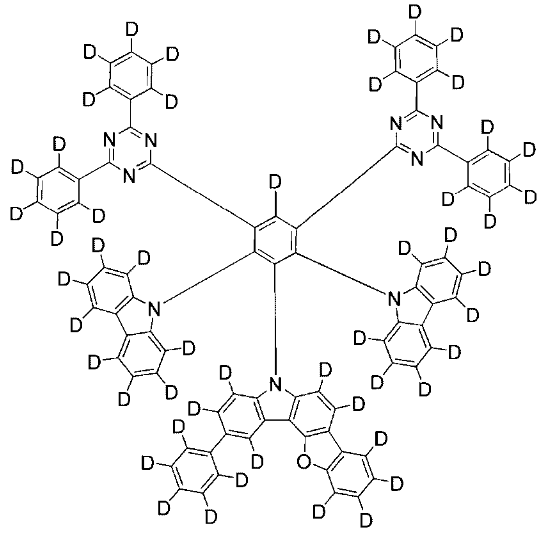

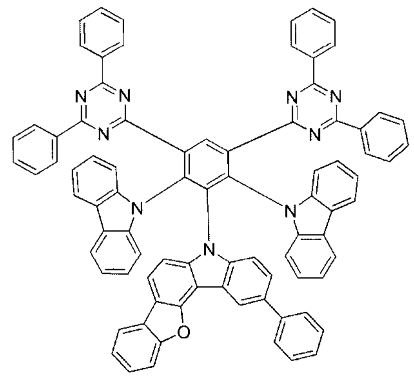

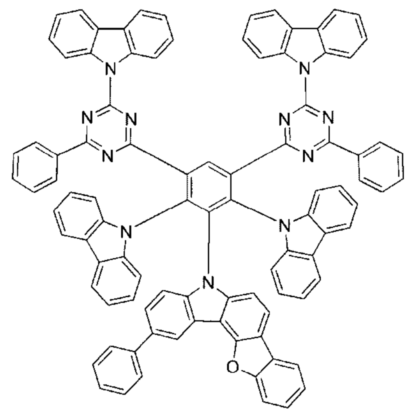

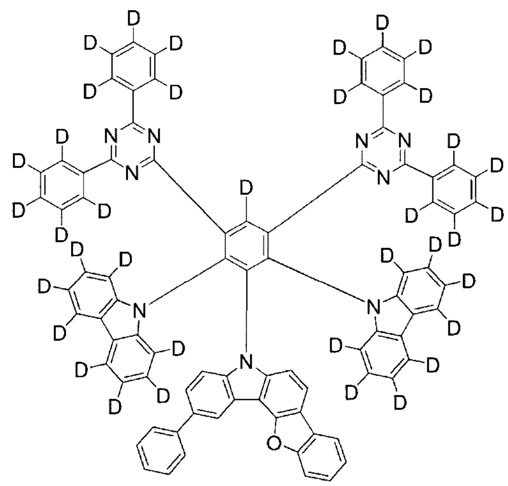

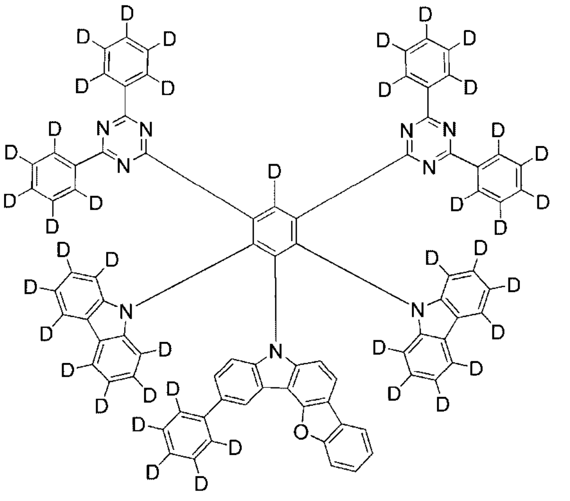

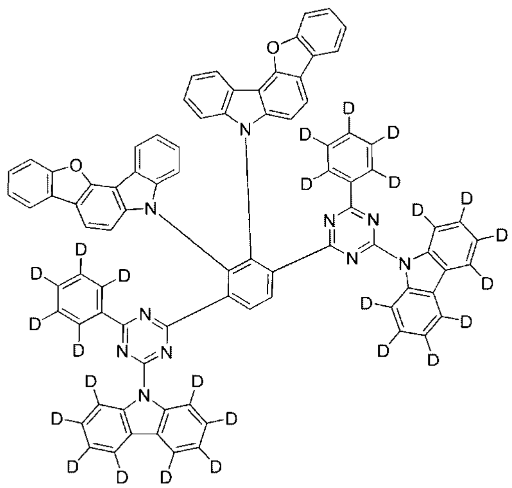

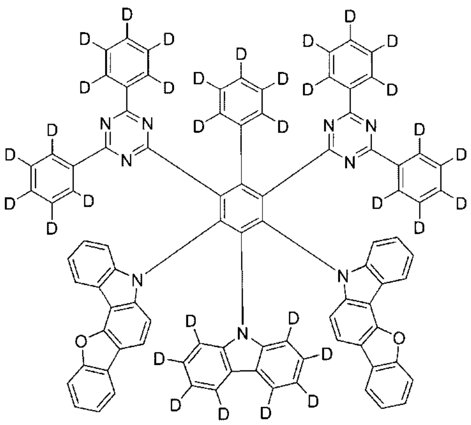

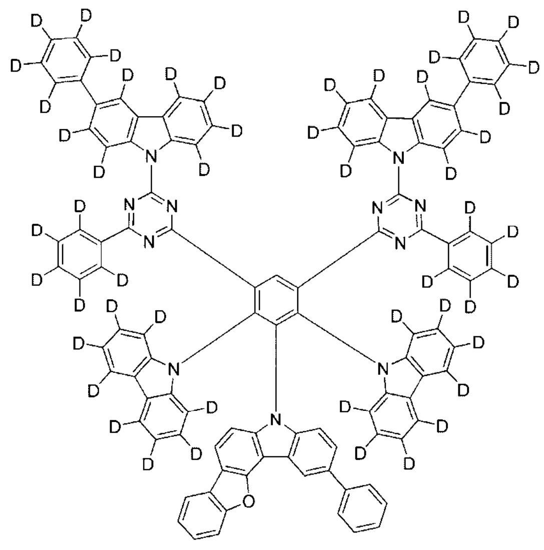

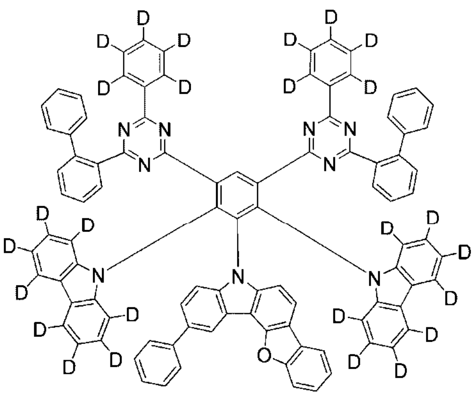

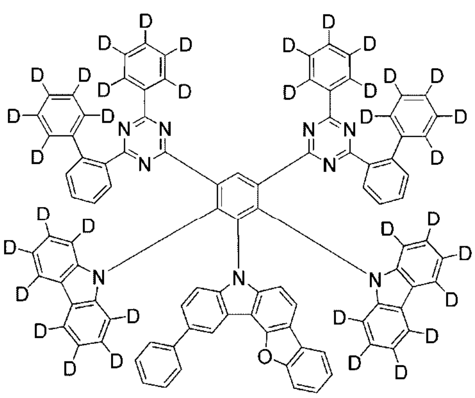

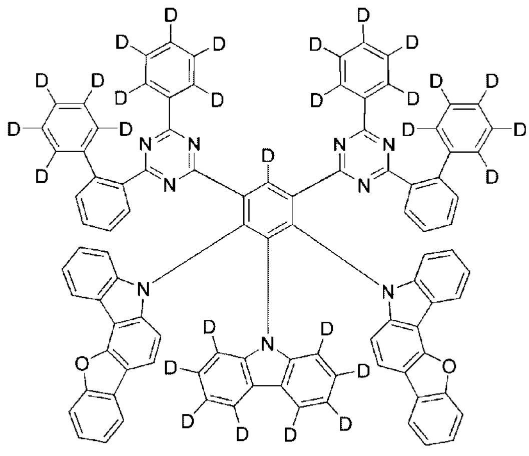

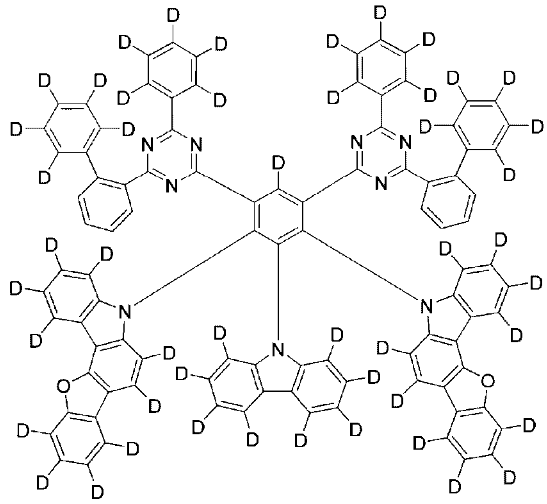

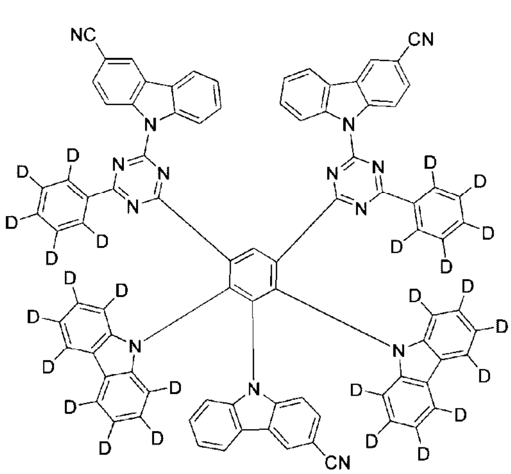

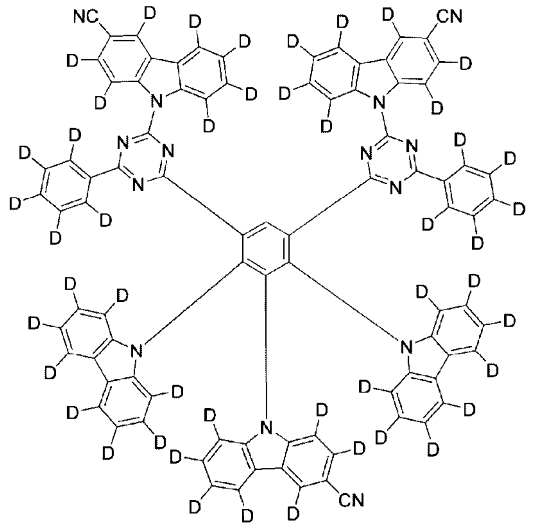

The molecular weight of the compound represented by the general formula (1) is preferably 1500 or less, more preferably 1200 or less, still more preferably 1000 or less, and even more preferably 900 or less, for example, in the case where an organic layer containing the compound represented by the general formula (1) is intended to be film-formed and used by a vapor deposition method. The lower limit of the molecular weight is the molecular weight of the compound having the smallest molecular weight represented by the general formula (1).

The compound represented by the general formula (1) may be formed into a film by a coating method regardless of the molecular weight. When the coating method is used, even a compound having a relatively large molecular weight can be formed into a film. The compound represented by the general formula (1) has an advantage of being easily dissolved in an organic solvent. For this reason, the compound represented by the general formula (1) is easily applicable to a coating method and is easily purified to increase its purity.

It is also conceivable to use a compound containing a plurality of structures represented by the general formula (1) in a molecule as a light-emitting material by applying the present invention.

For example, it is conceivable that a polymer obtained by allowing a polymerizable group to be present in the structure represented by the general formula (1) in advance and polymerizing the polymerizable group is used as the light-emitting material. For example, it is conceivable that a polymer having a repeating unit is obtained by preparing a monomer containing a polymerizable functional group at any site of the general formula (1) and polymerizing the monomer alone or copolymerizing the monomer with another monomer, and the polymer is used as the light-emitting material. Alternatively, it is also conceivable to obtain a dimer or a trimer by coupling compounds having a structure represented by the general formula (1) to each other and to use the dimer or the trimer as a light-emitting material.

Examples of the polymer having a repeating unit containing a structure represented by the general formula (1) include polymers containing a structure represented by any one of the following two general formulae.

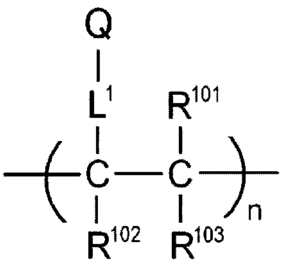 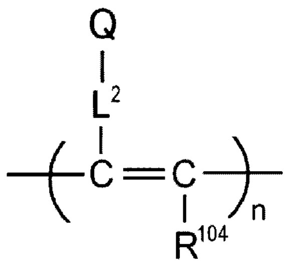

In the above general formulae, Q represents a group containing the structure represented by the general formula (1), and $L^1$ and $L^2$ each represent a linking group. The linking group preferably has 0 to 20 carbon atoms, more preferably 1 to 15 carbon atoms, and still more preferably 2 to 10 carbon atoms. The linking group preferably has a structure represented by $-X^{11}-L^{11}-$. Here, $X^{11}$ represents an oxygen atom or a sulfur atom, and is preferably an oxygen atom. $L^{11}$ represents a linking group, and is preferably a substituted or unsubstituted alkylene group or a substituted or unsubstituted arylene group, and more preferably a substituted or unsubstituted alkylene group having 1 to 10 carbon atoms or a substituted or unsubstituted phenylene group.

In the above general formulae, $R^{101}$, $R^{102}$, $R^{103}$, and $R^{104}$ each independently represent a substituent. The substituent is preferably a substituted or unsubstituted alkyl group having 1 to 6 carbon atoms, a substituted or unsubstituted alkoxy group having 1 to 6 carbon atoms, or a halogen atom, more preferably an unsubstituted alkyl group having 1 to 3 carbon atoms, an unsubstituted alkoxy group having 1 to 3 carbon atoms, a fluorine atom, or a chlorine atom, and still more preferably an unsubstituted alkyl group having 1 to 3 carbon atoms or an unsubstituted alkoxy group having 1 to 3 carbon atoms.

The linking group represented by $L^1$ and $L^2$ can be bonded to any site of the general formula (1) constituting Q. Two or more linking groups may be linked to one Q to form a cross-linked structure or a network structure.

Specific structural examples of the repeating unit include structures represented by the following formulae.

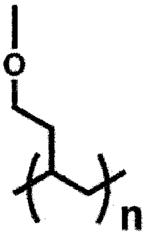 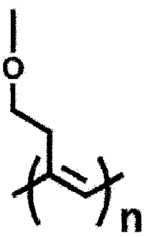 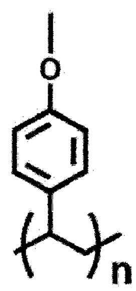 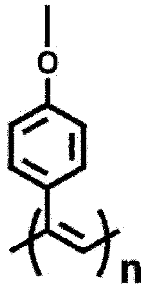

The polymer having a repeating unit including these formulae can be synthesized by introducing a hydroxyl group into any site of the general formula (1), reacting the following compound using the hydroxy group as a linker to introduce a polymerizable group, and polymerizing the polymerizable group.

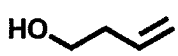 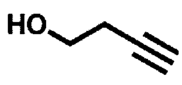 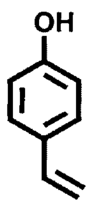 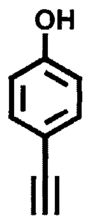

The polymer having a structure represented by the general formula (1) in the molecule may be a polymer having only a repeating unit that has the structure represented by the general formula (1), or may be a polymer containing a repeating unit that has any other structure. The repeating unit having the structure represented by the general formula (1) to be contained in the polymer may be a single kind or two or more kinds. The repeating unit not having the structure represented by the general formula (1) includes those derived from monomers used in general copolymerization. For example, it includes repeating units derived from monomers having an ethylenically unsaturated bond, such as ethylene or styrene.

In some embodiments, the compound represented by the general formula (1) is a light-emitting material.

In some embodiments, the compound represented by the general formula (1) is a compound capable of emitting

delayed fluorescence.

In some embodiments of the present disclosure, the compound represented by the general formula (1) is, when excited thermally or by an electronic means, able to emit light in a UV region, emit light of blue, green, yellow, orange, or red in a visible spectral region (e.g., about 420 nm to about 500 nm, about 500 nm to about 600 nm, or about 600 nm to about 700 nm), or emit light in a near IR region.

In some embodiments of the present disclosure, the compound represented by the general formula (1) is, when excited thermally or by an electronic means, able to emit light of red or orange in a visible spectral region (e.g., about 620 nm to about 780 nm, about 650 nm).

In some embodiments of the present disclosure, the compound represented by the general formula (1) is, when excited thermally or by an electronic means, able to emit light of orange or yellow in a visible spectral region (e.g., about 570 nm to about 620 nm, about 590 nm, about 570 nm).

In some embodiments of the present disclosure, the compound represented by the general formula (1) is, when excited thermally or by an electronic means, able to emit light of green in a visible spectral region (e.g., about 490 nm to about 575 nm, about 510 nm).

In some embodiments of the present disclosure, the compound represented by the general formula (1) is, when excited thermally or by an electronic means, able to emit light of blue in a visible spectral region (e.g., about 400 nm to about 490 nm, about 475 nm).

In some embodiments of the present disclosure, the compound represented by the general formula (1) is, when excited thermally or by an electronic means, able to emit light in a UV spectral region (e.g., about 280 to 400 nm).

In some embodiments of the present disclosure, the compound represented by the general formula (1) is, when excited thermally or by an electronic means, able to emit light in an IR spectral region (e.g., about 780 nm to 2 $\mu$m).

In some embodiments of the present disclosure, an organic semiconductor device using the compound represented by the general formula (1) can be produced. The organic semiconductor device referred to herein may be an organic optical device in which light is interposed or an organic device in which light is not interposed. The organic optical device may be an organic light-emitting device in which the device emits light, an organic light receiving device in which the device receives light, or a device in which energy transfer by light occurs in the device. In some embodiments of the present disclosure, an organic optical device such as an organic electroluminescent device or a solid-state imaging device (for example, a CMOS image sensor) can be produced by using the compound represented by the general formula (1). In some embodiments of the present disclosure, a CMOS (complementary metal-oxide film semiconductor) or the like using the compound represented by the general formula (1) can be produced.

Electronic characteristics of small-molecule chemical substance libraries can be calculated by known *ab initio* quantum chemistry calculation. For example, according to time-dependent density functional theory calculation using 6-31G* as a basis, and a functional group known as Becke's three parameters, Lee-Yang-Parr hybrid functionals, the Hartree-Fock equation (TD-DFT/B3LYP/6-31G*) is analyzed and molecular fractions (parts) having HOMO not lower than a specific threshold value and LUMO not higher than a specific threshold value can be screened.

With that, for example, in the presence of a HOMO energy (for example, ionization potential) of -6.5 eV or more, a donor part ("D") can be selected. On the other hand, for example, in the presence of a LUMO energy (for example, electron affinity) of -0.5 eV or less, an acceptor part ("A") can be selected. A bridge part ("B") is a strong conjugated system, for example, capable of strictly limiting the acceptor part and the donor part in a specific three-dimensional configuration, and therefore prevents the donor part and the acceptor part from overlapping in the $\pi$-conjugated system.

In some embodiments, a compound library is screened using one or more of the following characteristics.

1. Light emission around a specific wavelength
2. A triplet state over a calculated specific energy level
3. $\Delta E_{ST}$ value lower than a specific value
4. Quantum yield more than a specific value
5. HOMO level
6. LUMO level

In some embodiments, the difference ($\Delta E_{ST}$) between the lowest singlet excited state and the lowest triplet excited state at 77 K is less than about 0.5 eV, less than about 0.4 eV, less than about 0.3 eV, less than about 0.2 eV, or less than about 0.1 eV. In some embodiments, $\Delta E_{ST}$ value is less than about 0.09 eV, less than about 0.08 eV, less than about 0.07 eV, less than about 0.06 eV, less than about 0.05 eV, less than about 0.04 eV, less than about 0.03 eV, less than about 0.02 eV, or less than about 0.01 eV.

In some embodiments, the compound represented by the general formula (1) shows a quantum yield of more than 25%, for example, about 30%, about 35%, about 40%, about 45%, about 50%, about 55%, about 60%, about 65%, about 70%, about 75%, about 80%, about 85%, about 90%, about 95% or more.

[Method for Synthesizing Compound Represented by General Formula (1)]

The compound represented by the general formula (1) includes a novel compound.

The compound represented by the general formula (1) can be synthesized by combining known reactions. In the compound represented by the general formula (1), two or more of $R^1$ to $R^5$ are donor groups. For example, the compound represented by the general formula (1) in which a substituted or unsubstituted carbazol-9-yl group is a donor group can be synthesized by reacting a substituted or unsubstituted carbazole with a precursor in which the site of the donor group is a fluorine atom. For details of the reaction conditions, Synthesis Examples described later can be referred to.

[Composition Using Compound Represented by General Formula (1)]

In some embodiments, the compound represented by the general formula (1) is used along with one or more materials (e.g., small molecules, polymers, metals, metal complexes), by combining them, or by dispersing the compound, or by covalent-bonding with the compound, or by coating with the compound, or by carrying the compound, or by associating with the compound, and solid films or layers are formed. For example, by combining the compound represented by the general formula (1) with an electroactive material, a film can be formed. In some cases, the compound represented by the general formula (1) may be combined with a hole transport polymer. In some cases, the compound represented by the general formula (1) may be combined with an electron transport polymer. In some cases, the compound represented by the general formula (1) may be combined with a hole transport polymer and an electron transport polymer. In some cases, the compound represented by the general formula (1) may be combined with a copolymer having both a hole transport moiety and an electron transport moiety. In the embodiments mentioned above, the electrons and/or the holes formed in a solid film or layer can be interacted with the compound represented by the general formula (1).

[Film Formation]

In some embodiments, a film containing the compound represented by the general formula (1) can be formed in a wet process. In the wet process, a solution prepared by dissolving a composition containing the compound of the present invention is applied onto a surface, and then the solvent is removed to form a film. The wet process includes a spin coating method, a slit coating method, an inkjet method (a spraying method), a gravure printing method, an offset printing method, and a flexographic printing method, which, however, are not limitative. In the wet process, an appropriate organic solvent capable of dissolving a composition containing the compound of the present invention is selected and used. In some embodiments, a substituent (e.g., an alkyl group) capable of increasing the solubility in an organic solvent can be introduced into the compound contained in the composition.

In some embodiments, a film containing the compound of the present invention can be formed in a dry process. In some embodiments, a vacuum deposition method is employable as a dry process, which, however, is not limitative. In a case where the vacuum deposition method is employed, the compounds constituting the film may be subjected to co-deposition from individual vapor deposition sources, or may be subjected to co-deposition from a single vapor deposition source formed by mixing the compounds. In a case where the single vapor deposition source is used, a mixed powder prepared by mixing compound powders may be used, or a compression molded body prepared by compression-molding the mixed powder may be used, or a mixture prepared by heating and melting the compounds and cooling the resulting melt may be used. In some embodiments, by performing co-deposition under the condition that the vapor deposition rates (weight reduction rates) of plural compounds contained in a single vapor deposition source coincide or substantially coincide with each other, a film having a composition ratio corresponding to the compositional ratio of the plural compounds contained in the vapor deposition source can be formed. When plural compounds are mixed in the same compositional ratio as the compositional ratio of the film to be formed to prepare a vapor deposition source, a film having a desired compositional ratio can be formed in a simplified manner. In some embodiments, the temperature at which the compounds to be co-deposited have the same weight reduction ratio is specifically defined, and the temperature can be employed as the temperature of co-deposition.

[Use Examples of Compound Represented by General Formula (1)]

The compound represented by the general formula (1) is useful as a material for an organic light-emitting device. In particular, the compound is preferably used for an organic light-emitting diode or the like.

Organic Light-Emitting Diode:

One embodiment of the present invention relates to use of the compound represented by the general formula (1) of the present invention as a light-emitting material for an organic light-emitting device. In some embodiments, the

compound represented by the general formula (1) of the present invention can be effectively used as a light-emitting material in a light-emitting layer in an organic light-emitting device. In some embodiments, the compound represented by the general formula (1) includes a delayed fluorescent material that emits delayed fluorescence (delayed fluorescent substance). In some embodiments, the compound represented by the general formula (1) is applied to Phosphorescence Sensitized Fluorescence. In some embodiments, the present invention provides a delayed fluorescent substance having a structure represented by the general formula (1). In some embodiments, the present invention relates to use of the compound represented by the general formula (1) as a delayed fluorescent substance. In some embodiments, the compound represented by the general formula (1) of the present invention can be used as a host material, and can be used along with one or more light-emitting materials, and the light-emitting material can be a fluorescent material, a phosphorescent material, or a TADF. In some embodiments, the compound represented by the general formula (1) can be used as a hole transport material. In some embodiments, the compound represented by the general formula (1) can be used as an electron transport material. In some embodiments, the present invention relates to a method of generating delayed fluorescence from the compound represented by the general formula (1). In some embodiments, the organic light-emitting device containing the compound as a light-emitting material emits delayed fluorescence and shows a high light emission efficiency.

In some embodiments, the light-emitting layer contains the compound represented by the general formula (1), and the compound represented by the general formula (1) is aligned in parallel to a substrate. In some embodiments, the substrate is a film-forming surface. In some embodiments, the alignment of the compound represented by the general formula (1) relative to the film-forming surface has some influence on the propagation direction of light emitted by the aligned compounds, or determines the direction. In some embodiments, by aligning the propagation direction of light emitted by the compound represented by the general formula (1), the light extraction efficiency from the light-emitting layer can be improved.

For details of the use in organic light-emitting devices, reference can be made to [0063] to [0099] of WO2022/168956A1, which is incorporated herein by reference. For example, the compound represented by the general formula (1) can be used in combination with host materials described in [0123] to [0145] of WO2022/270602A1, which is incorporated herein by reference, or can be used in combination with dopants described in [0006] to [0129] of WO2022/270354A1, which is incorporated herein by reference.

Examples

The features of the present invention will be described more specifically with reference to Synthesis Examples and Examples given below. The materials, processes, procedures and the like shown below can be appropriately modified unless they deviate from the substance of the present invention. Accordingly, the scope of the present invention is not construed as being limited to the specific examples shown below. Hereunder, the light emission characteristics were evaluated using a source meter (available from Keithley Instruments, Inc.: 2400 series), a semiconductor parameter analyzer (available from Agilent Technologies, Inc., E5273A), an optical power meter device (available from Newport Corporation, 1930C), an optical spectroscope (available from Ocean Optic, Inc., USB2000), a spectroradiometer (available from Topcon Corporation, SR-3), and a streak camera (available from Hamamatsu Photonics K.K., Model C4334).

(Synthesis Example 1) Synthesis of Compound C1

Compound c

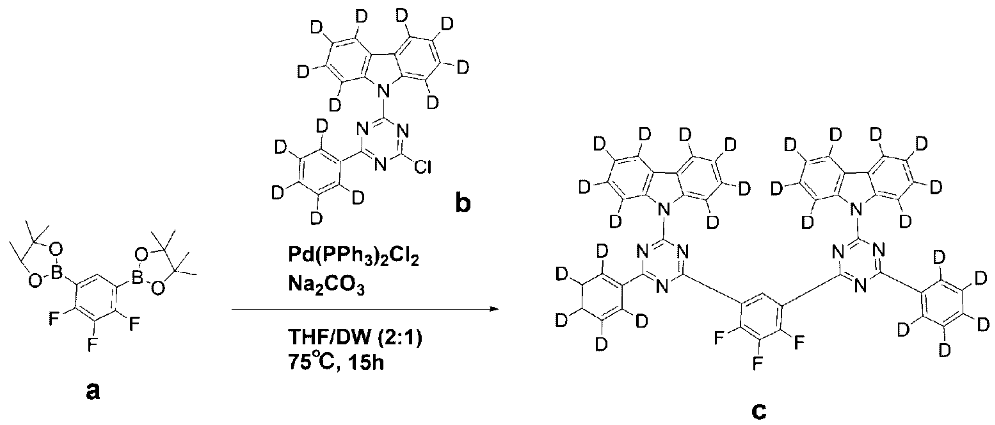


Compound a (5.29 g, 13.7 mmol) in a mixed solvent of tetrahydrofuran (THF, 140 mL) and water (70 mL) was subjected to nitrogen bubbling. To this solution, compound b (10.4 g, 28.1 mmol), bis(triphenylphosphine)palladium (II) dichloride (0.96 g, 1.37 mmol), and sodium carbonate (7.31 g, 69.0 mmol) were added under a nitrogen stream, and the mixture was heated and stirred at 75°C for 15 hours. The reaction vessel was cooled to room temperature and quenched by the addition of water. The solution was filtered and the obtained gray solid was separated by filtration, and washed with dichloromethane and ethyl acetate. The solid was dissolved in heated 1,2-dichlorobenzene (ODCB) and, when hot, passed through a funnel (Kiriyama funnel) packed with celite/silica gel/celite. The solution was concentrated under reduced pressure by an evaporator, and the obtained solid was washed with toluene and dried to obtain Compound c as a pale yellow solid (7.24 g, 9.06 mmol, yield 66%).
ASAP MS Spectral Analysis: $C_{48}HD_{26}F_3N_8$: theoretical value 798.39, observed value 799.46.

Compound C1

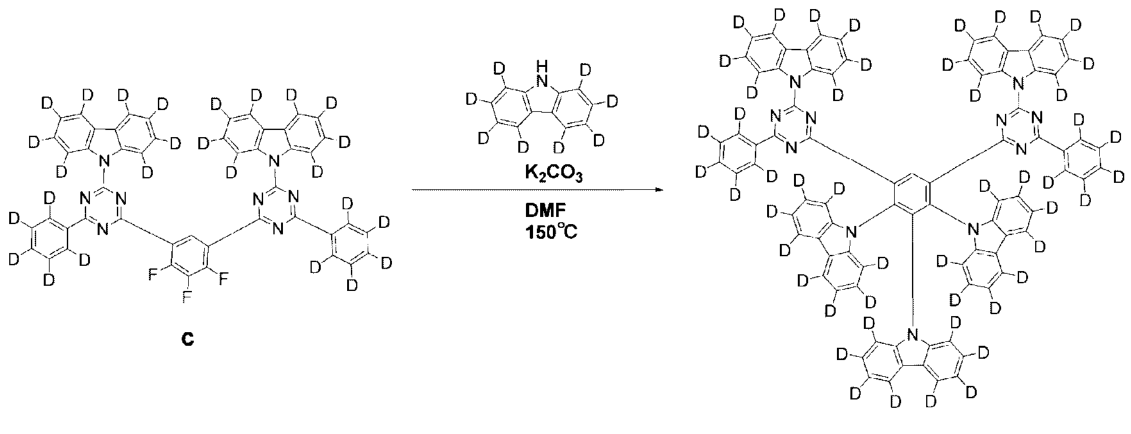


C1

Under a nitrogen stream, potassium carbonate (0.57 g, 4.15 mmol) was added to a solution of Compound c (0.83 g, 1.04 mmol) and carbazole-1,2,3,4,5,6,7,8-d8 (0.60 g, 3.42 mmol) in N,N-dimethylformamide (DMF, 40 mL), and the mixture was stirred at 150°C for 3 hours. The reaction solution was cooled to room temperature and quenched by the addition of water. The solution was filtered and the solid remaining in the funnel was washed with water. The solid was dissolved in dichloromethane and dried over magnesium sulfate. The solution was concentrated under reduced pressure in an evaporator and then purified by column chromatography (toluene:hexane = 1:1) to obtain a yellow Compound C1 (0.35 g, 0.276 mmol, yield 27%).

$^1$H-NMR (400 MHz, $CDCl_3$):$\delta$ 9.26 (s, 1H)
ASAP MS Spectral Analysis: $C_{84}HD_{50}N_{11}$: theoretical value 1263.7, observed value 1263.1.

(Synthesis Example 2) Synthesis of Compound C2

Compound C2

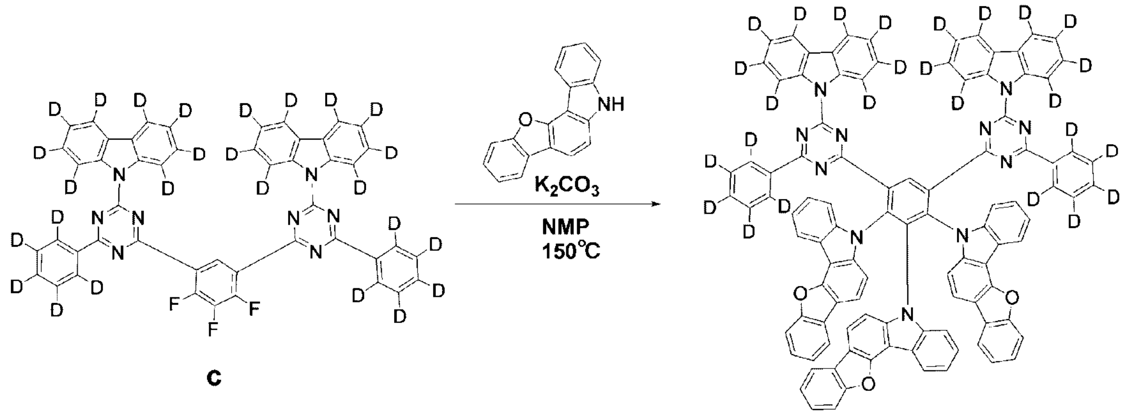


c

C2

Under a nitrogen stream, potassium carbonate (0.34 g, 2.50 mmol) was added to a solution of Compound c (0.50 g, 0.62 mmol) and SH-benzofuro[3,2-c]carbazole (0.48 g, 1.87 mmol) in N-methyl-2-pyrrolidone (NMP, 30 mL), and the mixture was stirred at 150°C for 3 hours. The reaction solution was cooled to room temperature and quenched by the addition of water. The solution was filtered and the solid remaining in the funnel was washed with water. The solid was dissolved in dichloromethane and dried over magnesium sulfate. The solution was concentrated under reduced pressure in an evaporator and then purified by column chromatography (toluene:hexane = 1:1) to obtain a yellow Compound C2 (0.44 g, 0.29 mmol, yield 47%).
ASAP MS Spectral Analysis: $C_{102}H_{31}D_{26}N_{11}O_3$: theoretical value 1509.6, observed value 1510.7.

(Synthesis Example 3) Synthesis of Compound C3

Compound d

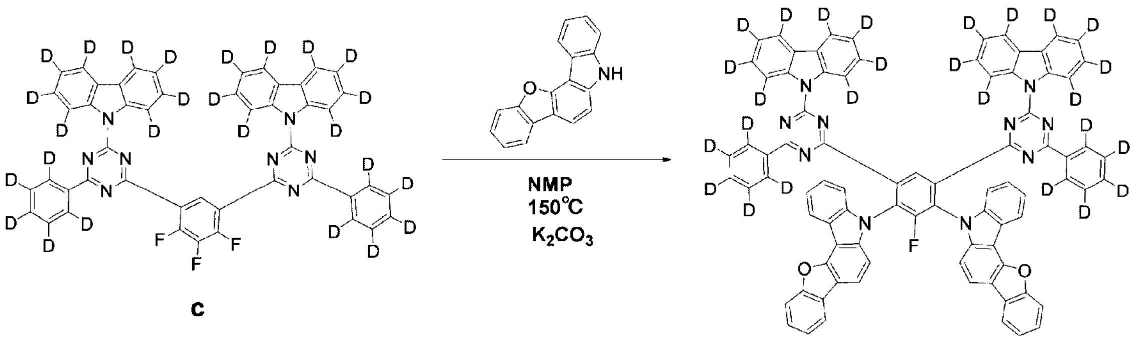


c

d

Under a nitrogen stream, potassium carbonate (0.86 g, 6.25 mmol) was added to a solution of Compound c (2.0 g, 2.50 mmol) and SH-benzofuro[3,2-c]carbazole (1.28 g, 5.00 mmol) in NMP (90 mL), and the mixture was stirred at 150°C for 3 hours. The reaction solution was cooled to room temperature and quenched by the addition of water. The solution was filtered and the solid remaining in the funnel was washed with water. The solid was dissolved in dichloromethane and dried over magnesium sulfate. The solution was concentrated under reduced pressure in an evaporator and then purified by column chromatography (toluene:hexane = 1:1) to obtain a yellow Compound d (1.93 g, 1.51 mmol, yield 61%).

$^1$H-NMR (400 MHz, $CDCl_3$): δ 9.40 (s, 1H), 8.40-8.30 (m, 2H), 8.12-7.90 (m, 4H), 7.80-7.30 (m, 14H).
ASAP MS Spectral Analysis: $C_{84}H_{21}D_{26}FN_{10}O_2$: theoretical value 1272.5, observed value 1272.8.

Compound C3

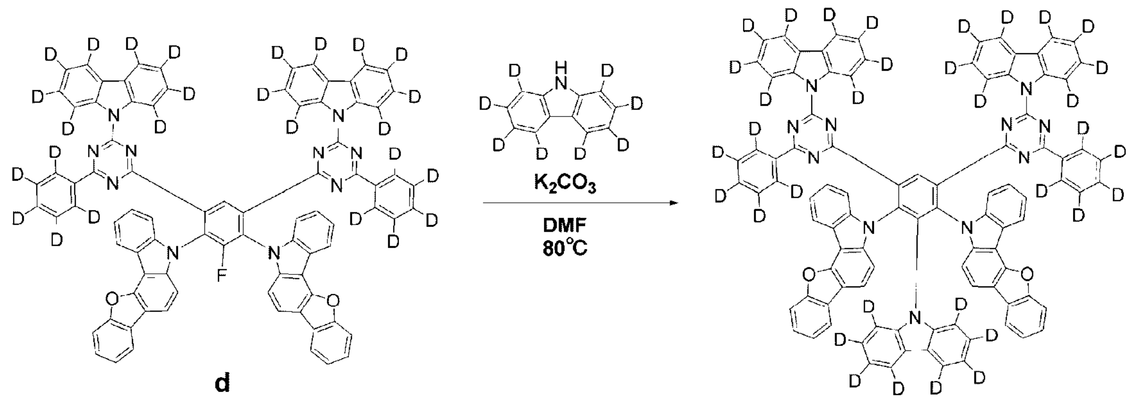


d

C3

Under a nitrogen stream, potassium carbonate (0.76 g, 5.52 mmol) was added to a solution of Compound d (1.76 g, 1.38 mmol) and carbazole-1,2,3,4,5,6,7,8-d8 (0.72 g, 4.14 mmol) in DMF (50 mL), and the mixture was stirred at 80°C for 72 hours. The reaction solution was cooled to room temperature and quenched by the addition of water. The solution was filtered and the solid remaining in the funnel was washed with water. The solid was dissolved in dichloromethane and dried over magnesium sulfate. The solution was concentrated under reduced pressure in an evaporator and then purified by column chromatography (toluene:hexane = 1:1) to obtain a yellow Compound C3 (0.30 g, 0.21 mmol, yield 15%).

$^1$H-NMR (400 MHz, $CDCl_3$): δ 9.37 (s, 1H), 7.97 (dd, J=4.8Hz, 1.2 Hz, 2H), 7.80 (d, J=8.4Hz, 2H), 7.61-7.56 (m, 2H), 7.51 (d, J=8.4Hz, 2H), 7.45-7.35 (m, 4H),7.34-7.22 (m, 4H), 7.10-7.00 (m, 4H).
ASAP MS Spectral Analysis: $C_{96}H_{21}D_{34}N_{11}O_2$: theoretical value 1427.6, observed value 1427.8.

(Synthesis Example 4) Synthesis of Compound C4

Compound e

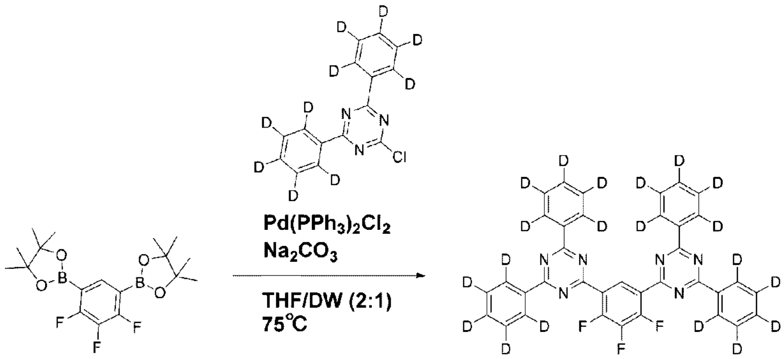


a

e

Compound a (2.30 g, 5.99 mmol) in a mixed solvent of THF (60 mL) and water (30 mL) was subjected to nitrogen bubbling. To this solution, 2-chloro-4,6-(diphenyl-2,3,4,5,6-d5)-1,3,5-triazine (3.39 g, 12.2 mmol), bis(triphenylphosphine) palladium (II) dichloride (0.42 g, 0.59 mmol), and sodium carbonate (3.17 g, 29.9 mmol) were added under a nitrogen stream, and the mixture was heated and stirred at 75°C for 18 hours. The reaction vessel was cooled to room temperature and quenched by the addition of water. The solution was filtered and the obtained gray solid was separated by filtration, and washed with dichloromethane and ethyl acetate. The solid was dissolved in heated ODCB and, when hot, passed through a funnel (Kiriyama funnel) packed with celite/silica gel/celite. The solution was concentrated under reduced pressure by an evaporator, and the obtained solid was washed with toluene and dried to obtain Compound e as a pale yellow solid (2.54 g, 4.13 mmol, yield 69%).
ASAP MS Spectral Analysis: $C_{36}HD_{20}F_3N_6$: theoretical value 614.30, observed value 615.40.

Compound f

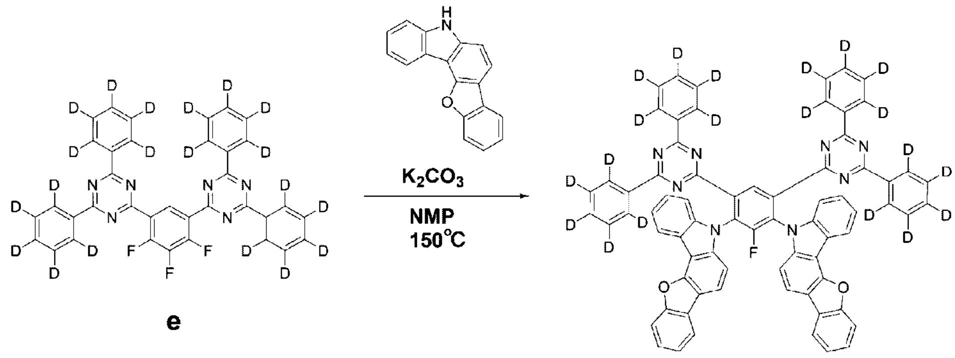


e

f

Under a nitrogen stream, potassium carbonate (1.12 g, 8.13 mmol) was added to a solution of Compound e (2.0 g, 3.25 mmol) and SH-benzofuro[3,2-c]carbazole (1.67 g, 6.50 mmol) in NMP (120 mL), and the mixture was stirred at 150°C for 3 hours. The reaction solution was cooled to room temperature and quenched by the addition of water. The solution was filtered through a funnel packed with celite (Kiriyama funnel), and the solid remaining in the funnel was washed with water. This was dissolved in dichloromethane and dried over magnesium sulfate. The solution was filtered, and the obtained solution was concentrated under reduced pressure in an evaporator and then purified by column chromatography (toluene:hexane = 1:1), and the obtained solid was washed with methanol to obtain a yellow Compound f (2.61 g, 2.39 mmol, yield 73%).

$^1$H-NMR(400 MHz, $CDCl_3$): δ 9.61 (d, J=1.6 Hz, 1H), 8.46 (d, J=7.6 Hz, 2H), 8.00-7.96 (m, 4H), 7.70 (d, J=8.4 Hz, 2H), 7.48-7.38 (m, 12H).

ASAP MS Spectral Analysis: $C_{72}H_{21}D_{20}FN_8O_2$: theoretical value 1088.46, observed value 1089.66.

Compound C4

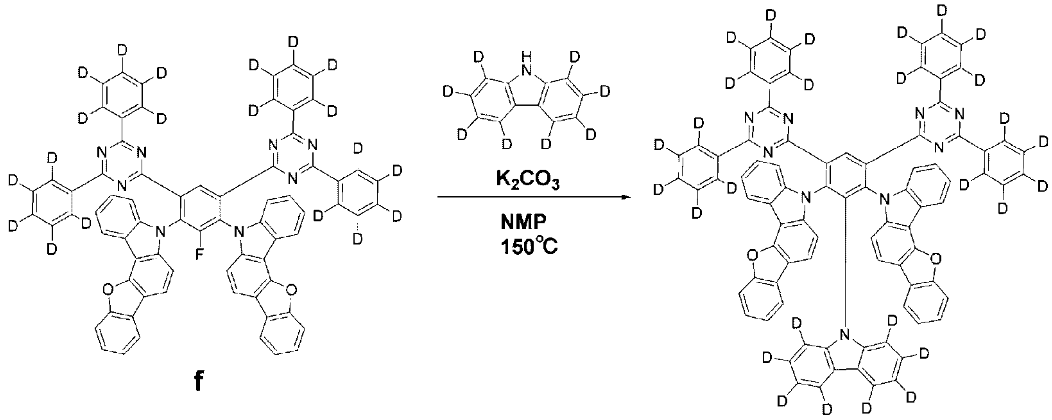


f

C4

Under a nitrogen stream, potassium carbonate (0.41 g, 2.96 mmol) was added to a solution of Compound f (1.61 g, 1.47 mmol) and carbazole-1,2,3,4,5,6,7,8-d8 (0.31 g, 1.77 mmol) in NMP (50 mL), and the mixture was stirred at 150°C for 3 hours. The reaction solution was cooled to room temperature and quenched by the addition of water. The solution was filtered and the solid remaining in the funnel was washed with water. The solid was dissolved in dichloromethane and dried over magnesium sulfate. The solution was concentrated under reduced pressure in an evaporator and then purified by column chromatography (toluene:hexane = 1:1) to obtain a yellow Compound C4 (1.59 g, 1.27 mmol, yield 86%).

$^1$H-NMR (400 MHz, $CDCl_3$): δ 9.49 (s, 1H), 8.01 (d, J=7 Hz, 2H), 7.83(d, J= 7Hz, 2H) 7.59-7.52(m, 4H), 7.38-7.28 (m, 8H), 7.10-7.12(m,4H).

ASAP MS Spectral Analysis: $C_{84}H_{21}D_{28}N_9O_2$: theoretical value 1243.58, observed value 1244.73.

(Synthesis Example 5) Synthesis of Compound C5

Compound C5

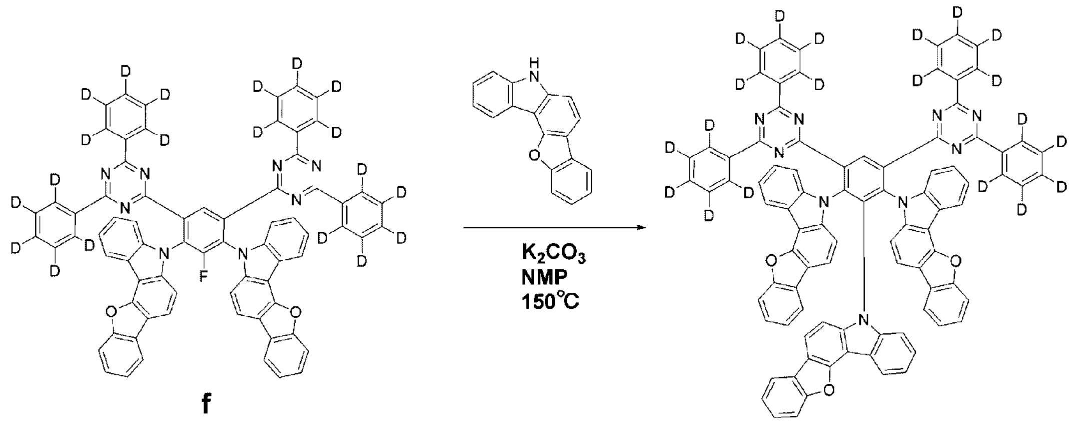


f

C5

Under a nitrogen stream, potassium carbonate (0.25 g, 1.83 mmol) was added to a solution of Compound f (1.0 g, 0.91 mmol) and SH-benzofuro[3,2-c]carbazole (0.28 g, 1.10 mmol) in NMP (50 mL), and the mixture was stirred at 150°C for 3 hours. The reaction solution was cooled to room temperature and quenched by the addition of water. The solution was filtered and the solid remaining in the funnel was washed with water. The solid was dissolved in dichloromethane and dried over magnesium sulfate. The solution was concentrated under reduced pressure in an evaporator, then, purified by column chromatography (toluene: hexane = 1:1), washed with methanol, and recrystallized (toluene) to obtain a yellow Compound C5 (0.99 g, 0.746 mmol, yield 82%).

$^1$H-NMR (400 MHz, $CDCl_3$): δ 9.53 (s, 1H), 8.0-6.76(m, 30H)
ASAP MS Spectral Analysis: $C_{90}H_{31}D_{20}N_9O_3$: theoretical value 1325.54, observed value 1327.79,

(Synthesis Example 6) Synthesis of Compound C6

Compound h

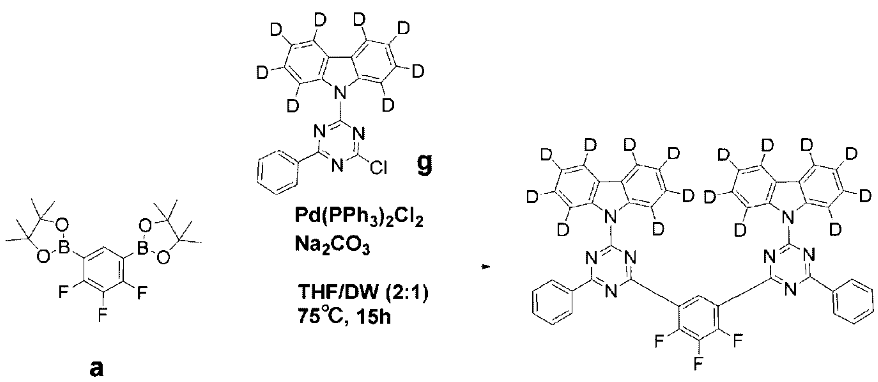


a

h

Compound a (4.84 g, 12.6 mmol) in a mixed solvent of THF (200 mL) and water (80 mL) was subjected to nitrogen bubbling. To this solution, compound g (9.40 g, 25.7 mmol), bis(triphenylphosphine)palladium (II) dichloride (0.88 g, 1.26 mmol), and sodium carbonate (6.69 g, 105.9 mmol) were added under a nitrogen stream, and the mixture was heated and stirred at 75°C for 15 hours. The reaction vessel was cooled to room temperature and quenched by the addition of water.

The solution was filtered, and the obtained gray solid was separated by filtration and washed with water and toluene to obtain Compound h as a gray solid (9.2 g, 11.6 mmol, yield 92%).
ASAP MS Spectral Analysis: $C_{48}H_{11}D_{16}F_3N_8$: theoretical value 788.3, observed value 789.62.

Compound C6

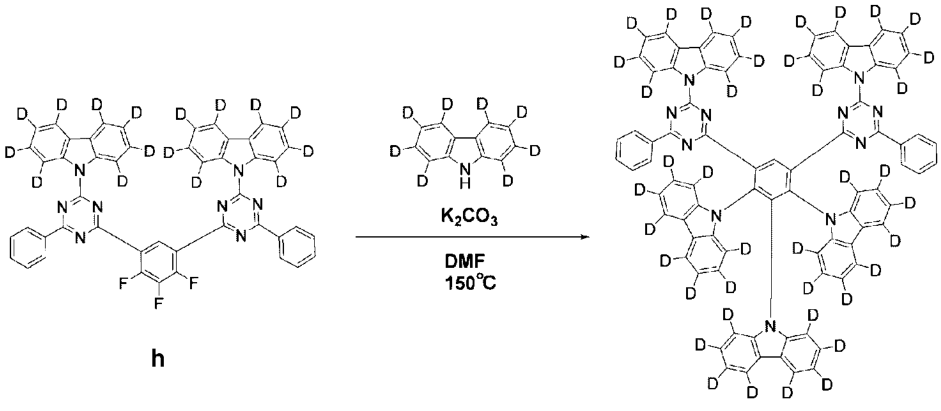


h

C6

Under a nitrogen stream, potassium carbonate (1.05 g, 7.60 mmol) was added to a solution of Compound h (1.50 g, 1.90 mmol) and carbazole-1,2,3,4,5,6,7,8-d8 (1.33 g, 7.60 mmol) in DMF (70 mL), and the mixture was stirred at 150°C for 3 hours. The reaction solution was cooled to room temperature and quenched by the addition of water. The solution was filtered and the solid remaining in the funnel was washed with water. The solid was dissolved in dichloromethane and dried over magnesium sulfate. The solution was concentrated under reduced pressure in an evaporator and then purified by column chromatography (toluene:hexane = 1:1) to obtain a yellow Compound C6 (0.66 g, 0.52 mmol, yield 28%).

$^1$H-NMR (400 MHz, $CDCl_3$): δ 9.25 (s, 1H), 7.52-7.48(m, 4H), 7.42-7.38 (m, 2H), 7.26-7.16(m,4H)
ASAP MS Spectral Analysis: $C_{84}H_{11}D_{40}N_{11}$: theoretical value 1253.6, observed value 1254.0.

(Synthesis Example 7) Synthesis of Compound C7

Compound j

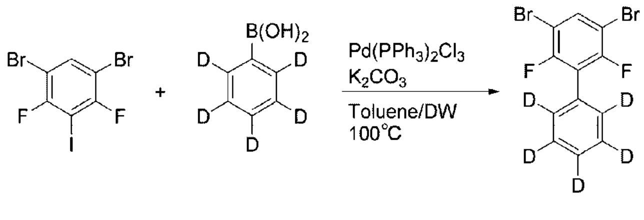


j

To a mixed solution of 1,5-dibromo-2,4-difluoro-3-iodobenzene (15.8 g, 39.9 mmol) in toluene (100 mL) and ion-exchanged water (30 mL), phenyl-d5-boronic acid (6.2 g, 48.8 mmol), bis(triphenylphosphine)palladium (II) dichloride (1.4 g, 2.0 mmol), and potassium carbonate (29.0 g, 210 mmol) were added, and the mixture was stirred at 100°C for 23 hours under a nitrogen atmosphere. The reaction solution was cooled to room temperature, and saturated saline water was added thereto to separate the solution into an organic phase and an aqueous phase. After the aqueous phase was extracted with toluene, the combined organic phase was dried over anhydrous magnesium sulfate and filtered, and then the filtrate was concentrated. The crude product was purified by silica gel chromatography (hexane) to obtain Compound j as a white solid (9.0 g, 25.5 mmol, yield 64%).

$^1$H-NMR (400 MHz, $CDCl_3$): δ 7.77 (t, J=6.8 Hz, 1H).
ASAP MS Spectral Analysis: $C_{12}HD_5Br_2F_2$: theoretical value 350.91, observed value 350.93 [M]

Compound k

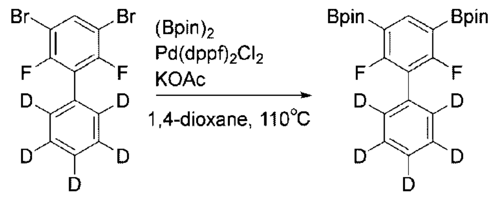


j k

A reaction mixture of Compound j (2.1 g, 5.98 mmol), [1,1'-bis(diphenylphosphino)ferrocene] dichloropalladium (II) (0.22 g, 0.30 mmol), potassium acetate (4.1 g, 42.0 mmol), bis(pinacolato)diboron (7.1 g, 27.9 mmol), and 1,4-dioxane (60 mL) was stirred at 110°C for 15 hours. The reaction mixture was cooled to room temperature, diluted with ethyl acetate, and filtered through silica. After the filtrate was concentrated, the obtained reaction mixture was dissolved in methylene chloride and subjected to silica filtration. The obtained solid was washed with hexane to obtain Compound k as a white solid (1.84 g, 4.11 mmol, yield 69%).

$^1$H-NMR (400 MHz, $CDCl_3$): δ 8.11 (t, J=7.2 Hz, 1H), 1.36 (s, 24H).

Compound m

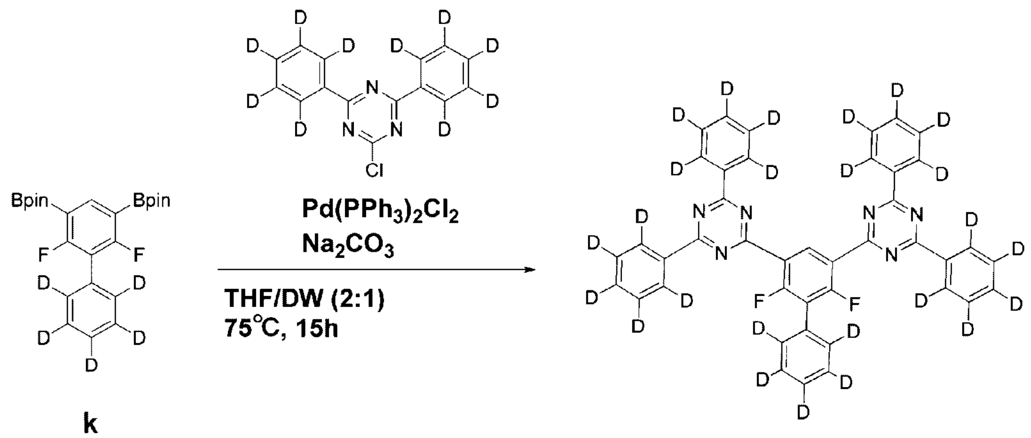


k

m

Compound k (8.00 g, 17.89 mmol) was dissolved in THF (138 mL) and ion-exchanged water (46 mL), and 2-chloro-4,6-bis(phenyl-d5)-1,3,5-triazine (4.35 g, 35.78 mmol), bis(triphenylphosphine)palladium (II) dichloride (0.63 g, 0.89 mmol) and sodium carbonate (9.48 g, 89.46 mmol) were added thereto, and the mixture was stirred at 75°C for 15 hours. The reaction vessel was cooled to room temperature, and the obtained gray solid was separated by filtration. The solid was washed with ion-exchanged water, methanol, and THF. The collected solid was stirred in hot toluene, and the solid was separated by filtration and washed with toluene to obtain Compound m as a black solid (4.1 g, 6.05 mmol, yield 34%).

ASAP MS Spectral Analysis: $C_{42}HD_{25}F_2N_6$: theoretical value 677, observed value 678 [$M+H^+$].

Compound C7

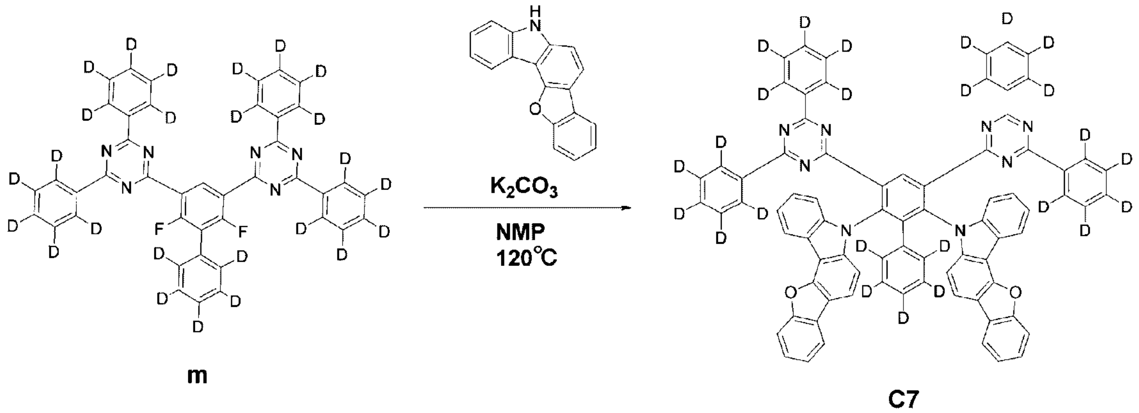


m

C7

Under a nitrogen stream, potassium carbonate (1.07 g, 7.74 mmol) was added to a solution of Compound m (1.75 g, 2.58 mmol) and SH-benzofuro[3,2-c]carbazole (1.60 g, 6.22 mmol) in NMP (90 mL), and the mixture was stirred at 120°C for 18 hours. The reaction solution was cooled to room temperature and quenched by the addition of water. The solution was filtered and the solid remaining in the funnel was washed with water. The solid was dissolved in dichloromethane and dried over magnesium sulfate. The solution was concentrated under reduced pressure in an evaporator, then, purified by column chromatography (toluene: hexane = 1:1), washed with methanol, and recrystallized (toluene) to obtain a yellow Compound C7 (0.80 g, 0.69 mmol, yield 27%).

$^1$H-NMR (400 MHz, $CDCl_3$): δ 9.51 (s, 1H), 7.57 (d, J=7.2Hz, 2H), 7.94-7.87 (m, 4H), 7.62 (d, J=7.2Hz, 2H), 7.38-7.24 (m, 12H).
ASAP MS Spectral Analysis: $C_{78}H_{21}D_{25}N_8O_2$: theoretical value 1151.5, observed value 1151.6.

(Synthesis Example 8) Synthesis of Compound C8

Compound n

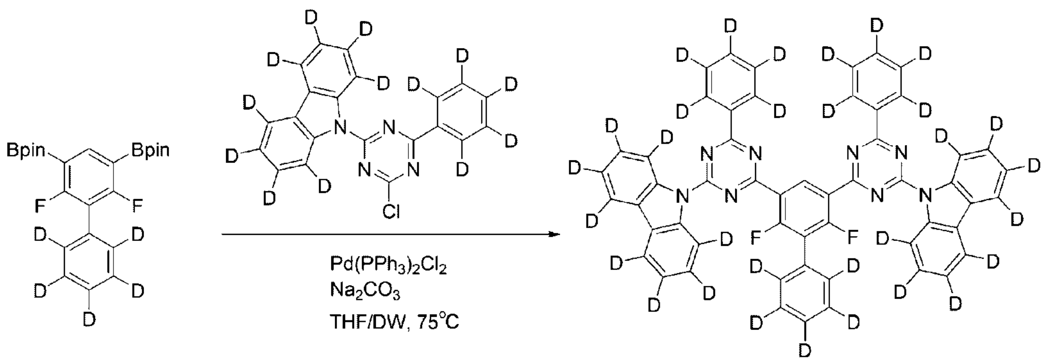


k

n

Compound k (1.81 g, 4.05 mmol) was dissolved in THF (30 mL) and ion-exchanged water (10 mL), and 9-(4-chloro-6-(phenyl-d5)-1,3,5-triazin-2-yl)-9H-carbazole-1,2,3,4,5,6,7,8-d8 (3.05 g, 8.25 mmol), bis(triphenylphosphine) palladium (II) dichloride (0.15 g, 0.21 mmol), and sodium carbonate (2.20 g, 20.8 mmol) were added thereto, and the mixture was stirred at 75°C for 16 hours. The reaction vessel was cooled to room temperature, and the obtained gray solid was separated by filtration. The solid was washed with ion-exchanged water, methanol, and THF. The collected solid was stirred in hot toluene, and the solid was separated by filtration and washed with toluene to obtain Compound n as a black solid (3.30 g, 3.82 mmol, yield 94%).
ASAP MS Spectral Analysis: $C_{54}HD_{31}F_2N_8$: theoretical value 861.47, observed value 862.72 [$M+H^+$].

Compound C8

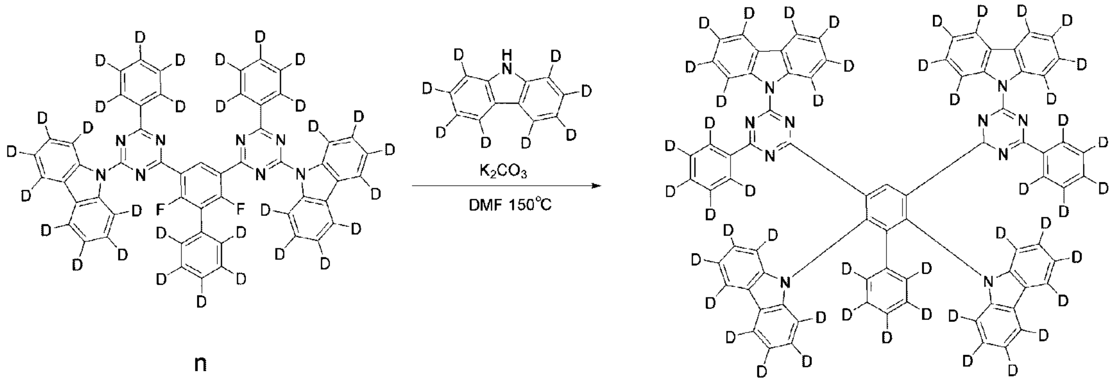


n

C8

To a mixture of Compound n (2.85 g, 3.30 mmol) and DMF (65 mL), carbazole-1,2,3,4,5,6,7,8-d8 (1.28 g, 7.30 mmol) and potassium carbonate (1.37 g, 9.91 mmol) were added, and the mixture was stirred at 150°C for 3 hours. The reaction solution was cooled to room temperature, and the solid was filtered and washed with ethyl acetate. The obtained filtrate was concentrated, methanol was added thereto, and the resulting solid was separated by filtration and washed with methanol. The obtained crude product was purified by column chromatography (toluene/hexane = 3:1), and the obtained solid was reprecipitated with ethyl acetate/hexane, thereby obtaining 2.13 g (1.81 mmol, yield 55%) of a pale green Compound C8.

$^1$H-NMR (400 MHz, DMSO-d6): δ9.20 (s, 1H).
ASAP MS Spectral Analysis: $C_{78}HD_{47}N_{10}$: theoretical value 1171.70, observed value 1172.15 [M+H$^+$].

(Synthesis Example 9) Synthesis of Compound C9

Compound p

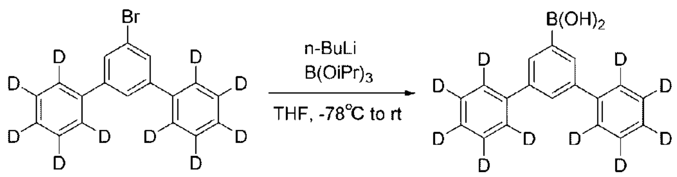


p

A 2.3M solution of n-butyllithium in cyclohexane (21 mL, 48.3 mmol) was slowly added dropwise to a THF (75 mL) solution of 5'-bromo-1,1':3',1"-terphenyl-2,2",3,3",4,4",5,5",6,6"-d10 (12.0 g, 37.6 mmol) under a nitrogen atmosphere at -78°C. After stirring for 80 minutes, triisopropyl borate (12.8 mL, 56.4 mmol) was added, and the temperature of the reactor was raised to room temperature. After stirring for 2 hours, the mixture was cooled to 0°C, and a 4N hydrochloric acid aqueous solution (70 mL) was added thereto. After stirring for 30 minutes at room temperature, the organic and aqueous phases were separated. The aqueous phase was extracted with diethyl ether, and the combined organic phase was washed with ion-exchanged water and saturated saline water. The organic phase was concentrated, and the obtained white solid was washed with hexane to obtain a white Compound p (9.30 g, 32.7 mmol, yield 87%).
$^1$H-NMR (400 MHz, DMSO-d6): δ8.26 (brs, 2H), 8.08 (d, J=1.6 Hz, 2H), 7.92-7.93 (m, 1H).

Compound q

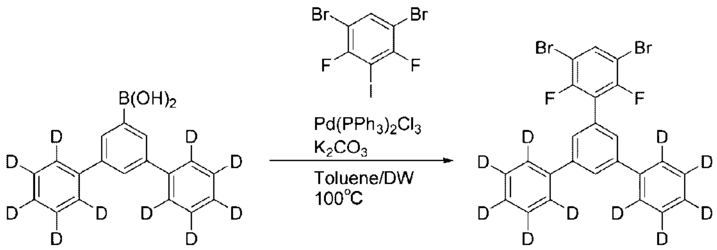


p q

To a mixed solution of 1,5-dibromo-2,4-difluoro-3-iodobenzene (5.1 g, 13.0 mmol) in toluene (30 mL) and ion-exchanged water (10 mL), Compound p (4.0 g, 14.0 mmol), bis(triphenylphosphine)palladium (II) dichloride (0.46 g, 0.64 mmol), and potassium carbonate (3.60 g, 26.0 mmol) were added, and the mixture was stirred at 100°C for 15 hours. The reaction solution was cooled to room temperature, and saturated saline water was added thereto to separate the solution into an organic phase and an aqueous phase. After the aqueous phase was extracted with ethyl acetate, the combined organic phase was dried over anhydrous magnesium sulfate and filtered, and then the filtrate was concentrated. The crude product was purified by silica gel chromatography (hexane/ethyl acetate = 40:1) and (hexane/methylene chloride = 10:1), and the obtained white solid was washed with hexane to obtain 4.84 g (9.48 mmol, yield 73%) of Compound q.

$^1$H-NMR (400 MHz, $CDCl_3$): δ 7.88 (t, J=2.0 Hz, 1H), 7.81 (t, J=6.8 Hz, 1H), 7.62 (q, J=2.0 Hz, 2H).
ASAP MS Spectral Analysis: $C_{24}H_4D_{10}Br_2F_2$: theoretical value 508.00, observed value 509.03 [M+H$^+$].

Compound r

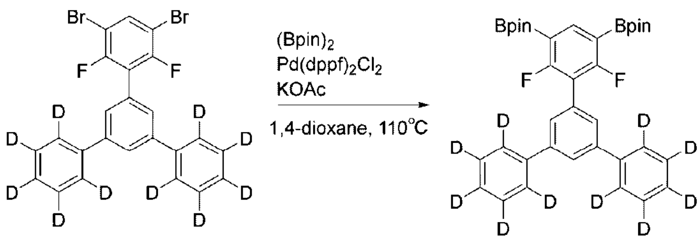


q r

A reaction mixture of Compound q (4.8 g, 9.40 mmol), [1,1'-bis(diphenylphosphino)ferrocene] dichloropalladium (II) (0.36 g, 0.50 mmol), potassium acetate (6.5 g, 66.2 mmol), bis(pinacolato)diboron (12.1 g, 47.6 mmol), and 1,4-dioxane (100 mL) was stirred at 110°C for 15 hours. The reaction mixture was cooled to room temperature, diluted with ethyl acetate, and filtered through silica. After the filtrate was concentrated, the obtained reaction mixture was dissolved in methylene chloride and subjected to silica filtration. The obtained solid was washed with hexane to obtain Compound r as a yellow solid (5.00 g, 8.27 mmol, yield 88%).

$^1$H-NMR (400 MHz, $CDCl_3$): δ 8.15 (t, J=6.8 Hz, 1H), 7.79-7.80 (m, 1H), 7.66 (brs, 2H), 1.26 (s, 24H).
ASAP MS Spectral Analysis: $C_{36}H_{28}D_{10}B_2F_2O_4$: theoretical value 604.37, observed value 605.47 [M+H$^+$].

Compound s

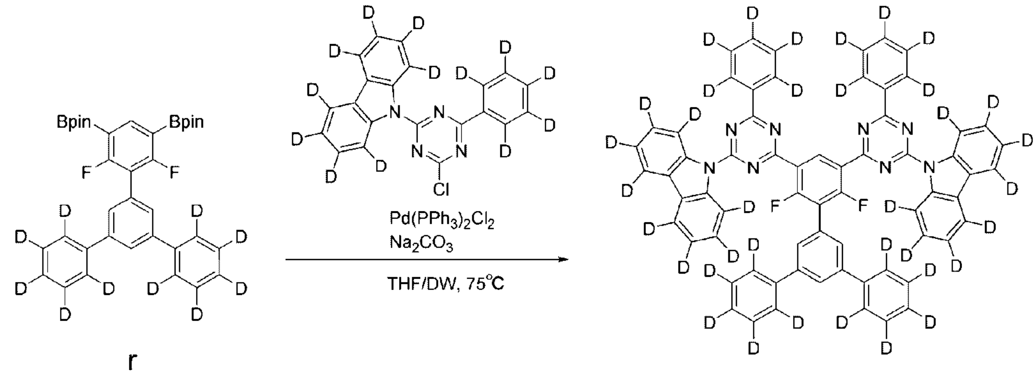


r

s

Compound r (4.98 g, 8.24 mmol) was dissolved in THF (60 mL) and ion-exchanged water (20 mL), and 9-(4-chloro-6-(phenyl-d5)-1,3,5-triazin-2-yl)-9H-carbazole-1,2,3,4,5,6,7,8-d8 (6.10 g, 16.5 mmol), bis(triphenylphosphine) palladium (II) dichloride (0.29 g, 0.41 mmol), and sodium carbonate (4.37 g, 41.2 mmol) were added thereto, and the mixture was stirred at 75°C for 20 hours. The reaction vessel was cooled to room temperature, and the obtained gray solid was separated by filtration. The solid was washed with THF and ion-exchanged water. The collected solid was stirred in hot toluene, and the solid was separated by filtration, washed with toluene, and dried to obtain Compound s as a gray solid (7.98 g, 7.83 mmol, yield 95%).
ASAP MS Spectral Analysis: $C_{66}H_4D_{36}F_2N_8$: theoretical value 1018.56, observed value 1019.83 [M+H$^+$].

Compound C9

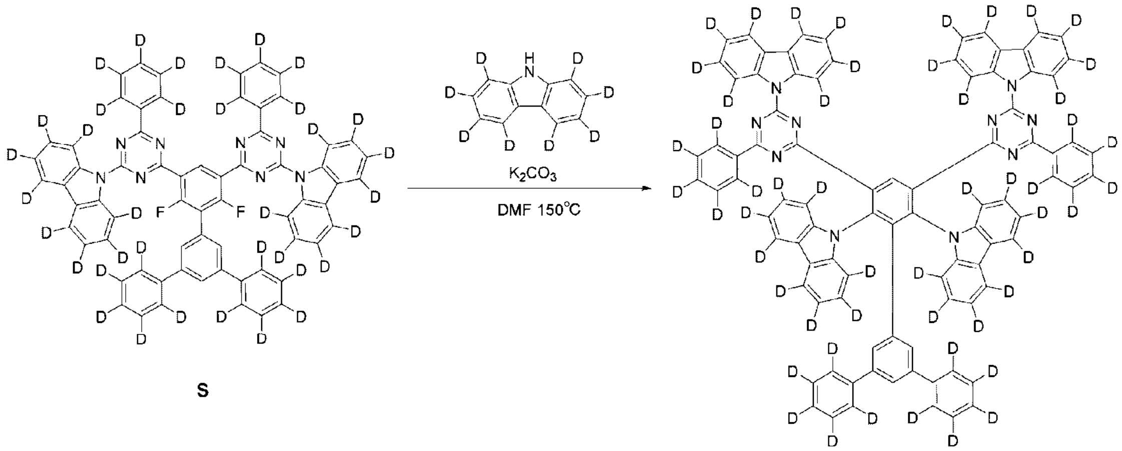


s

C9

To a mixture of Compound s (3.06 g, 3.00 mmol) and DMF (60 mL) were added carbazole-1,2,3,4,5,6,7,8-d8 (1.16 g, 6.61 mmol) and potassium carbonate (1.24 g, 8.97 mmol), and the mixture was stirred at 150°C for 3 hours. The reaction solution was cooled to room temperature, and the solid was filtered and washed with ethyl acetate. The obtained filtrate was concentrated, methanol was added thereto, and the resulting solid was separated by filtration and washed with methanol. The obtained crude product was purified by column chromatography (toluene/hexane = 1:1 to 2:1), and the obtained solid was reprecipitated with ethyl acetate/hexane, thereby obtaining 1.47 g (1.10 mmol, yield 37%) of a green Compound C9.

$^1$H-NMR (400 MHz, DMSO-d6): δ9.40 (s, 1H), 7.14 (d, J=1.6 Hz, 2H), 7.07 (d, J=1.6 Hz, 1H).
ASAP MS Spectral Analysis: $C_{90}H_4D_{52}N_{10}$: theoretical value 1328.80, observed value 1329.84 [M+H$^+$].

(Synthesis Example 10) Synthesis of Compound C10

Compound t

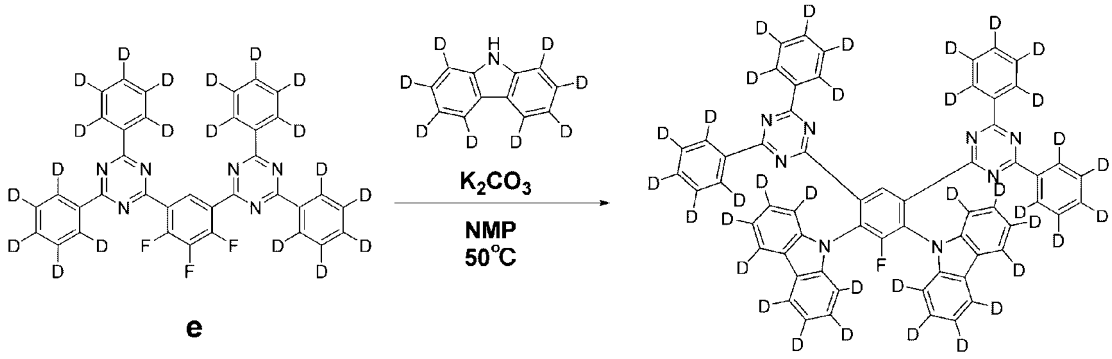


e

t

Under a nitrogen stream, potassium carbonate (3.37 g, 24.4 mmol) was added to a solution of Compound e (5.0 g, 8.13 mmol) and carbazole-1,2,3,4,5,6,7,8-d8 (2.85 g, 16.2 mmol) in NMP (30 mL), and the mixture was stirred at 50°C for 15 hours. The reaction solution was cooled to room temperature and quenched by the addition of water. The solution was filtered and the solid remaining in the funnel was washed with water and methanol. The solid was dissolved in heated ODCB, and the solution was charged and purified by column chromatography (toluene:hexane = 1:1). The purified product was further recrystallized from toluene to obtain a yellow Compound t (4.80 g, 5.18 mmol, yield 63%).

$^1$H-NMR (400 MHz, $CDCl_3$): δ 9.50 (dd, J=1.6 Hz, 1H).
ASAP MS Spectral Analysis: $C_{60}HD_{36}FN_8$: theoretical value 924.5, observed value 924.7.

Compound C10

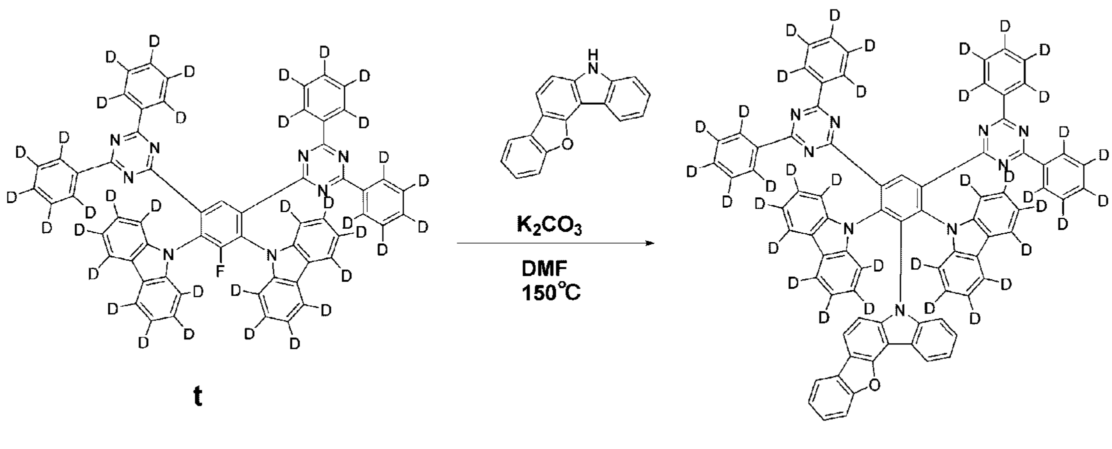


t

C10

Under a nitrogen stream, potassium carbonate (0.87 g, 6.27 mmol) was added to a solution of Compound t (1.45 g, 1.57 mmol) and 5H-benzofuro[3,2-c]carbazole (1.21 g, 4.70 mmol) in DMF (50 mL), and the mixture was stirred at 150°C for 3 hours. The reaction solution was cooled to room temperature and quenched by the addition of water. The solution was filtered and the solid remaining in the funnel was washed with water and methanol. The solid was dissolved in heated ODCB, and the solution was charged and purified by column chromatography (toluene:hexane = 1:1). The purified product was further recrystallized from toluene to obtain a yellow Compound C10 (1.47 g, 1.26 mmol, yield 81 %).
$^1$H-NMR (400 MHz, $CDCl_3$): δ 9.44 (s, 1H), 7.87 (d, J=7.2 Hz, 1H), 7.74(d, J= 7.2Hz, 1H) 7.54-7.49(m, 1H), 7.32-7.22 (m, 5H), 6.93 (t, J=7.2 Hz, 1H), 6.78 (t, J=7.2 Hz, 1H). ASAP MS Spectral Analysis: $C_{78}H_{11}D_{36}N_9O$: theoretical value 1161.6, observed value 1162.1.

(Synthesis Example 11) Synthesis of Compound C11

Compound C11

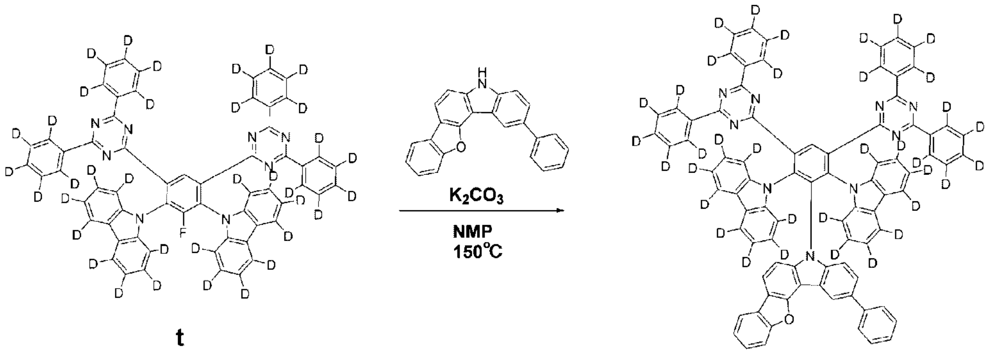


t

C11

Under a nitrogen stream, potassium carbonate (0.60 g, 4.32 mmol) was added to a solution of Compound t (1.0 g, 1.08 mmol) and 2-phenyl-5H-benzofuro[3,2-c]carbazole (1.08 g, 3.24 mmol) in DMF (40 mL), and the mixture was stirred at 150°C for 3 hours. The reaction solution was cooled to room temperature and quenched by the addition of water. The solution was filtered and the solid remaining in the funnel was washed with water. The solution was filtered and the solid remaining in the funnel was washed with water and methanol. The solid was dissolved in heated ODCB, and the solution was charged and purified by column chromatography (toluene:hexane = 1:1). The purified product was further recrystallized from toluene to obtain a yellow Compound C11 (0.98 g, 0.08 mmol, yield 73%).

$^1$H-NMR (400 MHz, $CDCl_3$): δ 9.45 (s, 1H), 8.08 (d, J=1.2 Hz, 1H), 7.75 (d, J=6.8 Hz, 1H), 7.54-7.51(m, 3H), 7.40(t, J=7.2 Hz, 2H) 7.34-7.24 (m, 6H), 7.04-7.00(m, 1H).
ASAP MS Spectral Analysis: $C_{84}H_{15}D_{36}N_9O$: theoretical value 1237.6, observed value 1239.2.

(Synthesis Example 12) Synthesis of Compound C12

Compound v

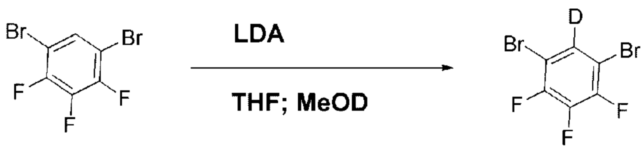


u

v

A solution of Compound u (25.7 g, 88.6 mmol) in THF (295 mL) was subjected to nitrogen bubbling, and the solution was cooled to -78°C. Under a nitrogen stream, 1M lithium diisopropylamide (LDA, 99.6 mL, 99.6 mmol) was added dropwise to this solution over 30 minutes. After the mixture was further stirred at -78°C for 1 hour, $CH_3OD$ (7.3 g, 221 mmol) was added thereto, and the mixture was stirred overnight. After the mixture was warmed to room temperature and a saturated aqueous ammonium chloride solution was added thereto, THF was distilled off by an evaporator. Dichloromethane and water were added to extract an organic layer, which was then washed with saturated saline water and dried over sodium sulfate. The solution was concentrated and purified by silica gel chromatography. The purified product was further purified by distillation under reduced pressure to obtain Compound v as a transparent liquid (10 g, 34.4 mmol, yield 36.6%).

Compound x

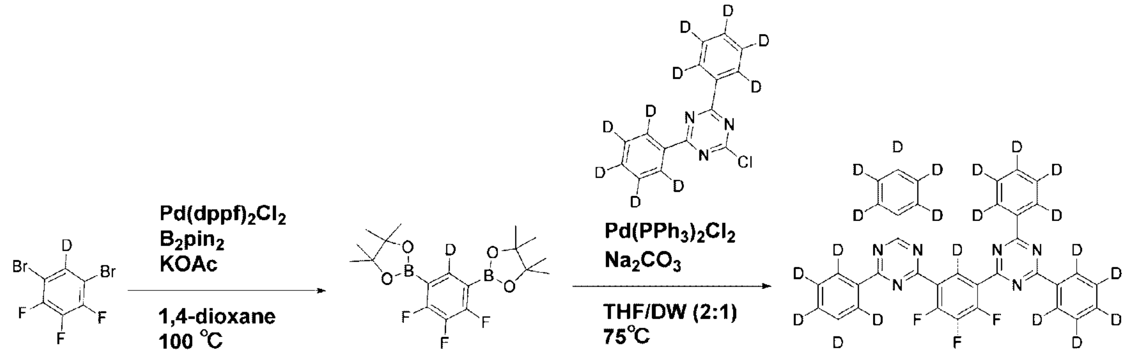


v w x

A reaction mixture of Compound v (9 g, 30.9 mmol), [1,1'-bis(diphenylphosphino)ferrocene] dichloropalladium (II) (2.3 g, 3.09 mmol), potassium acetate (21.1 g, 21.1 mmol), bis(pinacolato)diboron (39.1 g, 154 mmol), and 1,4-dioxane (310 mL) was stirred at 110°C for 15 hours. The reaction mixture was cooled to room temperature, diluted with ethyl acetate, and filtered through silica. The obtained solid was washed with hexane to obtain Compound w as a white solid (10.0 g, 25.9 mmol, yield 76%).

Compound w (10.0 g, 25.9 mmol) was dissolved in THF (260 mL) and ion-exchanged water (130 mL), and 2-chloro-4,6-bis(phenyl-d5)-1,3,5-triazine (17.9 g, 64.7 mmol), bis(triphenylphosphine)palladium (II) dichloride (1.81 g, 2.59 mmol) and sodium carbonate (8.22 g, 77.6 mmol) were added thereto, and the mixture was stirred at 75°C for 15 hours. The reaction vessel was cooled to room temperature, and the reaction solution was concentrated. The obtained gray solid was separated by filtration, and the solid was washed with ion-exchanged water, methanol, and THF. The collected solid was stirred in hot toluene, and the solid was separated by filtration and washed with toluene to obtain Compound x as a black solid (1.86 g, 3.02 mmol, yield 11.6%). ASAP MS Spectral Analysis: $C_{36}D_{21}F_3N_6$: theoretical value 615.31, observed value 616.41.

Compound y

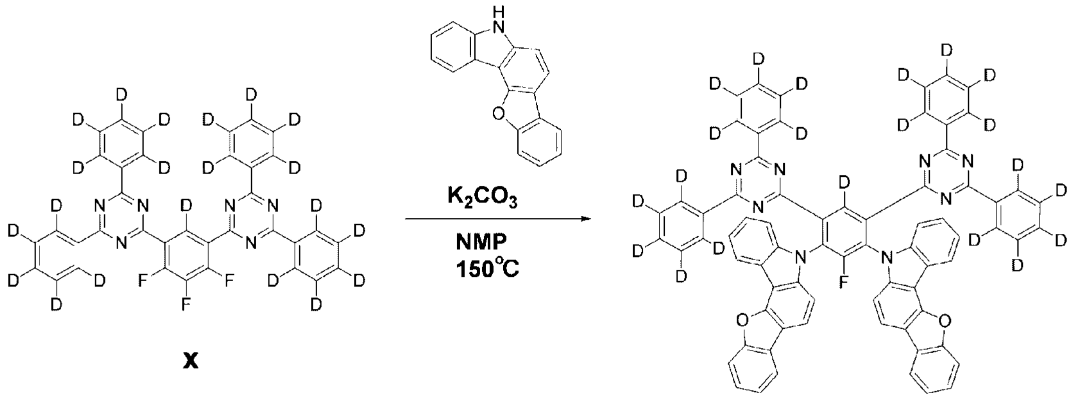


x

y

Under a nitrogen stream, a solution of Compound x (1.32 g, 2.14 mmol) and 5H-benzofuro[3,2-c]carbazole (1.37 g, 5.35 mmol) in THF (71 mL) was cooled to -25°C, and a solution of potassium tert-butoxide (0.72 g, 6.42 mmol) in THF (71 mL) was added dropwise. After the temperature was raised to room temperature, the mixture was stirred for 15 hours. After that, water was added, the suspension was filtered, and the solid remaining in the funnel was washed with water and methanol. This was dissolved in dichloromethane, purified by column chromatography (toluene:hexane = 1:1), and the obtained solid was washed with methanol to obtain a yellow Compound y (1.18 g, 1.08 mmol, yield 51%).

$^1$H-NMR(400 MHz, $CDCl_3$): 8.46 (d, J=7.6 Hz, 2H), 8.01-7.95 (m, 4H), 7.71 (d, J=8.4 Hz, 2H), 7.52-7.35 (m, 12H). ASAP MS Spectral Analysis: $C_{72}H_{20}D_{21}FN_8O_2$: theoretical value 1089.47, observed value 1090.61.

Compound C12

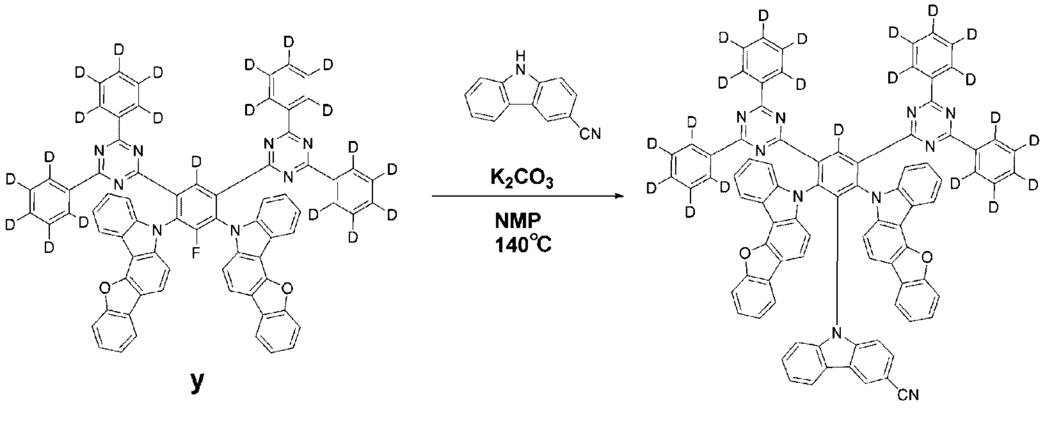


C12

Under a nitrogen stream, potassium carbonate (0.15 g, 1.09 mmol) was added to a solution of Compound y (0.60 g, 0.55 mmol) and 9H-carbazole-3-carbonitrile (0.16 g, 0.84 mmol) in NMP (5.5 mL), and the mixture was stirred at 140°C for 15 hours. The reaction solution was cooled to room temperature and quenched by the addition of water. The solution was filtered and the solid remaining in the funnel was washed with water and methanol. The solid was dissolved in dichloromethane and dried over magnesium sulfate. After filtration, the solution was concentrated under reduced pressure in an evaporator and then purified by column chromatography (toluene:hexane = 1: 1) to obtain a yellow Compound C12 (0.51 g, 0.40 mmol, yield 74%).

$^1$H-NMR (400 MHz, $CDCl_3$): δ 8.07-8.00 (m, 2H), 7.84 (dd, J=7.2 Hz, 12.8 Hz, 2H), 7.69 (s, 1H), 7.63-7.50(m, 4H), 7.43-7.19 (m, 11H), 7.16.-6.69(m, 7H).
ASAP MS Spectral Analysis: $C_{85}H_{27}D_{21}N_{10}O_2$: theoretical value 1261.53, observed value 1262.77.

(Synthesis Example 13) Synthesis of Compound C13

Compound C13

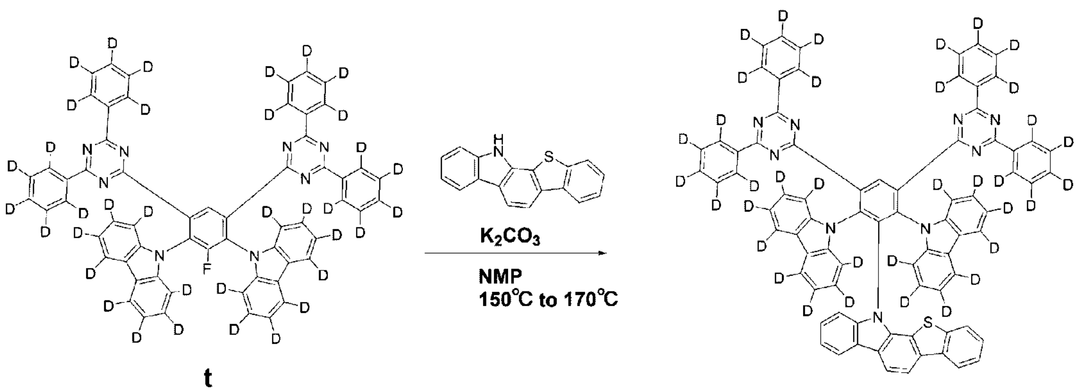


t

C13

Under a nitrogen stream, potassium carbonate (0.45 g, 3.23 mmol) was added to a solution of Compound t (1.0 g, 1.08 mmol) and 12H-[1]benzothieno[2,3-a]carbazole (0.59 g, 2.16 mmol) in NMP (11 mL), and the mixture was stirred at 150°C for 15 hours. Further, the temperature was raised to 170°C and stirred for 3 hours. The reaction solution was cooled to room temperature and quenched by the addition of water. The solution was filtered and the solid remaining in the funnel was washed with water and methanol. The solution was filtered and the solid remaining in the funnel was washed with water and methanol. The solid was dissolved in heated ODCB, and the solution was charged and purified by column chromatography (toluene:hexane = 1:1) to obtain a yellow solid Compound C13 (0.35 g, 0.29 mmol, yield 27%).

$^1$H-NMR (400 MHz, $CDCl_3$): δ 9.73 (s, 1H), 7.93-7.90 (m, 1H) 7.83-7.80 (m, 1H), 7.61 (m, 5H), 7.08 (d, J = 8 Hz, 1H), 6.90-6.84(m, 1H), 6.81-6.75 (m, 1H).
ASAP MS Spectral Analysis: $C_{78}H_{11}D_{36}N_9S$: theoretical value 1177.59, observed value 1178.71.

(Synthesis Example 14) Synthesis of Compound C14

Compound C14

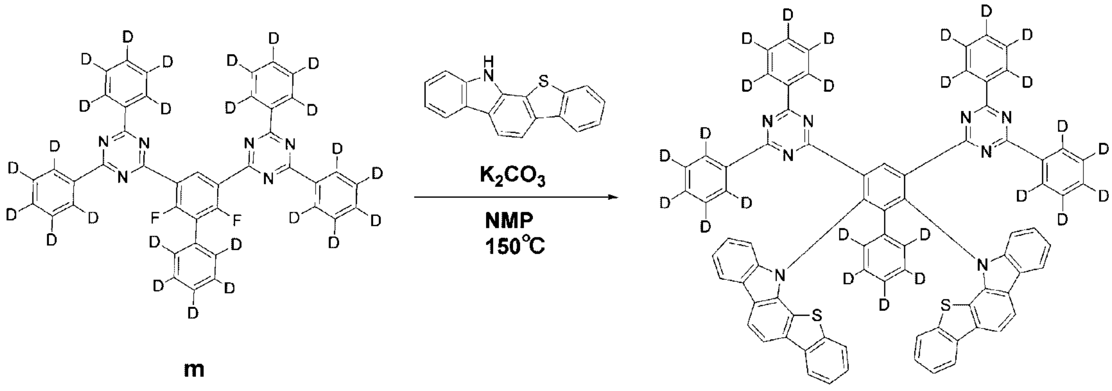


m

C14

Under a nitrogen stream, potassium carbonate (2.41 g, 8.85 mmol) was added to a solution of Compound m (2 g, 2.95 mmol) and 12H-[1]benzothieno[2,3-a]carbazole (1.60 g, 6.22 mmol) in NMP (90 mL), and the mixture was stirred at 150°C for 15 hours. The reaction solution was cooled to room temperature and quenched by the addition of water. The solid was dissolved in heated ODCB, and the solution was charged and purified by column chromatography (toluene:hexane = 1:1) to obtain a yellow Compound C14 (1.53 g, 1.57 mmol, yield 53%).

$^1$H-NMR (400 MHz, $CDCl_3$): δ10.18 (s, 1H, major rotamer), 9.95 (s, 1H, minor rotamer), 8.21-8.15 (m, 2H), 7.97-7.91(m, 7H), 7.81-7.77 (m, 1H), 7.54-7.29 (m, 8H), 7.22-7.17 (m, 2H).
ASAP MS Spectral Analysis: $C_{78}H_{21}D_{25}N_8S_2$: theoretical value 1183.49, observed value 1184.75.

(Synthesis Example 15) Synthesis of Comparative Compound A

Compound z

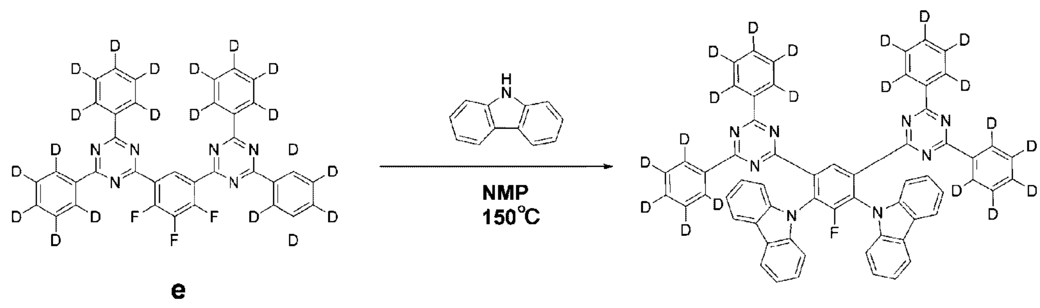


e

z

Under a nitrogen stream, potassium carbonate (0.84 g, 6.10 mmol) was added to a solution of Compound e (1.50 g, 2.44 mmol) and 9H-carbazole (0.81 g, 4.88 mmol) in NMP (90 mL), and the mixture was stirred at 150°C for 3 hours. The reaction solution was cooled to room temperature and quenched by the addition of water. The solution was filtered and the solid remaining in the funnel was washed with water. The solid was dissolved in dichloromethane and dried over magnesium sulfate. The solution was concentrated under reduced pressure in an evaporator and then purified by column chromatography (toluene:hexane = 1:1) to obtain a yellow Compound z (1.27 g, 1.39 mmol, yield 57%).

$^1$H-NMR (400 MHz, $CDCl_3$): δ 9.51 (d, J=1.6 Hz, 1H), 8.03 (d, J=8.0 Hz, 4H), 7.40-7.37 (m, 8H), 7.28-7.23 (m, 4H).
ASAP MS Spectral Analysis: $C_{60}H_{17}D_{20}FN_8$: theoretical value 908.4, observed value 909.75.

Comparative Compound A

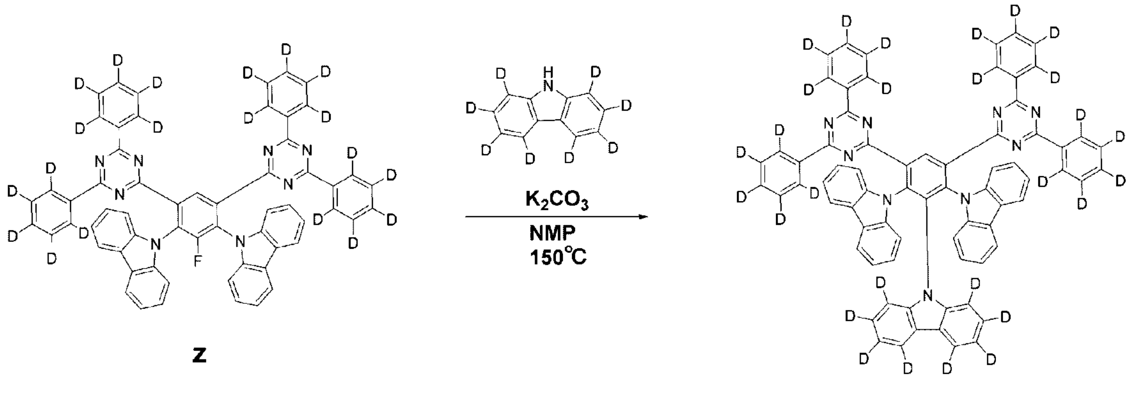


z

A

Under a nitrogen stream, potassium carbonate (0.31 g, 2.26 mmol) was added to a solution of Compound z (1.03 g, 1.13 mmol) and carbazole-1,2,3,4,5,6,7,8-d8 (0.29 g, 1.69 mmol) in NMP (50 mL), and the mixture was stirred at 130°C for 3 hours. The reaction solution was cooled to room temperature and quenched by the addition of water. The solution was filtered and the solid remaining in the funnel was washed with water. The solid was dissolved in dichloromethane and dried over magnesium sulfate. The solution was concentrated under reduced pressure in an evaporator and then purified by column chromatography (toluene:hexane = 1:1) to obtain a yellow Comparative compound A (0.78 g, 0.73 mmol, yield 65%).

$^1$H-NMR (400 MHz, $CDCl_3$): δ 9.40 (d, J=4.8 Hz, 1H), 7.57 (dd, J=4.8Hz, 1.2 Hz, 4H), 7.28-7.24 (m, 4H), 6.98-6.94 (m, 8H).
ASAP MS Spectral Analysis: $C_{72}H_{17}D_{28}N_9$: theoretical value 1063.5, observed value 1063.7.

(Test Example 1) Production and Evaluation of Organic Electroluminescent Device

On a glass substrate on which an anode made of indium-tin oxide (ITO) having a film thickness of 50 nm was formed, each thin film was laminated by a vacuum deposition method at a vacuum degree of $5.0 \times 10^{-5}$ Pa. First, HAT-CN was formed to a thickness of 10 nm on the ITO, NPD was formed to a thickness of 30 nm thereon, further Tris-PCz was formed to a thickness of 10 nm, and further H1 was formed to a thickness of 5 nm. Next, H1, Compound C1 and a dopant EM1 were co-deposited from different vapor deposition sources to form a layer with a thickness of 40 nm as a light-emitting layer. In the light-emitting layer, the content of H1 was 44.2% by mass, the content of Compound C1 was 55.0% by mass, and the content of EM1 was 0.8% by mass. Next, after SF3-TRZ was formed at a thickness of 10 nm, Liq and SF3-TRZ were co-deposited from different vapor deposition sources to form a layer with a thickness of 30 nm. The contents of Liq and SF3-TRZ in this layer were 30% by mass and 70% by mass, respectively. Furthermore, Liq was formed to a thickness of 2 nm, and aluminum (Al) was vapor-deposited to a thickness of 100 nm to form a cathode, thereby obtaining an organic electroluminescent device.
When an organic electroluminescent device produced using Compound C1 was driven, green delayed fluorescence was observed. When driven at 6.3 mA/cm$^2$, the measured voltage was 3.82 V. In addition, when driven at 6.3 mA/cm$^2$, the measured external quantum efficiency (EQE) was as high as 24.7%. These results show that the compound represented by the general formula (1) is useful.

(Test Example 2) Production and Evaluation of Organic Electroluminescent Devices

Instead of the light-emitting layer of the organic electroluminescent device of Test Example 1, H2, Compound C1, and the dopant EM1 were co-deposited from different vapor deposition sources to form a light-emitting layer with a thickness of 40 nm in which the content of H2 was 64.2% by mass, the content of Compound C1 was 35.0% by mass, and the content of EM1 was 0.8% by mass. Otherwise, an organic electroluminescent device was produced according to the same procedure as in Test Example 1.
Further, instead of forming the light-emitting layer of the organic electroluminescent device of Test Example 1,

H2, Compound C3, and the dopant EM1 were co-deposited from different vapor deposition sources to form a light-emitting layer with a thickness of 40 nm in which the content of H2 was 74.2% by mass, the content of Compound C3 was 25.0% by mass, and the content of EM1 was 0.8% by mass. Otherwise, an organic electroluminescent device was produced according to the same procedure as in Test Example 1. Furthermore, organic electroluminescent devices were produced in the same manner as above except for using Compound C4, Compound C5, Compound C12, Compound C14, and Comparative Compound A in place of Compound C3.

Each of these devices was driven at 25.2 mA/cm$^2$, and the time (LT95) from the start of driving until the light emission intensity reached 95% was measured. The measurement results are shown in Table 9 as relative values when LT95 of the organic electroluminescent device using Comparative Compound A is set to 1. The results in Table 9 show that an organic light-emitting device having a long lifetime can be provided by using the compound represented by the general formula (1).

[Table 9]

| Compound used | Lifetime (relative value) |
|---|---|
| Compound C1 | 12.7 |
| Compound C3 | 10.4 |
| Compound C4 | 11.6 |
| Compound C5 | 10.9 |
| Compound C12 | 14.5 |
| Compound C14 | 9.5 |
| Comparative Compound A | 1 |

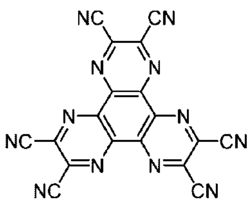
HAT-CN

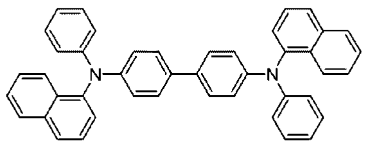
NPD

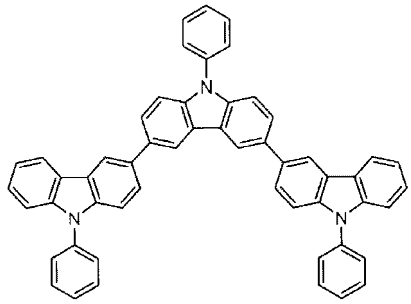
TrisPCz

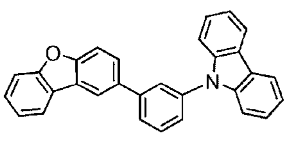
H1

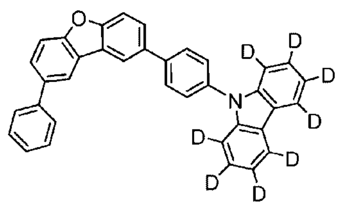
H2

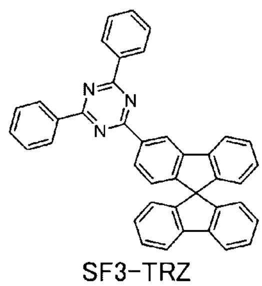
SF3-TRZ

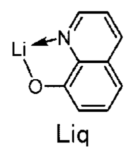
Liq

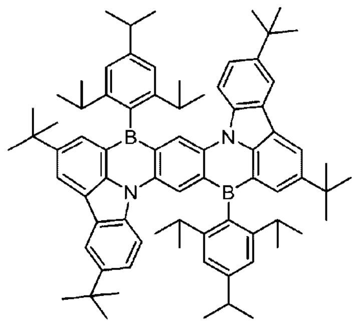

EM1

Industrial Applicability

By using the compound represented by the general formula (1), it is possible to provide a light-emitting device having excellent light emission characteristics. Therefore, the present invention has high industrial applicability.

## Claims

**1.** A compound represented by the following general formula (1):

General Formula (1)

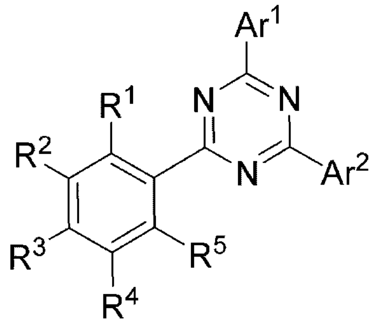

wherein $R^1$ to $R^5$ each independently represent a hydrogen atom, a deuterium atom, a substituted or unsubstituted alkyl group, a substituted or unsubstituted aryl group, a donor group, or a group represented by the following formula (2); $R^2$ or $R^3$ is a group represented by the following general formula (2), and two or more of $R^1$ to $R^5$ are donor groups; $Ar^1$ and $Ar^2$ each independently represent a substituted or unsubstituted aryl group, or a substituted or unsubstituted heteroaryl group;

General Formula (2)

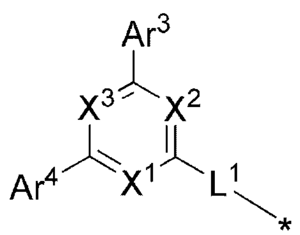

wherein $X^1$ to $X^3$ each independently represent N or C(R), and at least one of $X^1$ to $X^3$ is N; R represents a hydrogen atom, a deuterium atom, or a substituent; $Ar^3$ and $Ar^4$ each independently represent a substituted or unsubstituted aryl group, or a substituted or unsubstituted heteroaryl group; $L^1$ represents a single bond or a divalent linking group; * represents a bonding position;

provided that, in the compound represented by the general formula (1), at least one of $R^1$ to $R^5$ is a donor group having a fused ring structure of four or more rings, at least one of $Ar^1$ to $Ar^4$ is a substituted or unsubstituted

heteroaryl group (excluding a nitrogen-containing 6-membered ring group), or $R^1$ to $R^5$ and $Ar^1$ to $Ar^4$ satisfy both of these conditions.

**2.** The compound according to claim 1, wherein at least one of $R^1$ to $R^5$ is a donor group having a fused ring structure of four or more rings.

**3.** The compound according to claim 2, wherein at least one of $R^1$ to $R^5$ is a donor group represented by the following general formula (3):

General Formula (3)

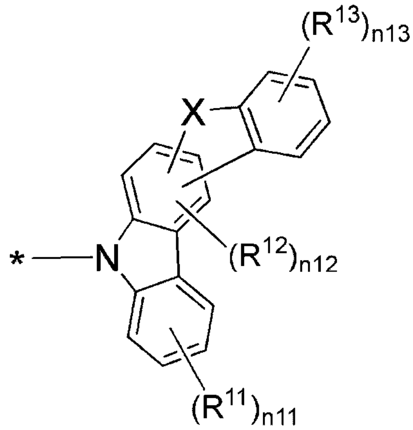

wherein X represents O, S or N-$R^{14}$; $R^{11}$ to $R^{13}$ each independently represent a deuterium atom, or a substituent; $R^{14}$ represents an aryl group optionally substituted with one or more selected from the group consisting of a deuterium atom, an alkyl group and an aryl group, or an alkyl group optionally substituted with one or more selected from the group consisting of a deuterium atom and an aryl group; $R^{11}$'s, $R^{12}$'s, and $R^{13}$'s each may be bonded to each other to form a cyclic structure; n11 and n13 each independently represent an integer of 0 to 4, and n12 represents an integer of 0 to 2.

**4.** The compound according to claim 2, wherein $Ar^1$ to $Ar^4$ are each independently a substituted or unsubstituted aryl group.

**5.** The compound according to claim 1, wherein at least one of $Ar^1$ to $Ar^4$ is a substituted or unsubstituted heteroaryl group (excluding a nitrogen-containing 6-membered ring group).

**6.** The compound according to claim 5, wherein at least one of $Ar^1$ to $Ar^4$ is a heteroaryl group having a 5-membered ring bonded via a nitrogen atom.

**7.** The compound according to claim 1, wherein $R^2$ is a group represented by the general formula (2).

**8.** The compound according to claim 1, wherein $R^3$ is a group represented by the general formula (2).

**9.** The compound according to claim 1, wherein the donor group is a substituted or unsubstituted carbazol-9-yl group.

**10.** The compound according to claim 1, wherein three of $R^1$ to $R^5$ are donor groups.

**11.** The compound according to claim 1, wherein $X^1$ to $X^3$ are N.

**12.** The compound according to claim 1, wherein $L^1$ is a single bond.

**13.** The compound according to claim 1, wherein $R^1$ is a hydrogen atom.

**14.** The compound according to claim 1, wherein the compound has at least one deuterium atom.

**15.** A light-emitting material comprising the compound according to any one of claims 1 to 14.

**16.** A delayed fluorescent substance comprising the compound according to any one of claims 1 to 14.

# INTERNATIONAL SEARCH REPORT

International application No.
**PCT/JP2024/012318**

**A. CLASSIFICATION OF SUBJECT MATTER**

***C07D 403/14***(2006.01)i; ***C07D 519/00***(2006.01)i; ***C09K 11/06***(2006.01)i
FI: C07D403/14 CSP; C09K11/06 640; C09K11/06; C07D519/00

According to International Patent Classification (IPC) or to both national classification and IPC

**B. FIELDS SEARCHED**

Minimum documentation searched (classification system followed by classification symbols)

C07D403/14; C07D519/00; C09K11/06

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | KR 10-2018-0047306 A (RESEARCH & BUSINESS FOUNDATION SUNGKYUNKWAN UNIVERSITY) 10 May 2018 (2018-05-10)<br>claims, examples | 1-16 |
| X | WO 2023/282224 A1 (KYULUX, INC.) 12 January 2023 (2023-01-12)<br>paragraphs [0048], [0051] | 1-16 |
| X<br>A | JP 2021-172629 A (TOYOBO CO., LTD.) 01 November 2021 (2021-11-01)<br>examples 2, 3, 4 | 1, 4, 7-13, 15, 16<br>2, 3, 5, 6, 14 |
| X<br>A | US 2019/0300508 A1 (KYULUX, INC.) 03 October 2019 (2019-10-03)<br>p. 102, upper left compounds | 1, 4, 7-13, 15, 16<br>2, 3, 5, 6, 14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 June 2024** | **18 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
**PCT/JP2024/012318**

C. DOCUMENTS CONSIDERED TO BE RELEVANT

| Category* | Citation of document, with indication, where appropriate, of the relevant passages | Relevant to claim No. |
|---|---|---|
| X<br>A | US 2020/0115364 A1 (KYULUX, INC.) 16 April 2020 (2020-04-16)<br>pp. 34-38 | 1, 4, 7-13, 15, 16<br>2, 3, 5, 6, 14 |
| X<br>A | KR 10-2019-0018397 A (LG CHEM, LTD.) 22 February 2019 (2019-02-22)<br>paragraph [0119] | 1, 4, 7-13, 15, 16<br>2, 3, 5, 6, 14 |
| P, X<br>P, A | CN 115894457 A (BEIJING ETERNAL MATERIAL TECHNOLOGY CO., LTD.) 04 April 2023 (2023-04-04)<br>claims | 1, 2, 4, 7-13, 15, 16<br>3, 5, 6, 14 |
| P, X<br>P, A | US 2023/0240140 A1 (LG CHEM, LTD.) 27 July 2023 (2023-07-27)<br>p. 69 | 1, 4, 7-13, 15, 16<br>2, 3, 5, 6, 14 |

Form PCT/ISA/210 (second sheet) (July 2022)

**INTERNATIONAL SEARCH REPORT**
**Information on patent family members**

International application No.
**PCT/JP2024/012318**

| Patent document cited in search report | Publication date (day/month/year) | Patent family member(s) | Publication date (day/month/year) |
| --- | --- | --- | --- |
| KR 10-2018-0047306 A | 10 May 2018 | (Family: none) | |
| WO 2023/282224 A1 | 12 January 2023 | CN 117581651 A | |
| JP 2021-172629 A | 01 November 2021 | WO 2021/220952 A1 | |
| US 2019/0300508 A1 | 03 October 2019 | (Family: none) | |
| US 2020/0115364 A1 | 16 April 2020 | JP 2020-525438 A | |
| | | JP 2020-525437 A | |
| | | US 2020/0115376 A1 | |
| | | US 2020/0119287 A1 | |
| | | WO 2018/237385 A1 | |
| | | WO 2018/237389 A1 | |
| | | WO 2018/237393 A1 | |
| | | EP 3642303 A1 | |
| | | KR 10-2020-0019694 A | |
| | | CN 110914378 A | |
| | | KR 10-2020-0023371 A | |
| | | CN 110997866 A | |
| KR 10-2019-0018397 A | 22 February 2019 | (Family: none) | |
| CN 115894457 A | 04 April 2023 | (Family: none) | |
| US 2023/0240140 A1 | 27 July 2023 | WO 2022/031033 A1 | |
| | | KR 10-2022-0017374 A | |
| | | CN 115606335 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019191665 A1
- WO 2022168956 A1
- WO 2022270602 A1
- WO 2022270354 A1


### Non-patent literature cited in the description


- **HANSCH, C.** *Chem. Rev.*, 1991, vol. 91, 165-195